# EUROPEAN PATENT APPLICATION

(11) **EP 4 461 729 A1**
(43) Date of publication of application: **13.11.2024**
(21) Application number: 23737139.8
(22) Date of filing: 06.01.2023
(51) Int. Cl.: C07D 401/12, C07D 405/12, C07D 237/34, C07D 237/26, C07D 471/04, C07D 495/04, C07D 487/04, C07D 401/04, C07D 409/12, C07D 405/14, C07D 403/04, C07D 253/07, A61K 31/502, A61K 31/5025, A61K 31/504, A61K 31/53, A61K 31/506, A61P 37/02

(54) **ANTIGEN BINDING PROTEIN TARGETING MSLN AND USE THEREOF**

(30) Priority: 07.01.2022 CN 202210015181; 07.01.2022 CN 202210015699; 20.05.2022 CN 202210563248; 26.07.2022 CN 202210895164; 08.09.2022 CN 202211093807
(71) Applicant: Transthera Sciences (Nanjing), Inc., Jiangbei New Area Nanjing Jiangsu 210032 (CN)
(72) Inventor: LI, Lin, Jiangsu 210032 (CN); WU, Frank, Jiangsu 210032 (CN)
(74) Representative: Hoeger, Stellrecht & Partner Patentanwälte mbB
(86) International application number: PCT/CN2023/070929
(87) International publication number: WO 2023/131277

(57) **Abstract**

The present application belongs to the technical field of medicines, relates to an NLRP3 inflammasome inhibitor and the uses thereof, and particularly relates to a compound represented by general formula (A) or a pharmaceutically acceptable salt, a stereoisomer and a tautomer thereof. The inhibitor has biological activity on NLRP3 inflammasome, and has important clinical development value for treatment of NLRP3-related diseases.

## Description

### CROSS REFERENCE TO RELATED APPLICATION

This application claims benefits and priorities of Chinese Patent Application No. 202210015181.X filed on January 7, 2022, Chinese Patent Application No. 202210015699.3 filed on January 7, 2022, Chinese Patent Application No. 2202210563248.3 filed on May 20, 2022, Chinese Patent Application No. 202210895164.X filed on July 26, 2022, and Chinese Patent Application No. 202211093807.5 filed on September 8, 2022, the disclosures of which are herein incorporated by reference in their entireties.

### TECHNICAL FIELD

The present invention belongs to the technical field of pharmaceuticals, and in particular, relates to an NLRP3 inflammasome inhibitor and application thereof.

### BACKGROUND OF THE INVENTION

Nucleotide-binding oligomerization domain (NOD)-like receptor protein 3 (NLRP3) belongs to the family of NOD-like receptors (NLRs), and is also known as "pyrin-containing domain protein 3". NLRP3 comprises three modules, namely a pyrin domain (PYD), a nucleotide-binding site domain (NBD), and a leucine-rich repeat (LRR). When stimulated by aseptic inflammation danger signals, NLRP3 interacts with an adaptor protein apoptosis-associated speck-like protein (ASC) and pro-caspase 1 to form a NLRP3 inflammasome. The activation of the NLRP3 inflammasome leads to the release of interleukin-1β (IL-1β) and interleukin-18 (IL-18).

The activation of the NLRP3 inflammasome typically requires two steps. The first step involves initiating signaling, in which the Toll-like receptor recognizes a pathogen-associated molecular pattern (PAMP) or a damage-associated molecular pattern (DAMP) and then transmits a signal into a cell to mediate the activation of a NF-κB signaling pathway, thereby upregulating the transcriptional levels of NLRP3 inflammasome-related components including inactive NLRP3 and pro-IL-1β and the like. The second step is to activate the signaling, in which after the P2X7 receptor or the like receives the signal stimulation from ATP, nigericin or the like, a NLRP3 monomer is oligomerized to form a NLRP3 oligomer, and then ASC and pro-caspase 1 are recruited to assemble an NLRP3 inflammasome complex. This triggers the conversion of pro-caspase 1 to caspase 1, as well as the production and secretion of mature IL-1β and IL-18.

The activation of the NLRP3 inflammasome is associated with a variety of diseases, for example, auto inflammatory fever syndrome such as cryopyrin-associated periodic syndrome (CAPS), sickle cell disease, systemic lupus erythematosus (SLE), chronic liver disease, non-alcoholic steatosis hepatitis (NASH), gout, pseudogout (chondrocalcinosis), type I and type II diabetes and related complications (for example, kidney disease and retinopathy), neuroinflammation-related disorders (for example, multiple sclerosis, brain infection, acute injury, neurodegenerative diseases, Alzheimer's disease), atherosclerosis and cardiovascular risk (for example, hypertension), hidradenitis suppurativa, wound healing and cicatrization, as well as cancers (for example, colorectal cancer, lung cancer, myeloproliferative neoplasm, leukemia, myelodysplastic syndrome (MDS), myelofibrosis). Most of the therapies include symptomatic treatment, slowing down the progression of the disease/disorder, and taking surgical procedures as a last resort.

At present, there are few varieties of NLRP3 inflammasome inhibitors under development, and it is clinically desired to develop an NLRP3 inflammasome inhibitor with higher activity and better druggability.

### SUMMARY OF THE INVENTION

The present invention studies the following compounds or the pharmacologically acceptable salts, stereoisomers or tautomers thereof. Studies have found that these compounds or the pharmacologically acceptable salts, stereoisomers or tautomers thereof have high bioactivity against NLRP3 inflammasomes, and show important clinical development value for the treatment of NLRP3-related diseases.

To achieve the object above, the present invention provides the following solution:
a compound of general formula (A) or a pharmacologically acceptable salt, stereoisomer or tautomer thereof: wherein, W is selected from

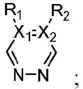

Y-W-R₃ (A)

X₁ and X₂ are each independently selected from C or N;
R₁ is selected from hydrogen, hydroxyl, amino, carboxyl, cyano, nitro, halogen, carbonyl, C₁₋₆ alkyl, halo C₁₋₆ alkyl, C₁₋₆ alkoxy, halo C₁₋₆ alkoxy, C₂₋₆ alkenyl, C₂₋₆ alkynyl, 3-7 membered heterocyclyl, 3-7 membered cycloalkyl, aryl, 5-7 membered heteroaryl, -N(C₁₋₆ alkyl)₂, -S-C₁₋₆ alkyl, or absent;
R₂ is selected from hydrogen, hydroxyl, amino, carboxyl, cyano, nitro, halogen, carbonyl, C₁₋₆ alkyl, halo C₁₋₆ alkyl, C₁₋₆ alkoxy, halo C₁₋₆ alkoxy, C₂₋₆ alkenyl, C₂₋₆ alkynyl, 3-7 membered heterocyclyl, 3-7 membered cycloalkyl, aryl, 5-7 membered heteroaryl, -N(C₁₋₆ alkyl)₂, -S-C₁₋₆ alkyl, or absent;
R₁ and R₂ are each optionally substituted by 1-3 substituents selected from hydroxyl, amino, carboxyl, cyano, nitro, halogen, carbonyl, C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, and 5-7 membered heteroaryl;
   or
R₁ and R₂, together with the carbon or nitrogen atom to which they are attached, form a 5-12 membered ring A; the 5-12 membered ring A is optionally substituted by 1-4 substituents selected from hydroxyl, amino, carboxyl, cyano, nitro, halogen, carbonyl, C₁₋₆ alkyl, -NH-C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂;
R₃ is selected from -(C₁₋₆ alkylene)₀₋₂-NR₄R₅, -(C₁₋₆ alkylene)₀₋₂-NR₄-COR₅, -(C₁₋₆ alkylene)₀₋₂-CO-NR₄-R₅, and - (C₁₋₆ alkylene)₀₋₂-NR₄-C₁₋₆ alkylene-R₅;
R₄ is selected from hydrogen or C₁₋₆ alkyl;
R₅ is selected from 3-7 membered heterocyclyl, 3-7 membered cycloalkyl, aryl, and 5-7 membered heteroaryl; R₅ is optionally substituted by 1-4 substituents selected from halogen, cyano, amino, hydroxyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₂₋₆ alkenylcarbonyl, sulfonyl, and C₁₋₆ alkylcarbonyl;
when R₅ is substituted,
the substituent on R₅, which is C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from halogen, cyano, amino, hydroxyl, carbonyl, C₁₋₆ alkyl, 3-6 membered cycloalkyl, 3-6 membered heterocyclyl, carboxyl, and C₁₋₆ alkylsulfonyl;
Y is selected from aryl, 5-14 membered heteroaryl, 3-14 membered heterocyclyl, and 3-12 membered cycloalkyl;
(1) when X₁ and X₂ are each selected from C,
Y is substituted by C₂₋₆ alkenyl or C₂₋₆ alkynyl, and is optionally substituted by 1-2 substituents selected from halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, and sulfonyl;
(2) when either one or two of X₁ and X₂ is/are selected from N,
Y is optionally substituted by 1-3 substituents selected from C₂₋₆ alkenyl, C₂₋₆ alkynyl, halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, sulfonyl, -N(C₁₋₆ alkyl)₂, and -S-C₁₋₆ alkyl;
in (1) and (2) above, when Y is substituted,
the substituent on Y, which is C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from halogen, cyano, amino, hydroxyl, carbonyl, C₁₋₆ alkyl, and 3-6 membered cycloalkyl; or
the substituent on Y, which is C₂₋₆ alkenyl or C₂₋₆ alkynyl, is optionally substituted by 1-3 substituents selected from halogen, cyano, amino, hydroxyl, carbonyl, unsubstituted or hydroxyl-substituted C₁₋₆ alkyl, unsubstituted or hydroxyl-substituted 3-6 membered cycloalkyl, and halo C₁₋₆ alkyl.

In some embodiments, the present invention further provides the compound of general formula (A) or the pharmacologically acceptable salt, stereoisomer or tautomer thereof: 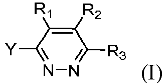 wherein,
R₁ is selected from hydrogen, hydroxyl, amino, carboxyl, cyano, nitro, halogen, C₁₋₆ alkyl, halo C₁₋₆ alkyl, C₁₋₆ alkoxy, halo C₁₋₆ alkoxy, C₂₋₆ alkenyl, C₂₋₆ alkynyl, 3-7 membered heterocyclyl, 3-7 membered cycloalkyl, aryl, and 5-7 membered heteroaryl;
R₂ is selected from hydrogen, hydroxyl, amino, carboxyl, cyano, nitro, halogen, C₁₋₆ alkyl, halo C₁₋₆ alkyl, C₁₋₆ alkoxy, halo C₁₋₆ alkoxy, C₂₋₆ alkenyl, C₂₋₆ alkynyl, 3-7 membered heterocyclyl, 3-7 membered cycloalkyl, aryl, and 5-7 membered heteroaryl;
R₁ and R₂ are each optionally substituted by 1-3 substituents selected from hydroxyl, amino, carboxyl, cyano, nitro, halogen, carbonyl, C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, and 5-7 membered heteroaryl;
   or
R₁ and R₂, together with the carbon atom to which they are attached, form a 5-12 membered ring A; the 5-12 membered ring A is optionally substituted by 1-4 substituents selected from hydroxyl, amino, carboxyl, cyano, nitro, halogen, carbonyl, C₁₋₆ alkyl, -NH-C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂;
R₃ is selected from -(C₁₋₆ alkylene)₀₋₂-NR₄R₅, -(C₁₋₆ alkylene)₀₋₂-NR₄-COR₅, -(C₁₋₆ alkylene)₀₋₂-CO-NR₄-R₅, and - (C₁₋₆ alkylene)₀₋₂-NR₄-C₁₋₆ alkylene-R₅;
R₄ is selected from hydrogen or C₁₋₆ alkyl;
R₅ is selected from 3-7 membered heterocyclyl, 3-7 membered cycloalkyl, aryl, and 5-7 membered heteroaryl; R₅ is optionally substituted by 1-4 substituents selected from halogen, cyano, amino, hydroxyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₂₋₆ alkenylcarbonyl, sulfonyl, and C₁₋₆ alkylcarbonyl;
Y is selected from aryl, 5-14 membered heteroaryl, 3-14 membered heterocyclyl, and 3-12 membered cycloalkyl, and Y is substituted by C₂₋₆ alkenyl or C₂₋₆ alkynyl, and is optionally substituted by 1-2 substituents selected from halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, and sulfonyl;
when R₅ is substituted,
the substituent on R₅, which is C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from halogen, cyano, amino, hydroxyl, carbonyl, C₁₋₆ alkyl, 3-6 membered cycloalkyl, and C₁₋₆ alkylsulfonyl;
when Y is substituted,
the substituent on Y, which is C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from halogen, cyano, amino, hydroxyl, carbonyl, C₁₋₆ alkyl, and 3-6 membered cycloalkyl;
the substituent on Y, which is C₂₋₆ alkenyl or C₂₋₆ alkynyl, is optionally substituted by 1-3 substituents selected from halogen, cyano, amino, hydroxyl, carbonyl, C₁₋₆ alkyl, 3-6 membered cycloalkyl, and halo C₁₋₆ alkyl.

In some embodiments, the present invention provides the compound of general formula (I) or the pharmacologically acceptable salt, stereoisomer or tautomer thereof: 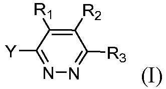 wherein,
R₁ is selected from hydrogen, hydroxyl, amino, carboxyl, cyano, nitro, halogen, C₁₋₆ alkyl, halo C₁₋₆ alkyl, C₁₋₆ alkoxy, halo C₁₋₆ alkoxy, C₂₋₆ alkenyl, C₂₋₆ alkynyl, 3-7 membered heterocyclyl, 3-7 membered cycloalkyl, aryl, and 5-7 membered heteroaryl;
R₂ is selected from hydrogen, hydroxyl, amino, carboxyl, cyano, nitro, halogen, C₁₋₆ alkyl, halo C₁₋₆ alkyl, C₁₋₆ alkoxy, halo C₁₋₆ alkoxy, C₂₋₆ alkenyl, C₂₋₆ alkynyl, 3-7 membered heterocyclyl, 3-7 membered cycloalkyl, aryl, and 5-7 membered heteroaryl;
R₁ and R₂ are each optionally substituted by 1-3 substituents selected from hydroxyl, amino, carboxyl, cyano, nitro, halogen, carbonyl, C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, and 5-7 membered heteroaryl;
   or
R₁ and R₂, together with the carbon atom to which they are attached, form a 5-12 membered ring A; the 5-12 membered ring A is optionally substituted by 1-4 substituents selected from hydroxyl, amino, carboxyl, cyano, nitro, halogen, carbonyl, C₁₋₆ alkyl, -NH-C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂;
R₃ is selected from -(C₁₋₆ alkylene)₀₋₂-NR₄R₅, -(C₁₋₆ alkylene)₀₋₂-NR₄-COR₅, and -(C₁₋₆ alkylene)₀₋₂-CO-NR₄-R₅; R₄ is selected from hydrogen or C₁₋₆ alkyl;
R₅ is selected from 3-7 membered heterocyclyl, 3-7 membered cycloalkyl, aryl, and 5-7 membered heteroaryl; R₅ is optionally substituted by 1-4 substituents selected from halogen, cyano, amino, hydroxyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₂₋₆ alkenylcarbonyl, sulfonyl, and C₁₋₆ alkylcarbonyl;
Y is selected from aryl, 5-14 membered heteroaryl, 3-14 membered heterocyclyl, and 3-12 membered cycloalkyl, and Y is substituted by C₂₋₆ alkenyl or C₂₋₆ alkynyl, and is optionally substituted by 1-2 substituents selected from halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, and sulfonyl;
when R₅ is substituted,
the substituent on R₅, which is C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from halogen, cyano, amino, hydroxyl, carbonyl, C₁₋₆ alkyl, and 3-6 membered cycloalkyl;
when Y is substituted,
the substituent on Y, which is C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from halogen, cyano, amino, hydroxyl, carbonyl, C₁₋₆ alkyl, and 3-6 membered cycloalkyl; or the substituent on Y, which is C₂₋₆ alkenyl or C₂₋₆ alkynyl, is not substituted.

In some embodiments, the compound of general formula (I) or the pharmacologically acceptable salt, stereoisomer or tautomer thereof is provided, 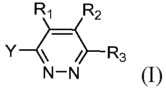 wherein,
R₁ is selected from hydrogen, hydroxyl, amino, carboxyl, cyano, nitro, halogen, C₁₋₆ alkyl, halo C₁₋₆ alkyl, C₁₋₆ alkoxy, halo C₁₋₆ alkoxy, C₂₋₆ alkenyl, C₂₋₆ alkynyl, 3-7 membered heterocyclyl, 3-7 membered cycloalkyl, aryl, and 5-7 membered heteroaryl;
R₂ is selected from hydrogen, hydroxyl, amino, carboxyl, cyano, nitro, halogen, C₁₋₆ alkyl, halo C₁₋₆ alkyl, C₁₋₆ alkoxy, halo C₁₋₆ alkoxy, C₂₋₆ alkenyl, C₂₋₆ alkynyl, 3-7 membered heterocyclyl, 3-7 membered cycloalkyl, aryl, and 5-7 membered heteroaryl;
R₁ and R₂ are each optionally substituted by 1-3 substituents selected from hydroxyl, amino, carboxyl, cyano, nitro, halogen, carbonyl, C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, and 5-7 membered heteroaryl;
   or
R₁ and R₂, together with the carbon atom to which they are attached, form a 5-12 membered ring A; the 5-12 membered ring A is optionally substituted by 1-4 substituents selected from hydroxyl, amino, carboxyl, cyano, nitro, halogen, carbonyl, C₁₋₆ alkyl, -NH-C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂;
R₃ is selected from -(C₁₋₆ alkylene)₀₋₂-NR₄R₅, -(C₁₋₆ alkylene)₀₋₂-NR₄-COR₅, and -(C₁₋₆ alkylene)₀₋₂-CO-NR₄-R₅; R₄ is selected from hydrogen or C₁₋₆ alkyl; R₅ is selected from 3-7 membered heterocyclyl, 3-7 membered cycloalkyl, aryl, and 5-7 membered heteroaryl; R₅ is optionally substituted by 1-4 substituents selected from halogen, cyano, amino, hydroxyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₂₋₆ alkenylcarbonyl, sulfonyl, and C₁₋₆ alkylcarbonyl;
Y is selected from aryl, 5-14 membered heteroaryl, 3-14 membered heterocyclyl, and 3-12 membered cycloalkyl, and Y is optionally substituted by 1-3 substituents selected from halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, C₂₋₆ alkenyl, C₂₋₆ alkynyl, and sulfonyl; and
the substituent to substitute R₅ or Y is C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, or sulfonyl, and is optionally substituted by 1-3 substituents selected from halogen, cyano, amino, hydroxyl, carbonyl, C₁₋₆ alkyl, and 3-6 membered cycloalkyl.

In some embodiments, the present invention further provides the compound or the pharmacologically acceptable salt, stereoisomer or tautomer thereof, which has a structure shown in general formula (B): 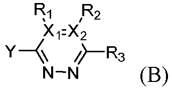
R₁ is selected from hydrogen, hydroxyl, amino, carboxyl, cyano, nitro, halogen, carbonyl, C₁₋₆ alkyl, halo C₁₋₆ alkyl, C₁₋₆ alkoxy, halo C₁₋₆ alkoxy, C₂₋₆ alkenyl, C₂₋₆ alkynyl, 3-7 membered heterocyclyl, 3-7 membered cycloalkyl, aryl, 5-7 membered heteroaryl, -N(C₁₋₆ alkyl)₂, -S-C₁₋₆ alkyl, or absent;
R₂ is selected from hydrogen, hydroxyl, amino, carboxyl, cyano, nitro, halogen, carbonyl, C₁₋₆ alkyl, halo C₁₋₆ alkyl, C₁₋₆ alkoxy, halo C₁₋₆ alkoxy, C₂₋₆ alkenyl, C₂₋₆ alkynyl, 3-7 membered heterocyclyl, 3-7 membered cycloalkyl, aryl, 5-7 membered heteroaryl, -N(C₁₋₆ alkyl)₂, -S-C₁₋₆ alkyl, or absent;
R₁ and R₂ are each optionally substituted by 1-3 substituents selected from hydroxyl, amino, carboxyl, cyano, nitro, halogen, carbonyl, C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, and 5-7 membered heteroaryl;
either one or two of X₁ and X₂ is/are selected from N; and
Y is substituted by hydroxyl and is optionally substituted by 1-2 substituents selected from C₂₋₆ alkenyl, C₂₋₆ alkynyl, halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, C₁₋₆ alkylamino, C₁₋₆ alkylcarbonylamino, C₁₋₆ alkylsulfonyl, aminocarbonyl, C₁₋₆ alkylaminocarbonyl, sulfonyl, -N(C₁₋₆ alkyl)₂, and -S-C₁₋₆ alkyl.

In some embodiments, Y is selected from phenyl, 5-7 membered heteroaryl, 3-8 membered heterocyclyl, and 3-7 membered cycloalkyl; Y is substituted by C₂₋₆ alkenyl, or C₂₋₆ alkynyl, and is optionally substituted by 1-2 substituents selected from halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, C₁₋₆ alkylamino, C₁₋₆ alkylcarbonylamino, C₁₋₆ alkylsulfonyl, aminocarbonyl, C₁₋₆ alkylaminocarbonyl, and sulfonyl;
when Y is substituted, the substituent on Y, which is C₁₋₆ alkyl, halo C₁₋₆ alkyl, C₁₋₆ alkoxy, 3-7 membered heterocyclyl, 3-7 membered cycloalkyl, or sulfonyl, is optionally substituted by 1-3 substituents selected from halogen, cyano, amino, hydroxyl, carbonyl, and C₁₋₆ alkyl; and
R₃ is selected from -NH-R₅, and R₅ is 3-7 membered heterocyclyl substituted by 1-2 substituents selected from C₁₋₆ alkyl.

In some embodiments, the ring A is selected from 5-12 membered cycloalkyl, 5-12 membered cycloalkenyl, 5-12 membered heterocyclyl, aryl, and 5-12 membered heteroaryl.

In some embodiments, the ring A is selected from 5-8 membered cycloalkyl, 5-8 membered cycloalkenyl, 5-8 membered heterocyclyl, phenyl, and 5-8 membered heteroaryl.

In some embodiments, the present invention provides a compound with a structure shown in general formula (II) or a pharmacologically acceptable salt, stereoisomer or tautomer thereof, 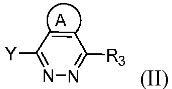 wherein,
a ring A is selected from 5-7 membered cycloalkenyl, 5-7 membered cycloalkyl, 5-7 membered heterocyclyl, phenyl, and 5-7 membered heteroaryl; and the ring A is optionally substituted by 1-4 substituents selected from hydroxyl, amino, carboxyl, cyano, nitro, halogen, C₁₋₆ alkyl, -NH-C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂.

In some embodiments, the ring A is selected from phenyl and 5-7 membered heteroaryl; and the ring A is optionally substituted by 1-4 substituents selected from hydroxyl, amino, carboxyl, cyano, nitro, halogen, C₁₋₆ alkyl, -NH-C₁₋₆ alkyl, halo C₁₋₆ alkyl, C₁₋₆ alkoxy, halo C₁₋₆ alkoxy, 3-7 membered heterocyclyl, 3-7 membered cycloalkyl, C₁₋₆ alkylsulfonyl, and -N(C₁₋₆ alkyl)₂.

In some embodiments, the ring A is selected from phenyl, 5-6 membered heteroaryl containing 1-2 heteratoms selected from O, S, or N, and 5-6 membered saturated ring.

In some embodiments, the ring A is optionally optionally substituted by 1-4 substituents selected from hydroxyl, amino, carboxyl, cyano, nitro, halogen, carbonyl, C₁₋₆ alkyl, -NH-C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂.

In some embodiments, the ring A is not substituted.

In some embodiments, the ring A is substituted by 1-4 substituents selected from hydroxyl, amino, carboxyl, cyano, nitro, halogen, C₁₋₆ alkyl, -NH-C₁₋₆ alkyl, halo C₁₋₆ alkyl, C₁₋₆ alkoxy, halo C₁₋₆ alkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂.

In some embodiments, the ring A is substituted by 1-2 substituents selected from cyano, C₁₋₆ alkoxy, C₁₋₆ alkyl, halo C₁₋₆ alkyl, C₁₋₆ alkylsulfonyl, -N(C₁₋₆ alkyl)₂.

In some embodiments, the ring A is substituted by 1 substituent selected from cyano, C₁₋₆ alkoxy, C₁₋₆ alkyl, halo C₁₋₆ alkyl, C₁₋₆ alkylsulfonyl, -N(C₁₋₆ alkyl)₂.

In some embodiments, the ring A is selected from phenyl, 5-6 membered heteroaryl containing 1-2 heteratoms selected from O, S, or N, and 5-6 membered saturated ring; and the ring A is not substituted or is substituted by 1 substituent selected from cyano, C₁₋₆ alkoxy, C₁₋₆ alkyl, halo C₁₋₆ alkyl, C₁₋₆ alkylsulfonyl, -N(C₁₋₆ alkyl)₂.

In some embodiments, the ring A is selected from 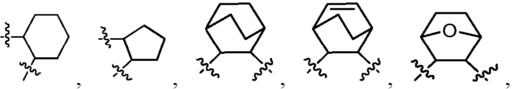 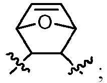 and the ring A is optionally substituted by 1-4 substituents selected from hydroxyl, amino, carboxyl, cyano, nitro, halogen, C₁₋₆ alkyl, -NH-C₁₋₆ alkyl, halo C₁₋₆ alkyl, C₁₋₆ alkoxy, halo C₁₋₆ alkoxy, 3-7 membered heterocyclyl, 3-7 membered cycloalkyl, C₁₋₆ alkylsulfonyl, and -N(C₁₋₆ alkyl)₂.

In some embodiments, the ring A is selected from 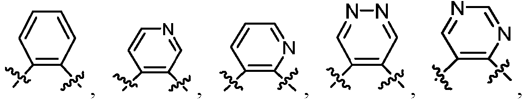 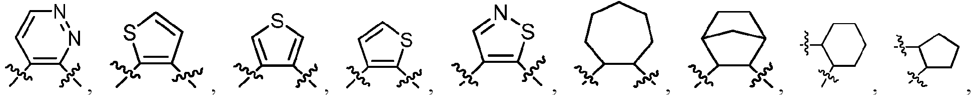 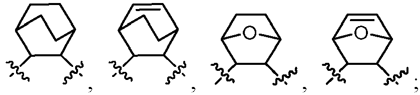 and the ring A is optionally substituted by 1-2 substituents selected from cyano, nitro, halogen, C₁₋₆ alkyl, halo C₁₋₆ alkyl, C₁₋₆ alkoxy, 3-7 membered cycloalkyl, 5-7 membered heteroaryl, C₁₋₆ alkylsulfonyl, and -N(C₁₋₆ alkyl)₂.

In some embodiments, the ring A is

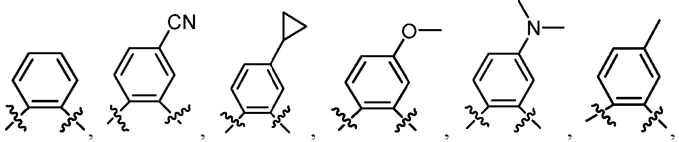

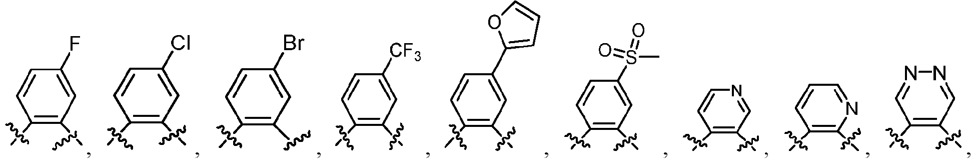

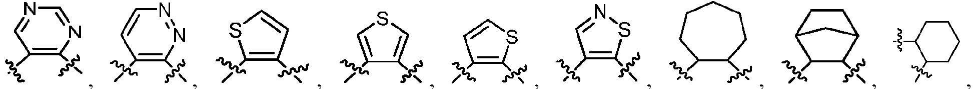

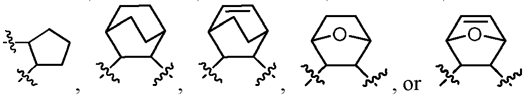

In some embodiments, R₃ is selected from -(C₁₋₆ alkylene)₀₋₂-NR₄R₅, -(C₁₋₆ alkylene)₀₋₂-NR₄-COR₅, -(C₁₋₆ alkylene)₀₋₂-CO-NR₄-R₅, and -(C₁₋₆ alkylene)₀₋₂-NR₄-C₁₋₆ alkylene-R₅; R₄ is selected from hydrogen or C₁₋₆ alkyl; and R₅ is selected from 3-7 membered heterocyclyl, 3-7 membered cycloalkyl, aryl, and 5-7 membered heteroaryl. In some embodiments, R₄ is selected from hydrogen.

In some embodiments, R₄ is selected from C₁₋₆ alkyl.

In some embodiments, R₄ is selected from methyl or ethyl.

In some embodiments, R₃ is selected from -NR₄R₅, and -NR₄-C₁₋₆ alkylene-R₅, R₄ is selected from hydrogen, and R₅ is selected from 3-7 membered heterocyclyl, 3-7 membered cycloalkyl, aryl, and 5-7 membered heteroaryl.

In some embodiments, R₃ is selected from -NR₄R₅, and -NR₄-C₁₋₆ alkylene-R₅, R₄ is selected from hydrogen, and R₅ is selected from 3-7 membered cycloalkyl, and 3-7 membered heterocyclyl; and preferably, R₃ is selected from -NHR₅, and R₅ is selected from 4-6 membered cycloalkyl and 4-6 membered heterocyclyl.

In some embodiments, R₅ is selected from 4-6 membered cycloalkyl, or 4-6 membered heterocyclyl containing 1 heteratom selected from O, N, or S.

In some embodiments, R₅ is optionally substituted by 1-4 substituents selected from halogen, cyano, amino, hydroxyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₂₋₆ alkenylcarbonyl, sulfonyl, and C₁₋₆ alkylcarbonyl; and when R₅ is substituted, the substituent on R₅, which is C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from halogen, cyano, amino, hydroxyl, carbonyl, C₁₋₆ alkyl, 3-6 membered cycloalkyl, 3-6 membered heterocyclyl, carboxyl, and C₁₋₆ alkylsulfonyl.

In some embodiments, R₅ is not substituted.

In some embodiments, R₅ is substituted by 1-2 substituents selected from halogen, cyano, amino, hydroxyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₂₋₆ alkenylcarbonyl, sulfonyl, and C₁₋₆ alkylcarbonyl.

In some embodiments, when R₅ is substituted, the substituent on R₅, which is C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, or sulfonyl, is not substituted. In some embodiments, when R₅ is substituted, the substituent on R₅, which is C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, or sulfonyl, is substituted by 1-3 substituents selected from halogen, cyano, amino, hydroxyl, carbonyl, C₁₋₆ alkyl, 3-6 membered cycloalkyl, 3-6 membered heterocyclyl, carboxyl, and C₁₋₆ alkylsulfonyl.

In some embodiments, R₅ is selected from

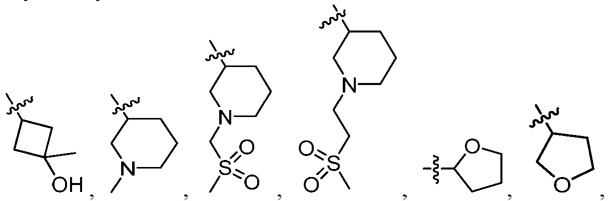

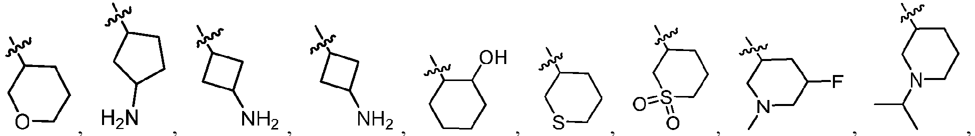

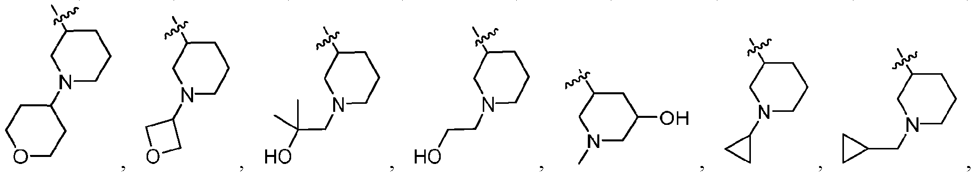

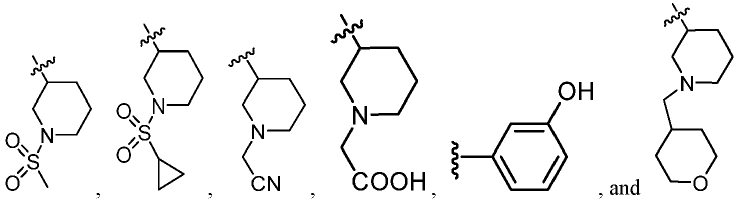

Preferably, R₅ is selected from

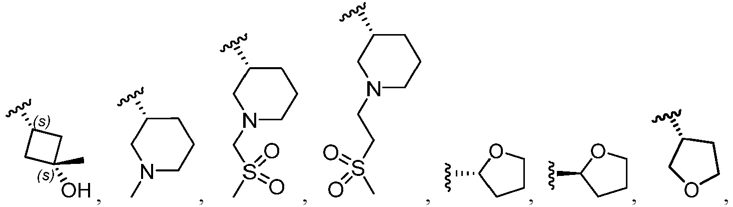

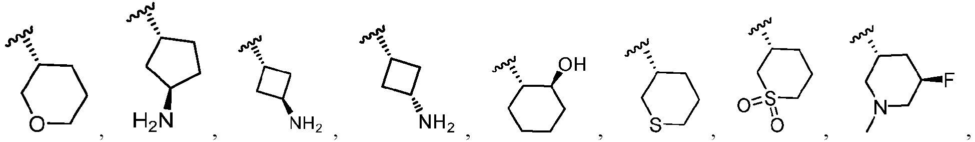

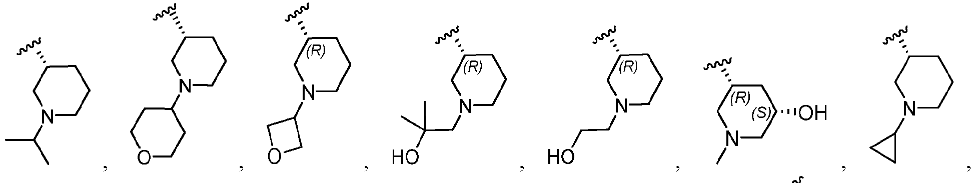

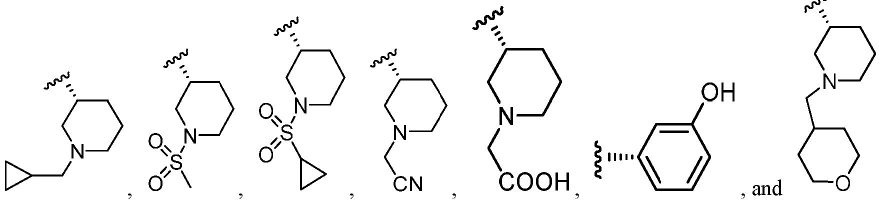

In some embodiments, Y is selected from aryl, 5-14 membered heteroaryl, 3-14 membered heterocyclyl, and 3-12 membered cycloalkyl, and Y is substituted by C₂₋₆ alkenyl or C₂₋₆ alkynyl, and is optionally substituted by 1-2 substituents selected from halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, and sulfonyl.

In some embodiments, Y is selected from phenyl, 5-7 membered heteroaryl, 3-8 membered heterocyclyl, and 3-7 membered cycloalkyl; Y is substituted by C₂₋₆ alkenyl, or C₂₋₆ alkynyl, and is optionally substituted by 1-2 substituents selected from halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, C₁₋₆ alkylamino, C₁₋₆ alkylcarbonylamino, C₁₋₆ alkylsulfonyl, aminocarbonyl, C₁₋₆ alkylaminocarbonyl, and sulfonyl.

In some embodiments, Y is selected from phenyl, 5-8 membered heteroaryl, 3-8 membered heterocyclyl, and 3-7 membered cycloalkyl.

In some embodiments, Y is selected from phenyl and 5-6 membered heteroaryl.

In some embodiments, Y is selected from phenyl and 5-6 membered heteroaryl containing 1-2 heteroatoms of N.

In some embodiments, Y is optionally substituted by 1-3 substituents selected from C₂₋₆ alkenyl, C₂₋₆ alkynyl, halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, sulfonyl, -N(C₁₋₆ alkyl)₂, and -S-C₁₋₆ alkyl.

In some embodiments, Y is substituted by 1 substituent selected from C₂₋₆ alkenyl and C₂₋₆ alkynyl, and is optionally substituted by 1-2 substituents selected from halogen, cyano, amino, hydroxyl, carbonyl, C₁₋₆ alkyl, halo C₁₋₆ alkyl, C₁₋₆ alkoxy, 3-7 membered heterocyclyl, and 3-7 membered cycloalkyl.

In some embodiments, Y is substituted by 1-2 substituents selected from C₂₋₆ alkenyl and C₂₋₆ alkynyl.

In some embodiments, Y is substituted by 1 substituent selected from C₂₋₆ alkenyl and C₂₋₆ alkynyl. In some embodiments, Y is optionally substituted by 1-3 substituents selected from halogen, cyano, amino, hydroxyl, carbonyl, C₁₋₆ alkyl, halo C₁₋₆ alkyl, C₁₋₆ alkoxy, 3-7 membered heterocyclyl, and 3-7 membered cycloalkyl.

In some embodiments, Y is optionally substituted by 1-2 substituents selected from halogen, cyano, amino, hydroxyl, carbonyl, C₁₋₆ alkyl, halo C₁₋₆ alkyl, C₁₋₆ alkoxy, 3-7 membered heterocyclyl, and 3-7 membered cycloalkyl.

In some embodiments, Y is substituted by 1-2 substituents selected from C₂₋₆ alkenyl and C₂₋₆ alkynyl, and is optionally substituted by 1-3 substituents selected from halogen, cyano, amino, hydroxyl, carbonyl, C₁₋₆ alkyl, halo C₁₋₆ alkyl, C₁₋₆ alkoxy, 3-7 membered heterocyclyl, and 3-7 membered cycloalkyl.

In some embodiments, Y is substituted by 1 substituent selected from C₂₋₆ alkenyl and C₂₋₆ alkynyl, and Y is optionally substituted by 1 substituent selected from halogen, cyano, amino, hydroxyl, carbonyl, C₁₋₆ alkyl, halo C₁₋₆ alkyl, C₁₋₆ alkoxy, 3-7 membered heterocyclyl, and 3-7 membered cycloalkyl. In some embodiments, Y is substituted by C₂₋₆ alkenyl or C₂₋₆ alkynyl, and is optionally substituted by 1-2 substituents selected from halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, sulfonyl, -N(C₁₋₆ alkyl)₂, and -S-C₁₋₆ alkyl.

In some embodiments, Y is substituted by 1 substituent selected from C₂₋₆ alkenyl or C₂₋₆ alkynyl, and Y is substituted by 1 hydroxyl; C₂₋₆ alkenyl or C₂₋₆ alkynyl is not substituted, or is substituted by 1-3 substituents selected from halogen, cyano, amino, hydroxyl, carbonyl, unsubstituted or hydroxyl-substituted C₁₋₆ alkyl, unsubstituted or hydroxyl-substituted 3-6 membered cycloalkyl, and halo C₁₋₆ alkyl.

In some embodiments, when Y is substituted, the substituent on Y, which is C₁₋₆ alkyl, halo C₁₋₆ alkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from halogen, cyano, amino, hydroxyl, carbonyl, C₁₋₆ alkyl, and 3-6 membered cycloalkyl.

In some embodiments, the substituent on Y, which is C₂₋₆ alkenyl or C₂₋₆ alkynyl, is substituted by 1-3 substituents selected from halogen, cyano, amino, hydroxyl, carbonyl, unsubstituted or hydroxyl-substituted C₁₋₆ alkyl, unsubstituted or hydroxyl-substituted 3-6 membered cycloalkyl, and halo C₁₋₆ alkyl.

In some embodiments, the substituent on Y, which is C₂₋₆ alkenyl or C₂₋₆ alkynyl, is not substituted.

In some embodiments, Y is selected from phenyl, 5-7 membered heteroaryl, 3-8 membered heterocyclyl, and 3-7 membered cycloalkyl; Y is substituted by C₂₋₆ alkenyl, or C₂₋₆ alkynyl, and is optionally substituted by 1-2 substituents selected from halogen, cyano, amino, hydroxyl, carbonyl, C₁₋₆ alkyl, halo C₁₋₆ alkyl, C₁₋₆ alkoxy, 3-7 membered heterocyclyl, 3-7 membered cycloalkyl, C₁₋₆ alkylamino, C₁₋₆ alkylcarbonylamino, C₁₋₆ alkylsulfonyl, aminocarbonyl, C₁₋₆ alkylaminocarbonyl, and sulfonyl; and
when Y is substituted, the substitute on Y, which is C₁₋₆ alkyl, halo C₁₋₆ alkyl, C₁₋₆ alkoxy, 3-7 membered heterocyclyl, 3-7 membered cycloalkyl, or sulfonyl, is optionally substituted by 1-3 substituents selected from halogen, cyano, amino, hydroxyl, carbonyl, and C₁₋₆ alkyl.

In some embodiments, Y is selected from phenyl and 5-6 membered heteroaryl, and Y is substituted by C₂₋₆ alkenyl or C₂₋₆ alkynyl, and is optionally substituted by 1-2 substituents selected from halogen, cyano, amino, hydroxyl, carbonyl, C₁₋₆ alkyl, halo C₁₋₆ alkyl, C₁₋₆ alkoxy, 3-7 membered heterocyclyl, and 3-7 membered cycloalkyl; and C₂₋₆ alkenyl and C₂₋₆ alkynyl are substituted by 1-3 substituents selected from halogen, cyano, amino, hydroxyl, carbonyl, C₁₋₆ alkyl, 3-6 membered cycloalkyl, and halo C₁₋₆ alkyl.

In some embodiments, Y is selected from

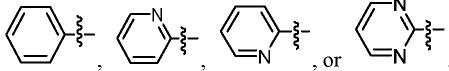

In some embodiments, when Y is substituted, the substitute on Y is selected from the group consisting of: hydroxyl,

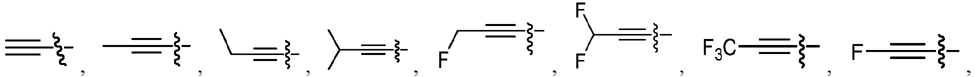

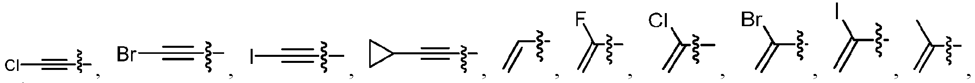

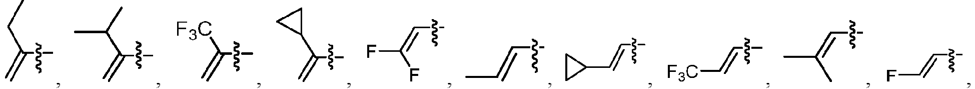

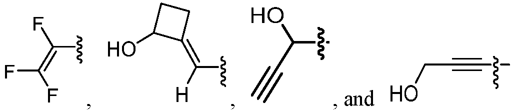

The present invention further provides the compound or the pharmacologically acceptable salt, stereoisomer or tautomer thereof, which has a structure shown in general formula (C): 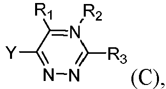 wherein the selection of R₁, R₂, R₃, or Y is as previously described.

In an embodiment of the present invention, the previously descried compound of formula (A), (B), (C), (I), or (II), or the pharmaceutically acceptable salt, stereoisomer or tautomer thereof can be found in Table 1:

**Table 1**

| No. | Structure | No. | Structure |
|---|---|---|---|
| 1 | 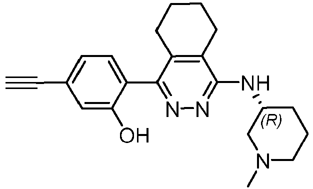 | 2 | 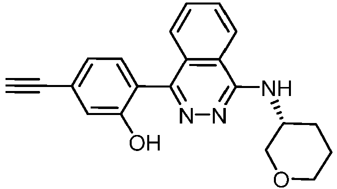 |
| 3 | 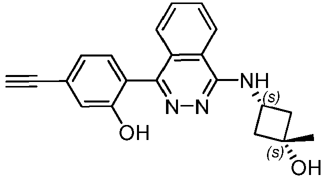 | 4 | 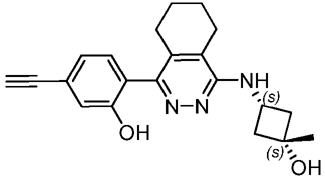 |
| 5 | 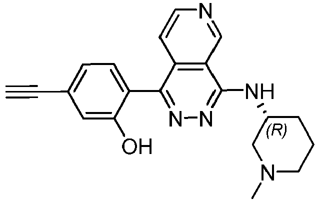 | 6 | 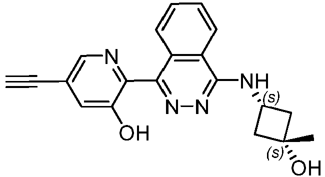 |
| 7 | 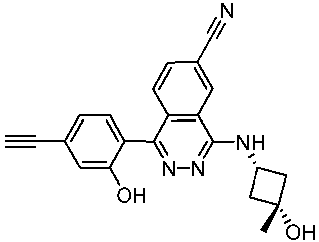 | 8 | 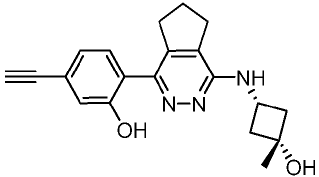 |
| 9 | 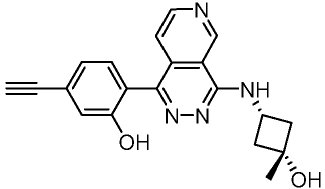 | 10 | 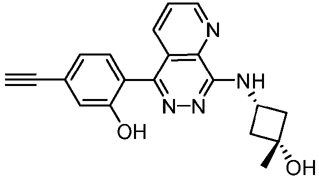 |
| 11 | 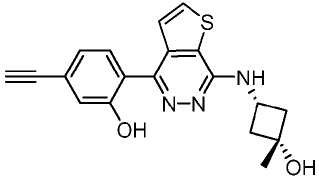 | 12 | 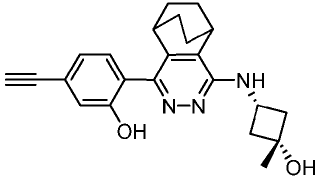 |
| 13 | 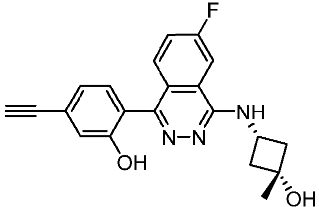 | 14 | 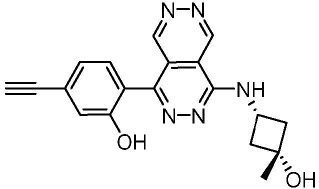 |
| 15 | 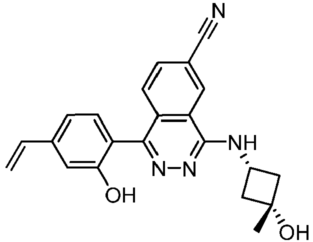 | 16 | 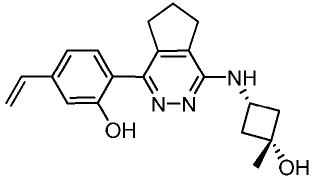 |
| 17 | 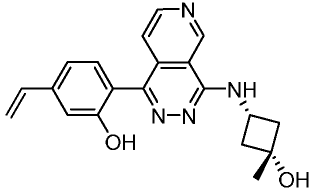 | 18 | 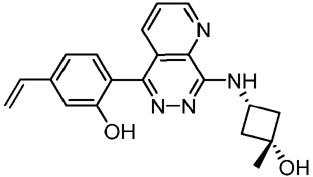 |
| 19 | 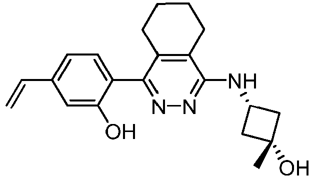 | 20 | 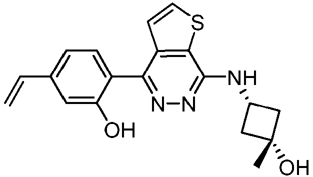 |
| 21 | 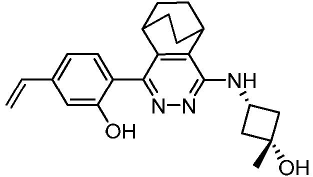 | 22 | 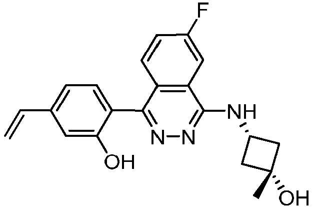 |
| 23 | 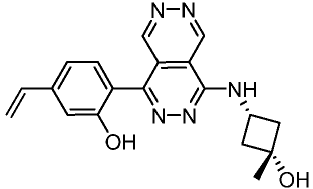 | 24 | 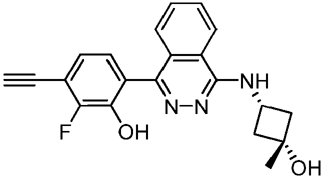 |
| 25 | 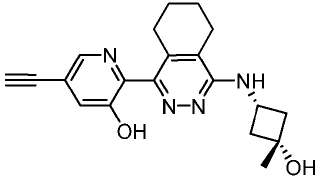 | 26 | 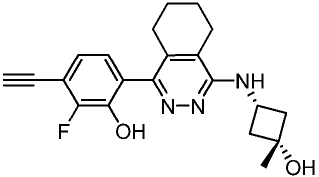 |
| 27 | 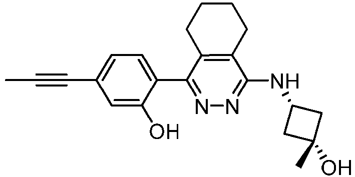 | 28 | 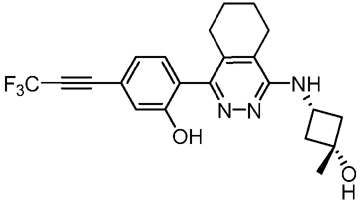 |
| 29 | 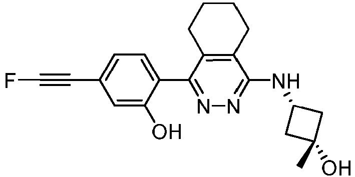 | 30 | 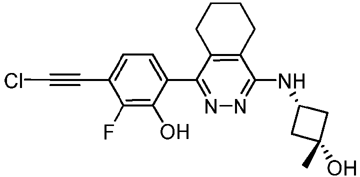 |
| 31 | 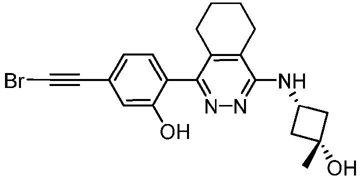 | 32 | 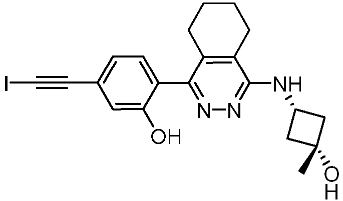 |
| 33 | 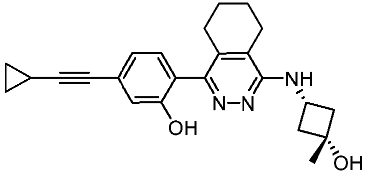 | 34 | 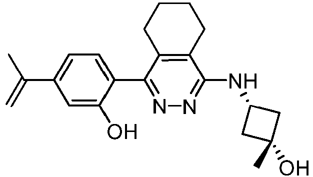 |
| 35 | 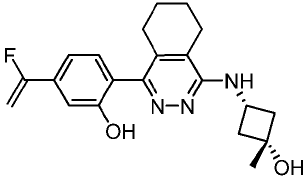 | 36 | 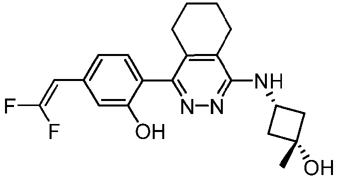 |
| 37 | 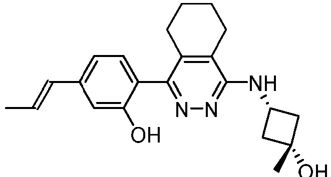 | 38 | 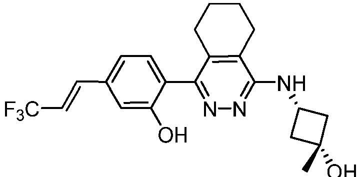 |
| 39 | 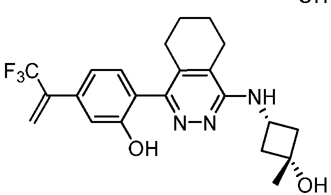 | 40 | 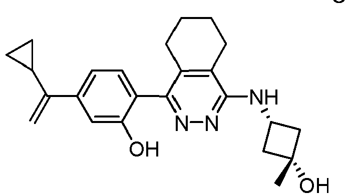 |
| 41 | 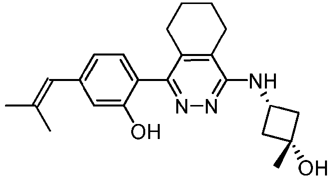 | 42 | 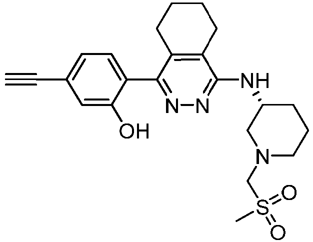 |
| 43 | 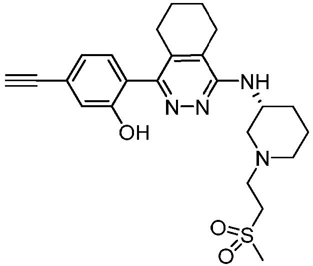 | 44 | 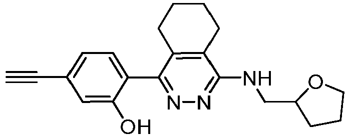 |
| 45 | 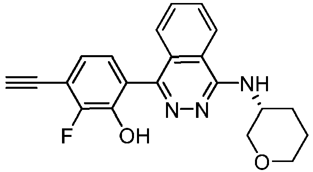 | 46 | 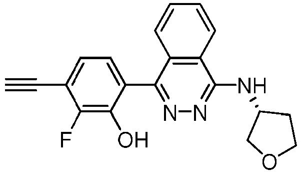 |
| 47 | 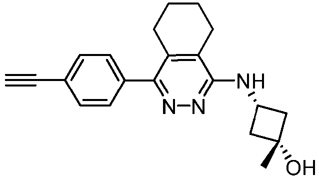 | 48 | 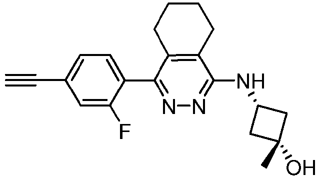 |
| 49 | 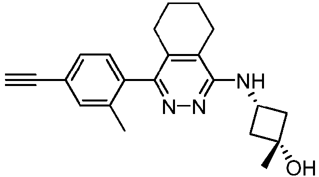 | 50 | 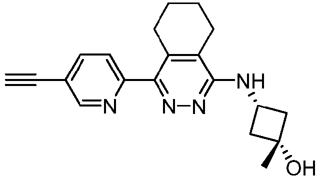 |
| 51 | 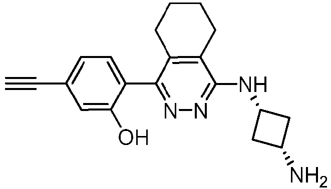 | 52 | 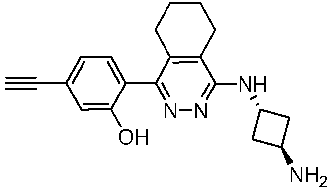 |
| 53 | 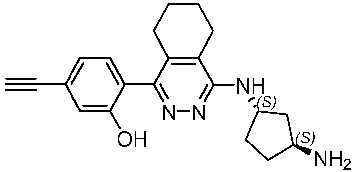 | 54 | 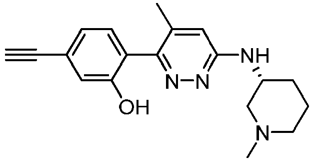 |
| 55 | 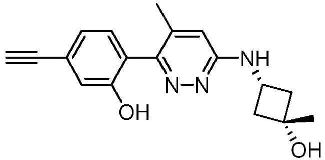 | 56 | 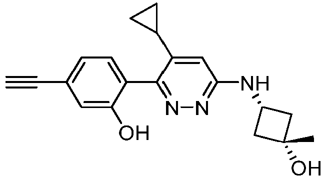 |
| 57 | 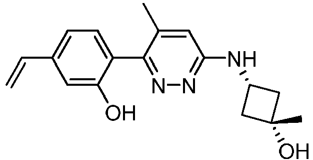 | 58 | 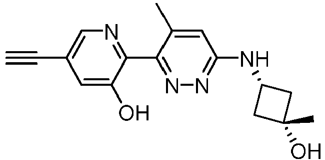 |
| 59 | 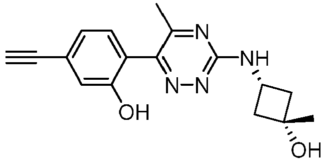 | 60 | 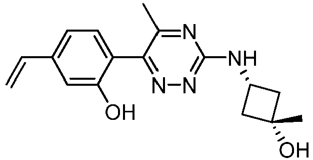 |
| 61 | 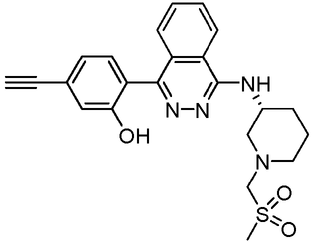 | 62 | 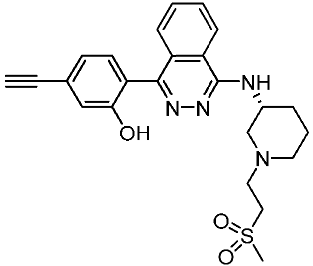 |
| 63 | 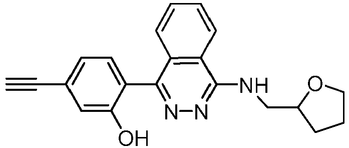 | 64 | 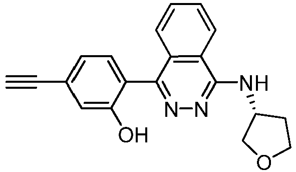 |
| 65 | 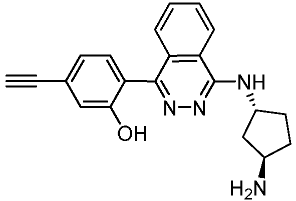 | 66 | 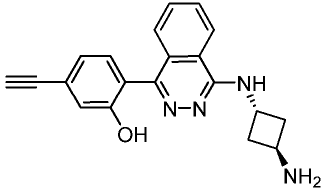 |
| 67 | 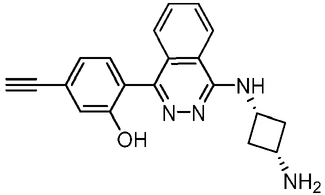 | 68 | 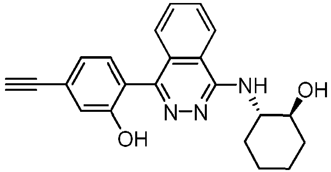 |
| 69 | 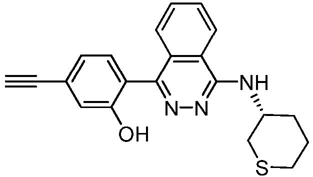 | 70 | 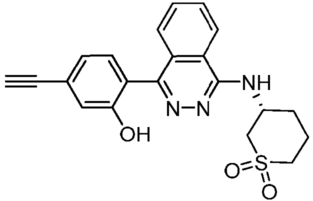 |
| 71 | 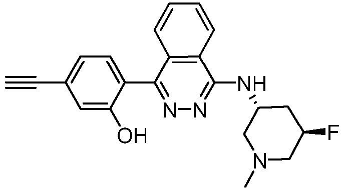 | 72 | 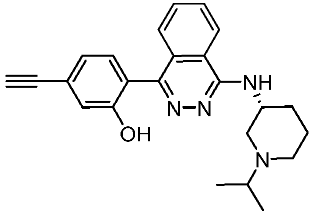 |
| 73 | 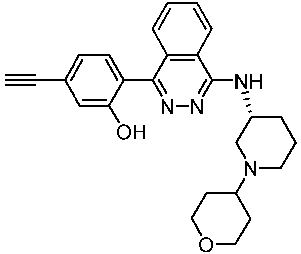 | 74 | 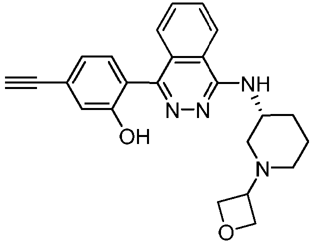 |
| 75 | 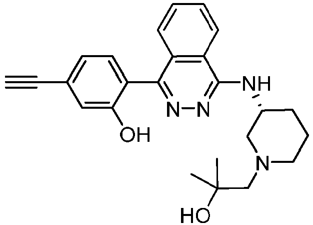 | 76 | 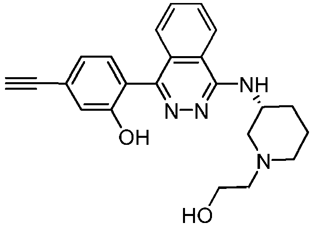 |
| 77 | 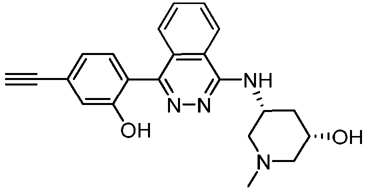 | 78 | 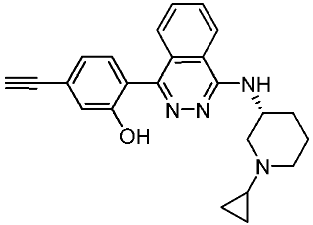 |
| 79 | 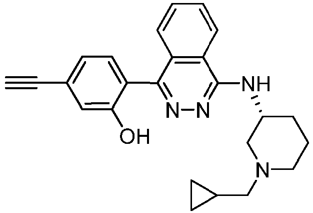 | 80 | 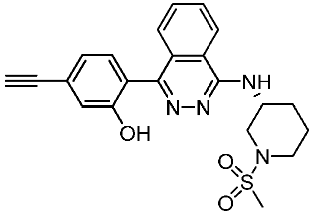 |
| 81 | 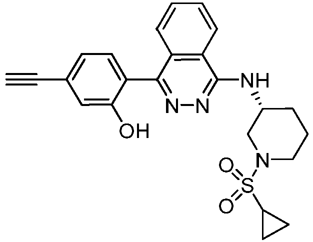 | 82 | 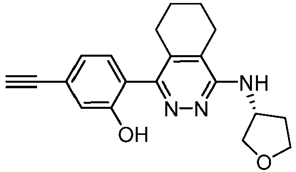 |
| 83 | 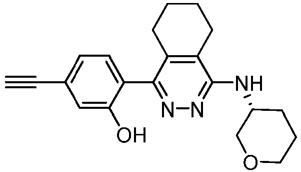 | 84 | 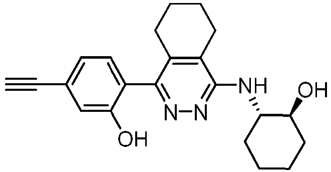 |
| 85 | 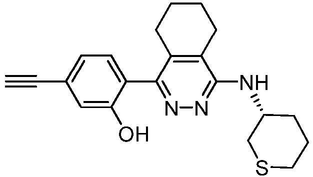 | 86 | 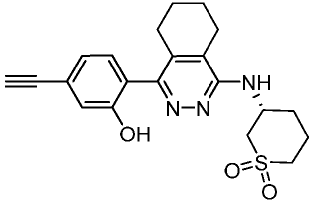 |
| 87 | 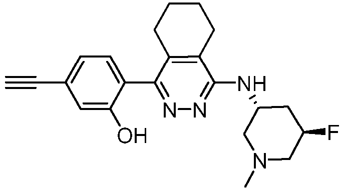 | 88 | 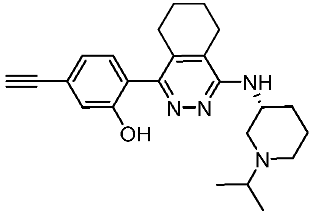 |
| 89 | 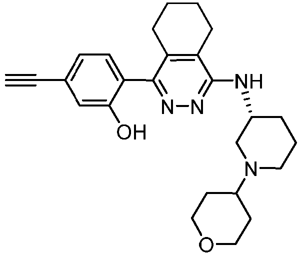 | 90 | 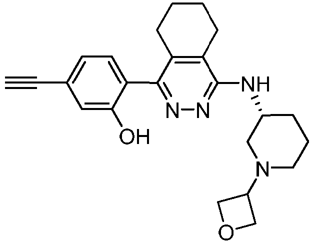 |
| 91 | 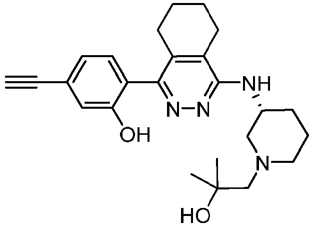 | 92 | 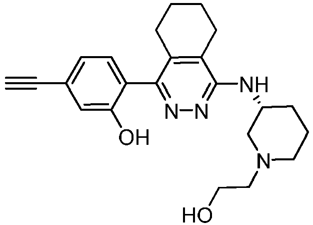 |
| 93 | 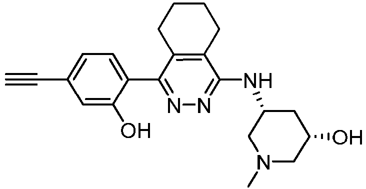 | 94 | 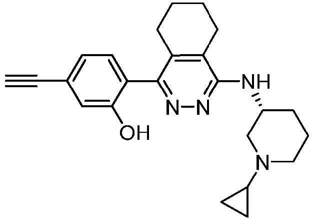 |
| 95 | 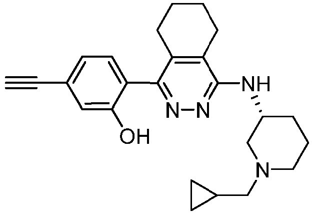 | 96 | 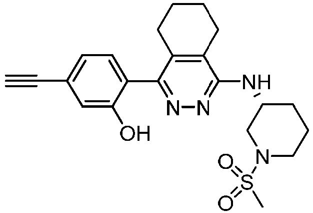 |
| 97 | 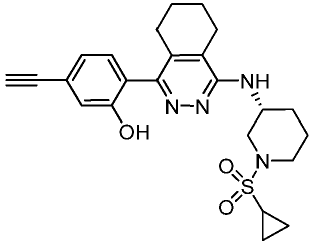 | 98 | 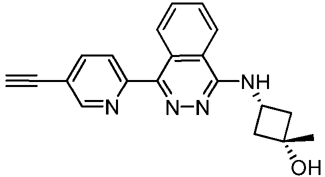 |
| 99 | 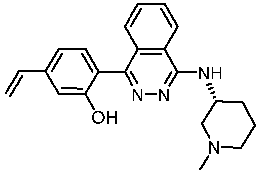 | 100 | 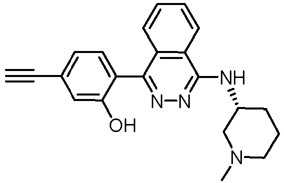 |
| 101 | 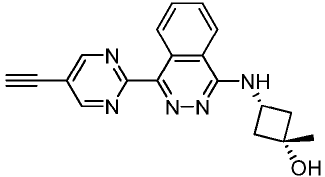 | 102 | 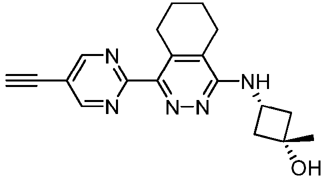 |
| 103 | 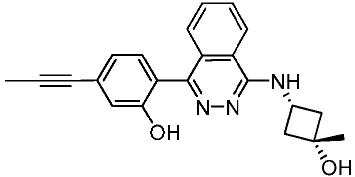 | 104 | 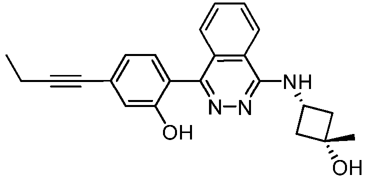 |
| 105 | 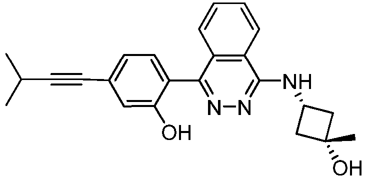 | 106 | 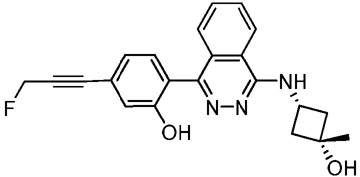 |
| 107 | 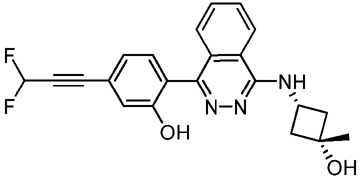 | 108 | 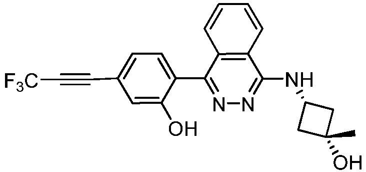 |
| 109 | 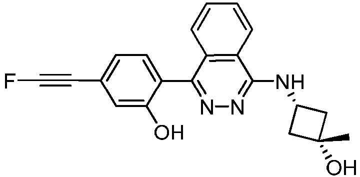 | 110 | 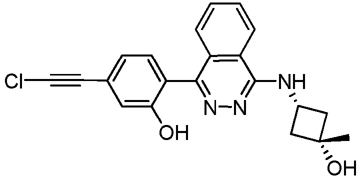 |
| 111 | 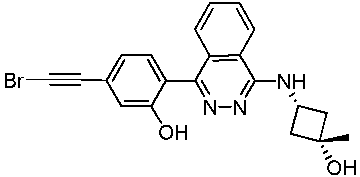 | 112 | 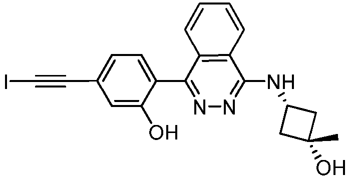 |
| 113 | 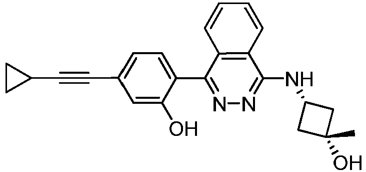 | 114 | 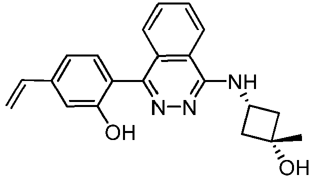 |
| 115 | 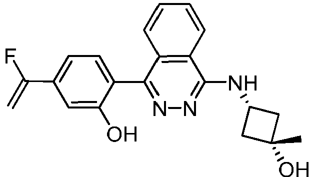 | 116 | 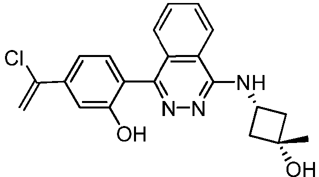 |
| 117 | 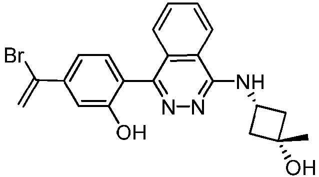 | 118 | 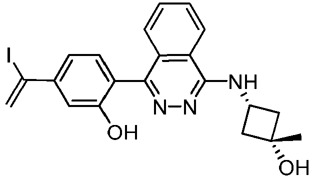 |
| 119 | 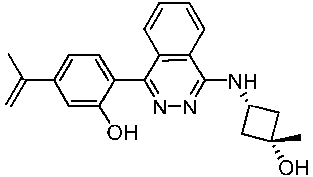 | 120 | 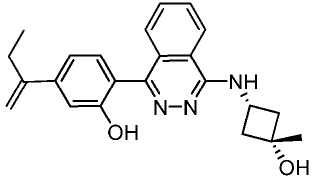 |
| 121 | 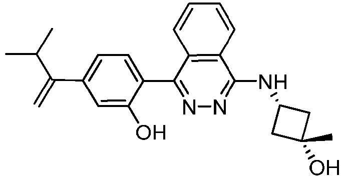 | 122 | 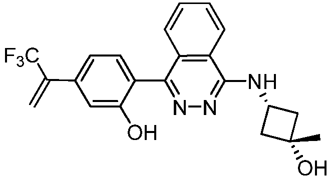 |
| 123 | 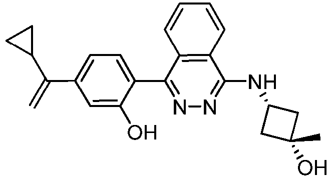 | 124 | 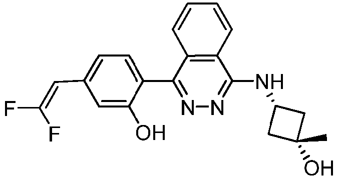 |
| 125 | 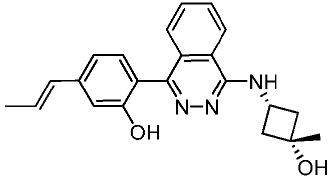 | 126 | 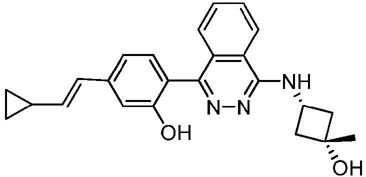 |
| 127 | 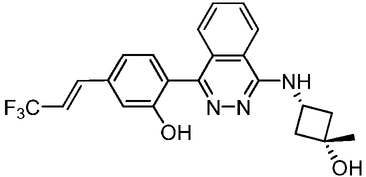 | 128 | 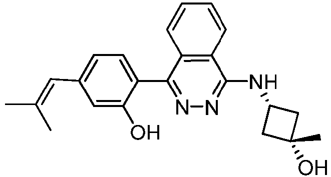 |
| 129 | 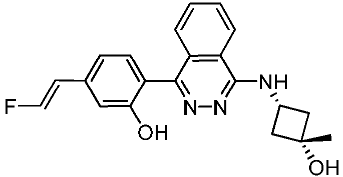 | 130 | 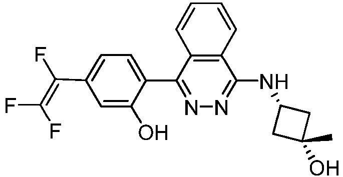 |
| 131 | 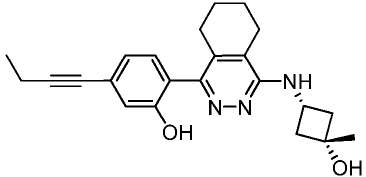 | 132 | 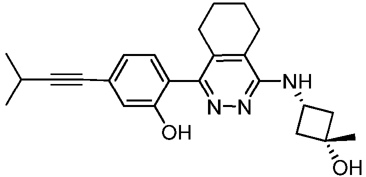 |
| 133 | 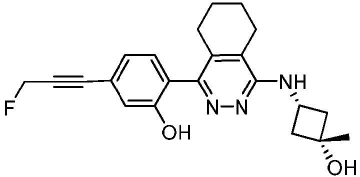 | 134 | 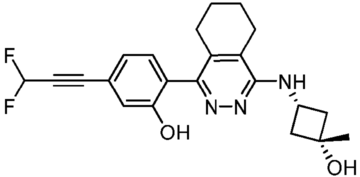 |
| 135 | 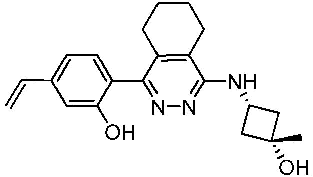 | 136 | 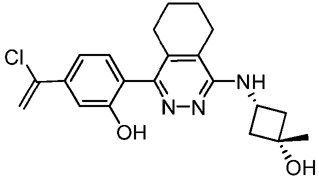 |
| 137 | 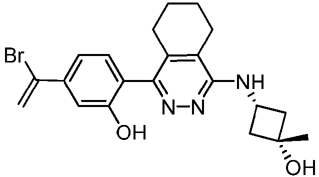 | 138 | 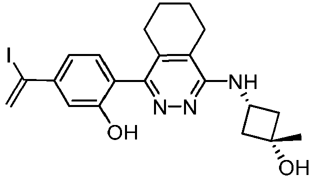 |
| 139 | 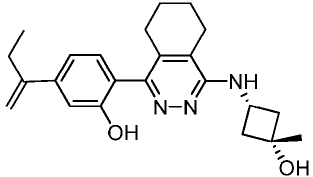 | 140 | 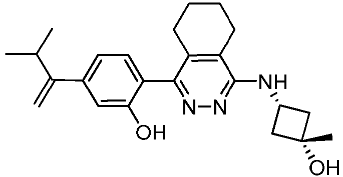 |
| 141 | 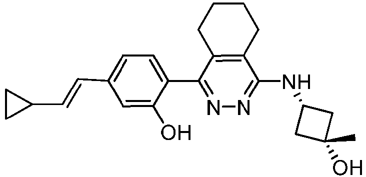 | 142 | 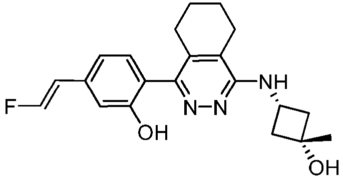 |
| 143 | 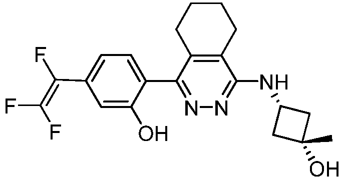 | 144 | 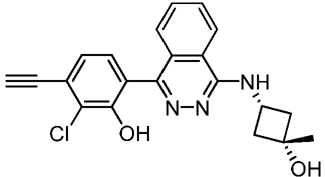 |
| 145 | 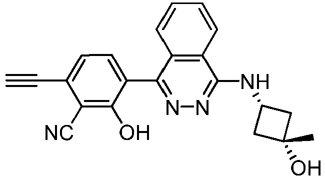 | 146 | 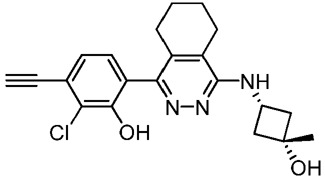 |
| 147 | 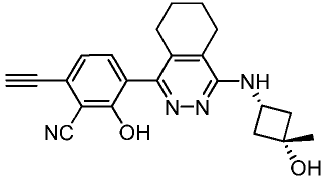 | 148 | 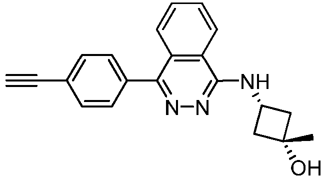 |
| 149 | 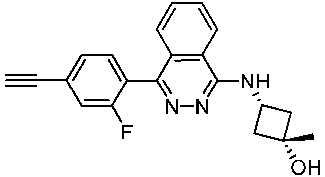 | 150 | 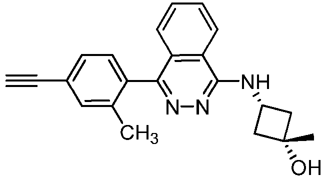 |
| 151 | 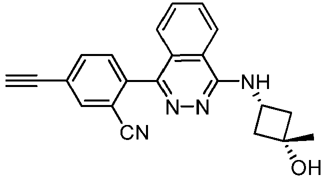 | 152 | 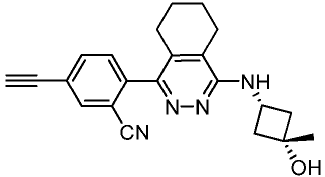 |
| 153 | 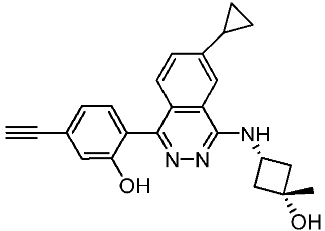 | 154 | 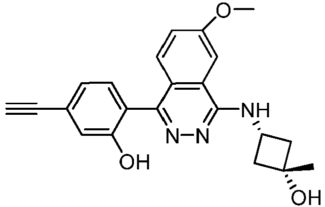 |
| 155 | 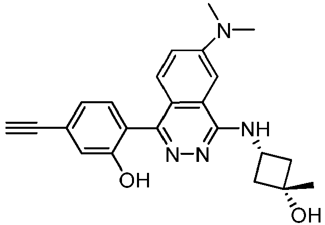 | 156 | 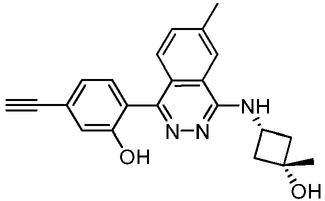 |
| 157 | 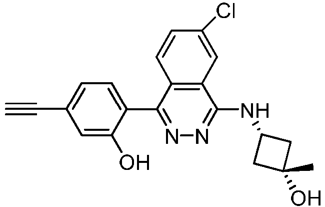 | 158 | 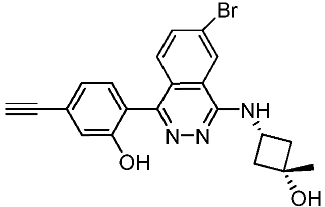 |
| 159 | 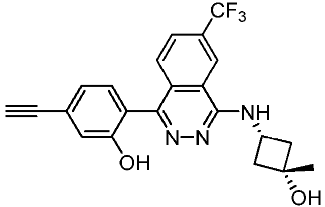 | 160 | 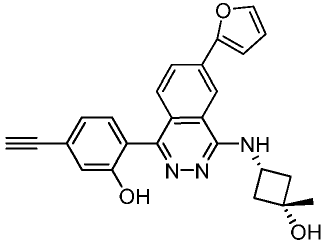 |
| 161 | 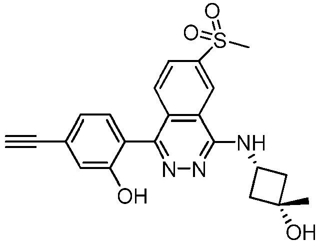 | 162 | 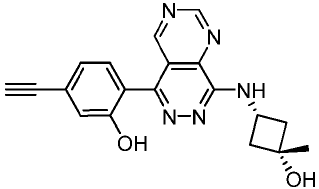 |
| 163 | 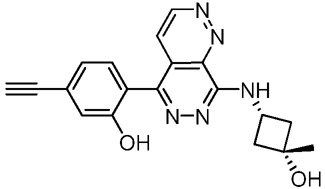 | 164 | 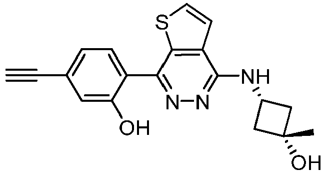 |
| 165 | 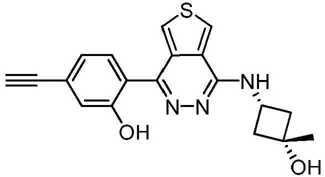 | 166 | 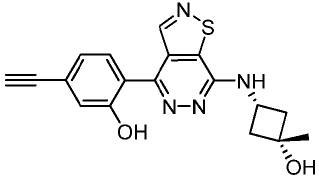 |
| 167 | 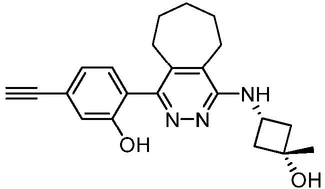 | 168 | 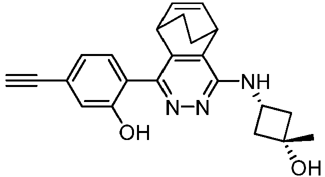 |
| 169 | 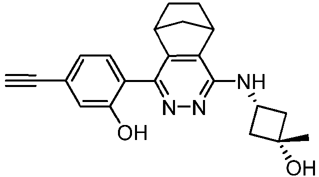 | 170 | 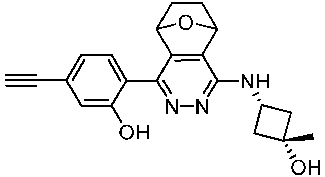 |
| 171 | 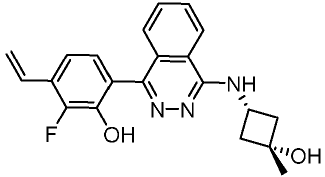 | 172 | 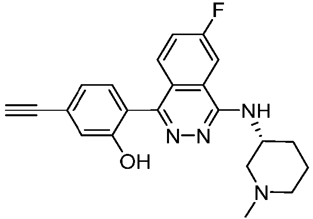 |
| 173 | 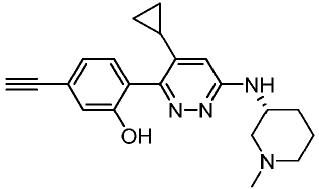 | 174 | 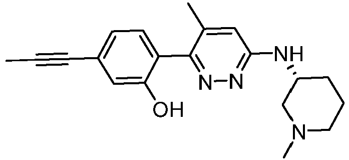 |
| 175 | 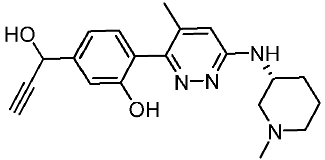 | 176 | 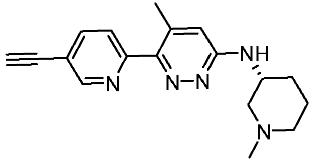 |
| 177 | 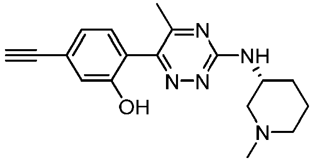 | 179 | 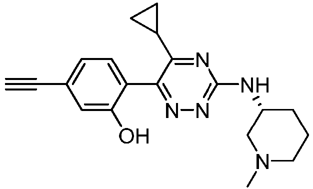 |
| 180 | 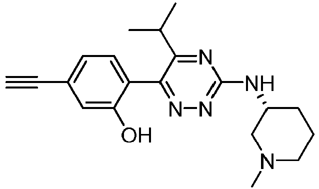 | 181 | 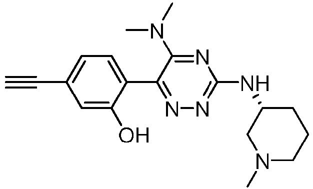 |
| 182 | 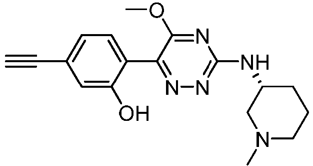 | 188 | 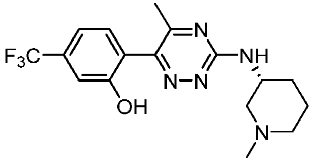 |
| 189 | 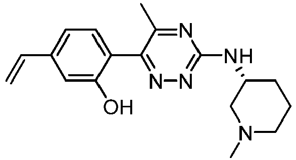 | 190 | 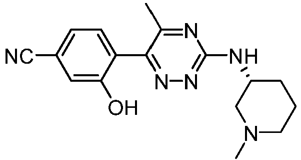 |
| 191 | 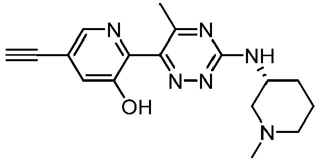 | 192 | 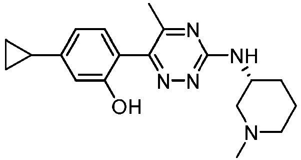 |
| 193 | 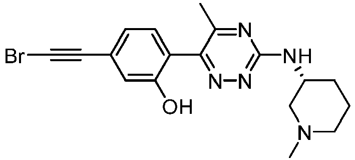 | 194 | 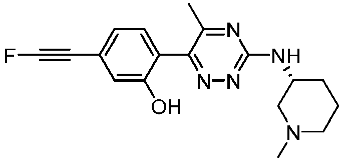 |
| 195 | 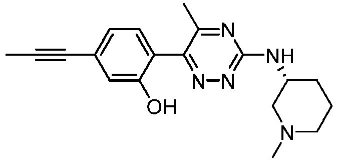 | 197 | 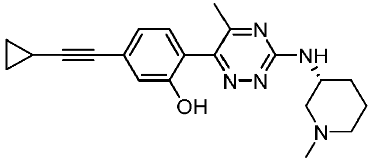 |
| 198 | 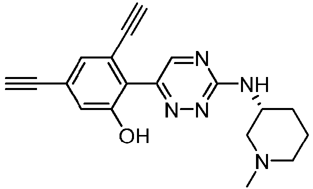 | 201 | 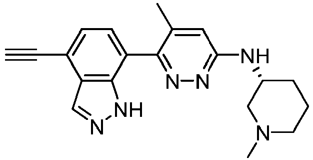 |
| 202 | 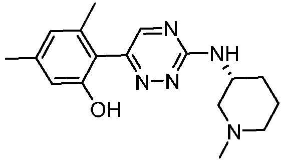 | 203 | 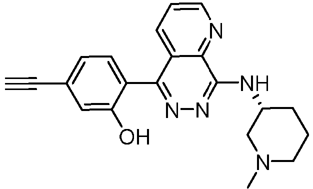 |
| 206 | 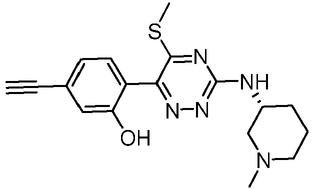 | 221 | 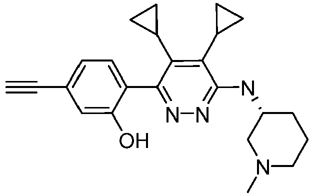 |
| 222 | 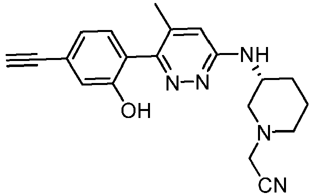 | 247 | 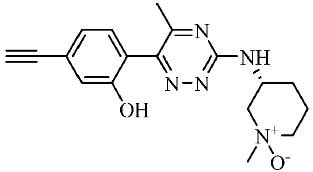 |
| 248 | 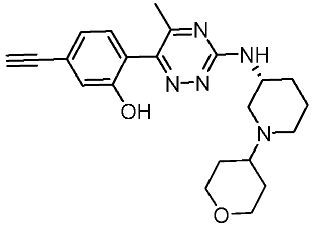 | 249 | 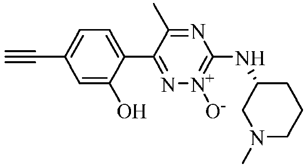 |
| 250 | 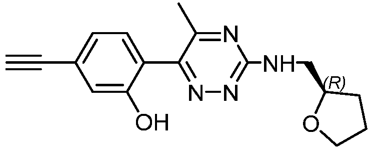 | 251 | 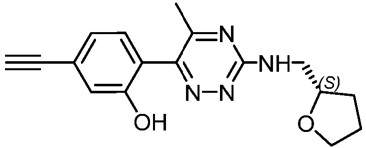 |
| 252 | 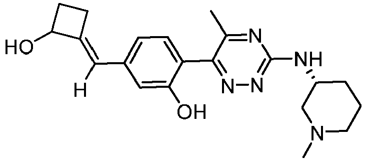 | 253 | 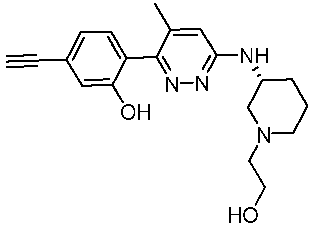 |
| 254 | 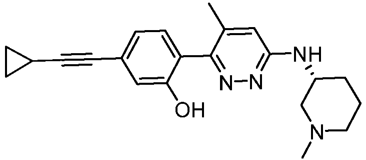 | 255 | 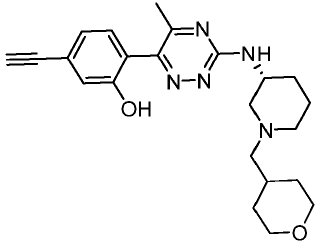 |
| 256 | 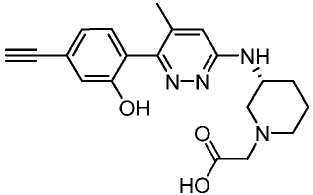 | 257 | 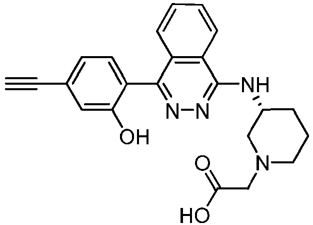 |
| 258 | 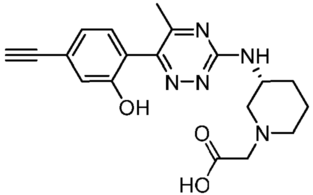 | 259 | 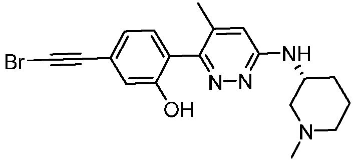 |
| 260 | 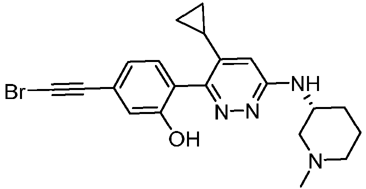 | 261 | 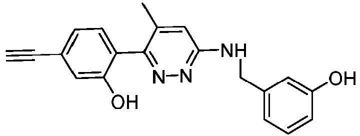 |
| 262 | 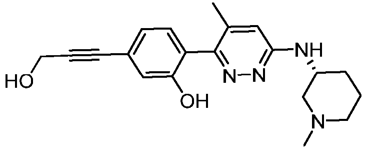 | 263 | 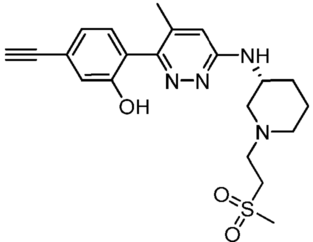 |
| 264 | 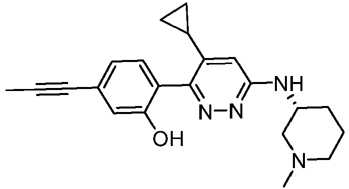 | 265 | 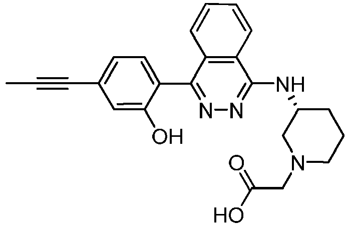 |
| 266 | 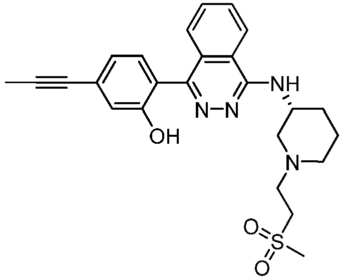 | 267 | 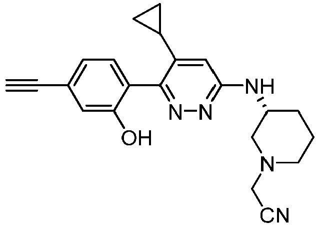 |
| 268 | 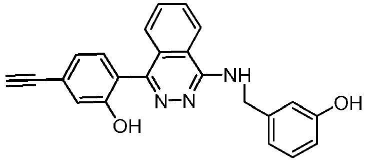 | 269 | 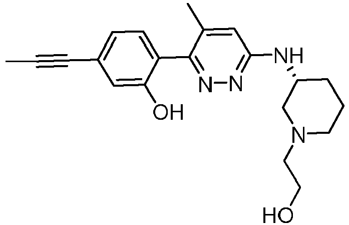 |
| 270 | 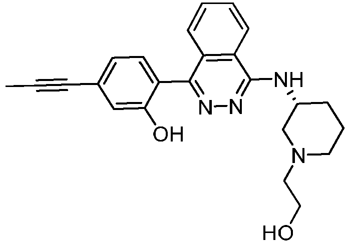 | 271 | 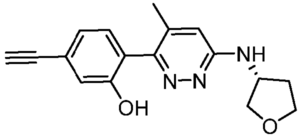 |

In an embodiment of the present invention, a pharmaceutical composition is provided, which comprises the compound or the pharmaceutically acceptable salt, stereoisomer or tautomer thereof as described above, and a pharmaceutically acceptable carrier.

In an embodiment of the present invention, the pharmaceutical composition may comprise one or more pharmaceutical carriers, and may be administered to patients or subjects in need of such treatment by means of oral, parenteral, rectal or transpulmonary administration. In case of oral administration, the pharmaceutical composition may be formulated into conventional solid preparations, such as tablets, capsules, pills, granules, or the like; or may also be formulated into oral liquid preparations, such as oral solutions, oral suspensions, syrup, or the like. When the pharmaceutical composition is formulated into oral preparations, suitable fillers, binders, disintegrants, lubricants or the like may be added. In case of parenteral administration, the pharmaceutical composition may be formulated into injections, including an injection solution, sterile powder for injection, and a concentrated solution for injection. When the pharmaceutical composition is formulated into injections, the production may be implemented by conventional methods in the existing pharmaceutical field, and during the formulation of the injections, additives may not be added, or may be added as appropriate depending on the nature of the medicament. In case of rectal administration, the pharmaceutical composition may be formulated into suppositories or the like. In case of transpulmonary administration, the pharmaceutical composition may be formulated into inhalers or spray or the like.

The present invention further provides use of the compound of general formula (A), (B), (C), (I) or (II) or the pharmaceutically acceptable salt, stereoisomer or tautomer thereof as described above, or the pharmaceutical composition as described above, in preparation of a medicament for prevention and/or treatment of NLRP3 inflammasome-related diseases.

The present invention further provides use of the compound or the pharmaceutically acceptable salt, stereoisomer or tautomer thereof as described above, or the pharmaceutical composition as described above, in preparation of a medicament for prevention and/or treatment of NLRP3 inflammasome-related diseases, immune diseases, inflammatory diseases, autoimmune diseases, or auto inflammatory diseases.

### DETAILED DESCRIPTION OF THE INVENTION

"Halogen" described in the present invention refers to fluorine, chlorine, bromine, and iodine.

"Hydroxy" described in the present invention refers to an -OH group.

"Cyano " described in the present invention refers to a -CN group.

"Amino" described in the present invention refers to an -NH₂ group.

"Carboxyl" described in the present invention refers to a -COOH group.

"Nitro" described in the present invention refers to an -NO₂ group.

"C₁₋₆ alkyl" described in the present invention refers to a linear or branched alkyl group derived from the partial removal of one hydrogen atom from hydrocarbon containing 1-6 carbon atoms, such as methyl, ethyl, n-propyl, isopropyl, n-butyl, isobutyl, sec-butyl, tert-butyl, n-amyl, isoamyl, 2-methylbutyl, neopentyl, 1-ethylpropyl, n-hexyl, isohexyl, 4-methylamyl, 3-methylamyl, 2-methylpentyl, 1-methylamyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methyl-2-methylpropyl, and the like.

"Ene" in "C₁₋₆ alkylene" described in the present invention refers to a divalent group derived from the removal of two hydrogen atoms from a C₁₋₆ alkyl group.

"Halo C₁₋₆ alkyl" described in the present invention refers to a C₁-C₆ alkyl group that is substituted by one or more halogen groups defined above. Examples of halo C₁₋₆ alkyl include, but are not limited to, trifluoromethyl, difluoromethyl, fluoromethyl, trichloromethyl, 2,2,2-trifluoroethyl, 1,3-dibromopropyl-2-yl, 3-bromo-2-fluoropropyl, and 1,4,4-trifluorobutyl-2-yl.

"C₁₋₆ alkoxy" described in the present invention refers to a group derived from attachment of the "C₁₋₆ alkyl" as previously defined to a parent molecule via an oxygen atom, namely the "C₁₋₆ alkyl-O-" group, such as methoxy, ethoxy, n-propoxy, isopropoxy, n-butoxy, tert-butoxy, n-pentoxy, neopentoxy, n-hexoxy or the like.

"Halo C₁₋₆ alkoxy" described in the present invention refers to the substitution of a C₁-C₆ alkoxy group by one or more halogen groups as defined above, and its examples include, but are not limited to, fluoromethoxy, chloromethoxy, trifluoromethoxy, trifluoroethoxy, fluoroethoxy, and fluoropropoxy.

"C₂₋₆ alkenyl" described in the present invention refers to a linear or branched olefin group derived from partial removal of one hydrogen atom from C₂₋₆ olefin containing at least one carbon-carbon double bond, such as vinyl, 1-propenyl, 2-propenyl, 1-butenyl, 2-butenylyl, 1,3-butadi-1-enyl, 1-penten-3-yl, 2-penten-1-yl, 3-penten-1-yl, 3-penten-2-yl, 1,3-pentadien-1-yl, 1,4-pentadien-3-yl, 1-hexen-3-yl, 1,4-hexadien-1-yl. Preferably, "C₂₋₆ alkenyl" contains a carbon-carbon double bond.

"C₂₋₆ alkynyl" described in the present invention refers to a linear or branched alkynyl group derived from partial removal of one hydrogen atom from C₂₋₆ alkyne containing at least one carbon-carbon triple bond, such as ethynyl, propynyl, butynyl, pentynyl, hexynyl and the like. Preferably, "C₂₋₆ alkynyl" contains a carbon-carbon triple bond. "C₁₋₆ alkylamino", "C₁₋₆ alkylcarbonylamino ", "C₁₋₆ alkylsulfonyl", "aminocarbonyl" described in the present invention refer to groups formed in the forms of C₁₋₆ alkyl-NH-, C₁₋₆ alkyl-C(O)-NH-, C₁₋₆ alkyl-S(O)₂-, NH₂-C(O)-, respectively.

It is understandable that in the present invention, when R₁ and R₂ in 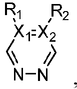 together with the carbon or nitrogen atom to which they are attached, form a ring, X₁ and X₂ form a double bond or single bond according to the law of chemical bonding.

"5-12 membered ring" described in the present invention includes a carbon or heterocyclic ring that may be formed chemically, such as 5-12 membered cycloalkyl, 5-7 membered cycloalkyl, 5-12 membered cycloalkenyl, 5-7 membered cycloalkenyl, 6-12 membered fused cycloalkyl, 5-12 membered heterocyclyl, 5-7 membered heterocyclyl, 6-12 membered fused heterocyclic ring, aryl, 5-12 membered heteroaryl, 8-12 membered heteroaryl, 5-7 membered heteroaryl, and the like.

"3-12 membered cycloalkyl" described in the present invention refers to a monovalent or divalent (as required) group (for example, 5-12 membered cycloalkyl) derived from 3-12 membered cycloalkane, and it may be a monocyclic, bicyclic or polycyclic cycloalkyl system. Unless otherwise specified, it includes all single and fused rings that may be formed (for example, 6-12 membered fused cycloalkyl), including those fused in merged, spiro, and bridged forms. The single ring system is generally a cyclic hydrocarbon group containing 3-12 carbon atoms, such as 3-8 or 3-6 carbon atoms. Examples of cycloalkyl include, but are not limited to: cyclopropanyl, cyclobutanyl, cyclopentanyl, cyclohexanyl, cycloheptanyl, cyclooctanyl, cyclopentanyl-1,3-diyl, cyclohexan-1,4-diyl, cycloheptan-1,4-diyl and the like. Fused cyclic cycloalkyl groups include merged cycloalkyl, bridged cycloalkyl, and spirocycloalkyl. Merged cycloalkyl groups may be 6-11 membered merged cycloalkyl groups such as 7-10 membered merged cycloalkyl, with the representative examples including but not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane. Spiro cycloalkyl groups may be 7-12 membered spiro cycloalkyl groups such as 7-11 membered spiro-alkyl, with the examples including bu not limited to: 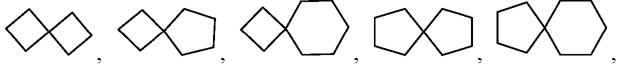 and 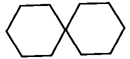 Bridged cycloalkyl groups may be 6-10 membered bridged cycloalkyl groups such as 7-10 membered bridged cycloalkyl, with the examples including but not limited to:

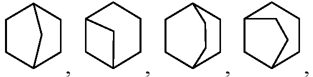

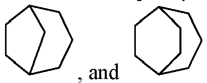

"3-7 membered cycloalkyl" described in the present invention refers to a monovalent or divalent (as desired) group derived from 3-7 membered cycloalkane. "3-7 membered cycloalkyl" may be 3, 4, 5, 6, or 7 membered cycloalkyl, and examples of 3-7 membered cycloalkyl include cyclopropanyl, cyclobutanyl, cyclopentenyl, and cyclohexanyl. "Cycloalkenyl" described in the present invention refers to a group obtained by endowing the above cycloalkyl group with at least one double bond. For example, it may be a "3-12 membered cycloalkenyl". That is, it may have 3, 4, 5, 6, 7, 8, 9, 10, 11 or 12 ring-forming carbon atoms. Unless otherwise specified, cycloalkenyl groups of certain members include all possible forms of single rings and fused rings (including those fused in the merged, spiro, and bridged forms) Cycloalkenyl may be 3-12 membered cycloalkenyl, 3-8 membered cycloalkenyl, 5-8 membered cycloalkenyl, 5-7 membered cycloalkenyl, 4-6 membered cycloalkenyl, 7-11 membered spiro cycloalkenyl, 7-11 membered merged cycloalkenyl, 6-11 membered bridged cyclobridges, or the like. Examples of cycloalkenyl may be listed as cyclobutenyl, cyclopentenyl, cyclopentadienyl, cyclohexenyl, 1,4-cyclohexadien-1-yl, cycloheptenyl, 1,4-cycloheptadien-1-yl, cyclooctenyl, 1,5-cyclooctadiene-1-yl, and the like, but they are not limited thereto.

"5-7 membered cycloalkenyl" described in the present invention refers to a group obtained by endowing a 5-7 membered cycloalkyl group with at least one double bond, such as cyclobutenyl, cyclopentenyl, cycloheptenyl, cycloheptenyl and the like.

"3-14 membered heterocyclyl" described in the present invention refers to a monovalent or divalent (as desired) group derived from 3-14 membered heterocycloalkane, i.e., a 3-14 membered nonaromatic cyclic group with at least one cyclocarbon atom substituted by a heteroatom selected from O, S, S(O), S(O)₂, C(O), and N; preferably, 1-3 heteroatoms are contained; and meanwhile, the substitution of ring-forming atoms including carbon atoms, nitrogen atoms and sulfur atoms by oxygen occur. "3-14 membered heterocyclyl" (for example, 5-14 membered heterocyclyl, 5-12 membered heterocyclyl) includes monocyclic heterocyclyl, bicyclic heterocyclyl, or polycyclic heterocyclyl, with one or more rings that may be saturated or partially saturated, but aromatic rings are not included. Unless otherwise specified, heterocyclyl of certain members (for example, 3-8 membered heterocyclyl, 3-7 membered heterocyclyl, 5-8 membered heterocyclyl, 5-7 membered heterocyclyl, 5-6 membered heterocyclyl, 4-6 membered heterocyclyl, and 6 membered heterocyclyl) includes all monocyclic, fused-cyclic (including those fused in merged, spiro, and bridged forms), saturated, and partially saturated forms that may be formed.

Monocyclic heterocyclyl may be 3-8 membered heterocyclyl (such as 5-7 membered heterocyclyl, 3-7 membered heterocyclyl, 4-7 membered heterocyclyl or 5-6 membered heterocyclyl), 3-8 membered nitrogen-containing heterocyclyl (such as 4-7 membered nitrogen-containing heterocyclyl or 5-6 membered nitrogen-containing heterocyclyl), 3-8 membered saturated heterocyclyl (such as 5-6 membered saturated heterocyclyl), and the like. Its examples include, but are not limited to, azacyclopropanyl, oxacyclopropanyl, thiacyclopropanyl, azacyclobutanyl, oxacyclobutanyl, thiacyclobutanyl, tetrahydrofuranyl, tetrahydropyrryl, tetrahydrothienyl, imidazolidinyl, pyrazolidinyl, 1,2-oxazolidinyl, 1,3-oxazolidinyl, 1,2-thiazolidinyl, 1,3-thiazolidinyl, tetrahydro-2H-pyranyl, tetrahydro-2H-thiapyranyl, piperidyl, piperazinyl, morpholinyl, 1,4-dioxacyclohetanyl, 1,4-oxathiacyclohetanyl, 4,5-dihydroisoxazolyl, 4,5-dihydrooxazolyl, 2,5-dihydrooxazolyl, 2,3-dihydrooxazolyl, 3,4-dihydro-2H-pyrryl, 2,3-dihydro-1H-pyrrolyl, 2,5-dihydro-1H-imidazolyl, 4,5-dihydro-1H-imidazolyl, 4,5-dihydro-1H-pyrazolyl, 4,5-dihydro-3H-pyrazolyl, 4,5-dihydrothiazolyl, 2,5-dihydrothiazolyl, 2H-pyranyl, 4H-pyranyl, 2H-thiapyranyl, 4H-thiapyranyl, 2,3,4,5-tetrahydropyridinyl, 1,2-isoxazinyl, 1,4-isoxazinyl, or 6H-1,3-oxazinyl, or the like.

The fused heterocyclyl (such as 6-12 membered fused heterocyclyl) includes merged heterocyclyl, spiro heterocyclyl, and bridged heterocyclyl, which may be saturated, partially saturated, or unsaturated, but not aromatic. The fused heterocyclyl may be 5-6 membered monocyclic heterocyclyl that is fused to a benzene ring, 5-6 membered monocyclic cycloalkyl, 5-6 membered monocyclic heterocycloalkenyl, or 5-6 membered monocyclic heteroaryl.

The merged heterocyclyl may be 6-12 membered merged heterocyclyl, such as 6-11 membered merged heterocyclyl or 7-10 membered meged heterocyclyl, 6-11 membered saturated merged heterocyclyl, and 6-11 membered nitrogen-containing merged heterocyclyl, and its representative examples include but are not limited to: 3-azabicyclo[3.1.0]hexanyl, 3,6-diazabicyclo[3.2.0]heptanyl, 3,8-diazabicyclo[4.2.0]octanyl, 3,7-diazabicyclo[4.2.0]octanyl, octahydropyrrolo[3,4-c]pyrrolyl, octahydropyrrolo[3,4-b]pyrrolyl, octahydropyrrolo[3,4-b] [1,4]oxazinyl, octahydro-1H-pyrrolo[3,4-c]pyridinyl, 2,3-dihydrobenzofuran-2-yl, 2,3-dihydrobenzofuran-3-yl, dihydroindol-1-yl, dihydroindol-2-yl, dihydroindol-3-yl, 2,3-dihydrobenzothiophen-2yl, octahydro-1H-indolyl, octahydrobenzofuranyl.

The spiro heterocyclyl may be 6-12 membered spiro heterocyclyl, such as 7-12 membered spiro heterocyclyl, 7-12 membered saturated spiro heterocyclyl, and 7-12 membered nitrogen-containing spiro heterocyclyl, and its examples include but are not limited to:

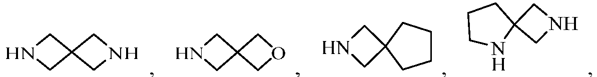

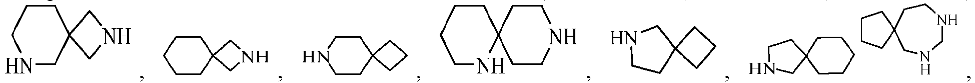

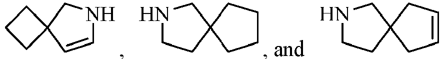

The bridged heterocyclyl may be 6-12 membered bridged heterocyclyl, such as 6-10 membered bridged heterocyclyl (for example, 6-10 membered nitrogen-containing bridged heterocyclyl, in particular 7 membered nitrogen-containing bridged heterocyclyl), and 7-10 membered bridged heterocyclyl, and its examples include but are not limited to:

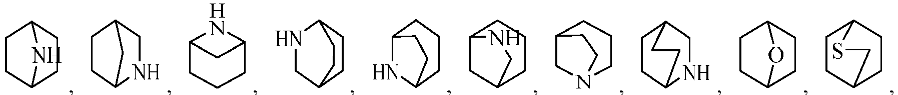

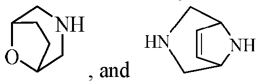

"Aryl" described in the present invention refers to a monovalent or divalent (as desired) cyclic aromatic group derived from an aromatic carbon cyclic hydrocarbon containing 6-14 carbon atoms, and it includes benzene, naphthyl, phenanthryl, and the like.

"5-14 membered heteroaryl" described in the present invention refers to an aromatic 5-14 membered cyclic group with at least one cyclocarbon atom substituted by a heteroatom selected from O, S, and N; "5-14 membered heteroaryl" may be 5, 6, 7, 8, 9, 10, 11, 12, 13, or 14 membered heteroaryl; preferably, 1-3 heteroatoms are contained; and meanwhile, substitutions of carbon atoms and sulfur atoms by oxygen and nitrogen occur, for example, the substitution of a carbon atom by C(O), or a sulfur atom by S(O) or S(O)₂. Heteroaryl groups include monoheteroaryl and fused heteroaryl, and unless otherwise specified, the heteroaryl of certain members includes all monocyclic, fused-cyclic, fully aromatic, or partially aromatic forms that may be formed. Monoheteroaryl may be 5-7 membered heteroaryl, such as 5-6 membered heteroaryl, and its examples include, but are not limited to, furanyl, imidazolyl, isoxazolyl, thiazolyl, isothiazolyl, oxadiazolyl, oxazolyl, pyridinyl, pyridazinyl, pyrimidinyl, pyrazinyl, pyrazolyl, pyrrolyl, tetrazolyl, thiadiazolyl, thienyl, triazol, and triazinyl.

In some embodiments, the fused heteroaryl refer to a group formed by the fusing of a monocyclic heteroaryl ring to phenyl, cycloalkenyl, hetearyl, cycloalkyl, or heterocyclyl. In some embodiments, the fused heteroaryl (such as 8-14 membered fused heteroaryl) may be 8-14 membered merged heteroaryl, such as 9-10 membered merged heteroaryl, and its examples include, but are not limited to, benzoimidazolyl, benzofuranyl, benzothienyl, benzooxadiazolyl, benzothiadiazolyl, benzothiazolyl, cinnolinyl, 5,6-dihydroquinolin-2-yl, 5,6-dihydroisoquinolin-1-yl, furanopyridyl, indazolyl, indolyl, isoindolyl, isoquinolinyl, naphthyridinyl, purinyl, quinolinyl, 5,6,7,8-tetrahydroquinolin-2-yl, 5,6,7,8-tetrahydroquinolinyl, 5,6,7,8-tetrahydroquinolin-4-yl, 5,6,7,8-tetrahydroisoquinolin-1-yl, thienopyridyl, 4,5,6,7-tetrahydro[c][1,2,5]oxadiazolyl, and 6,7-dihydro[c][1,2,5]oxadiazol-4(5H)ketone.

The heterocyclyl and heteroaryl groups of the present invention have ring-forming atoms (including carbon atoms, nitrogen atoms and sulfur atoms) substituted by oxygen.

"Pharmaceutically acceptable salt" described in the present invention refers to the pharmaceutical addition salts and solvates of acids and bases. Such pharmaceutical salts include the salts of the following acids: hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acid (such as acetic acid, HOOC-(CH₂)n-COOH (with n=0~4)), and the like. Such pharmaceutical salts also include the salts of the following alkalis: sodium, potassium, calcium, ammonium, and the like. Those skilled in the art know a variety of non-toxic pharmaceutical addition salts.

All numerical ranges described in the present invention each indicate the inclusion of two endpoints of the range, all integers within the range, and subranges formed by these integers. For example, "3-7 membered" includes 3, 4, 5, 6, and 7 membered; "1-4" includes 1, 2, 3, and 4; and "1-3" includes 1, 2, and 3.

The term "optional" or "optionally" refers to that the situation described subsequently may or may not occur, including both the occurrence and the non-occurrence of said situation.

"Stereoisomers" of the compound of the present invention refer to isomers resulting from different spatial arrangements of atoms in molecules. Enantiomers are produced when asymmetric carbon atoms are present in compounds; and cis-trans isomers are produced when a compound has a carbon-carbon double bond or a cyclic structure.

The term "tautomer" refers to a special kind of functional group isomerism where the isomers of different functional groups are in dynamic equilibrium and can be transformed quickly to each other. For example, when ketones or oximes are present, tautomers are produced, with representative examples including, for example: ketone-enol tautomers, phenol-ketone tautomers, nitroso-oxime tautomers, imine-enamine tautomers, and the like.

The enantiomers, diastereomers, racemic isomers, cis-trans isomers, tautomers, geometric isomers, epimers and their mixtures of all compounds are included in the scope of the present invention.

In the chemical configurations of the compounds described in the present invention, the bond " 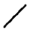 " indicates that the configuration is not specified. That is, if a chiral isomer is present in the chemical structure, the bond " 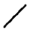 " may be " 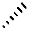 " or " 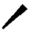 " or contains both configurations of " 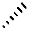 ", " and " 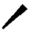 ". In the chemical structures of the compounds of the present invention, " 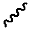 " indicates the linkage point with a parent molecule.

### Beneficial Effects of the Invention

Studies have found that the compounds provided by the present invention or the pharmaceutically acceptable salts, stereoisomers, or tautomers have good inhibitory activity against NLRP3 inflammasomes. Therefore, the compounds of the present invention can be used to prevent and/or treat NLRP3 inflammasome-related diseases.

### DETAILED DESCRIPTION OF THE PREFERRED EMBODIMENTS

For a clearer understanding of the objects, technical solutions and advantages of the present invention, the present invention will be further illustrated in detail. Obviously, the described embodiments are only part of rather than all of the embodiments of the present invention. Based on the embodiments of the present invention, every other embodiment achieved by those ordinarily skilled in the art without making creative efforts shall fall within the protection scope of the present invention.

The abbreviations and English expressions used in the present invention have the following meanings:
"THF" refers to tetrahydrofuran; "DMF" refers to N,N-dimethylformamide; "MeOH" refers to methanol; "EA" refers to ethyl acetate; "DCM" refers to dichloromethane; "DMAC" and "DMA" refer to N,N-dimethylacetamide; "MTBE" refers to methyl tert-butyl ether; "EtOH" refers to ethanol; "PE" refers to petroleum ether; "n-BuLi" refers to n-butyl lithium; "NMP" refers to N-methylpyrrolidone; "DBDMH" and "DBH" refer to dibromohydantoin; "mCPBA" refers to m-chloroperoxybenzoic acid; "BINAP" refers to 1,1'-binaphthalen-2,2'-bisdiphenylphosphine;"TMSOTf' refers to trimethylsilyl trifluoromethanesulfonate; "TBAF" refers to tetrabutylamine fluoride; "DIPEA" refers to N,N-diisopropylethylamine; "LDA" refers to lithium diisopropyl amide; and "B₂Pin₂" refers to bis(pinacolato)diboron.

"FBS" refers to fetal bovine serum; "PBS" refers to phosphate buffered salt solution; "PMA" refers to phorbol myristate acetate; "LPS" refers to lipopolysaccharide; and "Nigericin" refers to nigericin.

### Example 1: Synthesis of (R)-5-ethynyl-2-(4-((1-methylpiperidin-3-yl)amino)-5,6,7,8-tetrahydrophthalazin-1-yl)phenol (Compound 1)

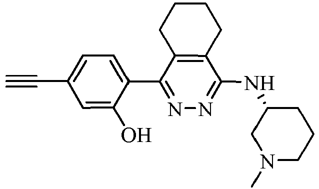

### Step 1: Synthesis of (R)-3-(ethoxymethoxy)-4-(4-((1-methylpiperidin-3-yl)amino)-5,6,7,8-tetrahydrophthalazin-1-yl)benzaldehyde

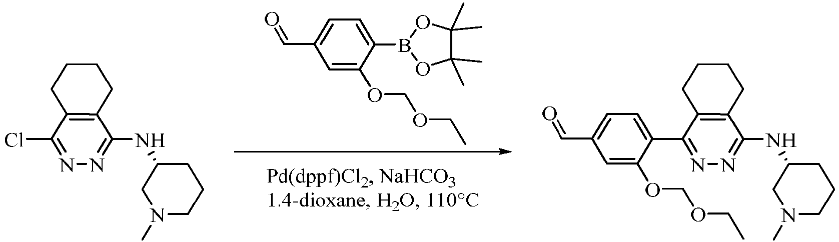

3-(ethoxymethoxy)-4-(4,4,5,5-tetramethyl-1,3,2-dioxaboracyclopentan-2-yl)benzaldehyde (393 mg, 1.28 mmol, 1.2 eq.), (R)-4-chloro-N-(1-methylpiperidin-3-yl)-5,6,7,8-tetrahydrophthalazin-1-amine (300 mg, 1.07 mmol, 1.0 eq.), Pd(dppf)Cl₂ (78.3 mg, 0.107 mmol, 0.1 eq.) and NaHCOs (180 mg, 2.14 mmol, 2.0 eq.) were successively added to 1,4-dioxane (6 mL) and water (3 mL), and heated to 110°C under the protection of nitrogen to react for 2 h. The reaction mixture was cooled to room temperature, quenched by adding water (30 mL), and extracted with EA (30 mL × 2); the organic phases were dried with anhydrous sodium sulfate and filtered; the filtrate was concentrated under reduced pressure; and the resulting crude product was separated by silica gel column chromatography (DCM:MeOH = 50:1-10:1) to obtain a product (320 mg, yield: 70.6%).

### Step 2: Synthesis of (R)-4-(2-(ethoxymethoxy)-4-ethynylphenyl)-N-(1-methylpiperidin-3-yl)-5,6,7,8-tetrahydrophthalazin-1 -amine

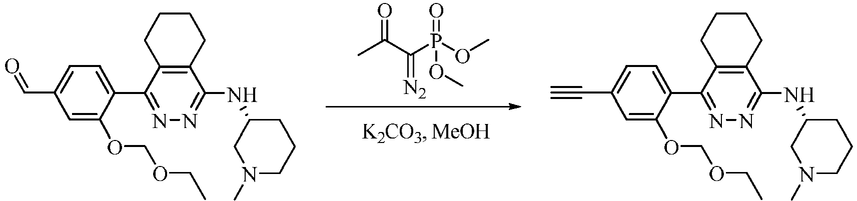

(R)-3-(ethoxymethoxy)-4-(4-((1-methylpiperidin-3-yl)amino)-5,6,7,8-tetrahydrophthalazin-1-yl)benzaldehyde (320 mg, 0.754 mmol, 1.0 eq.) was dissolved in MeOH(5 mL); K₂CO₃ (209 mg, 1.51 mmol, 2.0 eq.) and (1-diazo-2-oxopropyl)dimethyl phosphonate (217 mg, 1.13 mmol, 1.5 eq.) were added; and the resulting mixture was stirred at room temperature for 5 h. The resulting mixture was concentrated, dissolved with water, and extracted with DCM (20 mL × 3); the organic phase was dried with anhydrous sodium sulfate and filtered; the filtrate was concentrated under reduced pressure; and the resulting crude product was separated by silica gel column chromatography (DCM:MeOH = 50:1-20:1) to obtain a product (275 mg, yield: 86.8%).

### Step 3: Synthesis of (R)-5-ethynyl-2-(4-((1-methylpiperidin-3-yl)amino)-5,6,7,8-tetrahydrophthalazin-1-yl)phenol

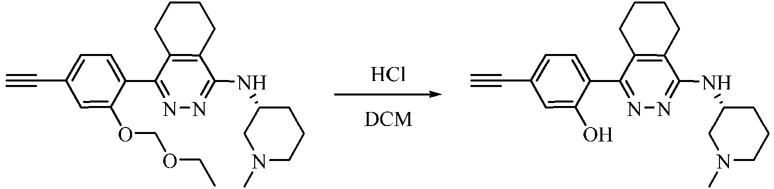

(R)-4-(2-(ethoxymethoxy)-4-ethynylphenyl)-N-(1-methylpiperidin-3-yl)-5,6,7,8-tetrahydrophthalazin-1-atnine (275 mg, 0.654 mmol, 1.0 eq.) was dissolved in DCM (5 mL); then, the 1,4-dioxane solution of hydrogen chloride (4 mol/L, 0.82 mL, 3.27 mmol, 5.0 eq.) was dropwise added; and the resutling mixture was stirred at room temperature for 2 h. The resulting mixture was adjusted to a pH value of 9 by using saturated Na₂CO₃ in water, and then extracted with DCM (20 mL × 3); the organic phases were dried with anhydrous sodium sulfate and filtered; the filtrate was concentrated under reduced pressure; and the resultant crude product was separated by silica gel column chromatography (DCM:MeOH = 50:1-10:1) to obtain a product (198 mg, yield: 83.5%).

¹HNMR (400 MHz, DMSO-*d₆*) δ(ppm): 10.11 (s, 1H), 7.11 (d, *J* = 7.6 Hz, 1H), 7.04 (s, 1H), 6.99-6.97 (m, 1H), 6.07 (s, 1H), 4.55 (s, 1H), 4.20 (s, 1H), 3.20 (s, 1H), 2.70 (s, 5H), 2.44 (s, 2H), 2.34-2.32 (m, 2H), 1.94 (s, 2H), 1.76 (s, 4H), 1.61 (d, *J=* 5.6 Hz, 3H).

Molecular formula: C₂₂H₂₆N₄O Accurate molecular weight: 362.21 LC-MS (Pos, m/z)=363.14[M+H]⁺.

### Example 2: Synthesis of (R)-5-ethynyl-2-(4-((tetrahydro-2H-pyran-3-yl)amino)phthalazin-1-yl)phenol (Compound 2)

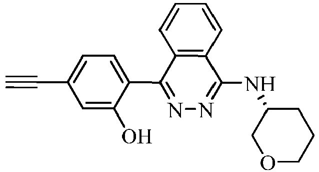

### Step 1: Synthesis of 4-(4-chlorophthalazin-1-yl)-3-(ethoxymethoxy)benzaldehyde

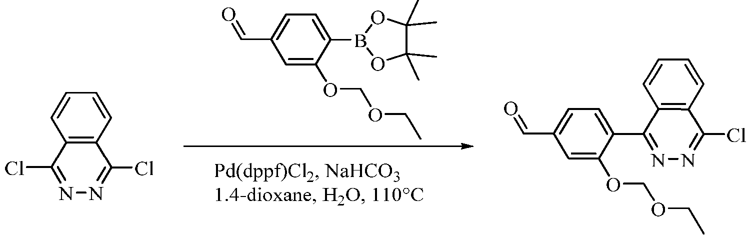

3-(ethoxymethoxy)-4-(4,4,5,5-tetramethyl-1,3,2-dioxaboracyclopentan-2-yl)benzaldehyde (462 mg, 1.51 mmol, 1.0 eq.), 1,4-dicholorophthalazine (300 mg, 1.51 mmol, 1.0 eq.), Pd(dppf)Cl₂ (110 mg, 0.151 mmol, 0.1 eq.) and NaHCOs (254 mg, 3.02 mmol, 2.0 eq.) were successively added to 1,4-dioxane (5 mL) and water (2.5 mL), and heated to 110°C under the protection of nitrogen to react for 2 h. The reaction mixture was cooled to room temperature, quenched by adding water (30 mL), and extracted with EA (30 mL × 2); the organic phases were dried with anhydrous sodium sulfate and filtered; the filtrate was concentrated under reduced pressure; and the resulting crude product was separated by silica gel column chromatography (DCM:MeOH = 100:1) to obtain a product (290 mg, yield: 56.1%).

### Step 2: Synthesis of (R)-3-(ethoxymethoxy)-4-(4-((tetrahydro-2H-pyran-3-yl)amino)phthalazin-1-yl)benzaldehyde

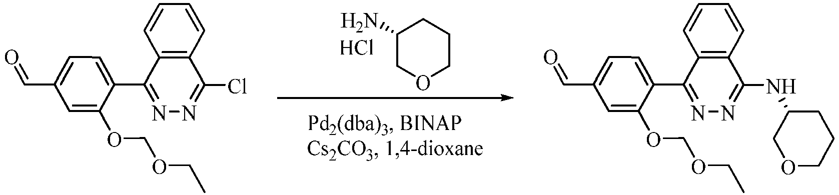

4-(4-chlorophthalazin-1-yl)-3-(ethoxymethoxy)benzaldehyde (240 mg, 0.700 mmol, 1.0 eq.), (R)-3-aminotetrahydropyrane hydrochloride (144 mg, 1.05 mmol, 1.5 eq.), Pd₂(dba)₃ (64.1 mg, 0.0700 mmol, 0.1 eq.), BINAP(87.1 mg, 0.140 mmol, 0.2 eq.) and Cs₂CO₃ (456 mg, 1.40 mmol, 2.0 eq.) were successively added to 1,4-dioxane (10 mL), and heated to 90°C under the protection of nitrogen to react for 2 h. The reaction mixture was cooled to room temperature, quenched by adding water (30 mL), and extracted with EA (20 mL × 2); the organic phases were dried with anhydrous sodium sulfate and filtered; the filtrate was concentrated under reduced pressure; and the resulting crude product was separated by silica gel column chromatography (DCM:MeOH = 100:1-20:1) to obtain a product (212 mg, yield: 74.3%).

### Step 3: Synthesis of (R)-4-(2-(ethoxymethoxy)-4-ethynylphenyl)-N-(tetrahydro-2H-pyran-3-yl)phthalazin-1-amine

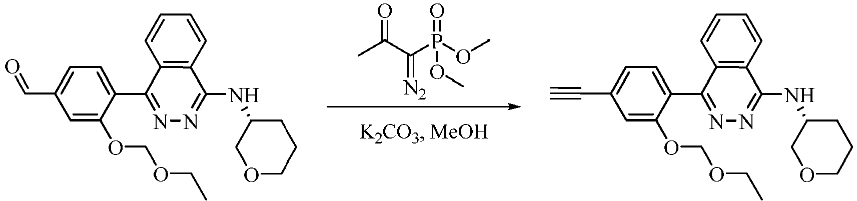

(*R*)-3-(ethoxymethoxy)-4-(4-((tetrahydro-2*H*-pyran-3-yl)amino)phthalazin-1-yl)benzaldehyde (210 mg, 0.515 mmol, 1.0 eq.) was dissolved in MeOH (4 mL); K₂CO₃ (142 mg, 1.03 mmol, 2.0 eq.) and (1-diazo-2-oxopropyl)dimethyl phosphonate (119 mg, 0.618 mmol, 1.2 eq.) were added; and the resulting mixture was stirred at room temperature for 16 h. The resulting mixture was concentrated, dissolved with water, and extracted with DCM (20 mL × 2); the organic phases were dried with anhydrous sodium sulfate and filtered; the filtrate was concentrated under reduced pressure; and the resulting crude product was separated by silica gel column chromatography (DCM:MeOH = 100:1-20:1) to obtain a product (122 mg, yield: 58.7%).

### Step 4: Synthesis of (R)-5-ethynyl-2-(4-((tetrahydro-2H-pyran-3-yl)atnino)phthalazin-1-yl)phenol

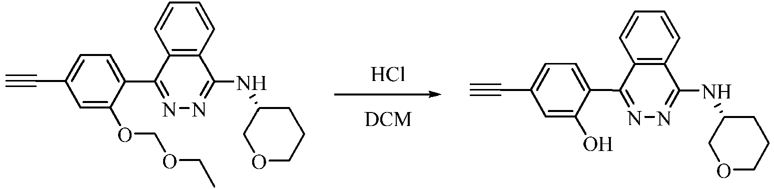

(*R*)-4-(2-(ethoxymethoxy)-4-ethynylphenyl)-*N*-(tetrahydro-2*H*-pyran-3-yl)phthalazin-1-amine (120 mg, 0.297 mmol, 1.0 eq.) was dissolved in DCM (4 mL); then, the 1,4-dioxane solution of hydrogen chloride (4 mol/L, 0.22 mL, 0.891 mmol, 3.0 eq.) was dropwise added; and the resulting mixture was stirred at room temperature for 1 h. The resulting mixture was adjusted to a pH value of 8 by using saturated NaHCOs in water, and then extracted with DCM (20 mL × 3); the organic phases were dried with anhydrous sodium sulfate and filtered; the filtrate was concentrated under reduced pressure; and the resulting crude product was separated by preparative thin-layer chromatography (DCM:MeOH = 20:1) to obtain a product (62.0 mg, yield: 60.4%).

¹HNMR (400 MHz, DMSO-*d₆*) δ(ppm): 10.02 (s, 1H), 8.41 (d, *J* = 8.4 Hz, 1H), 7.88-7.84 (m, 1H), 7.81-7.77 (m, 1H), 7.47 (d, *J* = 8.0 Hz, 1H), 7.30 (d, *J* = 7.6 Hz, 1H), 7.17 (d, *J* = 7.6 Hz, 1H), 7.09-7.06 (m, 2H), 4.41-4.39 (m, 1H), 4.24 (s, 1H), 4.10-4.07 (m, 1H), 3.84 (*d, J =* 11.2 Hz, 1H), 3.36 (s, 1H), 3.28-3.23 (m, 1H), 2.12-2.11 (m, 1H), 1.80-1.68 (m, 3H).

Molecular formula: C₂₁H₁₉N₃O₂ Accurate molecular weight: 345.15 LC-MS (Pos, m/z)=346.03[M+H]⁺.

### Example 3: Synthesis of 5-ethynyl-2-(4-(((cis)-3-hydroxyl-3-methylcyclobutyl)amino)phthalazin-1-yl)phenol (Compound 3)

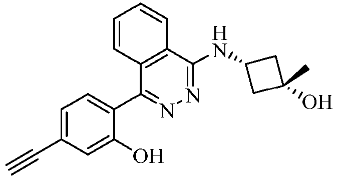

### Step 1: Synthesis of 3-(ethoxymethoxy)-4-(4-(((cis)-3-hydroxyl-3-methylcyclobutyl)amino)phthalazin-1-yl)benzaldehyde

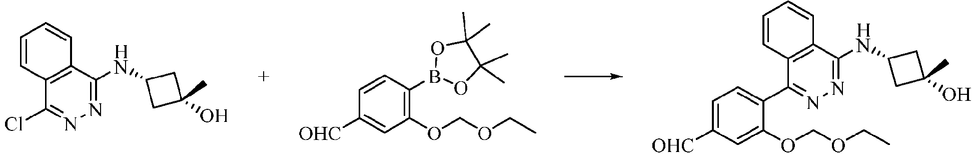

(*cis*)-3-((4-chlorophthalazin-1-yl)amino)-1-methylcyclobutan-1-ol (290.0 mg, 1.09 mmol, 1.0 eq.), 3-(ethoxymethoxy)-4-(4,4,5,5-tetratnethyl-1,3,2-dioxoboracyclopentan-2-yl)benzaldehyde (505.0 mg, 1.64 mmol, 1.5 eq.), sodium hydrogen carbonate(184.6 mg, 2.19 mmol. 2.0 eq.), water (5.0 mL) and Pd(dppf)Cl₂ (40.2 mg, 0.05 mmol, 0.05 eq.) were added to 1,4-dioxane (10.0 mL) and allowed to react for 3 h at 110°C under the protection of nitrogen; TLC monitoring showed that the reaction was complete; the system was cooled to room temperature; water (50.0 mL) was added; the mixture was extracted with ethyl acetate (100.0 mL); the organic phase was dried with anhydrous sodium sulfate and filtered; the filtrate was concentrated under reduced pressure; and the resulting crude product was purified by silica gel column chromatography (silica gel:100-200 meshes, dichloromethane :methanol = 100:1-40:1) to obtain a product (240.0 mg, yield: 53.5%).

### Step 2: Synthesis of (cis)-3-((4-(2-(ethoxymethoxy)-4-ethynylphenyl)phthalazin-1-yl)amino)-1-methylcyclobutan-1-ol

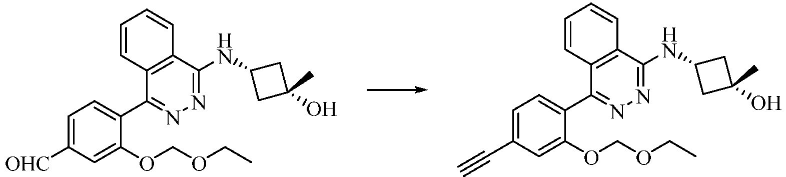

3-(ethoxymethoxy)-4-(4-(((1*s*,3*s*)-3-hydroxyl-3-methylcyclobutyl)amino)phthalazin-1-yl)benzaldehyde (240 mg, 0.58 mmol, 1.0 eq.),sodium carbonate(162.8 mg, 1.17 mmol, 2.0 eq.) and (1-diazo-2-oxopropyl)dimethyl phosphonate (169.7 mg, 0.88 mmol, 1.5 eq.) were added to methanol (10.0 mL), and stirred at room temperature for 2 h; LC-MS monitoring showed that the reaction was complete; water (50.0 mL) was added; the mixture was extracted with dichloromethane (100.0 mL); the organic phase was dried with anhydrous sodium sulfate and filtered; the filtrate was concentrated under reduced pressure; and the resulting crude product was purified by preparative thin-layer chromatography (dichloromethane :methanol = 10:1) to obtain a product (130.0 mg, yield: 54.7%).

### Step 3: Synthesis of 5-ethynyl-2-(4-(((cis)-3-hydroxyl-3-methylcyclobutyl)amino)phthalazin-1-yl)phenol

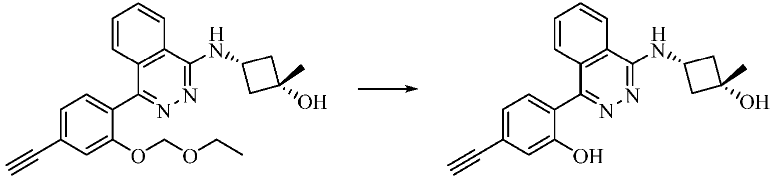

(*cis*)-3-((4-(2-(ethoxymethoxy)-4-ethynylphenyl)phthalazin-1-yl)amino)-1-methylcyclobutan-1-ol (130.0 mg, 0.32 mmol, 1.0 eq.) was added to dichloromethane (2.0 mL); a hydrogen chloride-1,4-dioxane solution (4.0 mol/L, 4.0 mL) was dropwise added to allow for reaction at room temperature for 2 h; TLC monitoring showed that the reaction was complete; the system was adjusted to pH = 7-8 by using saturated sodium hydrogen carbonate, and extracted with dichloromethane (50.0 mL); the organic phase was dried with anhydrous sodium sulfate and filtered; and the filtrate was concentrated under reduced pressure to obtain a product (50.0 mg, yield: 44.9%).

¹HNMR (400 MHz, DMSO-*d₆*) δ(ppm): 10.01 (s, 1H), 8.42-8.40 (d, *J*=8 Hz, 1H), 7.84 (t, *J*=8 Hz, 1H), 7.77 (t, *J*=8 Hz, 1H), 7.58-7.57 (d, *J*=4 Hz, 1H), 7.47-7.45 (d, *J*=8 Hz, 1H), 7.30-7.28 (d, *J*=8 Hz, 1H), 7.07-7.05 (m, 2H), 5.01 (s, 1H), 4.27-4.22 (m, 2H), 2.50-2.47 (m, 2H), 2.20-2.15 (m, 2H), 1.34 (s, 3H).

Molecular formula: C₂₁H₁₉N₃O₂ Accurate molecular weight: 345.15 LC-MS(Pos, *m*/*z*)=346.07[M+H⁺].

### Example 4: Synthesis of 5-ethynyl-2-(4-(((cis)-3-hydroxyl-3-methylcyclobutyl)amino)-5,6,7,8-tetrahydrophthalazin-1-yl)phenol (Compound 4)

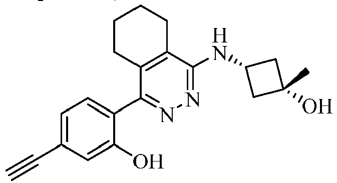

### Step 1: Synthesis of 3-(ethoxymethoxy)-4-(4-(((cis)-3-hydroxyl-3-methylcyclobutyl)amino)-5,6,7,8-tetrahydrophthalazin-1-yl)benzaldehyde

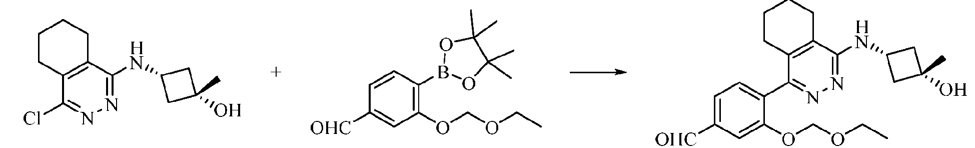

(cis)-3-((4- chloro-5, 6, 7, 8-tetrahydrophthalazin-1-yl)amino)-1-methylcyclobutan-1-ol (390.0 mg, 1.45 mmol, 1.0 eq.), 3- (ethoxymethoxy)-4-(4,4,5,5-tetramethyl-1,3,2-dioxoboracyclopentan-2-yl)benzaldehyde (624.3 mg, 2.03 mmol, 1.4 eq.), sodium hydrogen carbonate (244.5 mg, 2.91 mmol. 2.0 eq.), water (6.0 mL) and Pd(ddf)Cl₂ (53.2 mg, 0/07 mml, 0.05 eq.) were added to 1,4-dioxane (12.0 mL) to react for 4 h at 110°C under the protection of nitrogen; TLC monitoring showed that the reaction was complete; the system was cooled to room temperature; water (50.0 mL) was added; the mixture was extracted with ethyl acetate (100.0 mL); the organic phase was dried with anhydrous sodium sulfate and filtered; the filtrate was concentrated under reduced pressure; and the resulting crude product was purified by silica gel column chromatography (silica gel:100-200 meshes, dichloromethane :methanol = 80:1-30:1) to obtain a product (143.0 mg, yield: 23.8%).

### Step 2: Synthesis of 3-(cis)-3-((4-(2-(ethoxymethoxy)-4-ehtynylphenyl)-5,6,7,8-tetrahydrophthalazin-1-yl)amino-1- methylcyclobutan-1-ol

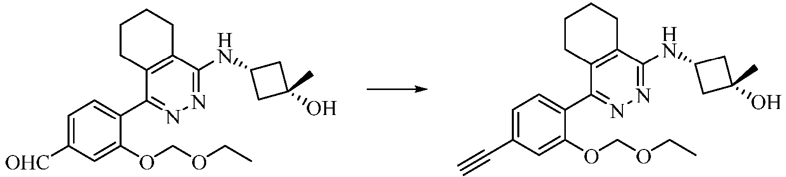

3-(ethoxymethoxy)-4-(4-(((1*s*,3*s*)-3-hydroxyl-3-methylcyclobutyl)amino)-5,6,7,8-tetrahydrophthalazin-1-yl)benzaldehyde (143.0 mg, 0.34 mmol, 1.0 eq.), sodium carbonate (95.9 mg, 0.69 mmol, 2.0 eq.) and (1-diazo-2-oxopropyl)dimethyl phosphonate (100.1 mg, 0.52 mmol, 1.5 eq.) were added to methanol (6.0 mL), stirred at room temperature for 14 h; LC-MS monitoring showed that the reaction was complete; water (50.0 mL) was added; the mixture was extracted with dichloromethane (100.0 mL); the organic phase was dried with anhydrous sodium sulfate and filtered; the filtrate was concentrated under reduced pressure; and the resulting crude product was purified by preparative thin-layer chromatography (dichloromethane:methanol = 10:1) to obtain a product (120.0 mg, yield: 84.8%).

### Step 3: Synthesis of 5-ethynyl-2-(4-(((cis)-3-hydroxyl-3-methylcyclobutyl)amino)-5,6,7,8-tetrahydrophthalazin-1-yl)phenol

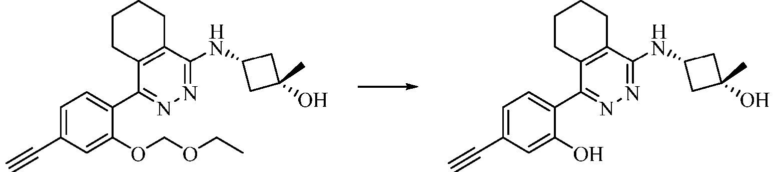

(*cis*)-3-((4-(2-(ethoxymethoxy)-4-ethynylphenyl)-5,6,7,8-tetrahydrophthalazin-1-yl)amino)-1-methylcyclobutan-1-ol (120.0 mg, 0.29 mmol, 1.0 eq.) was added to dichloromethane (2.0 mL); a hydrogen chloride-1,4-dioxane solution (4.0 mol/L, 4.0 mL) was dropwise added to allow for reaction at room temperature for 2 h; TLC monitoring showed that the reaction was complete; the system was adjusted to pH = 7-8 by using saturated sodium hydrogen carbonate, and extracted with dichloromethane (50.0 mL); the organic phase was dried with anhydrous sodium sulfate and filtered; and the filtrate was concentrated under reduced pressure to obtain a product (80.0 mg, yield: 77.8%).

¹HNMR (400 MHz, DMSO-*d₆*) δ(ppm): 9.99 (s, 1H), 7.13-7.11 (d, *J*=8 Hz, 1H), 6.99-6.96 (m, 2H), 6.08-6.06 (d, *J*=8 Hz, 1H), 4.91 (s, 1H), 4.17 (s, 1H), 4.11-4.06 (m, 1H), 2.42-2.37 (m, 4H), 2.33-2.30 (m, 2H), 2.09-2.04 (m, 2H), 1.76-1.73 (m, 2H), 1.60-1.58 (m, 2H), 1.30 (s, 3H).

Molecular formula: C₂₁H₂₃N₃O₂ Accurate molecular weight: 349.18 LC-MS(Pos, *m*/*z*)=350.14[M+H⁺].

### Example 5: Synthesis of (R)-2-(4-((1-methylpiperidin-3-yl)amino)phthalazin-1-yl)-5-ethenylphenol (Compound 99)

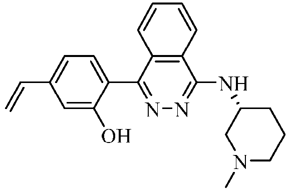

### Step 1: Synthesis of (R)-2-(4-((1-methylpiperidin-3-yl)amino)phthalazin-1-yl)-5-ethenylphenol

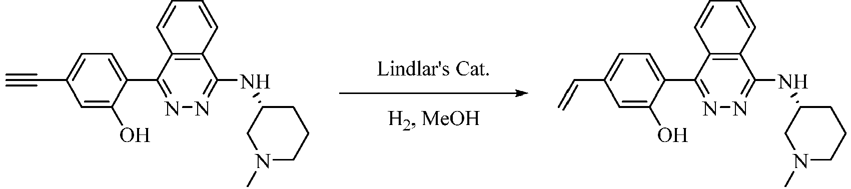

(R)-5-ethynyl-2-(4-((1-methylpiperidin-3-yl)amino)phthalazin-1-yl)phenol (300 mg, 0.837 mmol, 1.0 eq.) was dissolved in MeOH(10 mL); a Lindlar catalyst (30 mg, 10%) was added; and H₂ replacement was conducted to allow for reaction at room temperature for 1 h. The reaction mixture was subjected to suction filtration by means of diatomite; the filtrate was concentrated under reduced pressure; and the resulting crude product was separated by preparative thin-layer chromatography (DCM:MeOH = 10:1) to obtain a product (240 mg, yield: 79.6%).

¹HNMR (400MHz, DMSO-*d₆*) δ(ppm): 9.88 (s, 1H), 8.51 (s, 1H), 7.88-7.77 (m, 2H), 7.54 (d, *J =* 8.4 Hz, 1H), 7.42 (s, 1H), 7.28 (d, *J* = 7.6 Hz, 1H), 7.10-7.07 (m, 2H), 6.79-6.72 (m, 1H), 5.82 (*d, J =* 17.6 Hz, 1H), 5.31 (d*, J =* 11.2 Hz, 1H), 4.63 (s, 1H), 3.39 (d, *J =* 9.2 Hz, 1H), 3.02 (s, 1H), 2.55 (s, 5H), 1.92 (s, 2H),1.77-1.71 (m, 2H). Molecular formula: C₂₂H₂₄N₄O Accurate molecular weight: 360.20 LC-MS (Pos, m/z)=361.09[M+H]⁺.

### Example 6: Synthesis of (R)-5-ethynyl-2-(4-((1-methylpiperidin-3-yl)amino)phthalazin-1-yl)phenol (Compound 100)

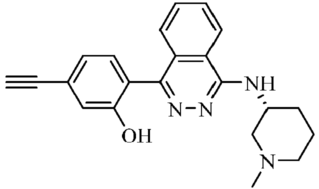

### Step 1: Synthesis of 4-bromo-3-hydroxybenzaldehyde

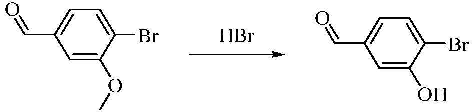

4-bromo-3-methoxybenzaldehyde (10.0 g, 46.5 mmol, 1.0 eq.) was added to HBr in water (60 mL) with a mass fraction of 40%, and heated to 100°C to react for 20 h. The reaction mixture was cooled to room temperature, quenched by adding water, and extracted with EA (50 mL × 2); the organic phases were dried with anhydrous sodium sulfate and filtered; the filtrate was concentrated under reduced pressure; and the resulting crude product was separated by silica gel column chromatography (PE:EA= 50:1-5:1) to obtain a product (7.80 g, yield: 83.4%). Step 2: Synthesis of 4-bromo-3-(ethoxymethoxy)benzaldehyde

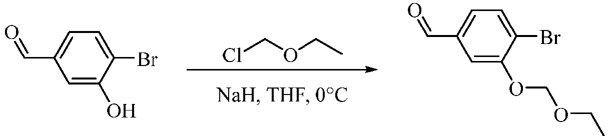

4-bromo-3-hydroxybenzaldehyde (7.80 g, 38.8 mmol, 1.0 eq.) was dissolved in THF (100 mL) and cooled to 0°C; NaH (2.33 g, 58.2 mmol, 1.5 eq.) with a mass fraction of 60% was portionwise added; the resulting mixture was stirred for 20 min; and chloromethyl ethyl ether (5.50 g, 86.2 mmol, 1.5 eq.) was then added to allow for reaction for 2 h. Saturated NH₄Cl in water was added for quenching; the mixture was separated; the aqueous phase was extracted with EA(30 mL × 2); the organic phases were combined, dried with anhydrous sodium sulfate, and filtered; the filtrate was concentrated under reduced pressure; and the resulting crude product was separated by silica gel column chromatography (PE:EA = 50:1-5:1) to obtain a product (7.60 g, yield: 75.6%).

### Step 3: Synthesis of 3-(ethoxymethoxy)-4-(4,4,5,5-tetramethyl-1,3,2-dioxaboracyclopentan-2-yl)benzaldehyde

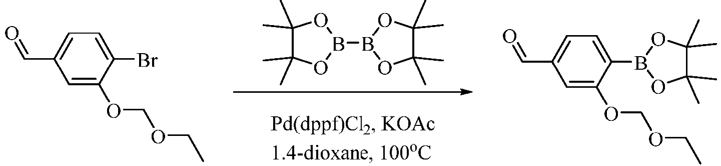

4-bromo-3-(ethoxymethoxy)benzaldehyde (5.00 g, 19.3 mmol, 1.0 eq.), bis(pinacolato)diboron (7.35 g, 29.0 mmol, 1.5 eq.), Pd(dppf)Cl₂ (1.41 g, 1.93 mmol, 0.1 eq.) and KOAc(3.79 g, 38.6 mmol, 2.0 eq.) were successively added to 1,4-dioxane (50 mL), and heated to 100°C under the protection of nitrogen to react for 20 h. The reaction mixture was cooled to room temperature, quenched by adding water (50 mL), and extracted with EA (50 mL × 2); the organic phases were dried with anhydrous sodium sulfate and filtered; the filtrate was concentrated under reduced pressure; and the resulting crude product was separated by silica gel column chromatography (PE:EA = 20:1-5:1) to obtain a product (5.20 g, yield: 88.0%).

### Step 4: Synthesis of (R)-3-(ethoxymethoxy)-4-(4-((1-methylpiperidin-3-yl)amino)phthalazin-1-yl)benzaldehyde

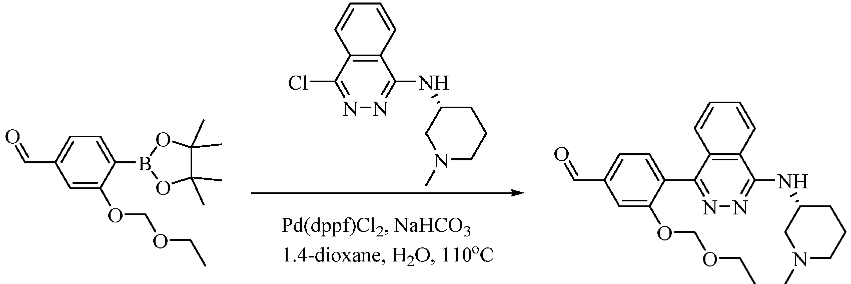

3-(ethoxymethoxy)-4-(4,4,5,5-tetramethyl-1,3,2-dioxaboracyclopentan-2-yl)benzaldehyde (1.66 g, 5.42 mmol, 1.5 eq.), (*R*)-4-chloro-*N*-(1-methylpiperidin-3-yl)phthalazin-1-amine (1.00 g, 3.61 mmol, 1.0 eq.), Pd(dppf)Cl₂ (264 mg, 0.361 mmol, 0.1 eq.) and NaHCOs (607 mg, 7.22 mmol, 2.0 eq.) were successively added to 1,4-dioxane (10 mL) and water (5 mL), and heated to 110°C under the protection of nitrogen to react for 3 h. The reaction mixture was cooled to room temperature, quenched by adding water (30 mL), and extracted with EA (30 mL × 3); the organic phases were dried with anhydrous sodium sulfate and filtered; the filtrate was concentrated under reduced pressure; and the resulting crude product was separated by silica gel column chromatography (DCM:MeOH = 50:1-10:1) to obtain a product (1.22 g, yield: 53.5%).

### Step 5: Synthesis of (R)-4-(2-(ethoxymethoxy)-4-ethynylphenyl)-N-(1-methylpiperidin-3-yl)phthalazin-1-amine

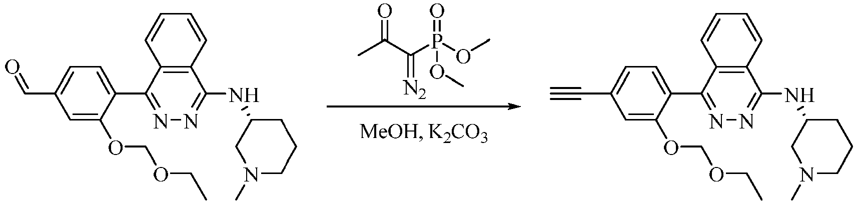

(*R*)-3-(ethoxymethoxy)-4-(4-((1-methylpiperidin-3-yl)amino)phthalazin-1-yl)benzaldehyde (1.20 g, 2.85 mmol, 1.0 eq.) was dissolved in MeOH (15 mL); K₂CO₃(788 mg, 5.70 mmol, 2.0 eq.) and (1-diazo-2-oxopropyl)dimethyl phosphonate (657 mg, 3.42 mmol, 1.2 eq.) were added; and the resulting mixture was stirred at room temperature for 2 h. The resulting mixture was concentrated, dissolved with water, and extracted with DCM (30 mL × 3); the organic phases were dried with anhydrous sodium sulfate and filtered; the filtrate was concentrated under reduced pressure; and the resulting crude product was separated by silica gel column chromatography (DCM:MeOH = 50:1-10:1) to obtain a product (1.08g, yield: 90.9%).

### Step 6: Synthesis of (R)-5-ethynyl-2-(4-((1-methylpiperidin-3-yl)amino)phthalazin-1-yl)phenol

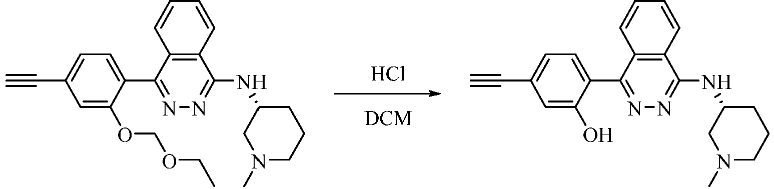

(R)-4-(2-(ethoxymethoxy)-4-ethynylphenyl)-N-(1-methylpiperidin-3-yl)phthalazin-1-amine (1.08 g, 2.95 mmol, 1.0 eq.) was dissolved in DCM (10 mL); then, the 1,4-dioxane solution of hydrogen chloride (4 mol/L, 3.24 mL, 12.9 mmol, 5.0 eq.) was dropwise added; and the resulting mixture was stirred at room temperature for 4 h. The resulting mixture was adjusted to a pH value of 8 by using saturated NaHCO₃ in water, and then extracted with DCM (30 mL × 3); the organic phases were dried with anhydrous sodium sulfate and filtered; the filtrate was concentrated under reduced pressure; and the resultant crude product was separated by silica gel column chromatography (DCM:MeOH = 50:1-10:1) to obtain aproduct (760 mg, yield: 81.8%).

¹HNMR (400 MHz, DMSO-*d₆*) δ(ppm): 10.10 (s, 1H), 8.53 (s, 1H), 7.88-7.78 (m, 2H), 7.49-7.47 (m, 2H), 7.29 (d, *J =* 7.6 Hz, 1H), 7.13 (s, 1H), 7.08-7.06 (m, 1H), 4.66 (s, 1H), 4.25 (s, 1H), 3.43 (d, *J =* 9.6 Hz, 1H), 3.06 (s, 1H), 2.59 (s, 5H), 1.99-1.92 (m, 2H), 1.79-1.74 (m, 2H).

Molecular formula: C₂₂H₂₂N₄O Accurate molecular weight: 358.18 LC-MS (Pos, m/z)=359.10[M+H]⁺.

### Example 7: Synthesis of (R)-5-ethynyl-2-(4-((1-methylpiperidin-3-yl)amino)pyridino[3,4-d]pyridazin-1-yl)phenol (Compound 5)

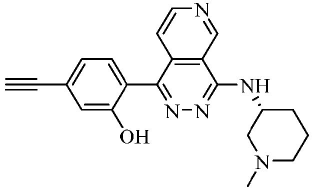

### Step 1: Synthesis of pyridino[3,4-d]pyridazin-1,4-diol

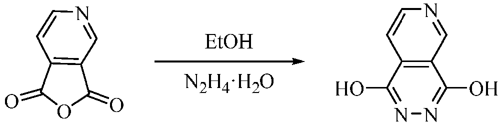

Pyridin-3,4-dimethyl anhydride (16.0 g, 0.11 mol, 1.0 eq.) was dissolved in EtOH (450 mL); hydrazine hydrate (27.0 g, 0.54 mol, 5.0 eq.) was added; and the resulting mixture was heated to 80°C to react for 7 h, after which a large amount of solids were precipitated. The mixture was cooled to room temperature and filtered by suction; and the filter cake was washed with EtOH and dried to obtain a product (15.0 g, yield: 85.0%).

### Step 2: Synthesis of 1,4-dicholoropyridino[3,4-d]pyridazine

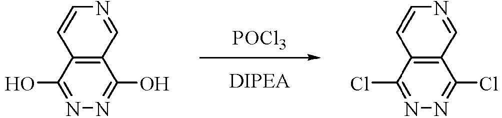

Pyridino[3,4-*d*]pyridazin-1,4-diol (1.0 g, 6.13 mmol, 1.0 eq.) was added to POCl₃ (4.70 g, 30.7 mmol, 5.0 eq ); then, DIPEA (785 mg, 6.13 mmol, 1.0 eq.) was added; the resulting mixture was heated to 100°C to allow for reaction for 2 h. The mixture was concentrated under reduced pressure, diluted with EA, adjusted to a pH value of alkalinity with NaHCOs in water, and extracted with DCM; and the organic phase was concentrated under reduced pressure, and separated by silica gel column chromatography (PE:EA = 5:1) to obtain a product (150 mg, yield: 12.5%).

### Step 3: Synthesis of 4-chloropyridino[3,4-d]pyridazin-1-ol

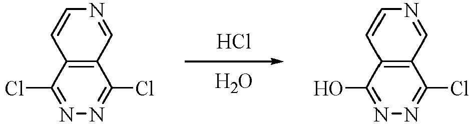

1,4-dicholoropyridino[3,4-*d*]pyridazine (5.0 g, 25.0 mmol, 1.0 eq.) was added to 1% dilute hydrochloric acid (150 mL), and then heated to 110°C to react for 2 h. The mixture was cooled to room temperature and filtered by suction; and the filter cake was recrystallized with acetic acid to obtain a product (1.50 g, yield: 33.0%).

### Step 4: Synthesis of (R)-3-((1-hydroxypyridino[3,4-d]pyridazin-4-yl)amino)piperidin-1-tert-butyl carboxylate

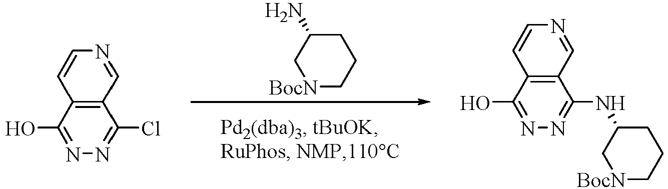

4-chloropyridino[3,4-*d*]pyridazin-1-ol (1.40 g, 7.73 mmol, 1.0 eq.), (*R*)-3-aminopiperidin-1-tert-butyl carboxylate(1.86 g, 9.28 mmol, 1.2 eq.), Pd₂(dba)₃(708 mg, 0.773 mmol, 0.1 eq.), RuPhos(721 mg, 1.55 mmol, 0.2 eq.) and tBuOK(2.20 g, 19.3 mmol, 2.5 eq.) were successively added to NMP(30 mL), and heated to 110°C under the protection of nitrogen to react for 2.5 h. The reaction mixture was cooled to room temperature, concentrated under reduced pressure, and separated by silica gel column chromatography (DCM:MeOH = 100:1- 30:1) to obtain a product (1.0 g, yield: 37.5%).

### Step 5: Synthesis of (R)-1-chloro-N-(piperidin-3-yl)pyridino[3,4-d]pyridazin-4-amine

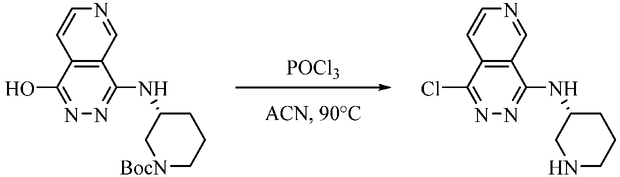

(*R*)-3-((1-hydroxypyridino[3,4-*d*]pyridazin-4-yl)amino)piperidin-1-tert-butyl carboxylate(582 mg, 1.60 mmol, 1.0 eq.) was added to ACN (10 mL); POCl₃ (736 mg, 4.80 mmol, 3.0 eq.) was added; and the resulting mixture was heated to 90°C to react for 4 h. The resulting mixture was cooled to room temperature, adjusted to a pH value of 8 by using saturated NaHCOs in water, and extracted with DCM (20 mL × 3); the organic phases were dried with anhydrous sodium sulfate and filtered; the filtrate was concentrated under reduced pressure; and the resultant was separated by silica gel column chromatography (DCM:MeOH = 50:1-10:1) to obtain a product (262 mg, yield: 62.1%).

### Step 6: Synthesis of (R)-1-chloro-N(1-methylpiperidin-3-yl)pyridino[3,4-d]pyridazin-4-amine

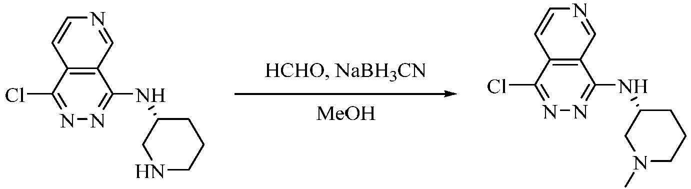

(*R*)-1-chloro-*N*-(piperidin-3-yl)pyridino[3,4-*d*]pyridazin-4-amine (262 mg, 0.993 mmol, 1.0 eq.) was dissolved in MeOH (5 mL); formaldehyde in water (37%, 121 mg, 1.49 mmol, 1.5 eq.) was added; and the resulting mixture was stirred at room temperature for 20 min. Then, NaBH₃CN (93.6 mg, 1.49 mmol, 1.5 eq.) was added to allow for reaction for 1 hour. The resulting mixture was quenched by using saturated NaHCOs in water, and extracted with DCM (20 mL × 2); the organic phases were dried with anhydrous sodium sulfate and filtered; the filtrate was concentrated under reduced pressure; and the resultant was separated by preparative thin-layer chromatography (DCM:MeOH = 10:1) to obtain a product (215 mg, yield: 77.9%).

### Step 7: Synthesis of (R)-3-(ethoxymethoxy)-4-(4-((1-methylpiperidin-3-yl)amino)pyridino[3,4-d]pyridazin-1-yl)benzaldehyde

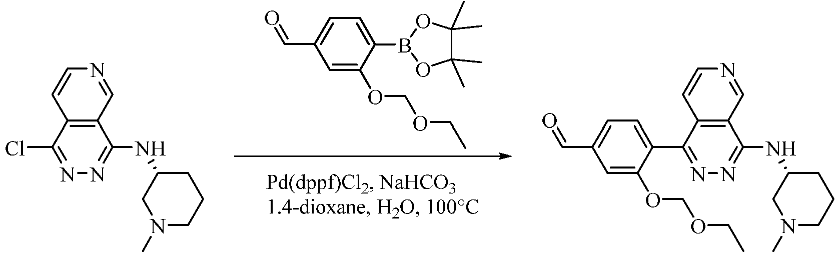

3-(ethoxymethoxy)-4-(4,4,5,5-tetramethyl-1,3,2-dioxoboracyclopentan-2-yl)benzaldehyde (284 mg, 0.929 mmol, 1.2 eq.), (*R*)-1-chloro-*N*-(1-methylpiperidin-3-yl)pyridino[3,4-*d*]pyridazin-4-amine (215 mg, 0.774 mmol, 1.0 eq.), Pd(dppf)Cl₂(56.6 mg, 0.0774 mmol, 0.1 eq.) and NaHCOs(130 mg, 1.55 mmol, 2.0 eq.) were successively added to 1,4-dioxane (4 mL); H₂O (2 mL) was added; and the resulting mixture was heated to 110°C under the protection of nitrogen to react for 3 h. The reaction mixture was cooled to room temperature, added with water (20 mL), and extracted with DCM (20 mL × 2); the organic phases were dried with anhydrous sodium sulfate and filtered; the filtrate was concentrated under reduced pressure; and the resultant was separated by silica gel column chromatography (DCM:MeOH = 100:1-10:1) to obtain a product (40.0 mg, yield: 12.3%).

### Step 8: Synthesis of (R)-1-(2-(ethoxymethoxy)-4-ethynylphenyl)-N-(1-methylpiperidin-3-yl)pyridino[3,4-d]pyridazin-4-amine

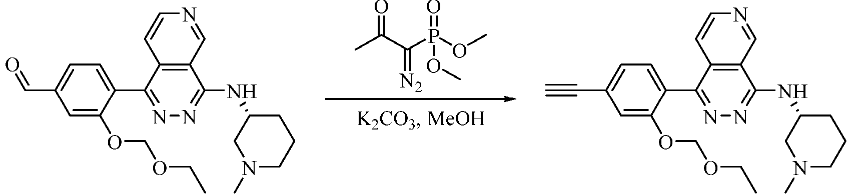

(*R*)-3-(ethoxymethoxy)-4-(4-((1-methylpiperidin-3-yl)amino)pyridino[3,4-*d*]pyridazin-1-yl)benzaldehyde (40.0 mg, 0.0949 mmol, 1.0 eq.) was dissolved in MeOH (2 mL); K₂CO₃ (26.3 mg, 0.190 mmol, 2.0 eq.) and (1-diazo-2-oxopropyl)dimethyl phosphonate (27.3 mg, 0.142 mmol, 1.5 eq.) were added; and the resulting mixture was stirred at room temperature for 3 h. The mixture was concentrated, dissolved by adding water, and extracted with DCM (5 mL×3); the organic phases were dried with anhydrous sodium sulfate and filtered; and the filtrate was concentrated under reduced pressure, and separated by preparative thin-layer chromatography (DCM:MeOH = 10:1) to obtain a product (22.0 mg, yield:55.5%).

### Step 9: Synthesis of (R)-5-ethynyl-2-(4-((1-methylpiperidin-3-yl)amino)pyridino[3,4-d]pyridazin-1-yl)phenol

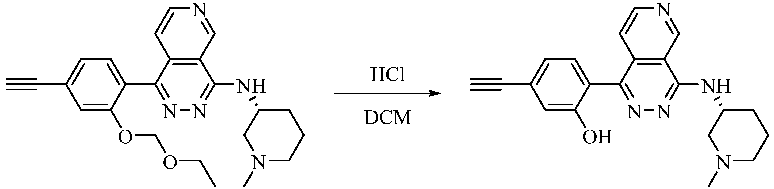

(*R*)-1-(2-(ethoxymethoxy)-4-ethynylphenyl)*-N-*(1-methylpiperidin-3-yl)pyridino[3,4-*d*]pyridazin-4-amine (122 mg, 0.0527 mmol, 1.0 eq.) was dissolved in DCM (2 mL); then, the 1,4-dioxane solution of hydrogen chloride (4 mol/L, 0.06 mL, 0.264 mmol, 5.0 eq.) was dropwise added; and the resulting mixture was stirred at room temperature for 1 h, adjusted to a pH value of 8 by using saturated NaHCOs in water, and extracted with a mixed solvent of DCM: MeOH = 10:1 (5 mL×3); the organic phases were dried with anhydrous sodium sulfate and filtered; and the filtrate was concentrated under reduced pressure, and separated by preparative thin-layer chromatography (DCM:MeOH = 10:1) to obtain a product (12.0 mg, yield: 63.4%).

¹HNMR (400 MHz, CD₃OD) δ(ppm): 9.72 (s, 1H), 8.86 (d, *J=* 8.0 Hz, 1H), 7.53-7.51 (m, 1H), 7.37 (d, *J=* 8.0 Hz, 1H), 7.16-7.14 (m, 1H), 7.12 (d, *J =* 1.2 Hz, 1H), 4.71-4.65 (m, 1H), 3.60 (s, 1H), 3.43 (d, *J =* 8.7 Hz, 1H), 3.02 (d, *J =* 10.0 Hz, 1H), 2.67 (d, *J* = 8.9 Hz, 2H), 2.60 (s, 3H), 2.16-2.14 (m, 1H), 2.06-2.01 (m, 1H), 1.88-1.78 (m, 2H). Molecular formula: C₂₁H₂₁N₅O Accurate molecular weight: 359.17 LC-MS(Pos, *m*/*z*)=360.13[M+H]⁺.

### Example 8: Synthesis of (R)-5-ethynyl-2-(5-methyl-3-((1-methylpiperidin-3-yl)amino)-1,2,4-triazin-6-yl)phenol (Compound 177)

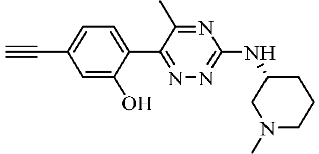

### Step 1: Synthesis of 6-heteronitrogen-2-thioureapyrimidine

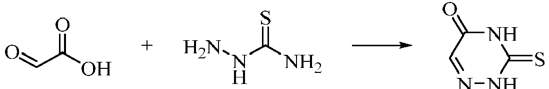

Aminothiourea (100g, 1.10 mol, 1.0 eq.) was dissolved in water (300 mL), and 50% glyoxylic acid (162.89 g, 1.1 mol, 1.0 eq.)was dropwise added to allow for reaction for 1.5 h at 100°C. A large amount of solids were then precipitated, cooled to room temperature, and filtered; the filter cake was portionwise added to NaOH (44g, 1.10 mol, 1.0 eq.) in water (300 mL) to allow for reaction for 1 h at 100°C; the reaction mixture was cooled to room temperature; the aqueous phase was adjusted to a pH value of 1 by using hydrochloric acid, after which a large amount of solids were precipitated; suction filtration was conducted; and the filter cake was dried to obtain a product (90 g, yield: 63.4%).

### Step 2: Synthesis of 3-(methylthio)-1,2,4-triazin-5(4H)-one

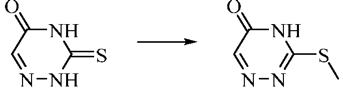

6-heteronitrogen-2-thioureapyrimidine (90 g, 696.91 mmol, 1.0 eq.) was dissolved in EtOH (1 L); sodium tert-butoxide (66.97 g, 696.91 mmol, 1.0 eq.) was portionwise added; the resulting mixture was stirred for 10 min; and iodomethane (98.93 g, 696.91 mmol, 1.0 eq.) was slowly dropwise added to allow for reaction for 50 min. TLC monitoring showed that the reaction was complete; suction filtration was conducted; the filtrate was concentrated under reduced pressure; the resulting mixture was first purified by silica gel column chromatography (MeOH:DCM = 1:15), then pulped with EA (100 mL), and subjected to suction filtration; and the filter cake was dried to obtain a product (30 g, yield: 30%).

### Step 3: Synthesis of 5,6-dicholoro-3-(methylthio)-1,2,4-triazine

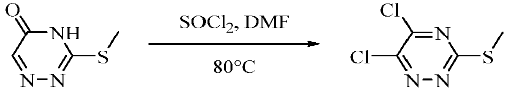

3-(methylthio)-1,2,4-triazin-5(4*H*)-one (8.50 g, 59.4 mmol, 1.0 eq.) was added to SOCl₂ (40 mL); DMF (0.4 mL) was added; and the resulting mixture was heated to 80°C to react for 2 h. The reaction mixture was cooled to room temperature, and concentrated under reduced pressure to obtain a crude product (12.2g).

### Step 4: Synthesis of 6-chloro-5-methyl-3-(methylthio)-1,2,4-triazine

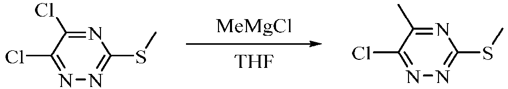

5,6-dicholoro-3-(methylthio)-1,2,4-triazine (12.2 g, 62.2 mmol, 1.0 eq.) was dissolved in THF (50 mL), and cooled to -60°C; and MeMgCl (20.7 mL, 62.2 mmol, 1.0 eq.) was dropwise added to allow for reaction for 0.5 h. Saturated NH₄Cl in water was added for quenching; the mixture was extracted with EA(50 mL × 2); the organic phases were dried with anhydrous sodium sulfate and filtered; and the filtrate was concentrated under reduced pressure, and separated by silica gel column chromatography (PE:EA = 100:1-10:1) to obtain a product (1.52 g, two-step yield: 20.0%).

### Step 5: Synthesis of 6-chloro-5-methyl-3-(methylsulfonyl)-1,2,4-triazine

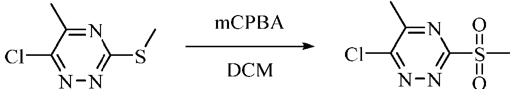

6-chloro-5-methyl-3-(methylthio)-1,2,4-triazine (1.52 g, 8.65 mmol, 1.0 eq.) was dissolved in DCM (50 mL), and stirred at room temperature; and metachloroperbenzoic acid (3.73 g, 21.6 mmol, 2.5 eq.) was added to allow for reaction for 2 h. Saturated NaHCOs in water was added to adjust to a pH value of 8; the resulting mixture was extracted with DCM (30 mL × 2); the organic phases were dried with anhydrous sodium sulfate and filtered; and the filtrate was concentrated under reduced pressure to obtain a product (1.38 g, yield: 76.8%).

### Step 6: Synthesis of (R)-3-((6-chloro-5-methyl-1,2,4-triazin-3-yl)amino)piperidin-1-tert-butyl carboxylate

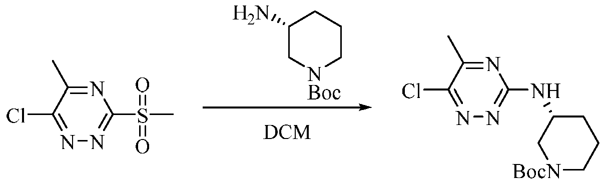

6-chloro-5-methyl-3-(methylsulfonyl)-1,2,4-triazine (1.38 g, 6.65 mmol, 1.0 eq.) was dissolved in DCM (30 mL); and (R)-3-aminopiperidin-1-tert-butyl carboxylate (2.00 g, 9.98 mmol, 1.5 eq.) was added to allow for reaction for 3 h at room temperature. The reaction mixture was concentrated under reduced pressure, and separated by silica gel column chromatography (PE:EA = 20:1-2:1) to obtain a product (540 mg, yield: 24.8%).

### Step 7: Synthesis of (R)-6-chloro-5-methyl-N-(piperidin-3-yl)-1,2,4-triazin-3-amine

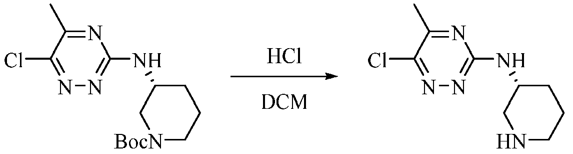

(R)-3-((6-chloro-5-methyl-1,2,4-triazin-3-yl)amino)piperidin-1-tert-butyl carboxylate (540 mg, 1.65 mmol, 1.0 eq.) was dissolved in DCM (5 mL); the 1,4-dioxane solution of hydrogen chloride (4 mol/L, 1.24 mL, 4.95 mmol, 3.0 eq.) was dropwise added; and the resulting mixture was stirred at room temperature for 1 h. The resulting mixture was adjusted to a pH value of 8 by using saturated NaHCOs in water, and extracted with DCM (20 mL × 3); the organic phases were dried with anhydrous sodium sulfate and filtered; the filtrate was concentrated under reduced pressure; and the resultant was separated by silica gel column chromatography (DCM:MeOH = 50:1-10:1) to obtain a product (332 mg, yield: 88.5%).

### Step 8: Synthesis of (R)-6-chloro-5-methyl-N-(1-methylpiperidin-3-yl)-1,2,4-triazin-3-atnine

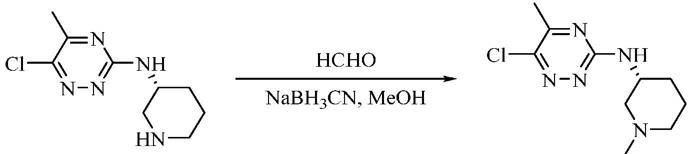

(R)-6-chloro-5-methyl-N-(piperidin-3-yl)-1,2,4-triazin-3-ainine (330 mg, 1.45 mmol, 1.0 eq.) was dissolved in MeOH (5 mL); formaldehyde in water (37%, 183 mg, 2.18 mmol, 1.5 eq.) was added; and the resulting mixture was stirred at room temperature for 20 min. Then, NaBH₃CN (137 mg, 2.18 mmol, 1.5 eq.) was added to allow for reaction for 1 hour. The resulting mixture was quenched by using saturated NaHCOs in water, and extracted with DCM (20 mL × 2); the organic phases were dried with anhydrous sodium sulfate and filtered; the filtrate was concentrated under reduced pressure; and the resultant was separated by silica gel column chromatography (DCM:MeOH = 50:1-10:1) to obtain a product (280 mg, yield: 77.9%).

### Step 8: Synthesis of (R)-3-(ethoxymethoxy)-4-(5-methyl-3-((1-methylpiperidin-3-yl)atnino)-1,2,4-triazin-6-yl)benzaldehyde

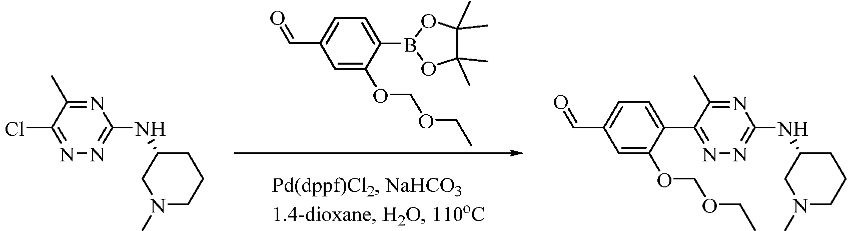

3-(ethoxymethoxy)-4-(4,4,5,5-tetramethyl-1,3,2-dioxoboracyclopentan-2-yl)benzaldehyde (380 mg, 1.24 mmol, 1.5 eq.), (R)-6-chloro-5-methyl-N (1-methylpiperidin-3-yl)-1,2,4-triazin-3-amine (200 mg, 0.827 mmol, 1.0 eq.), Pd(dppf)Cl₂ (60.5 mg, 0.0827 mmol, 0.1 eq.) and NaHCOs (139 mg, 1.65 mmol, 2.0 eq.) were successively added to 1,4-dioxane (6 mL); H₂O(3 mL) was added; and the resulting mixture was heated to 110°C under the protection of nitrogen to react for 1 h. The reaction mixture was cooled to room temperature, added with water (20 mL), and extracted with DCM (30 mL × 2); the organic phases were dried with anhydrous sodium sulfate and filtered; the filtrate was concentrated under reduced pressure; and the resultant was separated by silica gel column chromatography (DCM:MeOH = 100:1-10:1) to obtain aproduct (180 mg, yield: 56.4%).

### Step 10: Synthesis of (R)-6-(2-(ethoxymethoxy)-4-ethynylphenyl)-5-methyl-N-(1-methylpiperidin-3-yl)-1,2,4-triazin-3-amine

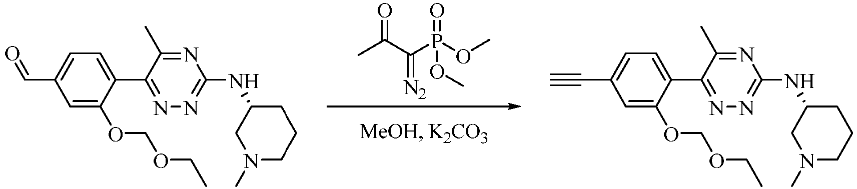

(*R*)-3-(ethoxymethoxy)-4-(5-methyl-3-((1-methylpiperidin-3-yl)amino)-1,2,4-triazin-6-yl)benzaldehyde (180 mg, 0.467 mmol, 1.0 eq.) was dissolved in MeOH (5 mL); K₂CO₃ (129 mg, 0.934 mmol, 2.0 eq.) and (1-diazo-2-oxopropyl)dimethyl phosphonate (135 mg, 0.701 mmol, 1.5 eq.) were added; and the resulting mixture was stirred at room temperature for 2 h. The resulting mixture was concentrated, dissolved with water, and extracted with EA (20 mL × 2); the organic phases were dried with anhydrous sodium sulfate and filtered; the filtrate was concentrated under reduced pressure; and the resultant was separated by silica gel column chromatography (DCM:MeOH = 50:1-10:1) to obtain a product (152 mg, yield: 85.3%).

### Step 11: Synthesis of (R)-5-ethynyl-2-(5-methyl-3-((1-methylpiperidin-3-yl)amino)-1,2,4-triazin-6-yl)phenol

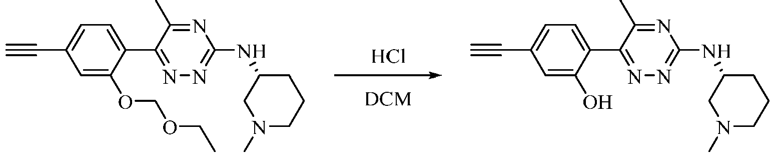

(*R*)-6-(2-(ethoxymethoxy)-4-ethynylphenyl)-5-methyl-*N*-(1-methylpiperidin-3-yl)-1,2,4-triazin-3-amine (152 mg, 0.398 mmol, 1.0 eq.) was dissolved in DCM (3 mL); the 1,4-dioxane solution of hydrogen chloride (4 mol/L, 0.3 mL, 1.19 mmol, 3.0 eq.) was dropwise added; and the resulting mixture was stirred at room temperature for 2 h. The resulting mixture was adjusted to a pH value of 8 by using saturated NaHCOs in water, and extracted with DCM (10 mL × 3); the organic phases were dried with anhydrous sodium sulfate and filtered; the filtrate was concentrated under reduced pressure; and the resultant was separated by silica gel column chromatography (DCM:MeOH = 50:1-10:1) to obtain a product (82.0 mg, yield: 63.6%).

¹HNMR(400 MHz, DMSO-*d₆*) δ(ppm): 10.10 (s, 1H), 7.45 (s, 1H), 7.27-7.25 (m, 1H), 7.04-7.03 (m, 2H), 4.21 (s, 1H), 3.99 (s, 1H), 2.89 (d, *J =* 7.0 Hz, 1H), 2.64 (d, *J =* 8 Hz, 1H), 2.19 (s, 3H), 2.18 (s, 3H), 1.90-1.85 (m, 3H), 1.72-1.68 (m, 1H), 1.57-1.49 (m, 1H), 1.36-1.30 (m, 1H).

Molecular formula: C₁₈H₂₁N₅O Accurate molecular weight: 323.17 LC-MS (Pos, m/z)=324.11[M+H]⁺.

### Example 9: Synthesis of (R)-2-(5-methyl-3-((1-methylpiperidin-3-yl)amino)-1,2,4-triazin-6-yl)-5-(trifluoromethyl)phenol (Compound 188)

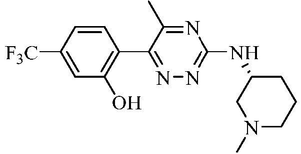

### Step 1: Synthesis of (R)-6-(2-(ethoxymethoxy)-4-(trifluoromethyl)phenyl)-5-methyl-N-(1-methylpiperidin-3-yl)-1,2,4-triazin-3-atnine

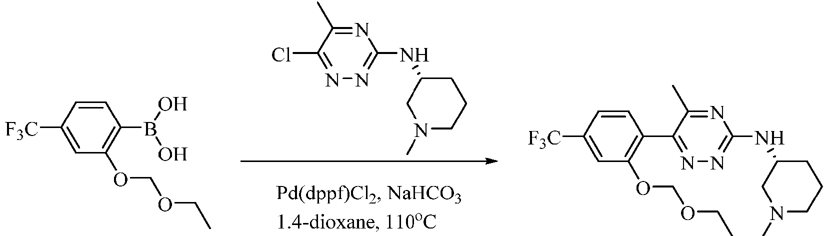

(2-(ethoxymethoxy)-4-(trifluoromethyl)phenyl)boric acid (131 mg, 0.496 mmol, 1.5 eq.), (R)-6-chloro-5-methyl-*N*-(1-methylpiperidin-3-yl)-1,2,4-triazin-3-amine (80 mg, 0.331 mmol, 1.0 eq.), Pd(dppf)Cl₂ (24.2 mg, 0.0331 mmol, 0.1 eq.) and NaHCO₃ (55.6 mg, 0.662 mmol, 2.0 eq.) were successively added to 1,4-dioxane (5 mL); H₂O(2.5 mL) was added; and the resulting mixture was heated to 110°C under the protection of nitrogen to react for 1 h. The reaction mixture was cooled to room temperature, added with water (10 mL), and extracted with DCM (15 mL × 2); the organic phases were dried with anhydrous sodium sulfate, filtered, and then concentrated; and the resultant was separated by silica gel column chromatography (DCM:MeOH = 100:1-10:1) to obtain a product (83.0 mg, yield: 58.9%).

### Step 2: Synthesis of (R)-2-(5-methyl-3-((1-methylpiperidin-3-yl)amino)-1,2,4-triazin-6-yl)-5-(trifluoromethyl)phenol

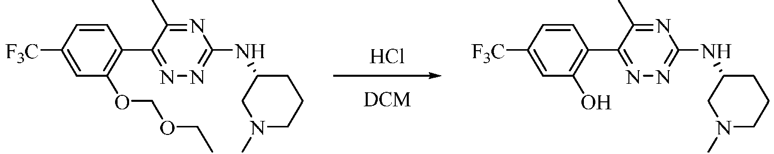

(*R*)-6-(2-(ethoxymethoxy)-4-(trifluoromethyl)phenyl)-5-methyl-*N-*1-methylpiperidin-3-yl)-1,2,4-triazin-3-amine (83 mg, 0.195 mmol, 1.0 eq.) was dissolved in DCM (3 mL); then, the 1,4-dioxane solution of hydrogen chloride (4 mol/L, 0.15 mL, 0.585 mmol, 3.0 eq.) was dropwise added; and the resulting mixture was stirred at room temperature for 2 h. The resulting mixture was adjusted to a pH value of 8 by using saturated NaHCO₃ in water, and extracted with DCM (10 mL × 3); the organic phases were dried with anhydrous sodium sulfate and filtered; the filtrate was concentrated under reduced pressure; and the resultant was separated by silica gel column chromatography (DCM:MeOH = 50:1-10:1) to obtain a product (47.0 mg, yield: 65.6%).

¹HNMR(400 MHz, DMSO-*d₆*) δ(ppm): 10.56 (s, 1H), 7.49 (d, *J =* 8 Hz, 2H), 7.28-7.25 (m, 2H), 4.02 (s, 1H), 2.92 (s, 1H), 2.67 (d, *J =* 10.6 Hz, 1H), 2.22 (s, 3H), 2.19 (s, 3H), 1.95-1.89 (m, 3H), 1.71-1.69 (m, 1H), 1.60-1.51 (m, 1H), 1.35-1.32(m, 1H).

Molecular formula: C₁₇H₂₀N₅O Accurate molecular weight: 367.16 LC-MS (Pos, m/z)=368.11[M+H]⁺.

### Example 10: Synthesis of (R)-5-ethynyl-2-(4-methyl-6-((1-methylpiperidin-3-yl)amino)pyridazin-3-yl)phenol (Compound 54)

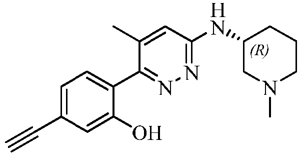

### Step 1: Synthesis of (R)-3-(ethoxymethoxy)-4-(4-methyl-6-((1-methylpiperidin-3-yl)atnino)pyridazin-3-yl)benzaldehyde

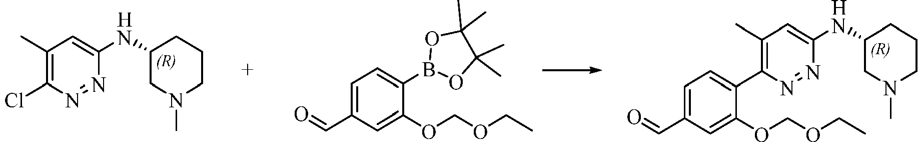

(*R*)-6-chloro-5-methyl-*N*-(1-methylpiperidin-3-yl)pyridazin-3-amine (300.0 mg, 1.24 mmol, 1.0 eq.), 3-(ethoxymethoxy)-4-(4,4,5,5-tetramethyl-1,3-dioxolam-2-yl)benzaldehyde (572.3 mg, 1.869 mmol, 1.5 eq.), sodium hydrogen carbonate (209.3 mg, 2.49 mmol. 2.0 eq.) and Pd(dppf)Cl₂ (45.5 mg, 0.06 mmol, 0.05 eq.) were added to the mixed solution of 1,4-dioxane (12.0 mL) and water (4.0 mL), and allowed to react for 4 h at 110°C under the protection of nitrogen; TLC monitoring showed that the reaction was complete; the system was cooled to room temperature; water (50.0 mL) was added; the mixture was extracted with ethyl acetate (100.0 mL); the organic phase was dried with anhydrous sodium sulfate and filtered; the filtrate was concentrated under reduced pressure; and the resulting crude product was purified by silica gel column chromatography (silica gel: 100-200 meshes, dichloromethane:methanol = 50:1-10:1) to obtain a product (283.0 mg, yield:59.0%).

### Step 2: Synthesis of (R)-6-(2-(ethoxymethoxy)-4-ethynylphenyl)-5-methyl-N-(1-methylpiperidin-3-yl)pyridazin-3-amine

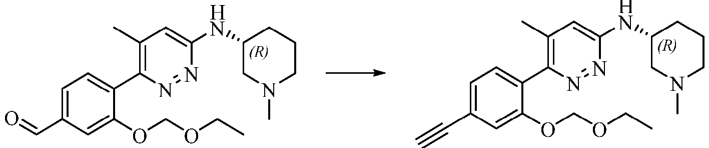

(*R*)-3-(ethoxymethoxy)-4-(4-methyl-6-((1-methylpiperidin-3-yl)amino)pyridazin-3-yl)benzaldehyde (283.0 mg, 0.73 mmol, 1.0 eq.) , anhydrous potassium carbonate (203.4 mg, 1.17 mmol, 2.0 eq.) and (1-diazo-2-oxopropyl)dimethyl phosphonate (212.1 mg, 1.10 mmol, 1.5 eq.) were added to methanol (10.0 mL), and allowed to react at room temperature for 12 h; TLC monitoring showed that the reaction was complete; saturated sodium chloride in water (50.0 mL) was added; the resulting mixture was extracted with dichloromethane (50.0 mL); the organic phases were dried with anhydrous sodium sulfate and filtered; the filtrate was concentrated under reduced pressure; and the resulting crude product was purified by silica gel column chromatography (silica gel:100-200 meshes, dichloromethane:methanol = 50:1-10:1) to obtain a product (190.0 mg, yield: 67.8%).

### Step 3: Synthesis of (R)-5-ethynyl-2-(4-methyl-6-((1-methylpiperidin-3-yl)amino)pyridazin-3-yl)phenol

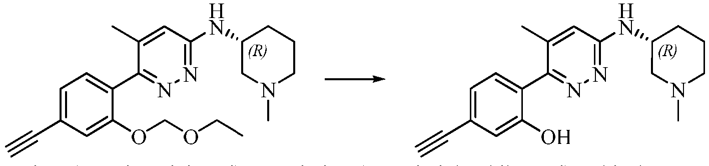

(*R*)-6-(2-(ethoxymethoxy)-4-ethynylphenyl)-5 -methyl-*N*-(1-methylpiperidin-3-yl)pyridazin-3-amine (190.0 mg, 0.49 mmol, 1.0 eq.) was added to dichloromethane (2.0 mL); the 1,4-dioxane solution of hydrogen chloride (4.0 mol/L, 4.0 mL) was dropwise added to allow for reaction for 1 h at room temperature; and TLC monitoring showed that the reaction was complete. The system was adjusted to pH = 7-8 by using saturated sodium hydrogen carbonate in water, and extracted with dichloromethane (50.0 mL); the organic phase was dried with anhydrous sodium sulfate and filtered; and the filtrate was concentrated under reduced pressure, and purified by preparative thin-layer chromatography (dichloromethane:methanol = 10:1) to obtain a product (110.0 mg, yield:68.7%).

¹HNMR (400 MHz, DMSO-*d₆*) δ(ppm): 10.09 (s, 1H), 7.18-7.16 (m, 1H), 7.00-6.98 (m, 2H), 6.68 (s, 1H), 6.60-6.58 (d, *J*=8 Hz, 1H), 4.17 (s, 1H), 4.04-4.01 (m, 1H), 2.86-2.84 (d, *J*=8 Hz, 1H), 2.51-2.50 (m, 1H), 2.17 (s, 3H), 1.99-2.02 (m, 4H), 1.89-1.81 (m, 2H), 1.73-1.69 (m, 1H), 1.55-1.52 (m, 1H), 1.30-1.24 (m, 1H).

Molecular formula: C₁₉H₂₂N₄O Accurate molecular weight: 322.18 LC-MS (Pos, m/z)=323.11[M+H]⁺.

### Example 11: Synthesis of (R)-5-ethynyl-2-(6-fluoro-4-((1-methylpiperidin-3-yl)amino)phthalazin-1-yl)phenol (Compound 172)

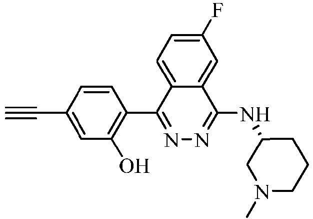

### Step 1: Synthesis of 2-(4-bromo-2-methoxybenzene formyl)-5-methyl fluorobenzoate

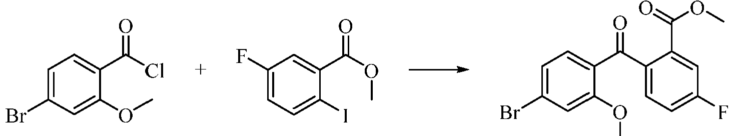

5-fluoro-2-methyl iodobenzoate (10.0 g, 35.71 mmol, 1.0 eq.) was dissolved in THF (200 mL), and cooled to -30°C under the protection of nitrogen; the tetrahydrofuran solution of isopropyl magnesium chloride lithium chloride (1.3 mol/L, 30.2 mL, 39.28 mmol, 1.1 eq.) was added, and stirred for 20 min; CuCN (3.2 g, 35.71 mmol, 1.0 eq.) was added and stirred for 20 min; 4-bromo-2-methoxybenzoyl chloride (10.7 g, 42.85 mmol, 1.0 eq.) was added to alow for reaction for 20 min; and TLC monitoring showed that the reaction was complete. The reaction mixture was poured into water (100 mL), stirred for 10 min, and subjected to suction filtration; the filtrate was separated; the aqueous phase was extracted with EA (100 mL × 2); the EA phase was then washed with potassium carbonate in water (100 mL × 2); and the organic phases were combined, dried, and concentrated to obtain a crude product (13.13 g, yield: 100%).

### Step 2: Synthesis of 4-(4-bromo-2-methoxyphenyl)-7-fluorophthalazin-1-ol

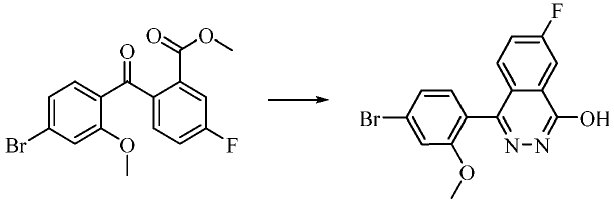

2-(4-bromo-2-methoxybenzene formyl)-5-methyl fluorobenzoate (13.13 g, 35.71 mmol, 1.0 eq.) and 85% hydrazine hydrate (4.20 g, 71.42 mmol, 2.0 eq.) were dissolved in ethanol (200 mL) to react at room temperature for 40 min; TLC monitoring showed that the reaction was complete; and the reaction mixture was cooled to room temperature and filtered; and the filter cake was dried to obtain a product (6.5 g, yield: 52.1%).

### Step 3: Synthesis of 1-(4-bromo-2-methoxyphenyl)-4-chloro-6-fluorophthalazine

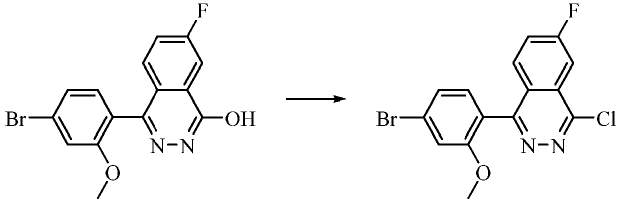

4-(4-bromo-2-methoxyphenyl)-7-fluorophthalazin-1-ol (6.5 g, 18.61 mmol, 1.0 eq.) and phosphorus oxychloride (5.71 g, 37.22 mmol, 2.0 eq.) were dissolved in acetonitrile (130 mL) to react for 2 h at 90°C; TLC monitoring showed that the reaction was complete; the reaction mixture was concentrated under reduced pressure; water (100 mL) was added; sodium carbonate was used to adjust the pH value to 10; the resulting mixture was extracted with EA (100 mL × 3); and the organic phases were dried and concentrated, and purified by silica gel column chromatography (DCM:PE = 1:1) to obtain a product (5.5 g, yield: 80.4%).

### Step 4: Synthesis of 1-(4-bromo-2-methoxyphenyl)-4,6-difluorophthalazine

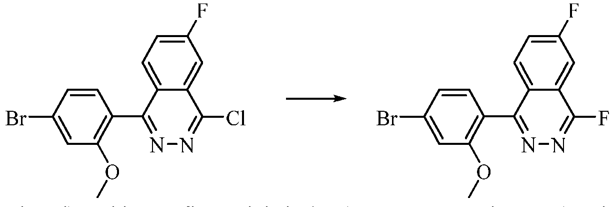

1-(4-bromo-2-methoxyphenyl)-4-chloro-6-fluorophthalazine (2.0 g, 5.44 mmol, 1.0 eq.) and tetramethylammonium fluoride (760 mg, 8.16 mmol, 1.5 eq.) were dissolved in DMF (20 mL) to react for 1 h at 100°C; LC-MS monitoring showed that the reaction was complete; the reaction mixture was poured into water (20 mL), and extracted with MTBE (20 mL × 2); the organic phases were combined, dried, concentrated, and purified by silica gel column chromatography (PE:EA = 5:1) to obtain a product (0.9 g, yield: 47.1%).

### Step 5: Synthesis of (R)-3-((4-(4-bromo-2-methoxyphenyl)-7-fluorophthalazin-1-yl)atnino)piperidin-1-tert-butyl carboxylate

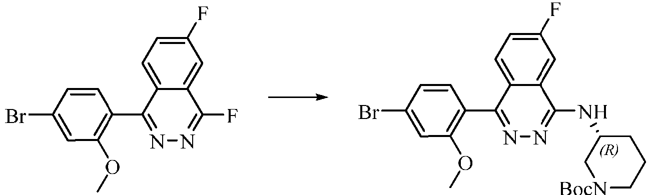

1-(4-bromo-2-methoxyphenyl)-4,6-difluorophthalazine (0.9 g, 2.56 mmol, 1.0 eq.), (*R*)-3-aminopiperidin-1-tert-butyl carboxylate (566 mg, 2.82 mmol, 1.1 eq.) and TEA(777 mg, 7.68 mmol, 3.0 eq.) were dissolved in 1,4-dioxane (10 mL) to react for 20 h at 80°C. LC-MS detection showed that the reaction was complete; the reaction mixture was concentrated under reduced pressure; the resulting crude product was pulped with a mixed solvent of PE and EA (10: 1, 20 mL), and subjected to suction filtration; and the filter cake was dried to obtain a product (1.0 g, yield: 73.5%).

### Step 6: Synthesis of (R)-3-((7-fluoro-4-(2-methoxy-4-((trimethylsilicyl)ethynyl)phenyl)phthalazin-1-yl)amino)piperidin-1-tert-butyl carboxylate

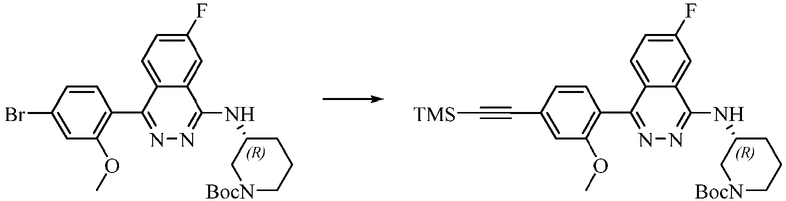

(*R*)-3-((4-(4-bromo-2-methoxyphenyl)-7-fluorophthalazin-1-yl)amino)piperidin-1-tert-butyl carboxylate(1.0 mg, 1.88 mmol, 1.0 eq.), PdCl₂(dppf)₂ (133 mg, 0.19 mmol, 0.1 eq.) and CuI (107 mg, 0.26 mmol, 0.3 eq.) were dissolved in diisopropylamine (10 mL); and trimethylsilylacetylene (1.85 mg, 18.80 mmol, 10 eq.) was added under the protection of nitrogen to allow for reaction for 16 h at 60°C. TLC detection showed that the reaction was complete; the reaction mixture was concentrated under reduced pressure; and the resulting crude product was purified by silica gel column chromatography (PE:EA = 2:1) to obtain a product (510 mg, yield: 49.5%).

### Step 7: Synthesis of (R)-5-ethynyl-2-(6-fluoro-4-(piperidin-3-ylamino)phthalazin-1-yl)phenol

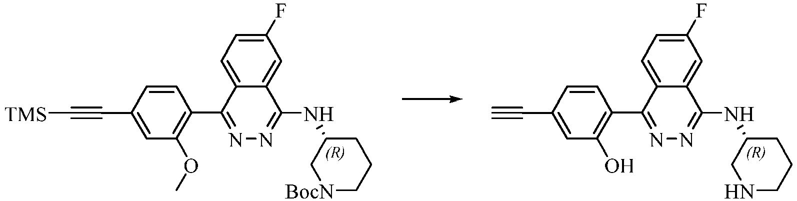

(*R*)-3-((7-fluoro-4-(2-methoxy-4-((trimethylsilicyl)ethynyl)phenyl)phthalazin-1-yl)amino)piperidin-1-tert-butyl carboxylate (0.5 g, 0.91 mmol, 1.0 eq.) was dissolved in DCM (10 mL); boron tribromide (456 mg, 1.82 mmol, 2.0 eq.) was added to allow for reaction for 3 h at 0°C; TLC detection showed that the reaction was complete; the reaction mixture was poured into water (20 mL), adjusted to a pH value of 9 by using NaHCO₃, and extracted with DCM (15 mL × 3); and the organic phases were combined, dried, and concentrated to obtain a product (300 mg, yield: 90.9%).

### Step 8: Synthesis of (R)-5-ethynyl-2-(6-fluoro-4-((1-methylpiperidin-3-yl)amino)phthalazin-1-yl)phenol

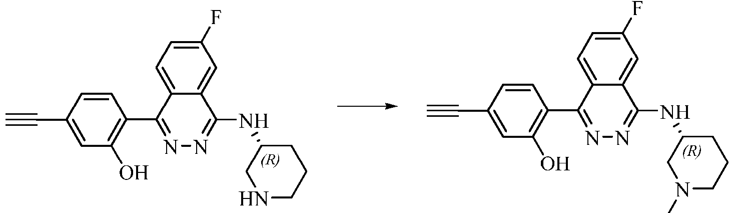

(*R*)-5-ethynyl-2-(6-fluoro-4-(piperidin-3-ylamino)phthalazin-1-yl)phenol (300 mg, 0.82 mmol, 1.0 eq.) and formaldehyde in water (37%)(67 mg, 0.82 mmol, 1.0 eq.) were dissolved in methanol (5 mL), and stirred at room temperature for 5min; and sodium cyanoborohydride (52 mg, 0.82 mmol, 1.0 eq.) was added to allow for reaction for 5 min at room temperature. LC-MS monitoring showed that the reaction was complete; the reaction mixture was concentrated under reduced pressure, added with water (10 mL), and extracted with DCM (10 mL × 3); the organic phases were combined, dried, and concentrated; and the resulting crude product was purified by preparative thin-layer chromatography (DCM:MeOH = 10:1) to obtain a product (108 mg, yield: 35.1%)

¹HNMR (400 MHz, DMSO-*d₆*) δ(ppm): 10.01 (s, 1H), 8.32-8.29 (d, 1H), 7.71-7.66 (t, 1H), 7.57-7.53 (m, 1H),7.31-7.29 (d, 1H), 7.13-7.07 (m, 3H), 4.43 (s, 1H), 4.23 (s, 1H), 3.17-3.14 (d ,1H), 2.80-2.78 (d, 1H), 2.28 (s, 3H), 2.02 (s, 2H), 1.81-1.78 (d, 1H), 1.68-1.59 (m, 1H), 1.49-1.46 (d, 1H), 1.24 (s, 1H).

Molecular formula: C₂₂H₂₁FN₄O Accurate molecular weight: 376.17 LC-MS (Pos, m/z)=377.11[M+H]⁺.

### Example 12: Synthesis of (R)-3-hydroxyl-4-(5-methyl-3-((1-methylpiperidin-3-yl)amino)-1,2,4-triazin-6-yl)benzonitrile (Compound 190)

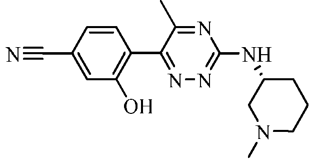

### Step 1: Synthesis of 5-methyl-3-(methylsulfonyl)-1,2,4-triazine

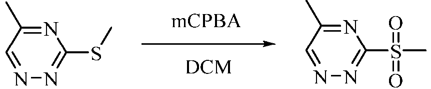

5-methyl-3-(methylthio)-1,2,4-triazine (1.5 g, 10.62 mmol, 1.0 eq.) was dissolved in DCM (50 mL); and metachloroperbenzoic acid (4.53 g, 22.30 mmol, 2.1 eq.) was added to allow for reaction for 0.5 h at room temperature. The reaction mixture was poured into water (20 mL), adjusted to a pH value of 9 by using NaHCO₃, and extracted with DCM (20 mL × 2); and the organic phases were dried, and concentrated to obtain a product (1.84 g, yield: 100%).

### Step 2: Synthesis of (R)-3-((5-methyl-1,2,4-triazin-3-yl)amino)piperidin-1-tert-butyl carboxylate

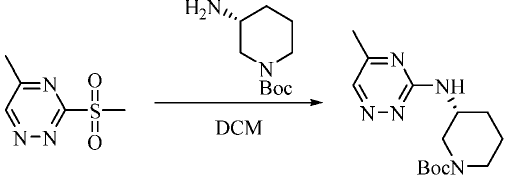

5-methyl-3-(methylsulfonyl)-1,2,4-triazine (1.84 g, 10.62 mmol, 1.0 eq.), (*R*)-3-aminopiperidin-1-tert-butyl carboxylate (2.13 g, 10.62 mmol, 1.5 eq.) and TEA (1.61 g, 15.93 mmol, 1.5 eq.) were dissolved in 1,4-dioxane (20 mL), and allowed to react for 1 h at 100°C. The reaction mixture was concentrated under reduced pressure, and the resulting crude product was purified by silica gel column chromatography (PE:EA=2:1) to obtain a product (700 mg, yield: 22.4%).

### Step 3: Synthesis of (R)-3-((6-bromo-5-methyl-1,2,4-triazin-3-yl)atnino)piperidin-1-tert-butyl carboxylate

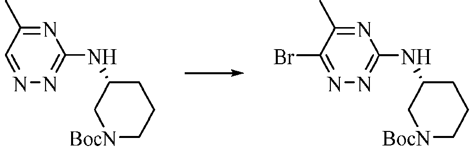

(*R*)-3-((5-methyl-1,2,4-triazin-3-yl)amino)piperidin-1-tert-butyl carboxylate (0.7 g, 2.38 mmol, 1.0 eq.) and NBS (466 mg, 2.62 mmol, 1.1 eq.) were dissolved in DMF (7 mL), and allowed to react at room temperature for 19 h. The reaction mixture was poured into water (20 mL), and extracted with MTBE (15 mL × 3); the organic phases were combined, dried, and concentrated; and the resulting crude product was purified by silica gel column chromatography (PE:EA = 5:1) to obtain a product (300 mg, yield: 33.9%).

### Step 4: Synthesis of (R)-3-((6-(4-cyano-2-(ethoxymethoxy)phenyl)-5-methyl-1,2,4-triazin-3-yl)amino)piperidin-1-tert-butyl carboxylate

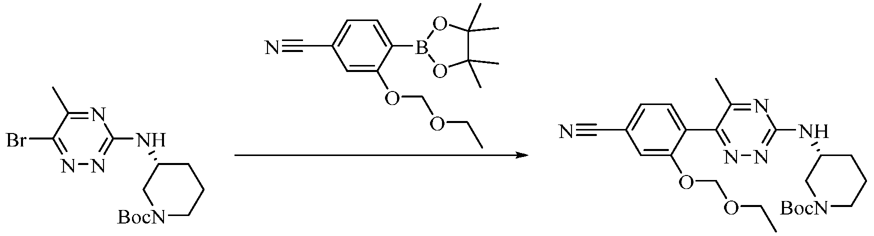

(*R*)-3-((6-bromo-5-methyl-1,2,4-triazin-3-yl)amino)piperidin-1-tert-butyl carboxylate (300 mg, 0.8 mmol, 1.5 eq.), 3-(ethoxymethoxy)-4-(4,4,5,5-tetramethyl-1,3,2-dioxoboracyclopentan-2-yl)benzonitrile (364 mg, 1.2 mmol, 1.5 eq.), Pd(dppf)Cl₂ (58 mg, 0.08 mmol, 0.1 eq.) and NaHCO₃ (134 mg, 1.6 mmol, 2.0 eq.) were added to 1,4-dioxane (6 mL) and H₂O (2 mL), and heated to 110°C under the protection of nitrogen to react for 1 h. The reaction mixture was poured into water (10 mL), and extracted with EA (20 mL × 2); the organic phases were combined, dried, and concentrated; and the resulting crude product was purified by silica gel column chromatography (PE:EA = 2:1) to obtain a product (280 mg, yield: 74.7%).

### Step 5: Synthesis of (R)-3-hydroxyl-4-(5-methyl-3-(piperidin-3-ylamino)-1,2,4-triazin-6-yl)benzonitrile

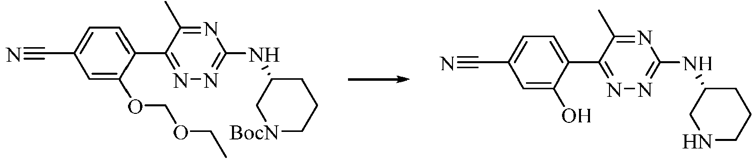

(*R*)-3-((6-(4-cyano-2-(ethoxymethoxy)phenyl)-5-methyl-1,2,4-triazin-3-yl)amino)piperidin-1-tert-butyl carboxylate (280 mg, 0.6 mmol, 1.0 eq.) was dissolved in DCM (3 mL); and then, the 1,4-dioxane solution of hydrogen chloride (4 mol/L, 1 mL) was dropwise added to allow for reaction for 1.5 h at room temperature. LC-MS monitoring showed that the reaction was complete; the reaction mixture was poured into water (10 mL), and reversely extracted with DCM (10 mL × 3); the aqueous phases were adjusted to a pH value of 10 by using NaHCOs in water, and extracted with DCM (10 mL × 4); and the organic phases were combined, dried, and concentrated to obtain a product (150 mg, yield: 80.6%).

### Step 6: Synthesis of (R)-3-hydroxyl-4-(5-methyl-3-((1-methylpiperidin-3-yl)amino)-1,2,4-triazin-6-yl)benzonitrile

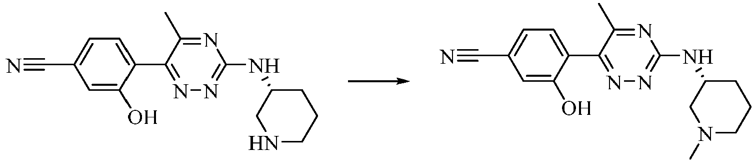

(*R*)-3-hydroxyl-4-(5-methyl-3-(piperidin-3-ylamino)-1,2,4-triazin-6-yl)benzonitrile (150 mg, 0.48 mmol, 1.0 eq.) and formaldehyde in water (37%) (39 mg, 0.48 mmol, 1.0 eq.) were dissolved in methanol (3 mL), and stirred at room temperature for 5min; and sodium cyanoborohydride (30 mg, 0.48 mmol, 1.0 eq.) was added to allow for reaction for 5 min at room temperature. TLC monitoring showed that the reaction was complete; the reaction mixture was poured into water (10 mL), and extracted with DCM (30 mL × 3); the organic phases were combined, dried, and concentrated; and the resulting crude product was purified by preparative thin-layer chromatography (DCM:MeOH = 10:1) to obtain a product (65 mg, yield: 41.9%).

¹HNMR (400 MHz, DMSO-*d₆*) δ(ppm): 10.71 (s, 1H), 7.48-7.46 (d, 1H), 7.40-7.38 (t, 1H), 7.33-7.31 (d, 1H), 4.06-4.01 (m, 1H), 2.96 (s, 1H), 2.71 (s, 1H), 2.25 (s, 3H), 2.18 (s ,3H), 1.99 (s, 2H), 1.88-1.86 (d, 1H), 1.75-1.71 (t, 1H), 1.61-1.52 (m, 1H), 1.40-1.30 (m, 1H).

Molecular formula: C₁₇H₂₀N₆O Accurate molecular weight: 324.17 LC-MS (Pos, m/z)=325.14[M+H]⁺.

### Example 13: Synthesis of (R)-3,5-dimethyl-2-(3-((1-methylpiperidin-3-yl)amino)-1,2,4-triazin-6-yl)phenol (Compound 202)

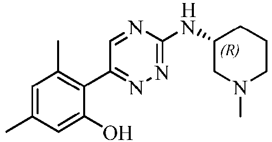

### Step 1: Synthesis of 6-bromo-3-(methylthio)-1,2,4-triazine

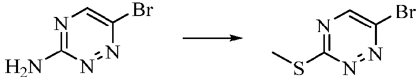

6-bromo-1,2,4-triazin-3-amine (3.0 g, 17.14 mmol, 1.0 eq.) and 1,2-dimethyl disulfide (2.4 g, 25.71 mmol, 1.5 eq.) was added to acetonitrile (20 mL); tert-butyl nitrite (3.5 g, 34.28 mmol, 2.0 eq.) was dropwise added to allow for reaction for 2 h at room temperature; TLC monitoring showed that the reaction was complete; methanol (20.0 mL) was used for quenching; the reaction mixture was concentrated under reduced pressure; and the resulting crude product was purified by silica gel column chromatography (silica gel:100-200 meshes, petroleum ether:ethyl acetate = 5:1) to obtain a product (1.5 g, yield: 42.4%).

### Step 2: Synthesis of 6-(2-(ethoxymethoxy)-4,6-dimethylphenyl)-3-(methylthio)-1,2,4-triazine

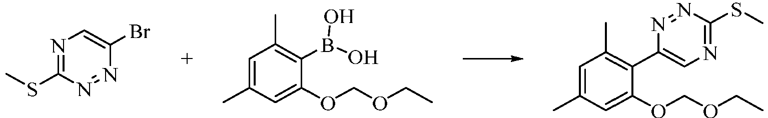

6-bromo-3-(methylthio)-1,2,4-triazine (230.0 mg, 1.11 mmol, 1.0 eq.), (2-(ethoxymethoxy)-4,6-dimethylphenyl)boric acid (250.0 mg, 1.11 mmol, 1.0 eq.), sodium hydrogen carbonate (187.5 mg, 2.22 mmol. 2.0 eq.) and Pd(dppf)Cl₂ (40.8 mg, 0.05 mmol, 0.05 eq.) were added to the mixed solution of 1,4-dioxane (12.0 mL) and water (6.0 mL) to react for 3 h at 110°C under the protection of nitrogen; TLC monitoring showed that the reaction was complete; the system was cooled to room temperature; water (50.0 mL) was added; the mixture was extracted with ethyl acetate (100.0 mL); the organic phase was dried with anhydrous sodium sulfate and filtered; the filtrate was concentrated under reduced pressure; and the resulting crude product was purified by silica gel column chromatography (silica gel: 100-200 meshes, petroleum ether:ethyl acetate = 10:1) to obtain a product (167.0 mg, yield: 49.0%).

### Step 3: Synthesis of 6-(2-(ethoxymethoxy)-4,6-dimethylphenyl)-3-(methylsulfonyl)-1,2,4-triazine

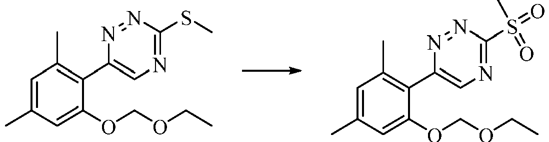

6-(2-(ethoxymethoxy)-4,6-dimethylphenyl)-3-(methylthio)-1,2,4-triazine (167.0 mg, 0.54 mmol, 1.0 eq.) was dissolved in dichloromethane (10.0 mL); metachloroperbenzoic acid (85%) (222.0 mg, 1.08 mmol, 2.0 eq.) , was added to allowed for reaction for 1 h at room temperature; TLC monitoring showed that the reaction was complete; saturated sodium hydrogen carbonate in water (50.0 mL) was added; the resulting mixture was extracted with dichloromethane (50.0 mL); the organic phase was dried with anhydrous sodium sulfate and filtered; the filtrate was concentrated under reduced pressure; and the resulting crude product was purified by silica gel column chromatography (silica gel: 100-200 meshes, petroleum ether:ethyl acetate = 5:1-2:1) to obtain a product (137.0 mg, yield: 74.4%).

### Step 4: Synthesis of (R)-3-((6-(2-(ethoxymethoxy)-4,6-dimethylphenyl)-1,2,4-triazin-3-yl)amino)piperidin-1-tert-butyl carboxylate

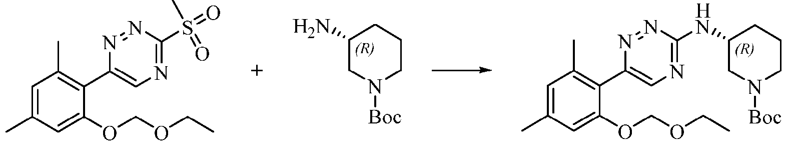

6-(2-(ethoxymethoxy)-4,6-dimethylphenyl)-3-(methylsulfonyl)-1,2,4-triazine (108.0 mg, 0.32 mmol, 1.0 eq.) and (*R*)-3-aminopiperidin-1-tert-butyl carboxylate(128.2 mg, 0.64 mmol, 2.0 eq.) were added to *N,N*-dimethylacetamide (4.0 mL) to react for 16 h at 108°C; and TLC monitoring showed that the reaction was complete. The system was cooled to room temperature, added with water (50.0 mL), and extracted with ethyl acetate (100.0 mL); the organic phase was dried with anhydrous sodium sulfate and filtered; the filtrate was concentrated under reduced pressure; and the resulting crude product was purified by preparative thin-layer chromatography (petroleum ether:ethyl acetate = 1:1) to obtain a product (60.0 mg, yield: 41.0%).

### Step 5: Synthesis of (R)-3,5-dimethyl-2-(3-(piperidin-3-ylamino)-1,2,4-triazin-6-yl)phenol

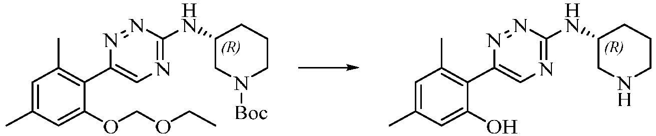

(*R*)-3-((6-(2-(ethoxymethoxy)-4,6-dimethylphenyl)-1,2,4-triazin-3-yl)amino)piperidin-1-tert-butyl carboxylate (60.0 mg, 0.13 mmol, 1.0 eq.) was added to dichloromethane (2.0 mL); the 1,4-dioxane solution of hydrogen chloride (4.0 mol/L)(3.0 mL) was dropwise added to allow for reaction for 1 h at room temperature; and TLC monitoring showed that the reaction was complete. The system was adjusted to pH = 7-8 by using saturated sodium hydrogen carbonate in water, and extracted with dichloromethane (50.0 mL); the organic phase was dried with anhydrous sodium sulfate and filtered; and the filtrate was concentrated under reduced pressure to obtain a product (39.0 mg, yield:99.4%).

### Step 6: Synthesis of (R)-3,5-dimethyl-2-(3-((1-methylpiperidin-3-yl)amino)-1,2,4-triazin-6-yl)phenol

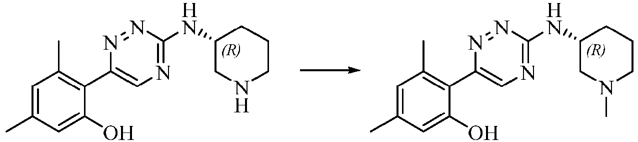

(*R*)-3,5-dimethyl-2-(3-(piperidin-3-ylamino)-1,2,4-triazin-6-yl)phenol (39.0 mg, 0.13 mmol, 1.0 eq.) and formaldehyde in water (37%) (13.7 mg, 0.16 mmol, 1.3 eq.) were added to methanol (5.0 mL), and allowed to react for 0.5 h at room temperature; then, sodium cyanoborohydride (12.2 mg, 0.19 mmol, 1.5 eq.) was added to allow to continue the reaction for 1 h; TLC monitoring showed that the reaction was complete; saturated sodium hydrogen carbonate in water (20.0 mL) was added; the resulting mixture was extracted with dichloromethane (50.0 mL); the organic phase was dried with anhydrous sodium sulfate and filtered; the filtrate was concentrated under reduced pressure; and the resulting crude product was purified by preparative thin-layer chromatography (dichloromethane:methanol = 10:1) to obtain a product (15.0 mg, yield: 50.0%).

¹HNMR (400 MHz, DMSO-*d₆*) δ(ppm): 9.43 (s, 1H), 8.22 (s, 1H), 7.59 (s, 1H), 6.60 (s, 2H), 4.02 (s, 1H), 3.02 (s, 1H), 2.76 (s, 1H), 2.31 (s, 3H), 2.23 (s, 3H), 2.06 (s, 4H), 1.91-1.88 (m, 1H), 1.78-1.74 (m, 1H), 1.59-1.53 (m, 1H), 1.40-1.34 (m, 2H).

Molecular formula: C₁₇H₂₃N₅O Accurate molecular weight: 313.19 LC-MS (Pos, m/z)=314.15[M+H]⁺.

### Example 14: Synthesis of (R)-2-(4-methyl-6-((1-methylpiperidin-3-yl)amino)pyridazin-3-yl)-5-(prop-1-yn-1-yl)phenol (Compound 174):

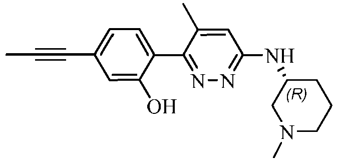

### Step 1: Synthesis of 1-bromo-2-(ethoxymethoxy)-4-ethynylbenzene:

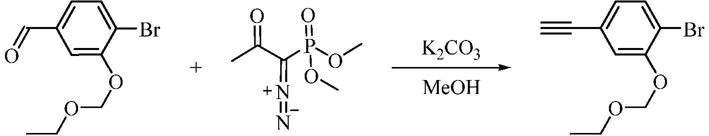

4-bromo-3-(ethoxymethoxy)benzaldehyde (8 g, 30.88 mmol, 1.0 eq.), (1-diazo-2-oxopropyl)dimethyl phosphonate (8.9 g, 46.32 mmol, 1.5 eq.) and anhydrous potassium carbonate (8.5 g, 61.76 mmol, 2.0 eq.) were added to methanol (150 mL), and allowed to react for 3h at room temperature. TLC monitoring showed the absence of raw materials; the reaction mixture was concentrated under reduced pressure, added with water (30 mL), and extracted with ethyl acetate (30 mL × 3); the organic phases were combined, dried with anhydrous sodium sulfate, and filtered; the filtrate was concentrated under reduced pressure; and the resulting crude product was purified by silica gel column chromatography (EA:PE = 1: 150) to obtain a product (3 g, yield: 38.5%).

### Step 2: Synthesis of 1-bromo-2-(ethoxymethoxy)-4-(prop-1-yn-1-yl)benzene

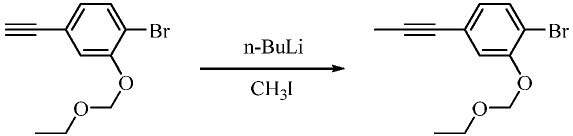

1-bromo-2-(ethoxymethoxy)-4-ethynylbenzene (500 mg, 1.96 mmol, 1.0 eq.) was dissolved in anhydrous THF (10 mL), and then cooled to -70°C; 1.6mol/L n-butyl lithium in a THF solution (1.35 mL, 1.1 eq.) was dropwise added to allow for reaction for 1 h at -75°C- -70°C; and iodomethane (834.6 mg, 5.88 mmol, 3.0 eq.) was dropwise added, after which, the mixture naturally rose to room temperature to allow for reaction for 16 h. LC-MS detection showed that 20% of raw materials were left; saturated ammonium chloride in water (50 mL) was added; the resulting mixture was extracted with ethyl acetate (50 mL × 2); the organic phases were combined, dried with anhydrous sodium sulfate, and filtered; the filtrate was concentrated under reduced pressure; and the resulting crude product was purified by silica gel column chromatography (EA:PE = 1:100) to obtain a product (485 mg, yield: 91.9%).

### Step 3: Synthesis of 2-(2-(ethoxymethoxy)-4-(prop-1-yn-1-yl)phenyl)-4,4,5,5-tetramethyl-1,3,2-dioxaboracyclopentane

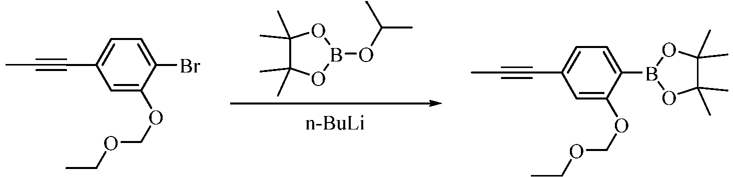

1-bromo-2-(ethoxymethoxy)-4-(prop-1-yn-1-yl)benzene (485 mg, 1.8 mmol, 1.0 eq.) was dissolved in anhydrous THF (10 mL) and cooled to -70°C; 1.6mol/Ln-buty lithium in a THF solution (1. 5 mL, 1.3 eq.) was dropwise added to allow for reaction for 1 h at -70°C; 2-isopropoxy-4,4,5,5-tetramethyl-1,3,2-dioxaboracyclopentane (502.4 mg, 2.7 mmol, 1.5 eq.) was dropwise added, after which the mixture naturally rose to room temperature to allow for reaction for 24 h. LC-MS detection showed the absence of raw materials; saturated ammonium chloride in water (30 mL) was added; the resulting mixture was extracted with ethyl acetate (30 mL × 3); the organic phases were combined, dried with anhydrous sodium sulfate, and filtered; the filtrate was concentrated under reduced pressure; and the resulting crude product was purified by silica gel column chromatography (EA:PE = 1:100-EA=100%) to obtain a product (383 mg, yield: 67.3%).

### Step 4: Synthesis of (R)-6-(2-(ethoxymethoxy)-4-(prop-1-yn-1-yl)phenyl)-5-methyl-N-(1-methylpiperidin-3-yl)pyridazin-3-amine

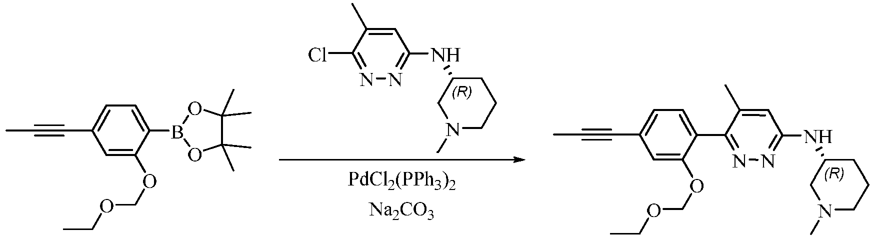

Bistriphenylphosphine palladium dichloride (70.2 mg, 0.1 mmol, 0.1 eq.), and anhydrous sodium carbonate (530 mg, 5 mmol, 5.0 eq.) were added to 1,4-dioxane (10 mL); under the protection of nitrogen, 2-(2-(ethoxymethoxy)-4-(prop-1-yn-1-yl)phenyl)-4,4,5,5-tetramethyl-1,3,2-dioxaboracyclopentane (379.4 mg, 1.2 mmol, 1.2 eq.), (R)-6-chloro-5-methyl-*N*-(1-methylpiperidin-3-yl)pyridazin-3-amine (240.7 mg, 1 mmol, 1.0 eq.) and water (2 mL) were added; and the resulting mixture was heated 100°C to react for 48 h under the protection of nitrogen. Water (30 mL) was added; the reaction mixture was extracted with DCM (30 mL × 2); the organic phases were combined, dried with anhydrous sodium sulfate, and filtered; the filtrate was concentrated under reduced pressure; and the resulting crude product was purified by preparative thin-layer chromatography (MeOH:DCM = 1:10) to obtain a product (150 mg, yield: 38%).

### Step 5: Synthesis of (R)-2-4-methl-6-((1-methylpiperidin-3 -yl)amino)pyridazin-3-yl)-5-(prop-1-yn-1-yl)phenol

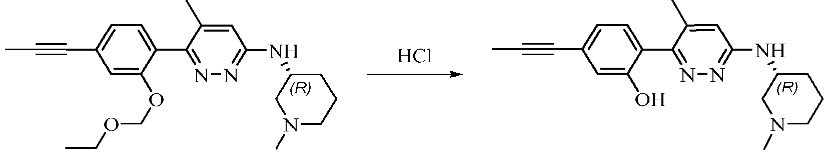

(*R*)-6-(2-(ethoxymethoxy)-4-(prop-1-yn-1-yl)phenyl)-5-methyl-*N*-(1-methylpiperidin-3-yl)pyridazin-3-amine (150 mg, 0.38 mmol, 1.0 eq.) was added to DCM (5 mL); and 4mol/L hydrogen chloride in a dioxane solution (3 mL) was dropwise added to allow for reaction for 1 h at room temperature. TLC detected the absence of raw materials; water (30 mL) was added; the mixture was reversely extracted with DCM (30 mL × 2); the aqueous phase was adjusted to pH = about 8 by adding sodium carbonate, and extracted with DCM (30 mL × 2); the organic phases were combined, dried with anhydrous sodium sulfate, and filtered; and the filtrate was concentrated under reduced pressure to obtain a product (100 mg, yield: 78.2%).

¹HNMR (400 MHz, DMSO-*d*₆) δ(ppm): 10.00 (s, 0.95H), 7.14-7.12 (d, 1H), 6.91-6.89 (d, 2H), 6.68 (s, 1H), 6.61-6.59 (d, 1H), 4.05-4.03 (m, 1H), 2.89-2.87 (m, 1H), 2.56 (m, 1H), 2.22 (s, 3H), 2.05-2.02 (m, 7H), 1.92 (m, 1H), 1.85-1.82 (m, 1H), 1.73-1.70 (m, 1H), 1.56-1.54 (m, 1H), 1.33-1.29 (m, 1H).

Molecular formula: C₂₀H₂₄N₄O Accurate mass: 336.20 LC-MS (Pos, *m*/*z*) =337.30[M+H]⁺.

### Example 15: Synthesis of (R)-2-(5-cyclopropyl-3-((1-methylpiperidin-3-yl)amino)-1,2,4-triazin-6-yl)-5-ethynylphenol (Compound 179):

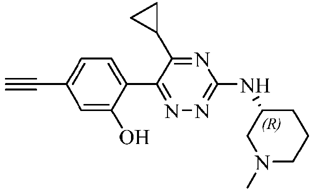

### Step 1: Synthesis of 2-cyclopropyl-2-oxoaldehyde

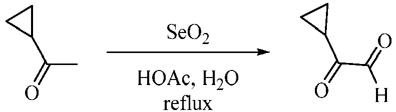

Selenium dioxide (18.5 g, 166.43 mmol, 1.4 eq.), acetic acid (5 g, 83.22 mmol, 0.7 eq.) and water (3.3 g, 184.26 mmol, 1.55 eq.) were added to 1,4-dioxane (75 mL), and heated to reflux for 2 h; 1-cyclopropyl ethanone (10 g, 118.88 mmol, 1.0 eq.) was added; and the resulting mixture was heated to reflux to allow for reaction for 22 h. The mixture was cooled to room temperature and filtered; the filter cake was rinsed with 1,4-dioxane; and the filtrate was used in the next step at a theoretical amount.

### Step 2: Synthesis of 5-cyclopropyl-3-(methylthio)-1,2,4-triazine

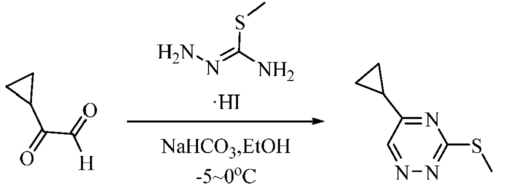

Water (150 mL) was added to the dioxane solution of 2-cyclopropyl-2-oxoaldehyde (118.88 mmol, 1.0 eq.); sodium hydrogen carbonate (20 g, 237.76 mmol, 2.0 eq.) was slowly added; the resulting mixture was cooled to -5-0°C; S-methylisothioaminourea hydriodate (30.5 g, 130.77 mmol, 1.1 eq.) in water (100 mL) was dropwise added; the temperature of the mixture naturally rose to room temperature to allow for reaction for 48 h; TLC monitoring showed the absence of raw materials; the mixture was extracted with ethyl acetate (200 mL × 3); the organic phases were combined, dried with anhydrous sodium sulfate, and filtered; the filtrate was concentrated under reduced pressure; and the resulting crude product was purified by silica gel column chromatography (EA:PE = 1:30-1:10) to obtain a product (12.6 g, two-step yield: 63.3%).

### Step 3: Synthesis of 5-cyclopropyl-3-(methylsulfonyl)-1,2,4-triazine

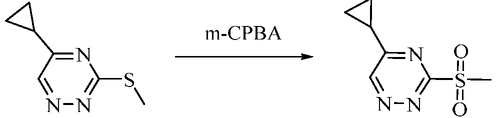

5-cyclopropyl-3-(methylthio)-1,2,4-triazine (2 g, 11.96 mmol, 1.0 eq.) was dissolved in DCM(40 mL); metachloroperbenzoic acid (6 g, 29.9 mmol, 2.5 eq.) with a mass fraction of 85% was portionwise added to allow for reaction for 1 h at room temperature; TCL monitoring showed that no raw material was left; saturated sodium carbonate in water (50 mL) was added; the mixture was stirred for 2 min, and extracted with DCM (50 mL × 3); the organic phases were combined, and washed with 10% sodium thiosulfate in water (50 mL); the organic phases were dried with anhydrous magnesium sulfate, and filtered; and the filtrate was concentrated under reduced pressure to obtain a product (2.1 g, yield: 88.2%).

### Step 4: Synthesis of (R)-3-((5-cyclopropyl-1,2,4-triazin-3-yl)amino)piperidin-1-tert-butyl carboxylate

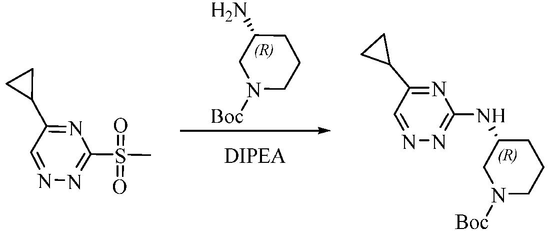

5-cyclopropyl-3-(methylsulfonyl)-1,2,4-triazine (2.1 g, 10.54 mmol, 1.0 eq.) was added to 1,4-dioxane (30 mL); (R)-3-aminopiperidin-1-tert-butyl carboxylate (4.2 g, 21.08 mmol, 2.0 eq.) and DIPEA(4.1 g, 31.62 mmol, 3.0 eq.) were added; and the resulting mixture was heated to 80°C to react for 20 h. TLC monitoring showed that no raw material was left; the reaction mixture was cooled to room temperature, added with water (50 mL), and extracted with ethyl acetate (50 mL × 3); the organic phases were combined, dried with anhydrous sodium sulfate, and filtered; the filtrate was concentrated under reduced pressure; and the resulting crude product was purified by silica gel column chromatography (EA:PE = 1:10-1:2) to obtain a product (2.5 g, yield: 73.5%).

### Step 5: Synthesis of (R)-3-((6-bromo-5-cyclopropyl-1,2,4-triazin-3-yl)amino)piperidin-1-tert-butyl carboxylate:

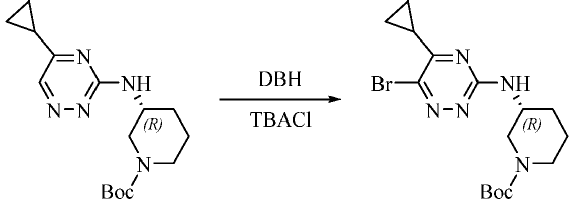

(*R*)-3-((5-cyclopropyl-1,2,4-triazin-3-yl)amino)piperidin-1-tert-butyl carboxylate (2.5 g, 7.83 mmol, 1.0 eq.), tetrabutylammonium chloride (2.2 g, 7.83 mmol, 1.0 eq.), anhydrous potassium carbonate (2.2 g, 15.66 mmol, 2.0 eq.) and dibromohydantoin (1.3 g, 4.7 mmol, 0.6 eq.) were added to acetonitrile (50 mL) to allow for reaction for 48 h at room temperature; the reaction mixture was added with water (50 mL), and extracted with ethyl acetate (50 mL × 3); the organic phases were combined, dried with anhydrous sodium sulfate, and filtered; the filtrate was concentrated under reduced pressure; and the resulting crude product was purified by silica gel column chromatography (EA:PE = 1:10-1:5) to obtain a product (1.5 g, yield: 48.4%).

### Step 6: Synthesis of (R)-6-bromo-5-cyclopropyl-N-(piperidin-3-yl)-1,2,4-triazin-3-amine:

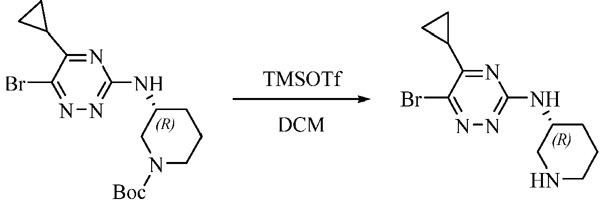

(*R*)-3-((6-bromo-5-cyclopropyl-1,2,4-triazin-3-yl)atnino)piperidin-1-tert-butyl carboxylate(1.5 g, 3.76 mmol, 1.0 eq.) was dissolved in DCM(20 mL); 2,6-dimethylpyridine (2.4 g, 22.56 mmol, 6.0 eq.) was added; the resulting mixture was cooled in an ice water bath; trimethylsilyl trifluoromethanesulfonate (2.5 g, 11.28 mmol, 3.0 eq.) was dropwise added to allow for reaction for 0.5 h; TCL monitoring showed that no raw material was left; the reaction mixture was added with water (50 mL), adjusted to pH = about 8 by using sodium carbonate, and extracted with DCM (50 mL × 3); the organic phases were combined, dried with anhydrous sodium sulfate, and filtered; the filtrate was concentrated under reduced pressure; and the resulting crude product was purified by silica gel column chromatography (MeOH:DCM = 1:100-1:20) to obtain a product (1.1 g, yield: 100%).

### Step 7: Synthesis of (R)-6-bromo-5-cyclopropyl-N-(1-methylpiperidin-3-yl)-1,2,4-triazin-3-amine:

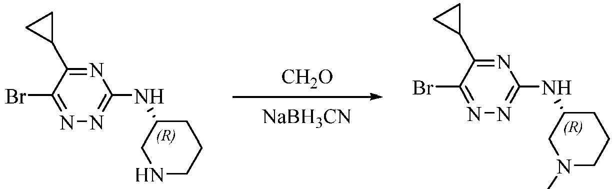

(*R*)-6-bromo-5-cyclopropyl-*N*-(piperidin-3-yl)-1,2,4-triazin-3-amine (1.1 g, 3.76 mmol, 1.0 eq.), acetic acid (225.6 mg, 3.76 mmol, 1.0 eq.) and 37% formaldehyde (305.2 mg, 3.76 mmol, 1.0 eq.) were added to methanol (7 mL) to allow for reaction at room temperature for 1 h; sodium cyanoborohydride (472.5 mg, 7.52 mmol, 2.0 eq.) was added; TLC monitoring showed that no raw material was left; saturated sodium carbonate in water (30 mL) was added; the resulting mixture was extracted with DCM (30 mL × 3); the organic phases were combined, dried with anhydrous sodium sulfate, and filtered; the filtrate was concentrated under reduced pressure; and the resulting crude product was purified by silica gel column chromatography (MeOH:DCM = 1:100-1:20) to obtain a product (776 mg, yield: 66.3%).

### Step 8: Synthesis of (R)-4-(5-cyclopropyl-3-((1-methylpiperidin-3-yl)amino)-1,2,4-triazin-6-yl)-3-(ethoxymethoxy)benzaldehyde:

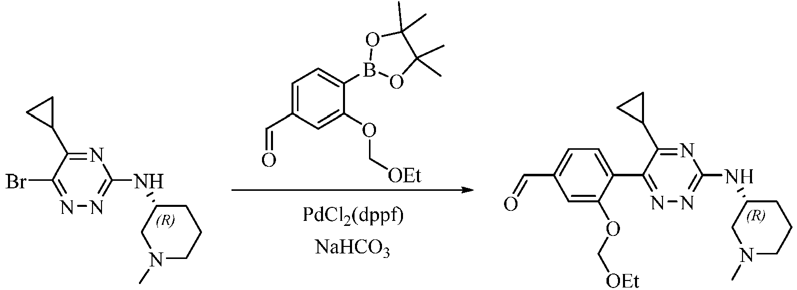

(*R*)-6-bromo-5-cyclopropyl-N-(1-methylpiperidin-3-yl)-1,2,4-triazin-3-atnine (776 mg, 2.47 mmol, 1.0 eq.), 3-(ethoxymethoxy)-4-(4,4,5,5-tetratnethyl-1,3,2-dioxaboracyclopentan-2-yl)benzaldehyde (1.1 g, 3.71 mmol, 1.5 eq.), sodium hydrogen carbonate(622.4 mg, 7.41 mmol, 1.0 eq.) and PdCl₂(dppf) (182.9 mg, 0.25 mmol, 0.1 eq.) were dissolved in the mixed solution of 1,4-dioxane (20 mL) and water (5 mL), and heated to 110°C under the protection of nitrogen to react for 2 h; TCL monitoring showed that no raw material was left; the reaction mixture was cooled to room temperature, added with water (50 mL), and extracted with DCM (50 mL × 3); the organic phases were combined, dried with anhydrous sodium sulfate, and filtered; the filtrate was concentrated under reduced pressure; and the resulting crude product was purified by silica gel column chromatography (MeOH:DCM = 1:100-1:20) to obtain a product (1.1 g, yield: 100%).

### Step 9: Synthesis of (R)-5-cyclopropyl-6-(2-(ethoxymethoxy)-4-ethynylphenyl)-N-(1-methylpiperidin-3-yl)-1,2,4-triazin-3-amine:

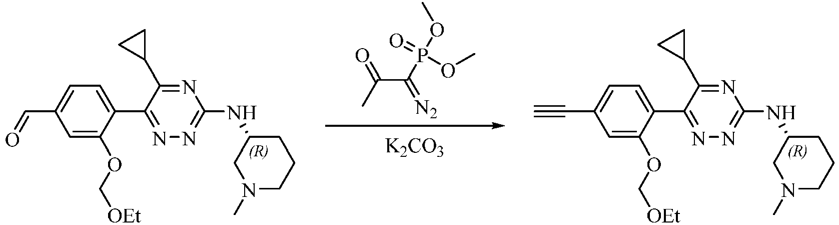

(*R*)-4-(5 -cyclopropyl-3-((1-methylpiperidin-3 -yl)amino)-1,2,4-triazin-6-yl)-3 -(ethoxymethoxy)benzaldehyde (1 g, 2.43 mmol, 1.0 eq.) was added to methanol (10 mL); anhydrous potassium carbonate (671.7 mg, 4.86 mmol, 2.0eq) and (1-diazo-2-oxopropyl)dimethyl phosphonate (699.2 mg, 3.64 mmol, 1.5 eq.) were added to allow for reaction at room temperature for 1 h; LC-MS monitoring showed that no raw material was left; the reaction mixture was concentrated under reduced pressure, added with water (50 mL), and extracted with ethyl acetate (50 mL × 3); the organic phases were combined, dried with anhydrous sodium sulfate, and filtered; and the filtrate was concentrated under reduced pressure to obtain a product (940 mg, yield: 94.9%).

### Step 10: Synthesis of (R)-2-(5-cyclopropyl-3-((1-methylpiperidin-3-yl)amino)-1,2,4-triazin-6-yl)-5-ethynylphenol:

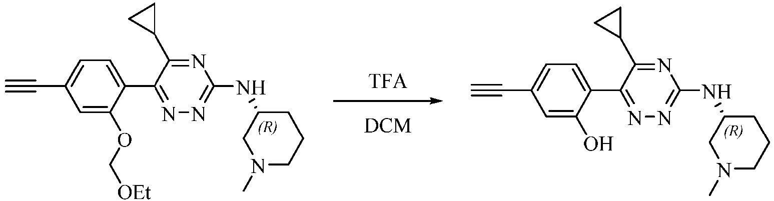

(*R*)*-*5-cyclopropyl-6-(2-(ethoxymethoxy)-4-ethynylphenyl)-*N*-(1-methylpiperidin-3 -yl)-1,2,4-triazin-3 -amine (324 mg, 0.79 mmol, 1.0 eq.) was added to DCM (9 mL); trifluoroacetic acid (1 mL) was dropwise added to allow for reaction at room temperature for 3 h; TLC monitoring showed the absence of raw materials; water (30 mL) was added; sodium carbonate was added to adjust pH to about 8; the resulting mixture was extracted with DCM (30 mL × 3); the organic phases were combined, dried with anhydrous sodium sulfate, and filtered; the filtrate was concentrated under reduced pressure; and the resulting crude product was purified by preparative thin-layer chromatography (MeOH:DCM = 1:7) to obtain a product (130 mg, yield:47.1%).

¹HNMR (400 MHz, DMSO-*d*₆) δ(ppm): 10.1 (s, 1H), 7.74-7.44 (d, 1H), 7.30-7.28 (d, 1H), 7.04 (d, 2H), 4.22 (s, 1H), 3.93-3.87 (m, 1H), 2.87 (m, 1H), 2.64-2.62 (m, 1H), 2.20 (s, 3H), 1.91-1.84 (m, 3H), 1.71-1.64 (m, 2H), 1.54-1.52 (m, 1H), 1.35-1.30 (m, 1H), 1.02-0.97 (m, 4H).

Molecular formula: C₂₀H₂₃N₅O Accurate mass: 249.19 LC-MS (Pos, *m*/*z*) =350.28 [M+H]⁺.

### Example 16: Synthesis of (R)-5-alkynyl-2-(8-((1-methylpiperidin-3-yl)amino)pyridino[2,3-d]pyridazin-5-yl)phenol (Compound 203):

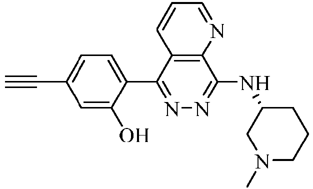

### Step 1: Synthesis of Intermediate 6,7-dihydropyridino[2,3-d]pyridazin-5,8-dione:

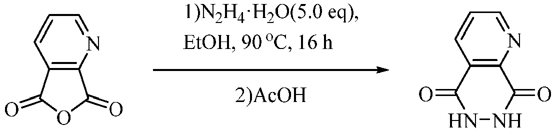

2,3-pyridinedicarboxylic anhydride (10.0 g, 67.06 mmol, 1.0 eq.) was dissolved in ethanol (150 mL); 85% hydrazine hydrate (19.74 g, 335.3 mmol, 5.0 eq.) was added to allow for reaction for 16 h at 90°C; and LC-MS detection showed that the reaction was complete. The reaction mixture was cooled and filtered; the filter cake was dissolved with water (100 mL), and adjusted to acidic pH with acetic acid; a large number of solids were precipitated, and then filtered; the filter cake was dried to obtain a product (7.95 g, yield: 72.6%).

### Step 2: Synthesis of Intermediate 5,8-dicholoropyridino[2,3-d]pyridazine

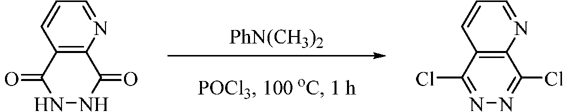

phosphorus oxychloride (70 mL) and *N,N*-dimethylaniline (7 mL) were added to 6,7-dihydropyridino[2,3-*d*]pyridazin-5,8-dione (7.16 g, 43.89 mmol, 1.0 eq.); the temperature of the resulting mixture was increased to 100°C to allow for reaction for 1 h; and TLC detection showed that the reaction was complete. The reaction mixture was concentrated, and poured into ice water. A large number of solids were precipitated, and then filtered; and the filter cake was dissolved with dichloromethane (100 mL), dried with anhydrous magnesium sulfate, and concentrated to obtain a product (6.0 g, yield: 68.3%).

### Step 3: Synthesis of Intermediate 5-chloropyridino[2.3-d]pyridazin-8-ol

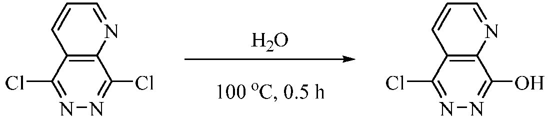

5,8-dicholoropyridino[2,3-*d*]pyridazine (6 g, 30 mmol, 1.0 eq.) was dissolved in 1% hydrochloric acid in water (75 mL) to allow for reaction for 1 h at 100°C; and TLC detection showed that the reaction was complete. The reaction mixture was concentrated, and treated by silica gel column chromatography to obtain a crude product; the crude product was dissolved in 1% NaOH in water (40 mL) to allow for reaction for 1 h at 100°C; the reaction mixture was cooled and filtered; and the filter cake is the product (3.6 g, yield: 66%).

### Step 4: Synthesis of Intermediate 4-(8-hydroxypyridino[2,3-d]pyridazin-5-yl)-3-methoxybenzaldehyde

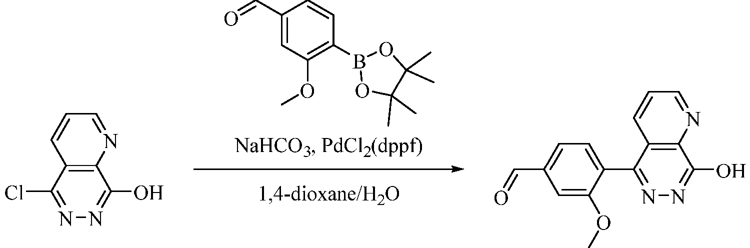

5-chloropyridino[2,3-*d*]pyridazin-8-ol (1.5 g, 8.26 mmol, 1.0 eq.) was dissolved in 1,4-dioxane (20 mL); 3-methoxy-4-(4,4,5,5-tetramethyl-1,3,2-dioxoboracyclopentan-2-yl)benzaldehyde (4.33 g, 16.52 mmol, 2.0 eq.), sodium hydrogen carbonate(1.39 g, 16.52 mmol, 2.0 eq.), PdCl₂(dppf)(604 mg, 0.826 mmol, 0.1 eq.) and water (5 mL) were added to the resulting mixture; under the protection of nitrogen, the temperature was increased to 100°C to allow for reaction for 1 h; and TLC detection showed that the reaction was complete. The reaction mixture was filtered by means of diatomite; the filtrate was added with water (10 mL), and extracted with dichloromethane (20 mL × 3); the organic phases were combined, dried, and concentrated; and the resulting crude product was purified by silica gel column chromatography (petroleum ether:ethyl acetate = 1: 1, dichloromethane:methanol = 100:1-30:1) to obtain a product (1.03 g, yield: 44.4%).

### Step 5: Synthesis of Intermediate 5-(4-alkynyl-2-methoxy)pyridino[2,3-d]pyridazin-8-phenol

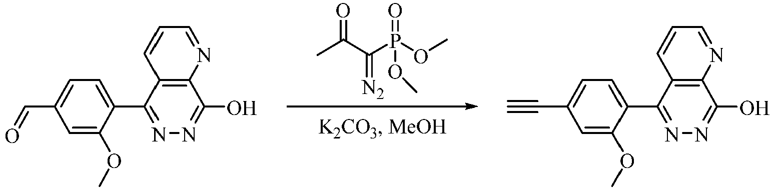

4-(8-hydroxypyridino[2,3-*d*]pyridazin-5-yl)-3-methoxybenzaldehyde (1.03 g, 3.66 mmol, 1.0 eq.) was dissolved in methanol (10 mL); potassium carbonate (1.01 g, 7.32 mmol, 2.0 eq.) and (1-diazo-2-oxopropyl)dimethyl phosphonate (1.05 g, 5.49 mmol, 1.5 eq.) were successively added to the resulting mixture to allow for reaction for 2 h at 25°C; and TLC detection showed that the reaction was basically complete. The reaction mixture was added with water (20 mL), and extracted with DCM (20 mL×3); the organic phases were combined, dried with anhydrous magnesium sulfate, and concentrated; and the resulting crude product was directly used in the next step.

### Step 6: Intermediate 8-chloro-5-(4-alkynyl-2-methoxy)pyridino[2,3-d]pyridazine

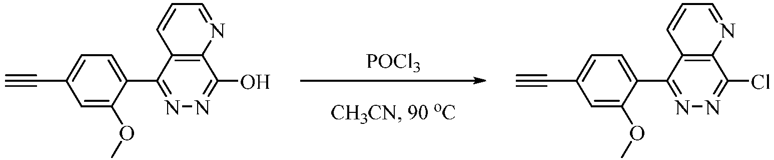

5-(4-alkynyl-2-methoxy)pyridino[2,3-*d*]pyridazin-8-phenol (3.66 mmol, 1.0 eq.) was dissolved in acetonitrile (10 mL); phosphorus oxychloride (1.12 g, 7.32 mmol, 2.0 eq.) was added to the resulting mixture to allow for reaction for 2 h at 100°C; and TLC detection showed that the reaction was complete. The reaction mixture was concentrated, and poured into ice water (10 mL), and extracted with MTBE (20 mL × 3); the organic phases were combined, dried with anhydrous sodium sulfate, and concentrated; and the resulting crude product was purified by silica gel column chromatography (PE: EA = 5:1) to obtain a product (300 mg, two-step yield: 27.8%).

### Step 7: Synthesis of Intermediate 2-(8-chloropyridino[2,3-d]pyridazin-5-yl)-5-ethynylphenol

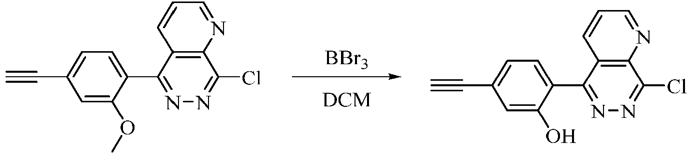

8-chloro-5-(4-alkynyl-2-methoxy)pyridino[2,3-*d*]pyridazine (300 mg, 1.01 mmol, 1.0 eq.) was dissolved in dichloromethane (3 mL); boron tribromide (759 mg, 3.03 mmol, 3.0 eq.) was dropwise added to the resulting mixture to allow for reaction for 30 min at 25°C; and TLC detection showed that the reaction was basically complete. The reaction mixture was poured into saturated sodium hydrogen carbonate in water (10 mL), and extracted with MTBE (10 mL × 3); and the organic phases were combined, dried with anhydrous sodium sulfate, and concentrated to obtain a crude product, which was directly used in a next step.

### Step 8: Synthesis of Compound (R)-3-((5-(4-ethynyl-2-hydroxyphenyl)pyridino[2,3-d]pyridazin-8-yl)amino)piperidin-1-tert-butyl carboxylate

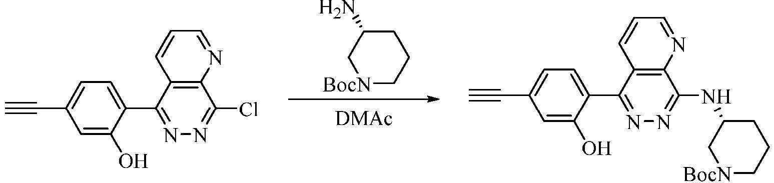

2-(8-chloropyridino[2,3-*d*]pyridazin-5-yl)-5-ethynylphenol (230 mg, 0.816 mmol, 1.0 eq.) was dissolved in DMAc(3 mL); (*R*)-1-tert-butoxycarbonyl-3-aminopiperidine (327 mg, 1.632 mmol, 2.0 eq.) was added to the resulting mixture to allow for reaction for 24 h at 100°C; and LC-MS detection showed that the reaction was basically complete. The reaction mixture was poured into water (10 mL), and filtered; the filter cake was dissolved with dichloromethane (10 mL), dried with anhydrous magnesium sulfate, and concentrated to obtain a crude product, which was directly used in a next step.

### Step 9: Synthesis of Intermediate (R)-5-alkynyl-2-(8-((piperidin-3-yl)atnino)pyridino[2,3-d]pyridazin-5-yl)phenol

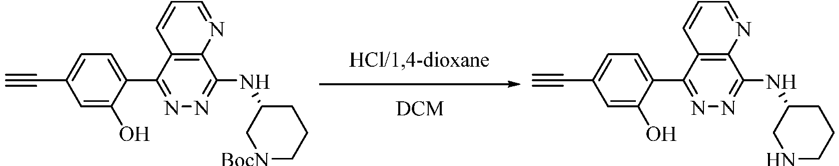

(*R*)-3-((5-(4-ethynyl-2-hydroxyphenyl)pyridino[2,3-*d*]pyridazin-8-yl)amino)piperidin-1-tert-butyl carboxylate(300 mg, 0.673 mmol, 1.0 eq.) was dissolved in dichloromethane (2 mL); the resulting mixture was dripped to the 1,4-dioxane solution of hydrogen chloride (2 mL, 4 mol/L) to allow for reaction for 5 min at 25°C; and TLC detection showed that the reaction was complete. The reaction mixture was added with water (10 mL), and reversely extracted with dichloromethane (10 mL × 2); and the aqueous phases were adjusted to alkaline pH by using saturated sodium hydrogen carbonate in water. The mixture was concentrated to obtain a crude product, which was dissoved with the mixed solution of dichloromethane/methanol, dried with anhydrous magnesium sulfate, filtered, and concentrated to obtain a product (232 mg, three-step yield: 66.5%).

### Step 10: Synthesis of Compound (R)-5-alkynyl-2-(8-((1-methylpiperidin-3-yl)amino)pyridino[2,3-d]pyridazin-5-yl)phenol

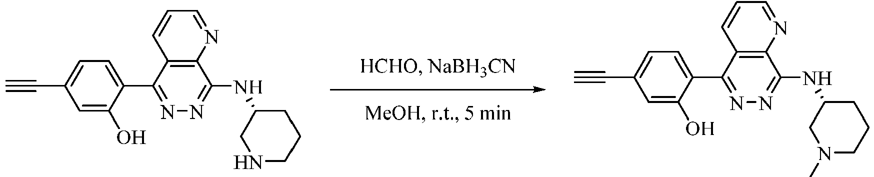

(*R*)-5-alkynyl-2-(8-((piperidin-3-yl)amino)pyridino[2,3-*d*]pyridazin-5-yl)phenol (232 mg, 0.671 mmol, 1.0 eq.) was dissolved in methanol (3 mL); 37% formaldehyde in water (55 mg, 0.671 mmol, 1.0 eq.) and sodium cyanoborohydride (46 mg, 0.738 mmol, 1.1 eq.) were added to the resulting mixture to allow for reaction for 5 min at normal temperature; and TLC detection showed that the reaction was complete. The reaction mixture was concentrated, added with saturated saline (5 mL), and extracted with the mixed solvent of DCM/MeOH = 7/1 (20 mL × 3); the organic phases were combined, dried and concentrated; and the resulting crude product was purified by preparative thin-layer chromatography (DCM:ammonia/MeOH solution (7 mol/L) = 14:1) to obtain a product (40 mg, yield: 16.6%).

¹H-NMR (400 MHz, DMSO-*d*₆) δ(ppm): 10.07 (s, 1H), 9.12-9.11 (d, 1H), 7.93-7.91 (m, 1H), 7.88-7.85 (m, 1H), 7.38 (s, 1H), 7.36 (s, 1H), 7.10 (s, 1H), 7.09 (s, 1H), 4.45 (s, 1H), 4.25 (s, 1H), 2.78-2.66 (m, 1H), 2.40-2.34 (m, 3H), 2.27 (s, 3H), 1.76 (s, 3H), 1.59 (m, 1H).

Molecular formula: C₂₁H₂₁N₅O Accurate molecular weight: 359.17 LC-MS(*m*/*z*): 360.08 [M+H]⁺

### Example 17: Synthesis of (R)-2-(4-cyclopropyl-6-((1-methylpiperidin-3-yl)amino)pyridazin-3-yl)-5-ethynylphenol (Compound 173):

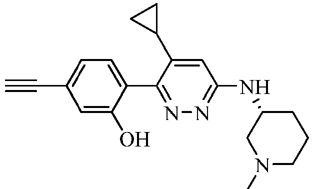

### Step 1: Synthesis of Intermediate 3,6-dicholoro-4-cyclopropylpyridazine

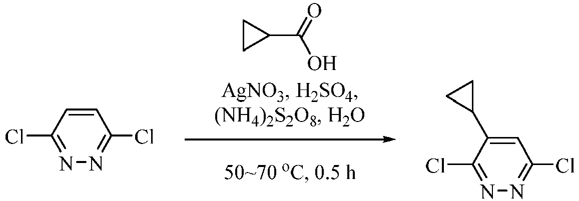

3,6-dicholoropyridazine (20.0 g, 134.2 mmol, 1.0 eq.) was dissolved in water (200 mL); cyclopropanecarboxylic acid (11.56 g, 134.2 mmol, 1.0 eq.) and silver nitrate (22.8 g, 134.2 mmol, 1.0 eq.) were added to the resutling mixture; at 50°C, concentrated sulfuric acid (21.6 mL, 402.6 mmol, 3.0 eq.) was dropwise added to the reaction mixture, after which the temperature was increased to 60°C; and ammonium persulfate (91.88 g, 402.6 mmol, 3.0 eq.) in water (200 mL) was dropwise added to the reaction mixture. After the dropwise addition, the temperature was incrased to 70°C to allow for reaction for 30 min, and TLC detection showed that the reaction was complete. The reaction mixture was adjusted to a pH value of 8 by using 1 mol/L sodium hydroxide in water, and extracted with ethyl acetate (400 mL × 2); the organic phases were combined, dried, and concentrated; and the resulting crude product was purified by silica gel column chromatography (petroleum ether:ethyl acetate = 50:1) to obtain a mixed product (18 g), which was stood overnight to allow for the precipitation of a large number of solids; the solids were filtered; the filter cake was pulped with the mixed solvent of petroleum ether/ethyl acetate to obtain a product (17 g, yield: 67.1%).

### Step 2: Synthesis of (R)-3-((6-chloro-5-cyclopropylpyridazin-3-yl)amino)piperidin-1-tert-butyl carboxylate

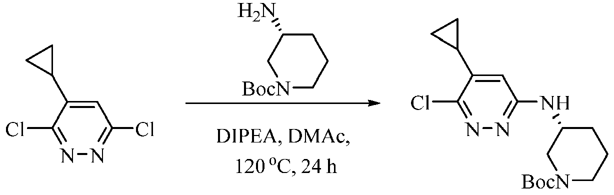

3,6-dicholoro-4-cyclopropylpyridazine (5.0 g, 26.45 mmol, 1.0 eq.) was dissolved in DMAc (50 mL); (R)-1-tert-butoxycarbonyl-3-aminopiperidine (26.5 g, 132.25 mmol, 5.0 eq.) and *N,N*-diisopropylethylamine (10.26 g, 79.35 mmol, 3.0 eq.) were added to the resulting mixture to allow for reaction for 24 h at 120°C; the reaction mixture was poured into water (100 mL), and extracted with MTBE (50 mL × 3); the organic phases were combined, dried, and concentrated; and the resulting crude product was purified by silica gel column chromatography (PE:EA = 10:1-4:1) to obtain a product (2.5 g, yield: 26.8%).

### Step 3: Synthesis of Intermediate (R)-3-((5-cyclopropyl-6-(2-(ethoxymethoxy)-4-formylphenyl)pyridazin-3-yl)amino)pineridin-1-tert-butyl carboxylate

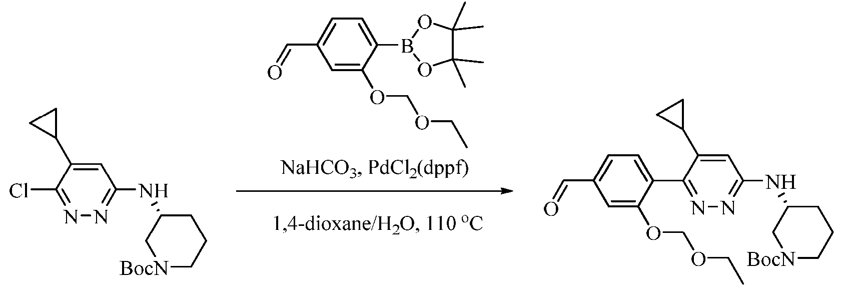

(*R*)-3-((6-chloro-5-cyclopropylpyridazin-3-yl)amino)piperidin-1-tert-butyl carboxylate(1.0 g, 3.95 mmol, 1.0 eq.) was dissolved in 1,4-dioxane (10 mL); 3-(ethoxymethoxy)-4-(4,4,5,5-tetramethyl-1,3,2-dioxaboracyclopentan-2-yl)benzaldehyde (1.45 g, 4.74 mmol, 1.2 eq.), sodium hydrogen carbonate (664 mg, 7.9 mmol, 2.0 eq.), PdCl₂(dppf) (289 mg, 0.395 mmol, 0.1 eq.) and water (2.5 mL) were added to the resulting mixture; under the protection of nitrogen, the temperature was increased to 110°C to allow for reaction for 1 h; and TLC detection showed that the reaction was complete. The reaction mixture was filtered by means of diatomite; the filtrate was added with water (20 mL), and extracted with MTBE (20 mL × 3); the organic phases were combined, dried, and concentrated; and the resulting crude product was purified by silica gel column chromatography (petroleum ether:ethyl acetate = 5:1-2:1) to obtain a product (760 mg, yield: 38.8%).

### Step 4: Synthesis of Intermediate (R)-3-((5-cyclopropyl-6-(2-(ethoxymethoxy)-4-ethynylphenyl)pyridazin-3-yl)amino)piperidin-1-tert-butyl carboxylate

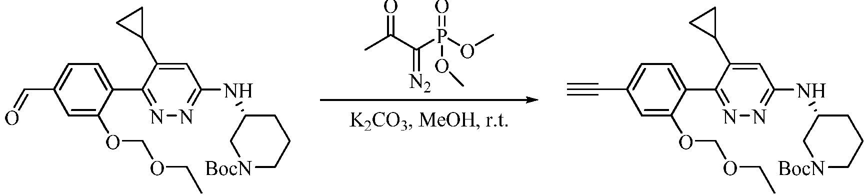

(*R*)-3 -((5 -cyclopropyl-6-(2-(ethoxymethoxy)-4-formylphenyl)pyridazin-3-yl)amino)piperidin-1-tert-butyl carboxylate (760 mg, 1.53 mmol, 1.0 eq.) was dissolved in methanol (8 mL); potassium carbonate (423 mg, 3.06 mmol, 2.0 eq.) and (1-diazo-2-oxopropyl)dimethyl phosphonate (422 mg, 2.3 mmol, 1.5 eq.) were successively added to the resulting mixture to allow for reaction for 5 min at 25°C; and TLC detection showed that the reaction was complete. The reaction mixture was added with water (10 mL), and extracted with dichloromethane (10 mL × 3); the organic phases were combined, dried with anhydrous magnesium sulfate, and concentrated; and the resulting crude product was purified by silica gel column chromatography (petroleum ether:ethyl acetate = 3:1-2:1) to obtain a product (340 mg, yield: 45.2%).

### Step 5: Synthesis of Intermediate (R)-2-(4-cyclopropyl-6-(piperidin-3-ylamino)pyridazin-3-yl)-5-ethynylphenol

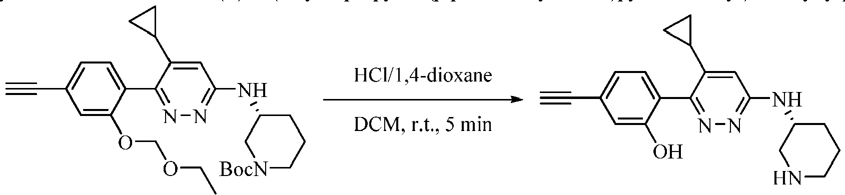

(*R*)-3 -((5 -cyclopropyl-6-(2-(ethoxymethoxy)-4-ethynylphenyl)pyridazin-3 -yl)amino)piperidin-1 -tert-butyl carboxylate (340 mg, 0.69 mmol, 1.0 eq.) was dissolved in dichloromethane (1.5 mL); the resulting mixture was dripped to the 1,4-dioxane solution of hydrogen chloride (1.5 mL, 4 mol/L), to allow for reaction for 5 min at 25°C; and TLC detection showed that the reaction was complete. The reaction mixture was concentrated; the concentrated solution was dissolved in dichloromethane (5 mL), then slowly dripped into saturated sodium hydrogen carbonate in water (20 mL), and extracted with dichloromethane:methanol = 10:1(20 mL × 3); and the organic phases were combined, dried, and concentrated to obtain a product (90 mg, yield: 39.1%).

### Step 6: Synthesis of Compound (R)-2-(4-cyclopropyl-6-((1-methylpiperidin-3-yl)amino)pyridazin-3-yl)-5-ethynylphenol

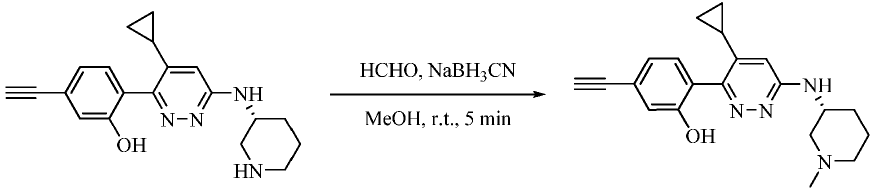

(*R*)-2-(4-cyclopropyl-6-(piperidin-3-ylamino)pyridazin-3-yl)-5-ethynylphenol (90 mg, 0.269 mmol, 1.0 eq.) was dissolved in methanol (1 mL); 37% formaldehyde in water (22 mg, 0.269 mmol, 1.0 eq.) and sodium cyanoborohydride (19 mg, 0.296 mmol, 1.1 eq.) were added to the resulting mixture, to allow for reaction for 5 min at 25°C; and TLC detection showed that the reaction was complete. The reaction mixture was concentrated, added with saturated saline (2 mL), and extracted with the mixed solvent of DCM:MeOH = 10:1 (10 mL × 3); the organic phases were combined and dried; and the resulting crude product was purified by preparative thin-layer chromatography (DCM:MeOH = 7:1) to obtain a product (31 mg, yield: 33.3%).

¹H-NMR (400 MHz, DMSO-*d*₆) δ(ppm): 10.09 (s, 1H), 7.24-7.20 (m, 1H), 7.01-6.99 (m, 2H), 6.59-6.58 (d, 1H), 6.34 (s, 1H), 4.18 (s, 1H), 4.11(s, 1H), 3.01 (m, 1H), 2.76-2.68 (m, 1H), 2.30 (s, 3H), 1.82-1.76 (m, 2H), 1.62-1.53 (m, 2H), 1.36-1.30 (m, 1H), 1.26-1.21 (m, 2H), 0.88-0.85 (m, 2H), 0.63-0.59 (m, 2H).

Molecular formula: C₂₁H₂₄N₄O Accurate molecular weight: 348.20 LC-MS(*m*/*z*): 349.11 [M+H]⁺

### Example 18: Synthesis of (R)-2-(4,5-dicyclopropyl-6-((1-methylpiperidin-3-yl)amino)pyridazin-3-yl)-5-ethynylphenol (Compound 221):

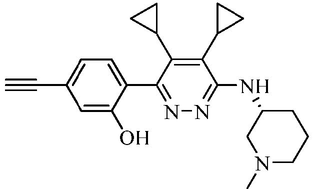

### Step 1: Synthesis of Intermediate 3,6-dicholoro-4,5-dicyclopropylpyridazine:

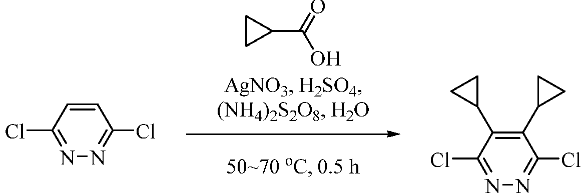

3,6-dicholoropyridazine (8.83 g, 59.24 mmol, 1.0 eq.) was dissolved in water (200 mL); cyclopropanecarboxylic acid (12.75 g, 148.1 mmol, 2.5 eq.) and silver nitrate (25.16 g, 148.1 mmol, 2.5 eq.) were added to the resutling mixture; at 50°C, concentrated sulfuric acid (24 mL, 444.3 mmol, 7.5 eq.) was dropwise added to the reaction mixture, after which the temperature was increased to 60°C; and ammonium persulfate (101 g, 444.3 mmol, 7.5 eq.) in water (200 mL) was dropwise added to the reaction mixture; and TLC detection showed that the reaction was complete. The reaction mixture was adjusted to a pH value of 8 by using 1 mol/L sodium hydroxide in water, and extracted with ethyl acetate (200 mL × 2); the organic phases were combined, dried, and concentrated; and the resulting crude product was purified by silica gel column chromatography (petroleum ether:ethyl acetate = 50:1) to obtain a product (5.69 g, yield: 41.9%).

### Step 2: Synthesis of Intermediate 4-(6-chloro-4,5-dicyclopropylpyridazin-3-yl)-3-(ethoxymethoxy)benzaldehyde

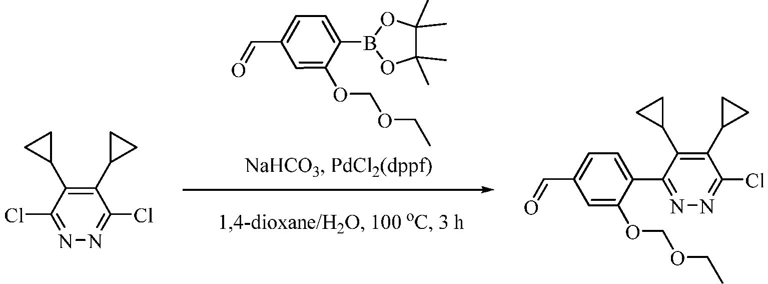

3,6-dicholoro-4,5-dicyclopropylpyridazine (2.0 g, 8.73 mmol, 1.0 eq.) was dissolved in 1,4-dioxane (20 mL); 3-(ethoxymethoxy)-4-(4,4,5,5-tetramethyl-1,3,2-dioxaboracyclopentan-2-yl)benzaldehyde (2.67 g, 8.73 mmol, 1.0 eq.), sodium hydrogen carbonate (1.47 g, 17.46 mmol, 2.0 eq.), PdCl₂(dppf) (639 mg, 0.873 mmol, 0.1 eq.) and water (5 mL) were added to the resulting mixture; under the protection of nitrogen, the temperature was increased to 100°C to allow for reaction for 3 h; and TLC detection showed that the reaction was complete. The reaction mixture was filtered by means of diatomite; the filtrate was added with water (20 mL), and extracted with MTBE (20 mL × 3); the organic phases were combined, dried, and concentrated; and the resulting crude product was purified by silica gel column chromatography (petroleum ether:ethyl acetate = 50:1-10:1) to obtain a product (1.19 g, yield: 36.6%).

### Step 3: Synthesis of (R)-3-((4,5-dicyclopropyl-6-(2-(ethoxymethoxy)-4-formylphenyl)pyridazin-3-yl)amino)piperidin-1-tert-butyl carboxylate

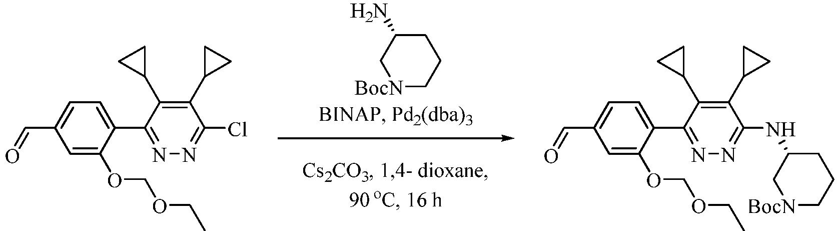

4-(6-chloro-4,5-dicyclopropylpyridazin-3-yl)-3-(ethoxymethoxy)benzaldehyde (1.19 g, 3.19 mmol, 1.0 eq.) was dissolved in 1,4-dioxane (12 mL); (R)-1-tert-butoxycarbonyl-3-atninopiperidine (639 mg, 3.19 mmol, 1.0 eq.), BINAP(199 mg, 0.319 mmol, 0.1 eq.), cesium carbonate(3.12 g, 9.57 mmol, 3.0 eq.) and Pd₂(dba)₃ (292 mg, 0.319 mmol, 0.1 eq.) were added to the resulting mixture, to allow for reaction for 16 h at 90°C under the protection of nitrogen; and TLC detection showed that the reaction was basically complete. The reaction mixture was filtered by means of diatomite; the filtrate was concentrated; and the resulting crude product was purified by silica gel column chromatography (PE:EA = 10:1-1:1) to obtain a product (1.14 g, yield: 66.7%).

### Step 4: Synthesis of Intermediate (R)-3-((4,5-dicyclopropyl-6-(2-(ethoxymethoxy)-4-ethynylphenyl)pyridazin-3-yl)amino)piperidin-1-tert-butyl carboxylate

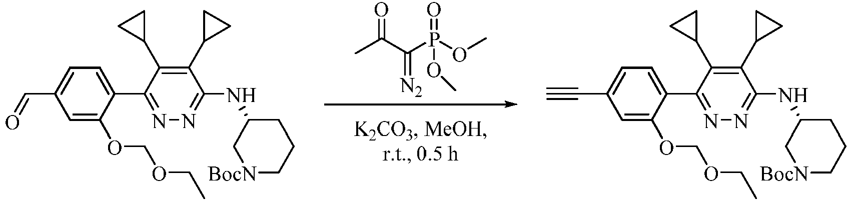

(*R*)-3 -((4,5 -dicyclopropyl-6-(2-(ethoxymethoxy)-4-formylphenyl)pyridazin-3-yl)amino)piperidin-1-tert-butyl carboxylate (1.14 g, 2.12 mmol, 1.0 eq.) was dissolved in methanol (10 mL); potassium carbonate (586 mg, 4.24 mmol, 2.0 eq.) and (1-diazo-2-oxopropyl)dimethyl phosphonate (611 mg, 3.18 mmol, 1.5 eq.) were successively added to the resulting mixture to allow for reaction for 30 min at 25°C; and TLC detection showed that the reaction was complete. The reaction mixture was added with water (20 mL), and extracted with MTBE (20 mL × 3); the organic phases were combined, dried with anhydrous sodium sulfate, and concentrated; and the resulting crude product was purified by silica gel column chromatography (petroleum ether:ethyl acetate = 5:1-3:1) to obtain a product (340 mg, yield:30.1%).

### Step 5: Synthesis of Intermediate (R)-2-(4,5-dicyclopropyl-6-(piperidin-3-ylatnino)pyridazin-3-yl)-5-ethynylphenol

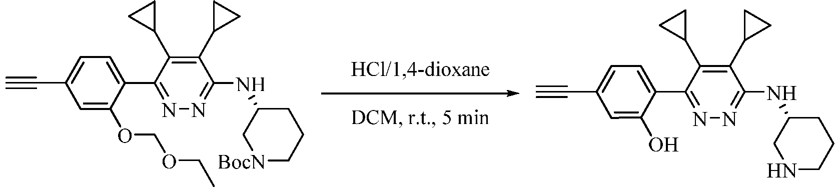

(*R*)-3 -((4,5 -dicyclopropyl-6-(2-(ethoxymethoxy)-4-ethynylphenyl)pyridazin-3-yl)amino)piperidin-1-tert-butyl carboxylate (340 mg, 0.638 mmol, 1.0 eq.) was dissolved in dichloromethane (2 mL); the resulting mixture was dripped to the 1,4-dioxane solution of hydrogen chloride (2 mL, 4 mol/L), to allow for reaction for 30 min at 25°C; and TLC detection showed that the reaction was complete. The reaction mixture was concentrated; the concentrated solution was dissolved in dichloromethane (5 mL), then slowly dripped into a saturated sodium hydrogen carbonate solution (10 mL), and extracted with dichloromethane methanol = 10:1(20 mL × 3); and the organic phases were combined, dried, and concentrated to obtain a product (120 mg, yield: 50.2%).

### Step 6: Synthesis of Compound (R)-2-(4,5-dicyclopropyl-6-((1-methylpiperidin-3-yl)atnino)pyridazin-3-yl)-5-ethynylphenol

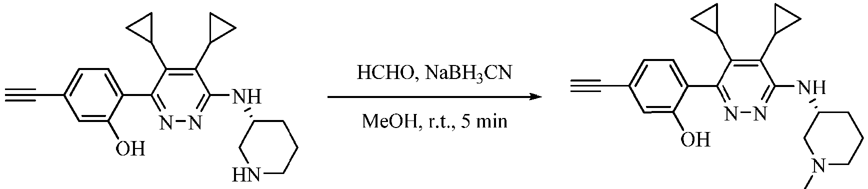

(*R*)-2-(4,5-dicyclopropyl-6-(piperidin-3-ylamino)pyridazin-3-yl)-5-ethynylphenol (120 mg, 0.32 mmol, 1.0 eq.) was dissolved in methanol (1 mL); 37% formaldehyde in water (26 mg, 0.32 mmol, 1.0 eq.) and sodium cyanoborohydride (22 mg, 0.35 mmol, 1.1 eq.) were added to the resulting mixture, to allow for reaction for 5 min at 25°C; and TLC detection showed that the reaction was complete. The reaction mixture was concentrated, added with saturated saline (2 mL), and extracted with the mixed solvent of DCM:MeOH = 10:1 (10 mL × 3); the organic phases were combined and dried; and the resulting crude product was purified by preparative thin-layer chromatography (DCM:MeOH = 8:1) to obtain a product (30 mg, yield: 24.2%).

¹H-NMR (400 MHz, DMSO-*d₆*) δ(ppm): 10.14 (s, 1H), 7.25-7.23 (m, 1H), 6.98-6.96 (m, 2H), 5.82 (s, 1H), 4.38-4.36 (m, 1H), 4.17 (s, 1H), 3.09-3.00 (m, 1H), 2.75-2.68 (m, 1H), 2.43 (s, 3H), 2.34-2.33 (m, 1H), 2.01-1.94 (m, 1H), 1.81 (m, 2H), 1.63 (m, 3H), 1.26-1.24 (m, 1H), 1.16-1.14 (m, 2H), 0.63-0.60 (m, 4H), 0.25-0.20 (m, 2H). Molecular formula: C₂₄H₂₈N₄O Accurate molecular weight: 388.23 LC-MS(*m*/*z*): 389.15 [M+H]⁺

### Example 19: Synthesis of (R)-5-ethynyl-2-(4-((1-(2-hydroxyethyl)piperidin-3-yl)amino)phthalazin-1-yl)phenol (Compound 76)

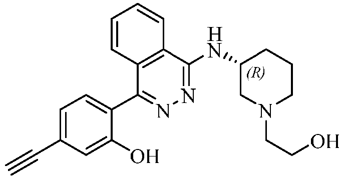

### Step 1: Synthesis of (R)-2-(3-((4-chlorophthalazin-1-yl)amino)piperidin-1-yl)ethan-1-ol

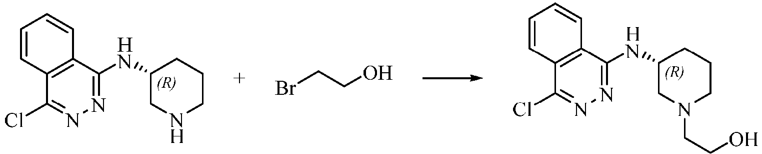

(*R*)-4-chloro-*N*-(piperidin-3-yl)phthalazin-1-amine (1.5 g, 5.70 mmol, 1.0 eq.), 2-bromo-1-ol (1.0 g, 8.56 mmol, 1.5 eq.) and triethylamine (1.15 g, 11.41 mmol, 2.0 eq.) were added to dichloromethane (15.0 mL), and stirred for 12 h at 50°C. TLC monitoring showed that the reaction was complete; saturated ammonium chloride in water (100.0 mL) was added; the resulting mixture was extracted with dichloromethane (100.0 mL); the organic phase was dried with anhydrous sodium sulfate and filtered; the filtrate was concentrated under reduced pressure; and the resulting crude product was purified by silica gel column chromatography (dichloromethane:methanol = 50:1-20:1) to obtain a product (1.2 g, yield: 68.5%).

### Step 2: Synthesis of (R)-3-(ethoxymethoxy)-4-(4-((1-(2-hydroxyethyl)piperidin-3-yl)amino)phthalazin-1-yl)benzaldehyde

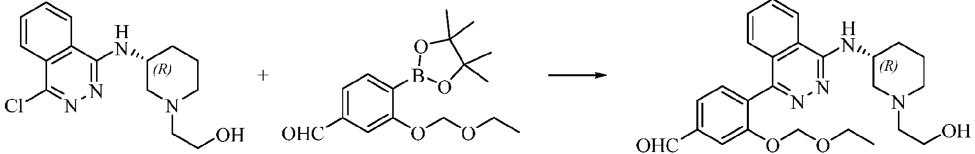

(*R*)-2-(3-((4-chlorophthalazin-1-yl)amino)piperidin-1-yl)ethan-1-ol (500.0 mg, 1.62 mmol, 1.0 eq.), 3-(ethoxymethoxy)-4-(4,4,5,5-tetratnethyl-1,3,2-dioxaboracyclopentan-2-yl)benzaldehyde (598.7 mg, 1.94 mmol, 1.2 eq.), sodium hydrogen carbonate (273.8 mg, 3.25 mmol, 2.0 eq.) and Pd(dppf)Cl₂ (59.6 mg, 0.08 mmol, 0.05 eq.) were added to the mixed solution of 1,4-dioxane (10.0 mL) and water (5.0 mL); nitrogen replacement was conducted for 2 min; the reaction was allowed to occur for 3 h at 110°C; TLC monitoring showed that the reaction was complete; the reaction mixture was added with water (50.0 mL), and extracted with ethyl acetate (100.0 mL); the organic phase was dried with anhydrous sodium sulfate and filtered; the filtrate was concentrated under reduced pressure; and the resulting crude product was purified by silica gel column chromatography (dichloromethane:methanol = 80:1-10:1) to obtain a product (400.0 mg, yield:54.4%).

### Step 3: Synthesis of (R)-2-(3-((4-(2-(ethoxymethoxy)-4-ethynylphenyl)phthalazin-1-yl)amino)piperidin-1-yl)ethan-1-ol

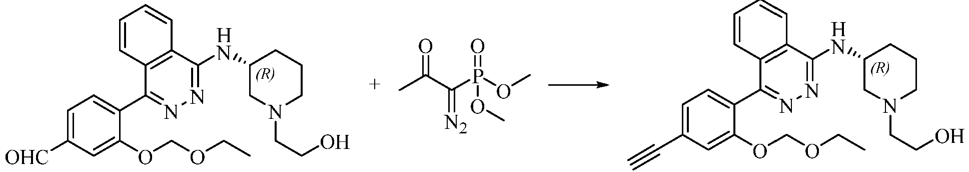

(*R*)-3-(ethoxymethoxy)-4-(4-((1-(2-hydroxyethyl)piperidin-3-yl)amino)phthalazin-1-yl)benzaldehyde (395.0 mg, 0.87 mmol, 1.0 eq.), (1-diazo-2-oxopropyl)dimethyl phosphonate (252.6 mg, 1.31 mmol, 1.3 eq.) and anhydrous potassium carbonate (242.1 mg, 1.75 mmol, 2.0 eq.) were added to methanol (10.0 mL) to allow for reaction for 2 h at room temperature; and TLC monitoring showed that the reaction was complete. The system was concentrated under reduced pressure; and the resulting crude product was purified by silica gel column chromatography (dichloromethane:methanol = 50:1-10:1) to obtain a product (293.0 mg, yield:74.9%).

### Step 4: Synthesis of (R) -5-ethynyl-2-(4-((1-(2-hydroxyethyl)piperidin-3-yl)amino)phthalazin-1-yl)phenol

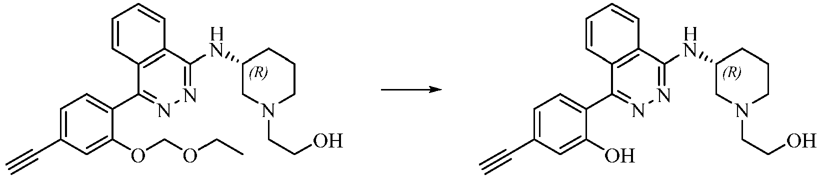

(*R*)-2-(3-((4-(2-(ethoxymethoxy)-4-ethynylphenyl)phthalazin-1-yl)amino)piperidin-1-yl)ethan-1-ol (293.0 mg, 0.65 mmol, 1.0 eq.) was added to dichloromethane (4.0 mL); the 1,4-dioxane solution of hydrogen chloride (4.0 mol/L, 3.0 mL) was dropwise added to allow for reaction for 2 h at room temperature; and TLC monitoring showed that the reaction was complete. The system was adjusted to pH = 8-9 by using saturated sodium carbonate in water, and extracted with dichloromethane (100.0 mL); the organic phase was dried with anhydrous sodium sulfate and filtered; and the filtrate was concentrated under reduced pressure to obtain a product (100.0 mg, yield:39.3%).

¹HNMR(400MHz, DMSO-*d₆*)δ(ppm): 10.00 (s, 1H), 8.40-8.38 (d, *J*=8 Hz, 1H), 7.87-7.77 (m, 2H), 7.47-7.45 (d, *J*=8 Hz, 1H), 7.30-7.28 (d, *J*=8 Hz, 1H), 7.18 (s, 1H), 7.08-7.06 (m, 2H), 4.48 (s, 2H), 4.24 (s, 1H), 3.58 (s, 2H), 3.38-3.36 (m, 2H), 2.94 (s, 1H), 2.67-2.61 (m, 1H), 2.33-1.99 (m, 3H), 1.79-1.52 (m, 3H).

Molecular formula: C₂₃H₂₄N₄O₂ Accurate molecular weight: 388.19 LC-MS(Pos, *m*/*z*)=389.14[M+H]⁺.

### Example 20: Synthesis of (R)-2-(3-((4-(4-ethynyl-2-hydroxyphenyl)phthalazin-1-yl)amino)piperidin-1-yl)acetic acid hydrochloride (hydrochloride of Compound 257)

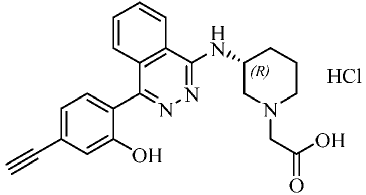

### Step 1: Synthesis of (R)-4-chloro-N (piperidin-3-yl)phthalazin-1-amine

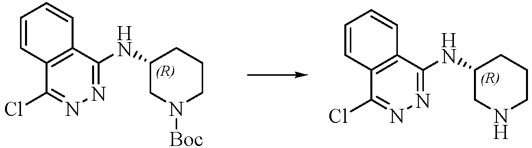

Tert-butyl(*R*)-3-((4-chlorophthalazin-1-yl)amino)piperidin-1-formate (5.0 g, 13.78 mmol, 1.0 eq.) was added to dichloromethane (50.0 mL); the 1,4-dioxane solution of hydrogen chloride (4.0 mol/L) (10.0 mL) was dropwise added to allow for reaction at room temperature for 2 h; TLC monitoring showed that the reaction was complete; the system was adjusted to pH = 8-9 by using saturated sodium carbonate in water, and extracted with dichloromethane (100.0 mL); the organic phase was dried with anhydrous sodium sulfate and filtered; and the filtrate was concentrated under reduced pressure to obtain a product (3.5 g, yield: 96.6%).

### Step 2: Synthesis of (R)-2-(3-((4-chlorophthalazin-1-yl)amino)piperidin-1-yl)ethyl acetate

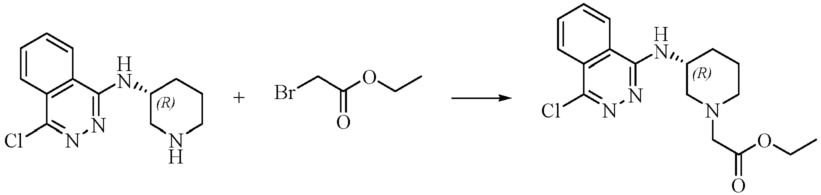

(*R*)-4-chloro-*N*-(piperidin-3-yl)phthalazin-1-amine (2.0 g, 7.61 mmol, 1.0 eq.), bromoethyl acetate (2.0 g, 12.18 mmol, 1.6 eq.) and triethylamine (1.54 g, 15.22 mmol, 2.0 eq.) were added to dichloromethane (20.0 mL), and stirred for 12 h at room temperature. TLC monitoring showed that the reaction was complete; saturated ammonium chloride in water (100.0 mL) was added; the resulting mixture was extracted with dichloromethane (100.0 mL); the organic phase was dried with anhydrous sodium sulfate and filtered; the filtrate was concentrated under reduced pressure; and the resulting crude product was purified by silica gel column chromatography (heptane:ethyl acetate = 5:1-1:1) to obtain a product (2.4 g, yield: 90.5%).

### Step 3: Synthesis of (R)-2-(3-((4-(2-(ethoxymethoxy)-4-formylphenyl)phthalazin-1-yl)amino)piperidin-1-yl)ethyl acetate

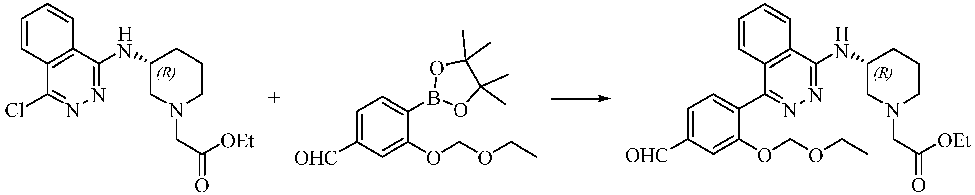

(*R*)-2-(3-((4-chlorophthalazin-1-yl)amino)piperidin-1-yl)ethyl acetate (1.2 g, 3.44 mmol, 1.0 eq.), 3-(ethoxymethoxy)-4-(4,4,5,5-tetramethyl-1,3,2-dioxaboracyclopentan-2-yl)benzaldehyde (1.36 g, 4.47 mmol, 1.3 eq.), sodium hydrogen carbonate (577.9 mg, 6.88 mmol, 2.0 eq.) and Pd(dppf)Cl₂ (125.8 mg, 0.17 mmol, 0.05 eq.) were added to the mixed solution of 1,4-dioxane (12.0 mL) and water (6.0 mL); nitrogen replacement was conducted for 2 min; the reaction was allowed to occur for 3 h at 110°C; TLC monitoring showed that the reaction was complete; the reaction mixture was added with water (50.0 mL), and extracted with ethyl acetate (100.0 mL); the organic phase was dried with anhydrous sodium sulfate and filtered; the filtrate was concentrated under reduced pressure; and the resulting crude product was purified by silica gel column chromatography (dichloromethane:methanol = 80:1-40:1) to obtain a product (1.1 g, yield: 65%).

### Step 4: Synthesis of (R)-2-(3-((4-(2-(ethoxymethoxy)-4-ethynylphenyl)phthalazin-1-yl)amino)piperidin-1-yl)ethyl acetate

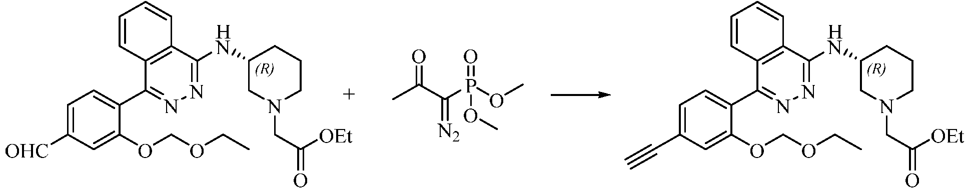

(*R*)-2-(3-((4-(2-(ethoxymethoxy)-4-formylphenyl)phthalazin-1-yl)amino)piperidin-1-yl)ethyl acetate (1.1 g, 2.23 mmol, 1.0 eq.), (1-diazo-2-oxopropyl)dimethyl phosphonate (557.6 mg, 2.90 mmol, 1.3 eq.) and anhydrous potassium carbonate (617.2 mg, 4.46 mmol, 2.0 eq.) were added to methanol (15.0 mL) to allow for reaction at room temperature for 2 h; TLC monitoring showed that the reaction was complete; the system was concentrated under reduced pressure; and the resulting crude product was purified by silica gel column chromatography (dichloromethane:methanol = 80:1-50:1) to obtain a product (550.0 mg, yield: 51.9%).

### Step 5: Synthesis of (R)-2-(3-((4-(4-ethynyl-2-hydroxyphenyl)phthalazin-1-yl)amino)piperidin-1-yl)ethyl acetate

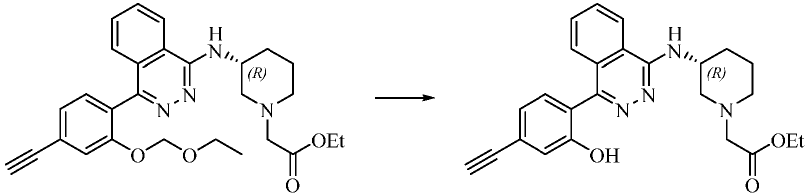

(*R*)-2-(3-((4-(2-(ethoxymethoxy)-4-ethynylphenyl)phthalazin-1-yl)amino)piperidin-1-yl)ethyl acetate (528.0 mg, 1.11 mmol, 1.0 eq.) was added to dichloromethane (5.0 mL); the 1,4-dioxane solution of hydrogen chloride (4.0 mol/L, 5.0 mL) was dropwise added to allow for reaction for 2 h at room temperature; and TLC monitoring showed that the reaction was complete. The system was adjusted to pH = 8-9 by using saturated sodium carbonate in water, and extracted with dichloromethane (100.0 mL); the organic phase was dried with anhydrous sodium sulfate and filtered; the filtrate was concentrated under reduced pressure; and the resulting crude product was purified by silica gel column chromatography (dichloromethane:methanol = 80:1) to obtain a product (370.0 mg, yield:79.9%).

### Step 6: Synthesis of (R)-2-(3-((4-(4-ethynyl-2-hydroxyphenyl)phthalazin-1-yl)amino)piperidin-1-yl)acetic acid hydrochloride

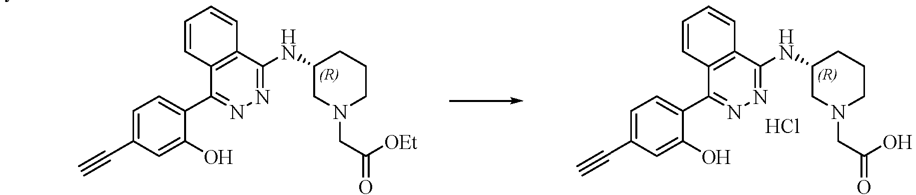

(*R*)-2-(3-((4-(4-ethynyl-2-hydroxyphenyl)phthalazin-1-yl)atnino)piperidin-1-yl)ethyl acetate (370.0 mg, 0.88 mmol, 1.0 eq.) was added to methanol (10.0 mL); lithium hydroxide monohydrate (111.8 mg, 2.66 mmol, 3.0 eq.) in water (5.0 mL) was dropwise added to allow for reaction at room temperature for 2 h; TLC monitoring showed that the reaction was complete; hydrochloric acid (2.0 mol/L) was dropwise added to the system to adjust pH to 2, after which white solids were precipitated and then filtered; and the filter cake was dried to obtain a product (365.0 mg, yield:93.8%).

¹HNMR(400MHz, DMSO-*d₆*)δ(ppm): 10.72 (s, 1H), 9.46 (s, 1H), 9.08 (s, 1H), 8.16-8.04 (m, 2H), 7.70-7.68 (d, *J*=8 Hz, 1H), 7.41-7.39 (d, *J*=8 Hz, 1H), 7.27 (s, 1H), 7.15-7.12 (m, 1H), 4.80 (s, 1H), 4.36 (s, 1H), 4.24-4.14 (m, 2H), 3.72-3.70 (d, *J*=8 Hz, 1H), 3.46-3.20 (m, 3H), 2.08-1.88 (m, 4H).

Molecular formula: C₂₃H₂₃ ClN₄O₃ Accurate molecular weight of free LC-MS (Pos, m/z)=403.11 [M+H]⁺. alkali: 402.17

### Example 21: Synthesis of 5-ethynyl-2-(4-((3-hydroxybenzyl)amino)phthalazin-1-yl)phenol (Compound 268)

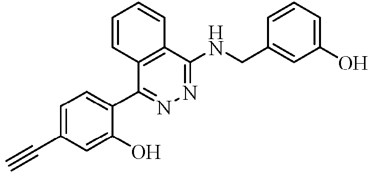

### Step 1: Synthesis of 3-(((4-chlorophthalazin-1-yl)amino)methyl)phenol

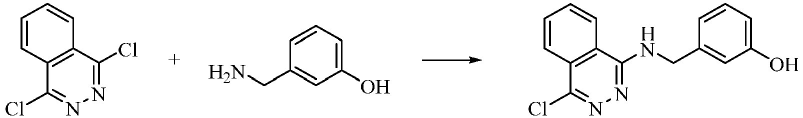

1,4-dicholorophthalazine (1.0 g, 5.02 mmol, 1.0 eq.), 3-(aminomethyl)phenol (618.8 mg, 5.02 mmol, 1.0 eq.) and *N,N*-diisopropylethylamine (1.29 g, 10.04 mmol, 2.0 eq.) were added to *N,N*-dimethylacetamide (10.0 mL), and stirred for 4 h at 120°C. TLC monitoring showed that the reaction was complate. The reaction mixture was added with water (100.0 mL), and extracted with ethyl acetate (100.0 mL); the organic phase was dried with anhydrous sodium sulfate and filtered; the filtrate was concentrated under reduced pressure; and the resulting crude product was purified by silica gel column chromatography (DCM:MeOH = 80:1-30:1) to obtain a product (714.0 mg, yield: 49.7%).

### Step 2: Synthesis of 3-(ethoxymethoxy)-4-(4-((3-hydroxybenzyl)amino)phthalazin-1-yl)benzaldehyde

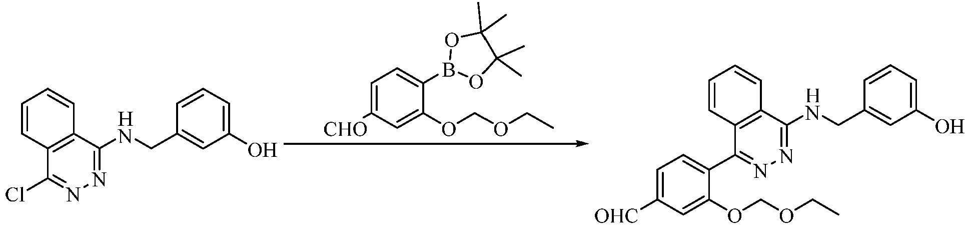

3-(((4-chlorophthalazin-1-yl)amino)methyl)phenol (388.8 mg, 1.36 mmol, 1.0 eq.), 3-(ethoxymethoxy)-4-(4,4,5,5-tetramethyl-1,3,2-dioxaboracyclopentan-2-yl)benzaldehyde (500.0 mg, 1.63 mmol, 1.2 eq.), sodium hydrogen carbonate (228.6 mg, 2.72 mmol, 2.0 eq.) and Pd(dppf)Cl₂ (49.7 mg, 0.06 mmol, 0.05 eq.) were added to the mixed solution of 1,4-dioxane (10.0 mL) and water (5.0 mL); nitrogen replacement was conducted for 2 min; the reaction was allowed to occur for 3 h at 110°C; TLC monitoring showed that the reaction was complete; the reaction mixture was added with water (50.0 mL), and extracted with ethyl acetate (100.0 mL); the organic phase was dried with anhydrous sodium sulfate and filtered; the filtrate was concentrated under reduced pressure; and the resulting crude product was purified by silica gel column chromatography (dichloromethane:methanol = 80:1-50:1) to obtain a product (180.0 mg, yield: 30.8%).

### Step 3: Synthesis of 3-((4-(2-(ethoxymethoxy)-4-ethynylphenyl)phthalazin-1-yl)amino)methyl)phenol

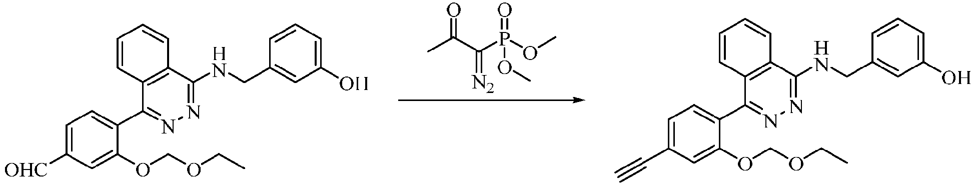

3-(ethoxymethoxy)-4-(4-((3-hydroxybenzyl)amino)phthalazin-1-yl)benzaldehyde (174.0 mg, 0.40 mmol, 1.0 eq.), (1-diazo-2-oxopropyl)dimethyl phosphonate (116.7 mg, 0.60 mmol, 1.5 eq.) and anhydrous potassium carbonate (140.0 mg, 1.01 mmol, 2.5 eq.) were added to methanol (10.0 mL) to allow for reaction for 2 h at room temperature; and TLC monitoring showed that the reaction was complete. The system was concentrated under reduced pressure; and the resulting crude product was purified by silica gel column chromatography (dichloromethane:methanol = 80:1-60:1) to obtain a product (158.0 mg, yield: 91.8%).

### Step 4: Synthesis of 5-ethynyl-2-(4-((3-hydroxybenzyl)amino)phthalazin-1-yl)phenol

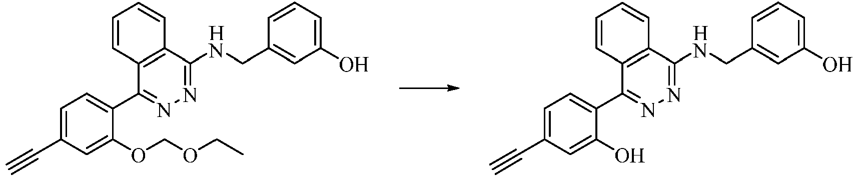

3-((4-(2-(ethoxymethoxy)-4-ethynylphenyl)phthalazin-1-yl)amino)methyl)phenol (158.0 mg, 0.37 mmol, 1.0 eq.) was added to dichloromethane (4.0 mL); the 1,4-dioxane solution of hydrogen chloride (4.0 mol/L, 2.0 mL) was dropwise added to allow for reaction for 1 h at room temperature; and TLC monitoring showed that the reaction was complete. The system was adjusted to pH = 8-9 by using saturated sodium carbonate in water, and extracted with dichloromethane (100.0 mL); the organic phase was dried with anhydrous sodium sulfate and filtered; the filtrate was concentrated under reduced pressure; and the resulting crude product was purified by silica gel column chromatography (dichloromethane:methanol = 80:1-50:1) to obtain a product (100.0 mg, yield: 73.5%).

¹HNMR(400MHz, DMSO-*d₆*)δ(ppm): 9.97 (s, 1H), 9.27 (s, 1H), 8.41-8.39 (d, *J*=8 Hz, 1H), 8.13-8.10 (m, 1H), 7.90-7.86 (m, 1H), 7.82-7.78 (m, 1H), 7.50-7.48 (d, *J*=8 Hz, 1H), 7.30-7.28 (d, *J*=8 Hz, 1H), 7.13-7.05 (m, 3H), 6.86-6.84 (d, *J*=8 Hz, 2H), 6.63-6.60 (m, 1H), 4.78-4.77 (d, *J*=4 Hz, 2H), 4.23 (s, 1H).

Molecular formula: C₂₃H₁₇N₃O₂ Accurate molecular weight: 367.13 LC-MS (Pos, m/z)=368.11[M+H]⁺.

### Example 22: Synthesis of (R)-2-(3-((4-(2-hydroxyl-4-(propyn-1-yl)phenyl)phthalazin-1-yl)amino)piperidin-1-yl)acetic acid (Compound 265)

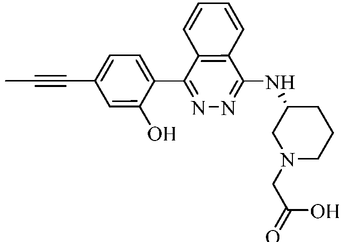

### Step 1: Synthesis of (R)-2-(3-((4-(2-(ethoxymethoxy)-4-(propyn-1-yl)phenyl)phthalazin-1-yl)amino)piperidin-1-yl)ethyl acetate

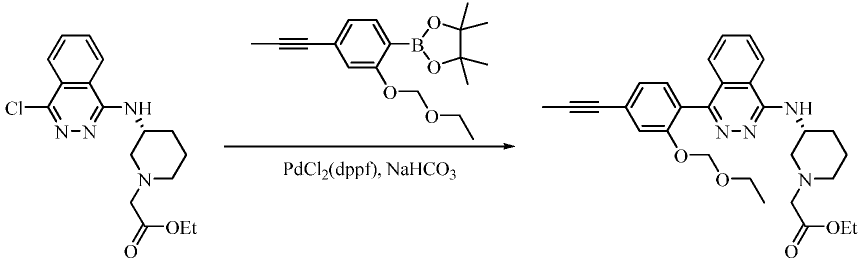

(*R*)-2-(3-((4-chlorophthalazin-1-yl)amino)piperidin-1-yl)ethyl acetate(279.5 mg, 1.20 mmol, 1.0 eq.), 2-(2-(ethoxymethoxy)-4-(propyn-1-yl)phenyl)-4,4,5,5-tetramethyl-1,3,2-dioxaboracyclopentane (380 mg, 0.80 mmol, 1.5 eq.), PdCl₂(dppf) (58.6 mg, 0.08 mmol, 0.1 eq.) and sodium hydrogen carbonate(134.6 mg, 1.60 mmol, 2.0 eq.) were added to the mixed solvent of 1,4-dioxane (4 mL) and water (1 mL), and heated to 100°C under the protection of nitrogen, to allow for reaction for 4 h. LC-MS monitoring showed that the reaction was complete; the reaction mixture was cooled to room temperature, added with water (5 mL), and extracted with a mixed solvent (DCM:MeOH = 10:1, 5 mL × 3); the organic phases were combined, dried with anhydrous sodium sulfate, and filtered; and the filtrate was concentrated under reduced pressure, and purified by preparative thin-layer chromatography (DCM:MeOH = 10:1) to obtain a product (310.2 mg, yield: 77.0%).

### Step 2: Synthesis of (R) -2-(3-((4-(2-hydroxyl-4-(propyn-1-yl)phenyl)phthalazin-1-yl)atnino)piperidin-1-yl)ethyl acetate

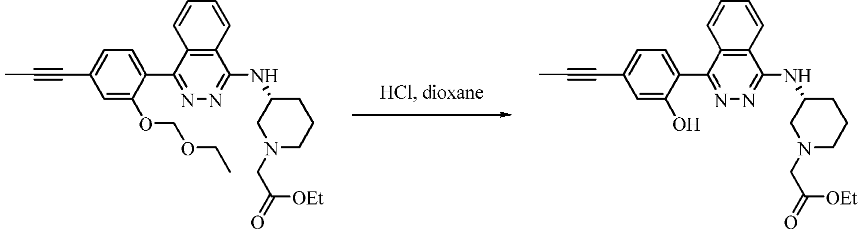

(*R*)-2-(3-((4-(2-(ethoxymethoxy)-4-(propyn-1-yl)phenyl)phthalazin-1-yl)amino)piperidin-1-yl)ethyl acetate (310.2 mg, 0.62 mmol, 1.0 eq.) was added to DCM (2.5 mL); and 4mol/L hydrogen chloride in a 1,4-dioxane solution (2.5 mL) was dropwise added to allow for reaction for 1 h at room temperature. LC-MS monitoring showed that the reaction was complete; the reaction mixture was added with water (5 mL), adjusted to a pH value of about 8 by adding sodium hydrogen carbonate, and extracted with a mixed solvent (DCM:MeOH = 10:1, 5 mL × 3); the organic phases were combined, dried with anhydrous sodium sulfate, and filtered; the filtrate was concentrated under reduced pressure; and the resulting crude product was purified by preparative thin-layer chromatography (DCM:MeOH = 10:1) to obtain a product (240 mg, yield: 87.5%).

### Step 3: Synthesis of (R)-2-(3-((4-(2-hydroxyl-4-(propyn-1-yl)phenyl)phthalazin-1-yl)amino)piperidin-1-yl)acetic acid

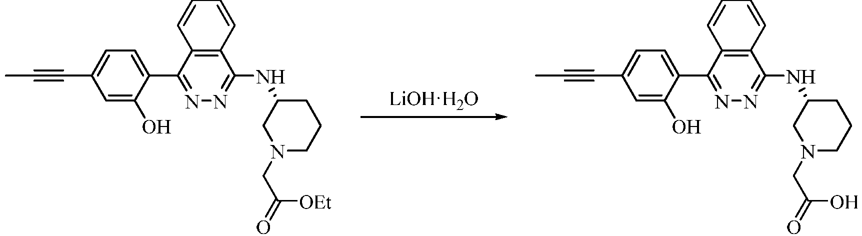

(*R*)-2-(3-((4-(2-hydroxyl-4-(propyn-1-yl)phenyl)phthalazin-1-yl)amino)piperidin-1-yl)ethyl acetate (240 mg, 0.54 mmol, 1.0 eq.) was added to MeOH (4 mL); and water (2 mL) and LiOH·H₂O (68.0 mg, 1.62 mmol, 3.0 eq.) were added to allow for reaction for 2 h at room temperature. LC-MS monitoring showed that the reaction was complete; the reaction mixture was concentrated under reduced pressure; the resulting crude product was added with water (5 mL), and adjusted to a pH value of about 2 by adding 2N HCl, by which solids were precipitated. The solids were dissolved by addition of DMAc(3 mL), and purified by reversed-phase column chromatography (C18 column, MeCN:H₂O = 0-4:1, 30 min) to obtain a product (129.6 mg, yield: 57.6%).

¹HNMR (400 MHz, DMSO-*d*₆) δ(ppm): 8.43 (d, *J =* 8.4 Hz, 1H), 7.90-7.80 (m, 2H), 7.51-7.49 (m, 2H), 7.25 (d, *J* = 7.6 Hz, 1H), 7.03 (m, 1H), 6.98-6.96 (m, 1H), 4.61 (s, 1H), 3.52 (s, 2H), 3.43 (d, *J =* 8.0 Hz, 1H), 3.13-3.10 (m, 1H), 2.73-2.68 (m, 2H), 2.08-2.02 (m, 5H), 1.88-1.85 (m, 1H), 1.78-1.75 (m, 1H), 1.64-1.61 (m, 1H).

Molecular formula: C₂₄H₂₄N₄O₃ Accurate molecular weight: 416.18 LC-MS (Pos, m/z) =417.11[M+H]⁺.

### Example 23: Synthesis of (R)-2-(4-((1-(2-(methylsulfonyl)ethyl)piperidin-3-yl)amino)phthalazin-1-yl)-5-(propyn-1-yl)phenol (Compound 266)

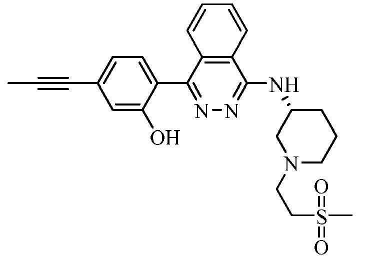

### Step 1: Synthesis of (R)-4-chloro-N-(piperidin-3-yl)phthalazin-1-amine:

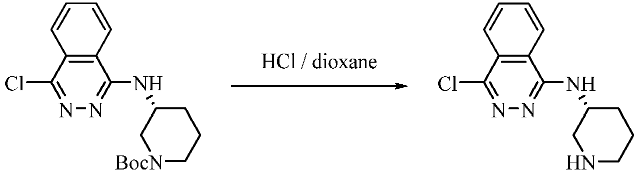

(*R*)-3-((4-chlorophthalazin-1-yl)amino)piperidin-1-tert-butyl carboxylate (4.965 g, 13.68 mmol, 1.0 eq.) was dissolved in DCM (25 mL); 4 mol/L hydrogen chloride in a 1,4-dioxane solution (15.0 mL) was dropwise added to allow for reaction for 2 h at room temperature. LC-MS monitoring showed that the reaction was complete; the reaction mixture was added with water (25 mL), adjusted to a pH value of about 8 by adding sodium hydrogen carbonate, and extracted with a mixed solvent (DCM:MeOH = 10: 1, 25 mL × 3); the organic phases were combined, dried with anhydrous sodium sulfate, and filtered; and the filtrate was concentrated under reduced pressure to obtain a product (3.376 g, yield: 93.9%).

### Step 2: Synthesis of (R)-4-(2-(ethoxymethoxy)-4-(propyn-1-yl)phenyl)-N-(piperidin-3-yl)phthalazin-1-amine

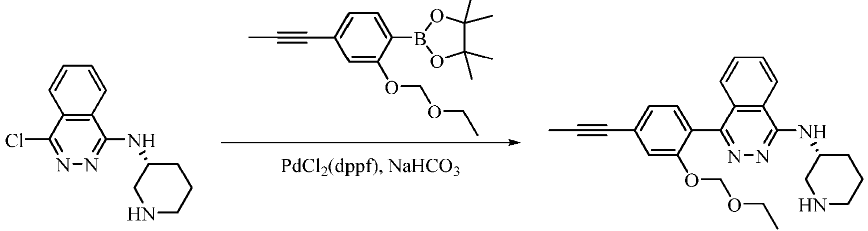

(*R*)-4-chloro-*N*-(piperidin-3-yl)phthalazin-1-amine (210.5 mg, 1.20 mmol, 1.0 eq.), 2-(2-(ethoxymethoxy)-4-(propyn-1-yl)phenyl)-4,4,5,5-tetramethyl-1,3,2-dioxaboracyclopentane (380 mg, 0.80 mmol, 1.5 eq.), PdCl₂(dppf) (58.6 mg, 0.08 mmol, 0.1 eq.) and sodium hydrogen carbonate(134.6 mg, 1.60 mmol, 2.0 eq.) were added to the mixed solvent of 1,4-dioxane (4 mL) and water (1 mL), and heated to 100°C under the protection of nitrogen to allow for reaction for 4 h. LC-MS monitoring showed that the reaction was complete; the reaction mixture was cooled to room temperature, added with water (5 mL), and extracted with a mixed solvent (DCM:MeOH = 10:1, 5 mL × 3); the organic phases were combined, dried with anhydrous sodium sulfate, and filtered; and the filtrate was concentrated under reduced pressure, and purified by preparative thin-layer chromatography (DCM:MeOH = 10: 1) to obtain a product (245.8 mg, yield: 73.6%)

### Step 3: Synthesis of (R)-4-(2-(ethoxymethoxy)-4-(propyn-1-yl)phenyl)-N-(1-(2-(methylsulfonyl)ethyl)piperidin-3-yl)phthalazin-1-amine

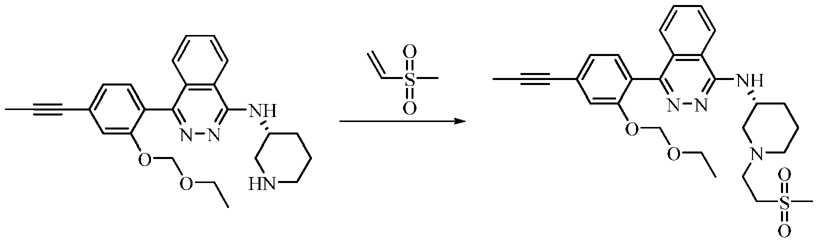

(*R*)-4-(2-(ethoxymethoxy)-4-(propyn-1-yl)phenyl)-*N*-(piperidin-3-yl)phthalazin-1-amine (245.8 mg, 0.59 mmol, 1.0 eq.) was dissolved in DCM (3 mL); and (methylsulfonyl)ethylene (162.2 mg, 1.53 mmol, 2.6 eq.) was dropwise added to allow for reaction for 48 h at room temperature. LC-MS monitoring showed that the reaction was complete; and the reaction mixture was concentrated under reduced pressure, and purified by preparative thin-layer chromatography (DCM:MeOH = 10:1) to obtain a product (154.4 mg, yield: 50.0%).

### Step 4: Synthesis of (R)-2-(4-((1-(2-(methylsulfonyl)ethyl)piperidin-3-yl)atnino)phthalazin-1-yl)-5-(propyn-1-yl)phenol

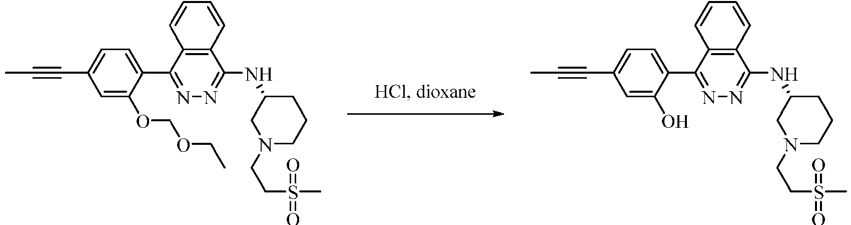

(*R*)-4-(2-(ethoxymethoxy)-4-(propyn-1-yl)phenyl)-*N-*(1-(2-(methylsulfonyl)ethyl)piperidin-3-yl)phthalazin-1-amine (2.8 g, 9.48 mmol, 1.0 eq.) was dissolved in DCM (1.5 mL); and 4mol/L hydrogen chloridethe in a 1,4-dioxane solution (1.5 mL) was dropwise added to allow for reaction for 2 h at room temperature. LC-MS monitoring showed that the reaction was complete; the reaction mixture was added with water (1.5 mL), adjusted to a pH value of about 8 by adding sodium hydrogen carbonate, and extracted with a mixed solvent (DCM:MeOH = 10:1, 1.5 mL × 3); the organic phases were combined, dried with anhydrous sodium sulfate, and filtered; and the filtrate was concentrated under reduced pressure, and purified by preparative thin-layer chromatography (DCM:MeOH = 10:1) to obtain a product (57.6 mg, yield: 42.0%).

¹HNMR (400 MHz, DMSO-*d*₆) δ(ppm): 9.90 (s, 1H), 8.38 (d, *J=* 8.4 Hz, 1H), 7.87-7.77 (m, 2H), 7.48 (d, *J=* 8 Hz, 1H), 7.25 (d, *J =* 7.6 Hz, 1H), 7.14-7.12 (m, 1H), 6.99-6.96 (m, 2H), 4.43-4.42 (m, 1H), 3.35-3.33 (m, 2H), 3.18-3.16 (m, 1H), 3.07 (s, 3H), 2.86-2.77 (m, 3H), 2.12-2.08 (m, 5H), 2.00-1.98 (m, 1H), 1.80-1.77 (m, 1H), 1.61-1.52 (m, 2H).

Molecular formula: C₂₅H₂₈N₄O₃S Accurate molecular weight: 464.19 LC-MS (Pos, *m*/*z*) =465.09[M+H]⁺.

### Example 24: Synthesis of (R)-2-(5-methyl-3-(1-methylpiperidin-3-yl)amino)-1,2,4-triazin-6-yl)-5-(prop-1-yn-1yl)phenol (Compound 195)

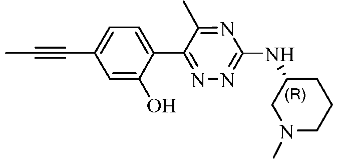

### Step 1: Synthesis of (R)-6-(2-(ethoxymethoxy)-4-(prop-1-yn-1-yl)phenyl)-5-methyl-N-(1-methylpiperidin-3-yl)-1,2,4-triazin-3-atnine

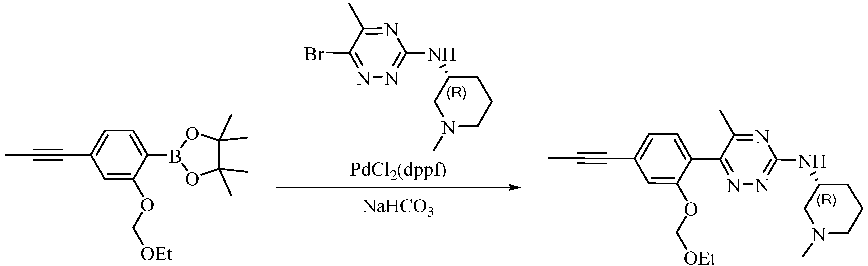

(*R*)-6-bromo-5-methyl-N-(1-methylpiperidin-3-yl)-1,2,4-triazin-3-amine (200 mg, 0.7 mmol, 1.0 eq.), 2-(2-(ethoxymethoxy)-4-(prop-1-yn-1-yl)phenyl)-4,4,5,5-tetramethyl-1,3,2-dioxaboracyclopentane (332 mg, 1.05 mmol, 1.5 eq.), PdCl₂(dppf) (51.2 mg, 0.07 mmol, 0.1 eq.) and sodium hydrogen carbonate (176.4 mg, 2.1 mmol, 3.0 eq.) were added to the mixed solvent of 1,4-dioxane (5 mL) and water (1 mL), and heated to 100°C under the protection of nitrogen to allow for reaction for 3 h. LC-MS monitoring showed the absence of raw materials; the reaction mixture was cooled to room temperature, added with water (30 mL), and extracted with DCM (30 mL × 3); the organic phases were combined, dried with anhydrous sodium sulfate, and filtered; the filtrate was concentrated under reduced pressure; and the resulting crude product was purified by preparative thin-layer chromatography (MeOH:DCM = 1:10) to obtain a product (217 mg, yield: 78.4%).

### Step 2: Synthesis of (R)-2-(5-methyl-3-(1-methylpiperidin-3-yl)amino)-1,2,4-triazin-6-yl)-5-(prop-1-yn-1yl)phenol

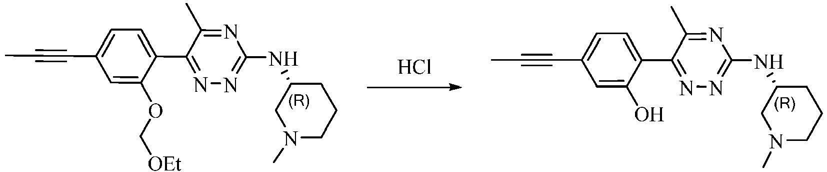

(*R*)-6-(2-(ethoxymethoxy)-4-(prop-1-yn-1-yl)phenyl)-5-methyl-*N*-(1-methylpiperidin-3-yl)-1,2,4-triazin-3-amine (217 mg, 0.55 mmol, 1.0 eq.) was added to DCM (10 mL); 4 mol/L hydrogen chloride in a 1,4-dioxane solution (4 mL) was dropwise added to allow for reaction for 1 h at room temperature. TLC detected the absence of raw materials; the reaction mixture was added with water (30 mL), adjusted to a pH value of about 8 by addition of sodium carbonate, and extracted with DCM (30 mL × 3); the organic phases were combined, dried with anhydrous sodium sulfate, and filtered; the filtrate was concentrated under reduced pressure, to obtain a crude product; the crude product was pulped by addition of tert-butylmethyl ether (4 mL), and filtered; and the filter cake was dried to obtain a product (130 mg, yield: 70%).

¹HNMR (400 MHz, DMSO-*d*₆) δ(ppm): 9.97 (s, 1.0H), 7.44-7.42 (d, 1H), 7.22-7.20 (s, 1H), 6.94-6.93 (d, 2H), 3.99 (m, 1H), 2.89-2.88 (m, 1H), 2.65-2.62 (m, 1H), 2.18 (s, 6H), 2.06 (s, 3H), 1.86 (m, 3H), 1.72-1.68 (m, 1H), 1.55-1.52 (m, 1H), 1.33-1.29 (m, 1H).

Molecular formula: C₁₉H₂₃N₅O Accurate molecular weight: 337.19 LC-MS (Pos, m/z) = 338.33[M+H]⁺.

### Example 25: Synthesis of (R)-5-(cyclopropylethynyl)-2-(5-methyl-3-((1-methylpiperidin-3-yl)amino)-1,2,4-triazin-6-yl)phenol (Compound 197)

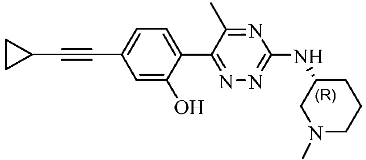

### Steps:

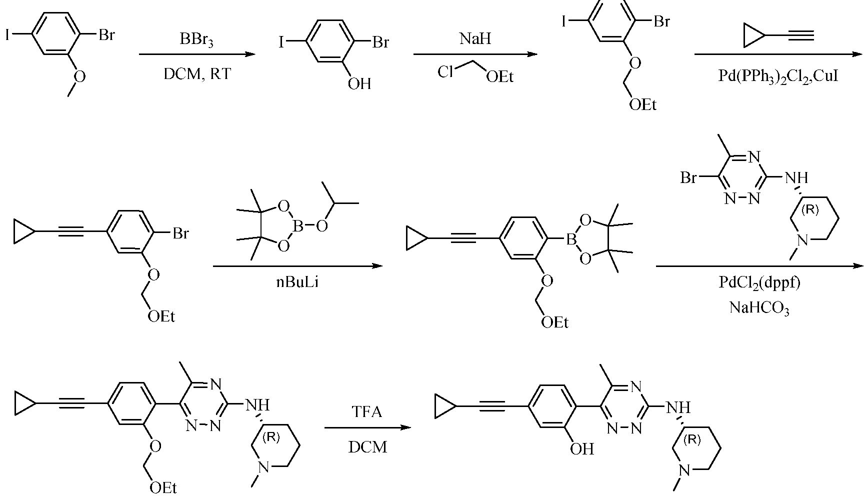

### Step 1: Synthesis of 2-bromo-5-iodophenol

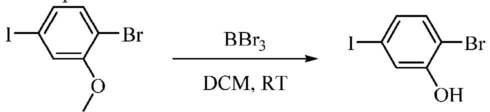

2-bromo-5-iodophenylmethyl ether(3.8 g, 12.14 mmol, 1.0 eq.) was dissolved in DCM (40 mL), and cooled to - 10°C; boron tribromide (6.1 g, 24.28 mmol, 2.0 eq.) was dropwise added; and after dropwise addition, the temperature was naturally increased to room temperature to allow for reaction for 17 h. TLC monitoring showed the absence of raw materials; methanol (10 mL) was dropwise added; the resulting mixture was stirred for 10 min, added with water (30 mL), and extracted with DCM (30 mL × 2); the organic phases were combined, dried with anhydrous magnesium sulfate, and filtered; and the filtrate was concentrated under reduced pressure to obtain a product (3.6 g, yield: 100%).

### Step 2: Synthesis of 1-bromo-2-(ethoxymethoxy)-4-iodobenzene

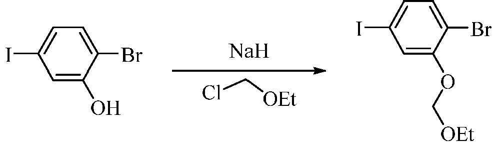

2-bromo-5-iodophenol (3.6 g, 12.14 mmol, 1.0 eq.) was dissolved in anhydrous THF (40 mL), and 60% sodium hydride (728.4 mg, 18.21 mmol, 1.5 eq.) was slowly added to allow for reaction for 0.5 h at room temperature. Chloromethyl ethyl ether (1.7 g, 18.21 mmol, 1.5 eq.) was dropwise added to allow for reaction for 1 h at room temperature. LC-MS detection showed the absence of raw materials; the reaction mixture was added with a saturated ammonium chloride solution (40 mL), and extracted with ethyl acetate (50 mL × 3); the organic phases were combined, dried with anhydrous sodium sulfate, and filtered; the filtrate was concentrated under reduced pressure; and the resulting crude product was purified by silica gel column chromatography (EA:PE = 1:10) to obtain a product (4.3 g, yield: 100%).

### Step 3: Synthesis of 1-bromo-4-(cyclopropylethynyl)-2-(ethoxymethoxy)benzene

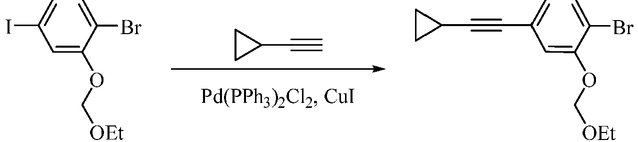

1-bromo-2-(ethoxymethoxy)-4-iodobenzene (3.6 g, 10.08 mmol, 1.0 eq.), bis(triphenylphosphine)palladium dichloride (701.9 mg, 1 mmol, 0.1 eq.), cuprous iodide (380.9 mg, 2 mmol, 0.2 eq.) and DIPEA (1.9 g, 15.12 mmol, 1.5 eq.) were dissolved in anhydrous THF (40 mL), and stirred for 10 min under the protection of nitrogen; and cyclopropyl acetylene (733 mg, 11.09 mmol, 1.1 eq.) was added to allow for reaction for 16 h at room temperature. TLC detection showed the absence of raw materials; the reaction mixture was added with water (100 mL), and extracted with ethyl acetate (100 mL × 2); the organic phases were combined, dried with anhydrous sodium sulfate, and filtered; the filtrate was concentrated under reduced pressure; and the resulting crude product was purified by silica gel column chromatography (EA:PE = 1:200-1:100) to obtain a product (2.8 g, yield: 93.3%).

### Step 4: Synthesis of 2-(4-(cyclopropylethynyl)-2-(ethoxymethoxy)phenyl)-4,4,5,5-tetramethyl-1,3,2-dioxaboracyclopentane

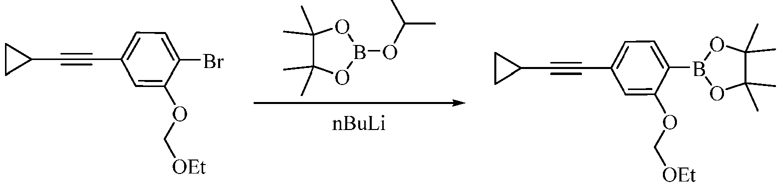

1-bromo-4-(cyclopropylethynyl)-2-(ethoxymethoxy)benzene (2.8 g, 9.48 mmol, 1.0 eq.) was dissolved in anhydrous THF (30 mL), and cooled to -75°C; 1.6 mol/L n-buty lithium in a THF solution (7.7 mL, 12.32 mmol, 1.3 eq.) was dropwise added, and after dropwise addition, the temperature was held at -70°C to allow for reaction for 1h; 2-isopropoxy-4,4,5,5-tetramethyl-1,3,2-dioxaboracyclopentane (2.6 g, 14.22 mmol, 1.5 eq.) was dropwise added, and after dropwise addition, the temperature was held at -70°C to allow for reaction for 1h; and the temperature was naturally increased to room temperature to allow for reaction for 15h. TLC detection showed the absence of raw materials; the reaction mixture was added with saturated ammonium chloride in water (30 mL), stirred for 5 min, and extracted with ethyl acetate (50 mL × 3); the organic phases were combined, dried with anhydrous sodium sulfate, and filtered; the filtrate was concentrated under reduced pressure; and the resulting crude product was purified by silica gel column chromatography (EA:PE = 1:100-EA=100%) to obtain a product (2.3 g, yield: 71.9%).

### Step 5: Synthesis of (R)-6-(4-(cyclopropylethynyl)-2-(ethoxymethoxy)phenyl)-5-methyl-N-(1-methylpiperidin-3-yl)-1,2,4-triazin-3-amine

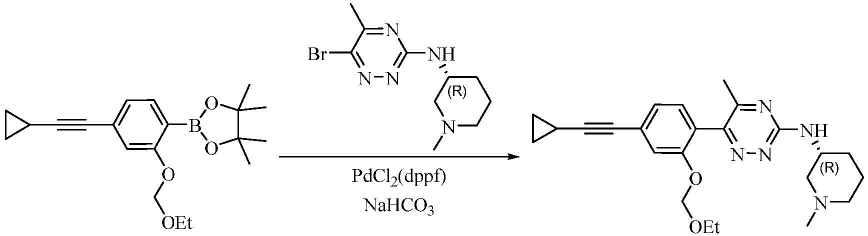

(*R*)-6-bromo-5-methyl-N-(1-methylpiperidin-3-yl)-1,2,4-triazin-3-amine (286.2 mg, 1 mmol, 1.0 eq.), 2-(4-(cyclopropylethynyl)-2-(ethoxymethoxy)phenyl)-4,4,5,5-tetratnethyl-1,3,2-dioxaboracyclopentane (684.5 mg, 2 mmol, 2.0 eq.), sodium hydrogen carbonate (252 mg, 3 mmol, 3.0 eq.) and PdCl₂(dppf) (73.2 mg, 0.1 mmol, 0.1 eq.) were added to the mixed solution of 1,4-dioxane (10 mL) and water (2 mL), and heated to 100°C under the protection of nitrogen to allow for reaction for 3 h. LC-MS monitoring showed the absence of raw materials; the reaction mixture was cooled to room temperature, added with water (50 mL), and extracted with ethyl acetate (50 mL × 3); the organic phases were combined, dried with anhydrous sodium sulfate, and filtered; the filtrate was concentrated under reduced pressure; and the resulting crude product was purified by silica gel column chromatography (MeOH:DCM = 1:100-1:20) to obtain a product (412 mg, yield: 97.7%).

### Step 6: Synthesis of (R)-5-(cyclopropylethynyl)-2-(5-methyl-3-((1-methylpiperidin-3-yl)amino)-1,2,4-triazin-6-yl)phenol

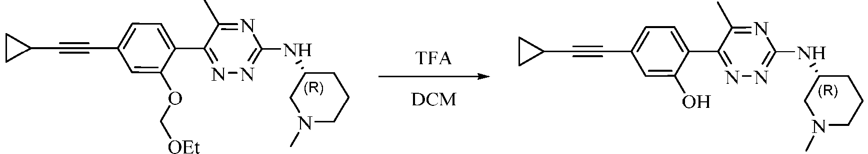

(*R*)-6-(4-(cyclopropylethynyl)-2-(ethoxymethoxy)phenyl)-5-methyl-*N-*(1-methylpiperidin-3-yl)-1,2,4-triazin-3-amine (410 mg, 0.97 mmol, 1.0 eq.) was added to DCM (5 mL), and cooled to about 0°C by means of ice water; the solution of TFA (0.5 mL) in DCM (5 mL) was dropwise added to allow for reaction for 8h at room temperature. LC-MS detection showed that about 5% of the raw materials were left; the reaction mixture was added with saturated sodium hydrogen carbonate in water (30 mL), and extracted with DCM (30 mL×5); the organic phases were combined, dried with anhydrous sodium sulfate, and filtered; the filtrate was concentrated under reduced pressure; and the resulting crude product was purified by preparative thin-layer chromatography (MeOH:DCM = 1:10) to obtain a product (125 mg, yield: 35.4%).

¹HNMR (400 MHz, DMSO-*d*₆) δ(ppm): 9.98 (s, 1H), 7.40 (d, 1H), 7.20-7.18 (d, 1H), 6.92-6.90 (d, 2H), 3.98 (m, 1H), 2.88 (m, 1H), 2.65-2.63 (m, 1H), 2.18-2.17 (s, 6H), 1.86-1.84 (m, 3H), 1.71-1.68 (m, 1H), 1.58-1.52 (m, 2H), 1.34-1.28 (m, 1H), 0.91-0.88 (m, 2H), 0.75-0.74 (m, 2H).

Molecular formula: C₂₁H₂₅N₅O Accurate molecular weight: 363.21 LC-MS (Pos, m/z) =364.30[M+H]⁺.

### Example 26: Synthesis of (R)-5-(cyclopropylethynyl)-2-(4-methyl-6-((1-methylpiperidin-3-yl)amino)pyridazin-3-yl)phenol (Compound 254)

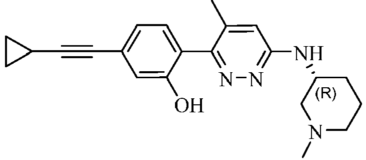

### Step 1: Synthesis of (R)-6-(4-(cyclopropylethynyl)-2-(ethoxymethoxy)phenyl)-5-methyl-N-(1-methylpiperidin-3-yl)pyridazin-3-amine

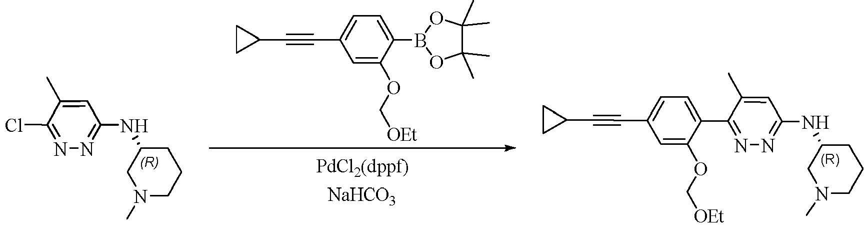

(*R*)-6-chloro-5-methyl-N (1-methylpiperidin-3-yl)pyridazin-3-atnine (142.6 mg, 0.59 mmol, 1.0 eq.), 2-(4-(cyclopropylethynyl)-2-(ethoxymethoxy)phenyl)-4,4,5,5-tetratnethyl-1,3,2-dioxaboracyclopentane (403.8 mg, 1.18 mmol, 2.0 eq.), sodium hydrogen carbonate (147.8 mg, 1.77 mmol, 3.0 eq.) and PdCl₂(dppf) (43.9 mg, 0.06 mmol, 0.1 eq.) were added to the mixed solution of 1,4-dioxane (10 mL) and water (2 mL), and heated to 100°C under the protection of nitrogen to allow for reaction for 3 h. LC-MS monitoring showed the absence of raw materials; the reaction mixture was cooled to room temperature, added with water (30 mL), and extracted with DCM (30 mL × 2); the organic phases were combined, dried with anhydrous sodium sulfate, and filtered; the filtrate was concentrated under reduced pressure; and the resulting crude product was purified by silica gel column chromatography (MeOH:DCM = 1:100-1:20) to obtain a product (183 mg, yield: 73.7%).

### Step 2: Synthesis of (R)-5-(cyclopropylethynyl)-2-(4-methyl-6-((1-methylpiperidin-3-yl)amino)pyridazin-3-yl)phenol

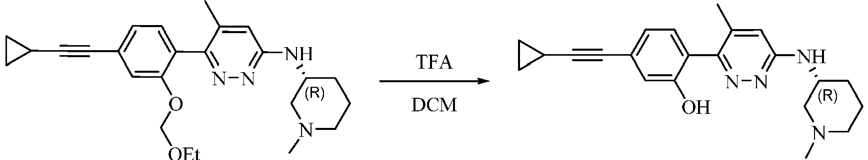

(*R*)-6-(4-(cyclopropylethynyl)-2-(ethoxymethoxy)phenyl)-5-methyl-*N*-(1-methylpiperidin-3-yl)pyridazin-3-amine (183 mg, 0.43 mmol, 1.0 eq.) was added to DCM (5 mL), and cooled by means of ice water; and the solution of TFA (0.5 mL) in DCM (5 mL) was dropwise added to allow for reaction for 8 h at room temperature. LC-MS detection showed that about 5% of the raw materials were left; the reaction mixture was added with saturated sodium hydrogen carbonate in water (30 mL), stirred for 5 min, and extracted with DCM (50 mL×3); the organic phases were combined, dried with anhydrous sodium sulfate, and filtered; the filtrate was concentrated under reduced pressure; and the resulting crude product was purified by preparative thin-layer chromatography (MeOH:DCM = 1:10) to obtain a product (78 mg, yield: 50%).

¹HNMR (400 MHz, DMSO-*d*₆) δ(ppm): 10.01 (s, 1H), 7.12-7.10 (d, 1H), 6.88-6.86 (d, 2H), 6.67 (s, 1H), 6.62-6.60 (d, 1H), 4.04-4.00 (m, 1H), 2.87-2.85 (m, 1H), 2.51(m, 1H), 2.18 (s, 3H), 2.00 (m, 4H), 1.89-1.82 (m, 2H), 1.72-1.69 (m, 1H), 1.57-1.52 (m, 2H), 1.29-1.27 (m, 1H), 0.91-0.88 (m, 2H), 0.75-0.73 (m, 2H).

Molecular formula: C₂₂H₂₆N₄O Accurate molecular weight: 362.21 LC-MS (Pos, m/z) =363.07[M+H]⁺.

### Example 27: Synthesis of (R)-2-(4-((1-(2-hydroxyethyl)piperidin-3-yl)amino)phthalazin-1-yl)-5-(prop-1-yn-1-yl)phenol (Compound 270)

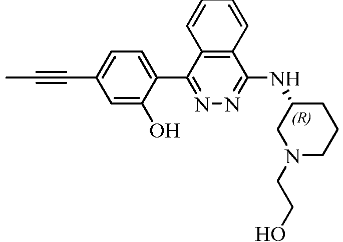

### Step 1: Synthesis of (R)-2-(3-((4-(2-(ethoxymethoxy)-4-(prop-1-yn-1-yl)phenyl)phthalazin-1-yl)amino)piperidin-1-yl)ethan-1-ol

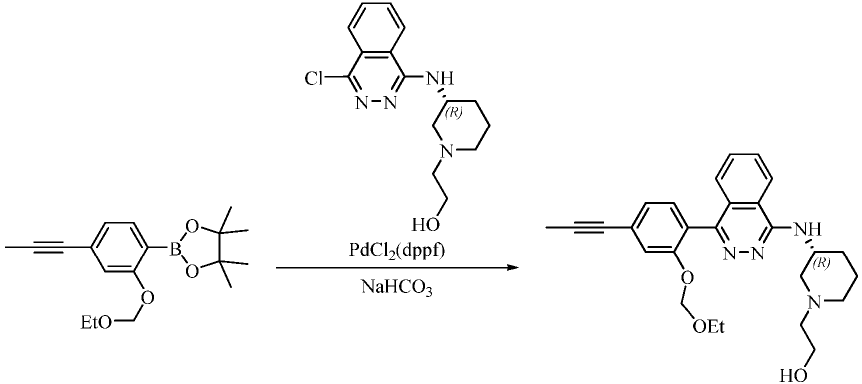

(*R*)-2-(3-((4-chlorophthalazin-1-yl)amino)piperidin-1-yl)ethan-1-ol (200 mg, 0.65 mmol, 1.0 eq.), 2-(2-(ethoxymethoxy)-4-(prop-1-yn-1-yl)phenyl)-4,4,5,5-tetramethyl-1,3,2-dioxaboracyclopentane (306.7 mg, 0.97 mmol, 1.5 eq.), sodium hydrogen carbonate (163.8 mg, 1.95 mmol, 3.0 eq.) and PdCl₂(dppf) (43.9 mg, 0.06 mmol, 0.1 eq.) were added to the mixed solution of 1,4-dioxane (10 mL) and water (2 mL), and heated to 100°C under the protection of nitrogen to allow for reaction for 18 h. LC-MS monitoring showed the absence of raw materials; the reaction mixture was cooled to room temperature, added with water (30 mL), and extracted with DCM (30 mL × 2); the organic phases were combined, dried with anhydrous sodium sulfate, and filtered; the filtrate was concentrated under reduced pressure; and the resulting crude product was purified by silica gel column chromatography (MeOH:DCM = 1:100-1:10) to obtain a product (166 mg, yield: 55.4%).

### Step 2: Synthesis of (R)-2-(4-((1-(2-hydroxyethyl)piperidin-3-yl)amino)phthalazin-1-yl)-5-(prop-1-yn-1-yl)phenol

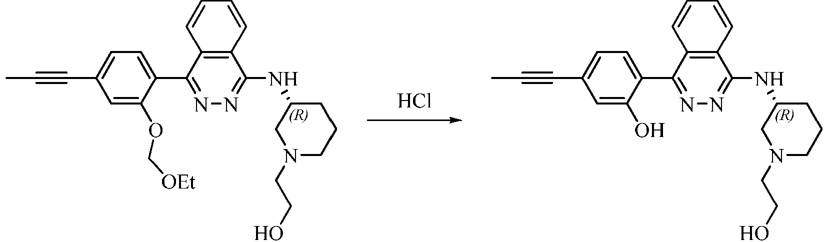

(*R*)-2-(3-((4-(2-(ethoxymethoxy)-4-(prop-1-yn-1-yl)phenyl)phthalazin-1-yl)amino)piperidin-1-yl)ethan-1-ol (166 mg, 0.36 mmol, 1.0 eq.) was added to DCM (5 mL); 4 mol/L hydrogen chloride in a 1,4-dioxane solution (3 mL) was dropwise added to allow for reaction for 1 h at room temperature. LC-MS detection showed the absence of raw materials; the reaction mixture was added with water (30 mL), adjusted to pH = about 7 by adding sodium hydrogen carbonate, and extracted with DCM (30 mL × 3); the organic phases were combined, dried with anhydrous sodium sulfate, and filtered; the filtrate was concentrated under reduced pressure; and the resulting crude product was purified by preparative thin-layer chromatography (MeOH:DCM = 1:10) to obtain a product (72 mg, yield: 49.7%). ¹HNMR (400 MHz, DMSO-*d*₆) δ(ppm): 9.88 (s, 1H), 8.38-8.36 (d, 1H), 7.86-7.76 (d, 2H), 7.48-7.46 (d, 1H), 7.26-7.24 (d, 1H), 7.09-7.07 (d, 1H), 6.98-6.96 (d, 1H), 4.44 (m, 2H), 3.53-3.52 (m, 2H), 3.16-3.14 (m, 1H), 2.83-2.81 (m, 1H), 2.47-2.44 (m, 2H), 2.08 (m, 5H), 1.97 (m, 1H), 1.76-1.73 (m, 1H), 1.61-1.49 (m, 2H).

Molecular formula: C₂₄H₂₆N₄O₂ Accurate molecular weight: 402.21 LC-MS (Pos, *m*/*z*) =403.35[M+H]⁺.

### Example 28: Synthesis of (R)-5-bromoethynyl-2-(4-methyl-6-(1-methylpiperidin-3-yl)amino)pyridazin-3-yl)phenol (Compound 259)

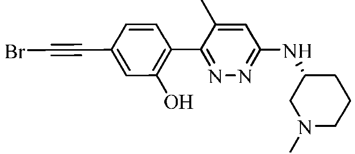

### Steps:

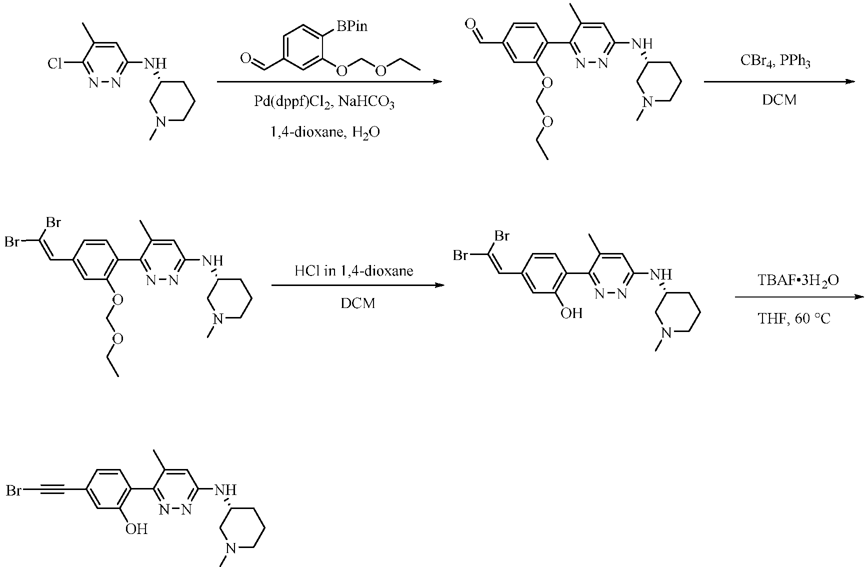

### Step 1: Synthesis of (R)-3-(ethoxymethoxy)-4-(4-methyl-6-(1-methylpiperidin-3-yl)amino)pyridazin-3-yl)benzaldehyde

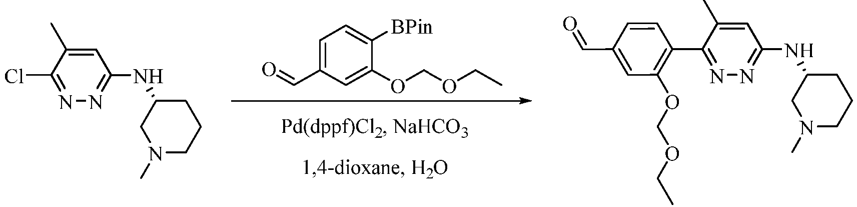

(*R*)-6-chloro-5-methyl-*N*-(1-methylpiperidin-3-yl)pyridazin-3-amine (650 mg, 2.70 mmol, 1.0 eq.) was dissolved in 1,4-dioxane (7 mL) and water (3 mL); 3-(ethoxymethoxy)-4-(4,4,5,5-tetramethyl-1,3,2-dioxaboracyclopentan-2-yl)benzaldehyde (1.16 g, 3.78 mmol, 1.4 eq.), sodium hydrogen carbonate (454 mg, 5.40 mmol, 2.0 eq.) and Pd(dppf)Cl₂ (198 mg, 0.27 mmol, 0.1 eq.) were successively added; and the resulting mixture was stirred for 2 h at 90°C under the protection of nitrogen. LCMS detection showed that the reaction was complete. The reaction mixture was concentrated, added with water (5 mL), and extracted with DCM (5 mL × 3); the organic phases were dried and filtered by suction; the filtrate was concentrated; and the resulting crude product was purified by silica gel column chromatography (DCM:MeOH = 20:1-10:1) to obtain a product (520 mg, yield: 50.0%).

### Step 2: Synthesis of (R)-6-(4-(2,2-dibromoethenyl)-2-(ethoxymethoxy)phenyl)-5-methyl-N-(1-methylpiperidin-3-yl)pyridazin-3-amine

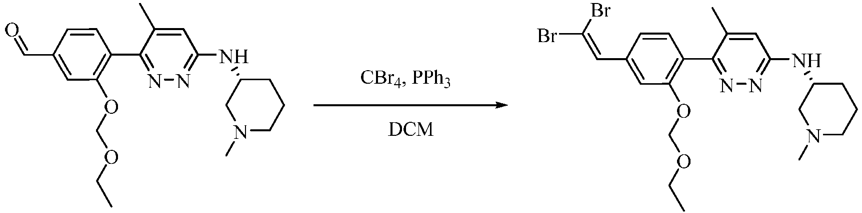

(*R*)-3-(ethoxymethoxy)-4-(4-methyl-6-(1-methylpiperidin-3-yl)atnino)pyridazin-3-yl)benzaldehyde (400 mg , 1.04 mmol, 1.0 eq.) obtained from the previous step was dissolved in dichloromethane (5 mL); carbon tetrabromide (2.07 g, 6.24 mmol, 6.0 eq.) was added; in the ice water bath, triphenylphosphine (3.27 g, 12.5 mmol, 12.0 eq.) in a dichloromethane solution (12 mL) was dropwise added; after dropwise addition, the resulting mixture was stirred for 30 min; the ice water bath is removed, and the mixture was continuously stirred for 1 h; TLC detection showed that the reaction was complete; the reaction mixture was diluted by adding dichloromethane; and the resulting crude product was treated by silica gel column chromatography (dichloromethane:methanol = 40:1-20:1) to obtain a crude product (900 mg).

### Step 3: Synthesis of (R)-5-(2,2-dibromoethenyl)-2-(4-methyl-6-((1-methylpiperidin-3-yl)amino)pyridazin-3-yl)phenol

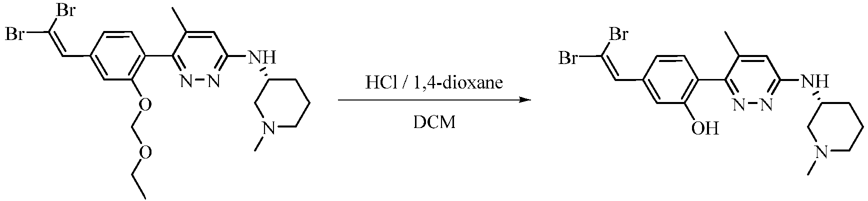

The crude product of (*R*)-6-(4-(2,2-dibromoethenyl)-2-(ethoxymethoxy)phenyl)-5-methyl-*N*-(1-methylpiperidin-3-yl)pyridazin-3-amine (700 mg) was dissolved in dichloromethane (7 mL); 4 mol/L hydrogen chloride in a 1,4-dioxane solution (1 mL) was added; the resulting mixture was stirred for 1 h at room temperature; and LC-MS detection showed that the reaction was complete. The reaction mixture was poured into water, and adjusted to a pH value of about 8 by using sodium hydrogen carbonate; the resulting mixture was separated, extracted with dichloromethane (5 mL × 2), dried, filtered by suction, and concentrated; and the resulting crude product was treated by silica gel column chromatography (dichloromethane:methanol = 40:1-20:1) to obtain a product (390 mg, two-step yield: 99.9%).

### Step 4: Synthesis of (R)-5-bromoethynyl-2-(4-methyl-6-(1-methylpiperidin-3-yl)amino)pyridazin-3-yl)phenol

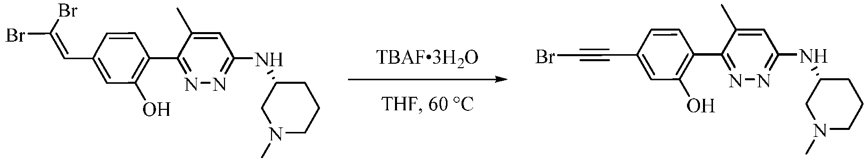

(*R*)-5-(2,2-dibromoethenyl)-2-(4-methyl-6-((1-methylpiperidin-3-yl)amino)pyridazin-3-yl)phenol (200 mg, 0.41 mmol, 1.0 eq.) obtained from the previous step was dissolved in tetrahydrofuran (3 mL); TBAF trihydrate (786 mg, 2.49 mmol, 6.0 eq.) was added; the resulting mixture was stirred for 4 h at 60°C; and TLC detection showed that the reaction was complete. The reaction mixture was concentrated; and the resulting crude product was purified by preparative thin-layer chromatography (DCM:7% ammonia in MeOH = 20:1) to obtain a product (100 mg, yield: 60.8%).

¹H-NMR (400 MHz, DMSO-*d*₆) δ(ppm): 10.20 (s, 1H), 7.17-7.15 (m, 1H), 7.01 (s, 2H), 6.83 (s, 1H), 6.70 (s, 1H), 4.15 (s, 1H), 3.18 (s, 2H), 2.87 (s, 1H), 2.44-2.24 (m, 4H), 2.02 (s, 3H), 1.87-1.81 (m, 2H), 1.67 (s, 1H), 1.40 (s, 1H)_{∘}

Molecular formula: C₁₉H₂₁BrN₄O Accurate molecular weight: 400.09 LC-MS(*m*/*z*):401.07, 403.06 [M+H]⁺

### Example 29: Synthesis of Compound (R)-5-bromoethynyl-2-(4-cyclopropyl-6-(1-methylpiperidin-3-yl)amino)pyridazin-3-yl)phenol (Compound 260)

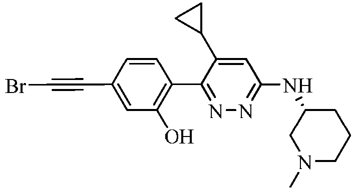

### Steps:

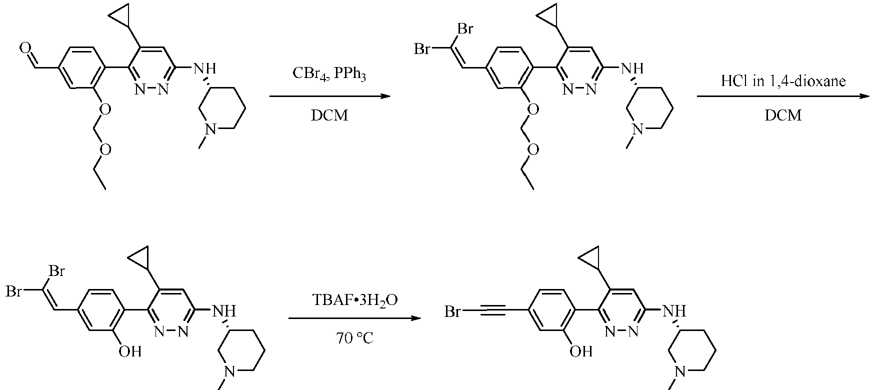

### Step 1: Synthesis of (R)-5-cyclopropyl-(6-(4-(2,2-dibromoethenyl)-2-(ethoxymethoxy)phenyl) -N-(1-methylpiperidin-3-yl)pyridazin-3-amine

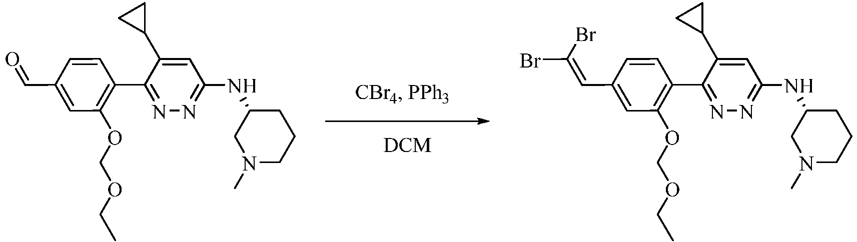

(*R*)-4-(4-cyclopropyl-6-((1-methylpiperidin-3-yl)atnino)pyridazin-3-yl)-3-(ethoxymethoxy)benzaldehyde (300 mg, 0.73 mmol, 1.0 eq.) obtained from the previous step was dissolved in dichloromethane (4 mL); carbon tetrabromide (1.45 g, 4.38 mmol, 6.0 eq.) was added; in the ice water bath, triphenylphosphine (2.30 g, 8.77 mmol, 12.0 eq.) in a dichloromethane solution (9 mL) was dropwise added; after dropwise addition, the resulting mixture was stirred for 30 min; the ice water bath was removed, and the mixture was continuously stirred for 30 min; LC-MS detection showed that the reaction was complete; the reaction mixture was diluted by adding dichloromethane; and the resulting crude product was treated by silica gel column chromatography (dichloromethane:methanol = 50:1-10:1) to obtain a crude product (560 mg) containing a product.

### Step 2: Synthesis of (R)-2-(4-cyclopropyl-6-((1-methylpiperidin-3-yl)amino)pyridazin-3-yl)-5-(2,2-dibromoethenyl)phenol

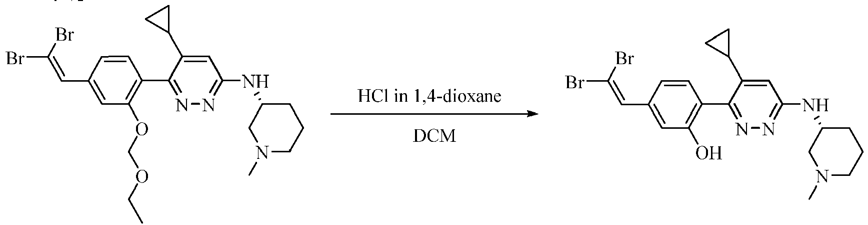

The crude product of (*R*)-5-cyclopropyl-(6-(4-(2,2-dibromoethenyl)-2-(ethoxymethoxy)phenyl)-*N*-(1-methylpiperidin-3-yl)pyridazin-3-amine (560 mg) was dissolved in dichloromethane (5 mL); 4 mol/L hydrogen chloride in a 1,4-dioxane solution (0.5 mL) was added; the resulting mixture was stirred for 1 h at room temperature; and LC-MS detection showed that the reaction was complete. The reaction mixture was poured into water, and adjusted to a pH value of about 8 by using sodium hydrogen carbonate; the resulting mixture was separated, and extracted with dichloromethane (5 mL × 2); the organic phases were dried, filtered by suction, and concentrated; and the resulting crude product was purified by silica gel column chromatography (dichloromethane:methanol = 20:1-10:1) to obtain a product (300 mg, two-step yield: 80.8%).

### Step 3: Synthesis of Compound (R)-5-bromoethynyl-2-(4-cyclopropyl-6-(1-methylpiperidin-3-yl)atnino)pyridazin-3-yl)phenol

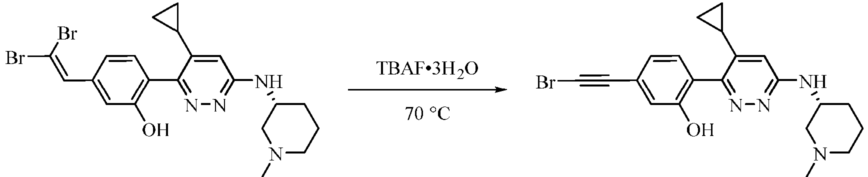

(*R*)-2-(4-cyclopropyl-6-((1-methylpiperidin-3-yl)atnino)pyridazin-3-yl)-5-(2,2-dibromoethenyl)phenol (300 mg, 0.59 mmol, 1.0 eq.) obtained from the previous step was dissolved in tetrahydrofuran (3 mL); TBAF trihydrate (931 mg, 2.95 mmol, 6.0 eq.) was added and stirred overnight at 70°C; and LC-MS detection showed that the reaction was complete. The reaction mixture was concentrated; and the resulting crude product was purified by preparative thin-layer chromatography (DCM:7% ammonia in MeOH = 20:1) to obtain a product (90 mg, yield: 35.7%).

¹H-NMR (400 MHz, DMSO-*d*₆) δ(ppm): 10.14 (s, 1H), 7.22 (d, *J*=7.68 Hz, 1H), 7.02-6.99 (m, 2H), 6.55 (d, *J*=7.48 Hz, 1H), 6.33 (s, 1H), 4.07 (s, 1H), 2.90 (s, 1H), 2.61 (s, 1H), 2.25 (s, 3H), 2.16-2.01 (m, 2H), 1.80-1.72 (m, 2H), 1.58-1.51 (m, 2H), 1.34-1.25 (m, 1H), 0.88-0.85 (m, 2H), 0.61-0.60 (m, 2H),_{∘}

Molecular formula: C₂₁H₂₃BrN₄O Accurate molecular weight: 426.11 LC-MS(*m*/*z*):427.10, 429.08 [M+H]⁺

### Example 30: Synthesis of (R)-5-(3-hydroxyprop-1-yn-1-yl)-2-(4-methyl-6-((1-methylpiperidin-3-yl)aminopyridazin-3-yl)phenol (Compound 262)

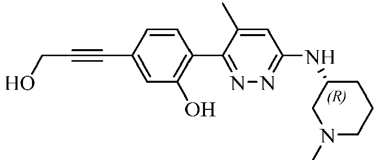

### Step 1: Synthesis of 3-(4-bromo-3-(ethoxymethoxy)phenyl)prop-2-yn-1-yl acetate

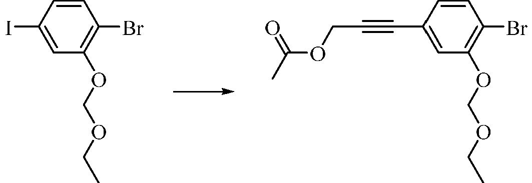

1-bromo-2-(ethoxymethoxy)-4-iodobenzene (5.0 g, 14.00 mmol, 1.0 eq.), PdCl₂(PPh₃)₂ (983 mg, 1.40 mmol, 0.1 eq.), CuI(267 mg, 1.40 mmol, 0.1 eq.) and DIPEA(5.43 g, 42.00 mmol, 3.0 eq.) were dispersed in THF (50 mL), and cooled to 0-5°C under the protection of nitrogen to allow for reaction for 20 min. Propargyl acetate (1.65 mg, 16.8 mmol, 1.2 eq.) was added to allow for reaction for 40 min; and TLC monitoring showed that the reaction was complete. The reaction mixture was poured into water (100 mL), and extracted with EA (50 mL × 2); and the organic phases were dried, and concentrated to obtain a crude product (4.58 g, yield: 100%).

### Step 2: Synthesis of 3-(4-bromo-3-(ethoxymethoxy)phenyl)prop-2-yn-1-ol

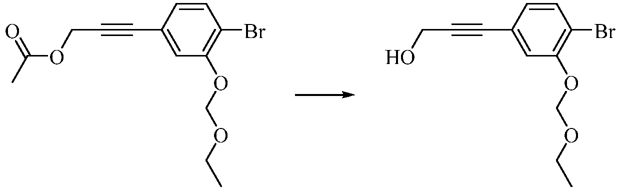

3-(4-bromo-3-(ethoxymethoxy)phenyl)prop-2-yn-1-yl acetate (4.58 g, 14.00 mmol, 1.0 eq.) and lithium hydroxide monohydrate (1.76 g, 42.00 mmol, 3.0 eq.) were dissolved in methanol (50 mL) and water (10 mL), and allowed to react for 5 min at 50°C; TLC monitoring showed that the reaction was complete; the reaction mixture was concentrated under reduced pressure; the resulting crude product was dispersed with water (30 mL), and extracted with EA (30 mL × 2); the organic phases were dried, concentrated, and purified by silica gel column chromatography (EA:PE = 1:4) to obtain a product (2.3 g, yield: 56.7%).

### Step 3: Synthesis of 1-bromo-2-(ethoxymethoxy)-4-(3-(ethoxymethoxy)prop-1-yn-1-yl)benzene

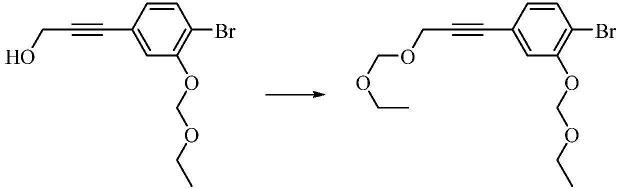

3-(4-bromo-3-(ethoxymethoxy)phenyl)prop-2-yn-1-ol (2.3 g, 8.07 mmol, 1.0 eq.) was dissolved in THF (25 mL); 60% NaH (484 mg, 12.10 mmol, 1.5 eq.) was added to allow for reaction for 0.5 h at room temperature; and chloromethyl ethyl ether (1.14 g, 12.10 mmol, 1.5 eq.) was added to allow for reaction at room temperature for 1 h. TLC monitoring showed that the reaction was complete; the reaction mixture was poured into water (50 mL), and extracted with EA (20 mL × 2); and the organic phases were dried, concentrated, and purified by silica gel column chromatography (EA:PE = 70: 1) to obtain a product (2.0 g, yield: 72.5%).

### Step 4: Synthesis of 2-(2-(ethoxymethoxy)-4-(3-(ethoxymethoxy)prop-1-yn-1-yl)phenyl)-4,4,5,5-tetramethyl-1,3,2-dioxaboracyclopentane

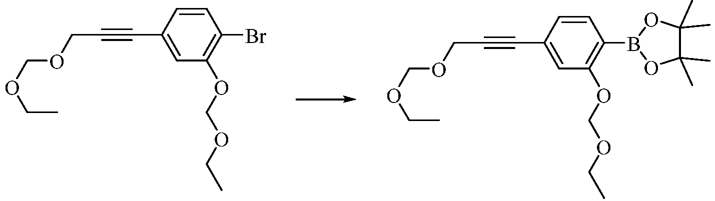

1-bromo-2-(ethoxymethoxy)-4-(3-(ethoxymethoxy)prop-1-yn-1-yl)benzene (2.0 g, 5.83 mmol, 1.0 eq.) was dissolved in THF (20 mL), and cooled to -65°C under the protection of nitrogen; n-butyl litium (1.6 mol/L, 5.5 mL) was dropwise added; and the resulting mixture was stirred to allow for reaction for 1 h. Isopropoxyboronic acid pinacol ester (1.63 g, 8.74 mmol, 1.5 eq.) was added to allow for reaction for 1.5 h at -65°C; TLC monitoring showed that the reaction was complete; the reaction mixture was poured into water (20 mL), and extracted with EA (20 mL × 2); and the organic phases were dried, concentrated, and purified by silica gel column chromatography (EA:PE = 10:1) to obtain a product (1.45 g, yield: 63.9%).

### Step 5: Synthesis of (R)-6-(2-(ethoxymethoxy)-4-(3-(ethoxymethoxy)prop-1-yn-1-yl)phenyl)-5-methyl-N-(1-methylpiperidin-3 -yl)pyridazin-3 -amine

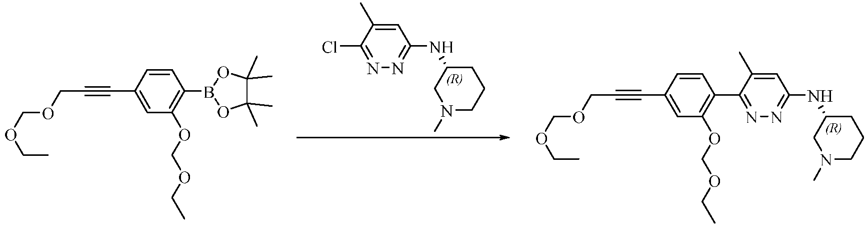

(*R*)-6-chloro-5-methyl-N (1-methylpiperidin-3-yl)pyridazin-3-atnine (130 mg, 0.54 mmol, 1.0 eq.), (R)-6-(2-(ethoxymethoxy)-4-(3-(ethoxymethoxy)prop-1-yn-1-yl)phenyl)-5-methyl-*N*-(1-methylpiperidin-3-yl)pyridazin-3-amine (316 mg, 0.81 mmol, 1.5 eq.), Pd(dppf)Cl₂(40 mg, 0.054 mmol, 0.1 eq.) and NaHCOs(91 mg, 1.08 mmol, 2.0 eq.) were added to 1,4-dioxane (5 mL) and H₂O(1 mL), and heated to 110°C under the protection of nitrogen to allow for reaction for 20 h. The reaction mixture was poured into water (10 mL), and extracted with EA (20 mL × 3); and the organic phases were dried, and concentrated to obtain a crude product (253 mg, yield: 100%).

### Step 6: Synthesis of (R)-5-(3-hydroxyprop-1-yn-1-yl)-2-(4-methyl-6-((1-methylpiperidin-3-yl)atninopyridazin-3-yl)phenol

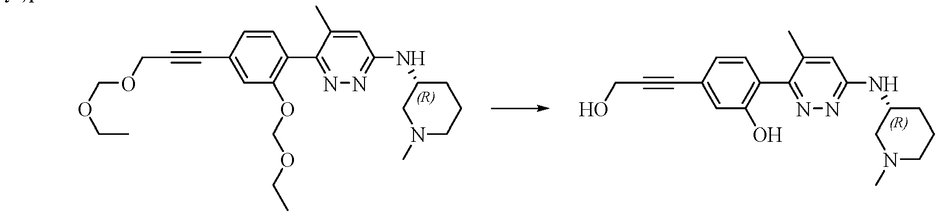

(*R*)-6-(2-(ethoxymethoxy)-4-(3-(ethoxymethoxy)prop-1-yn-1-yl)phenyl)-5-methyl-*N*-(1-methylpiperidin-3-yl)pyridazin-3-amine (253 mg, 0.54 mmol, 1.0 eq.) was dissolved in DCM (2 mL); and the 1,4-dioxane solution of hydrogen chloride (4 mol/L, 5 mL) was added to allow for reaction for 10 min at room temperature. The reaction mixture was poured into water (10 mL), and extracted with DCM (10 mL × 2); the aqueous phase was reserved, adjusted to a pH value of about 9 by using NaHCOs, and then extracted with DCM (15 mL×4); the organic phases were dried and concentrated; and the resulting crude product was purified by preparative thin-layer chromatography (MeOH:DCM = 1:10) to obtain a product (30 mg, yield: 15.8%).

¹HNMR (400MHz, DMSO-*d₆*, D₂O exchange) δ(ppm): 7.15-7.13 (d, 1H), 6.97-6.95 (t, 2H), 6.73 (s, 1H), 4.29 (s, 2H), 4.26-4.20 (t, 1H), 3.65 (s, 1H), 3.42-3.35 (m, 1H), 2.87 (s, 1H), 2.78 (s ,3H), 2.68 (s, 1H), 2.02 (s, 4H), 1.79-1.77 (d, 2H), 1.47-1.44 (d, 1H).

Molecular formula: C₂₀H₂₄N₄O₂ Accurate molecular weight: 352.19 LC-MS(Pos, *m*/*z*)=353.18[M+H]⁺.

### Example 31: Synthesis of Compound (R)-5-ethynyl-2-(4-methyl-6-(1-(2-(methylsulfonyl)ethyl)piperidin-3-amino)pyridazin-3-yl)phenol (Compound 263)

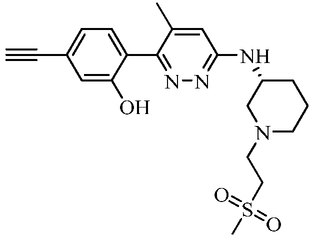

### Steps:

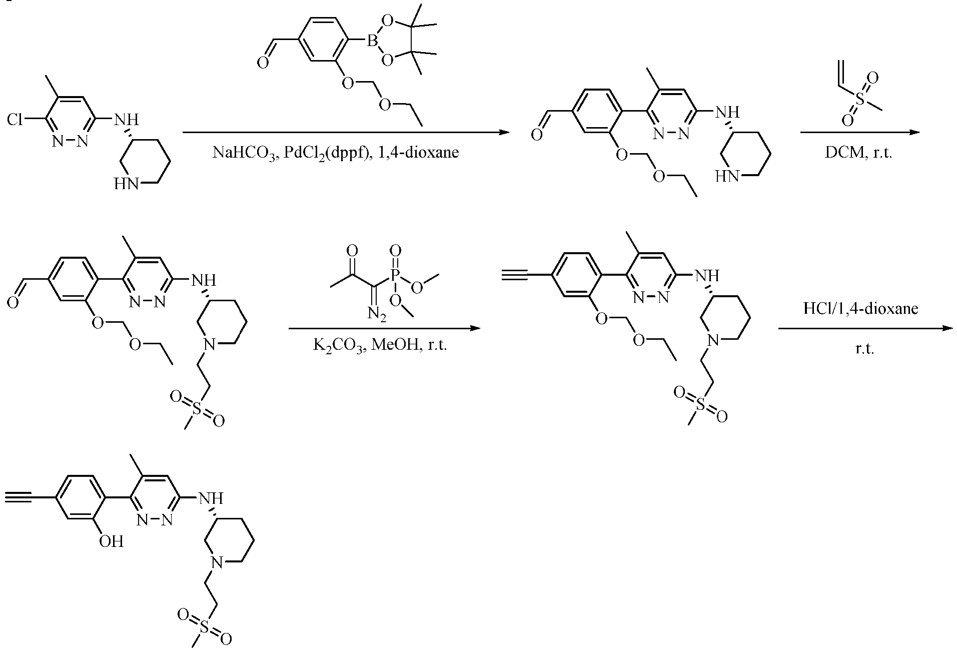

### Step 1: Synthesis of Intermediate (R)-3-(ethoxymethoxy)-4-(4-methyl-6-(piperidin-3-ylamino)pyridazin-3-yl)benzaldehyde

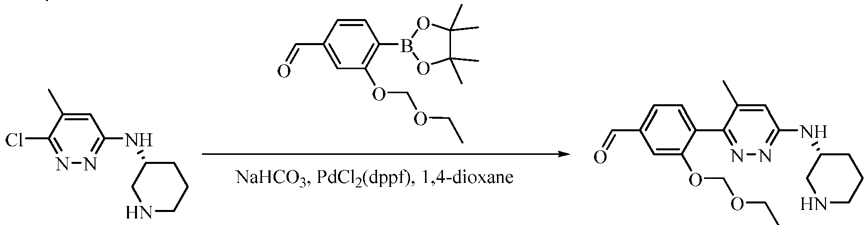

(R)-6-chloro-5-methyl-A-(piperidin-3-yl)pyridazin-3-amine (200 mg, 0.88 mmol, 1.0 eq.) was dissolved in 1,4-dioxane (2 mL); 3-(ethoxymethoxy)-4-(4,4,5,5-trimethyl-1,3,2-dioxoboracyclopentan-2-yl)benzaldehyde (404 mg, 1.32 mmol, 1.5 eq.), sodium hydrogen carbonate (148 mg, 1.76 mmol, 2.0 eq.), PdCl₂(dppf) (64 mg, 0.088 mmol, 0.1 eq.) and water (0.5 mL) were added to the resulting mixture; under the protection of nitrogen, the temperature was increased to 100°C to allow for reaction for 2 h; and TLC detection showed that the reaction was complete. The reaction mixture was filtered by means of diatomite; the filtrate was dried, and concentrated; and the resulting crude product was purified by silica gel column chromatography (dichloromethane:methanol = 100:1-7:1) to obtain a product (250 mg, yield: 76.7%).

### Step 2: Synthesis of Intermediate (R)-3-(ethoxymethoxy)-4-(4-methyl-6-(1-(2-(methylsulfonyl)ethyl)piperidin-3-amino)pyridazin-3 -yl)benzaldehyde

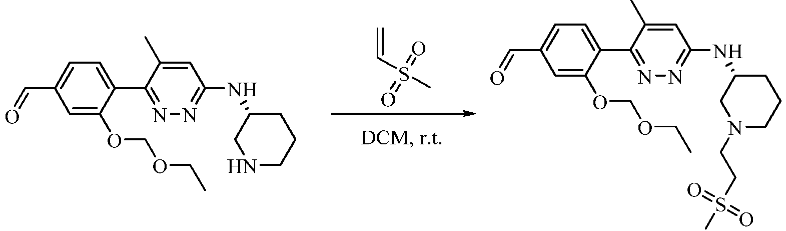

(*R*)-3-(ethoxymethoxy)-4-(4-methyl-6-(piperidin-3-ylamino)pyridazin-3-yl)benzaldehyde (250 mg , 0.675 mmol, 1.0 eq.) was dissolved in dichloromethane (3 mL); methyl vinyl sulfone (72 mg, 0.675 mmol, 1.0 eq) was added to the resulting mixture to allow for reaction for 2 h at 25°C; and TLC detection showed that the reaction was complete. The reaction mixture was concentrated to obtain a product (300 mg of crude product, yield: 93.5%), which was directly used in a next step.

### Step 3: Synthesis of Intermediate (R)-6-(2-ethoxymethoxy)-4-ethynylphenyl)-5-methyl-N-(1-(2-methylsulfonyl)ethyl)piperidin-3-yl)pyridazin-3-amine

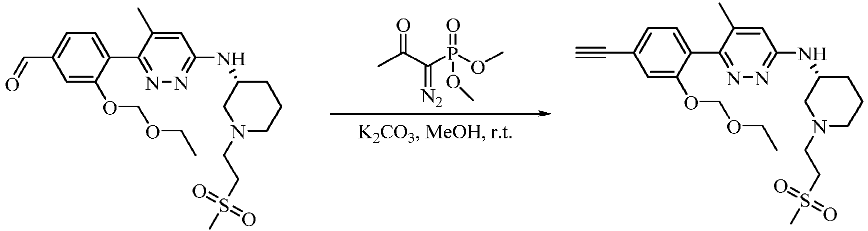

(*R*)-3-(ethoxymethoxy)-4-(4-methyl-6-(1-(2-(methylsulfonyl)ethyl)piperidin-3-atnino)pyridazin-3-yl)benzaldehyde (300 mg, 0.629 mmol, 1.0 eq.) was dissolved in methanol (3 mL); potassium carbonate (174 mg, 1.258 mmol, 2.0 eq.) and (1-diazo-2-oxopropyl)dimethyl phosphonate (182 mg, 0.944 mmol, 1.5 eq.) were successively added to the resulting mixture, to allow for reaction for 5 min at 25°C; and LC-MS detection showed that the reaction was complete. The reaction mixture was added with water (10 mL), and extracted with dichloromethane (10 mL × 3); the organic phases were combined, dried with anhydrous magnesium sulfate, and concentrated; and the resulting crude product was purified by silica gel column chromatography (dichloromethane:methanol = 50:1) to obtain a product (148 mg, yield: 49.8%).

### Step 4: Synthesis of Compound (R)-5-ethynyl-2-(4-methyl-6-(1-(2-(methylsulfonyl)ethyl)piperidin-3-amino)pyridazin-3 -yl)phenol

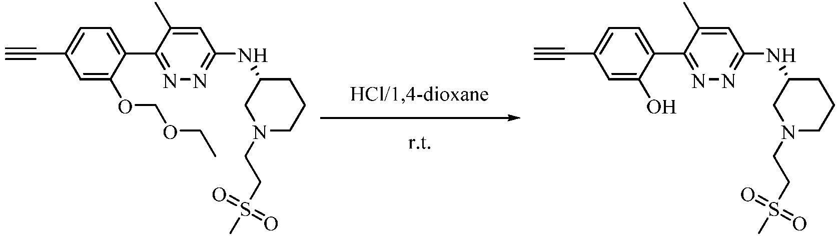

(*R*)-6-(2-ethoxymethoxy)-4-ethynylphenyl)-5-methyl-*N*-(1-(2-methylsulfonyl)ethyl)piperidin-3-yl)pyridazin-3-amine (148 mg, 0.313 mmol) was dissolved in dichloromethane (1 mL); the resulting mixture was dripped into the 1,4-dioxane solution of hydrogen chloride (1 mL, 4 mol/L), to allow for reaction for 5 min at 25°C; and LC-MS detection showed that the reaction was complete. The reaction mixture was poured into saturated sodium hydrogen carbonate in water (5 mL), and extracted with dichloromethane/methanol (10:1, 10 mL×5); the organic phases were combined, dried with anhydrous magnesium sulfate, filtered, and concentrated; and the resulting crude product was purified by silica gel column chromatography (dichloromethane:methanol = 50:1-20:1) to obtain a product (59 mg, yield: 45%).

¹H-NMR (400 MHz, DMSO-*d*₆) δ(ppm): 10.09 (s, 1H), 7.19-7.17 (d, 1H), 7.00-6.99 (m, 2H), 6.66-6.62 (m, 2H), 4.20 (s, 1H), 4.02-4.00 (m, 1H), 3.33-3.24 (m, 2H), 3.04 (s, 3H), 3.03-3.00 (m, 1H), 2.76-2.72 (m, 3H), 2.11-2.06 (m, 1H), 2.02 (s, 3H), 1.99 (m, 1H), 1.89-1.86 (m, 1H), 1.74-1.71 (m, 1H), 1.58-1.49 (m, 1H), 1.33-1.24 (m, 1H). Molecular formula: C₂₁H₂₆N₄O₃S Accurate molecular weight: 414.17 LC-MS(*m*/*z*): 415.16 [M+H]⁺

### Example 32: Synthesis of Compound (R)-2-(4-cyclopropyl-6-((1-methylpiperidin-3-yl)amino)pyridazin-3-yl)-5-(propyl-1-alkyn-1-yl)phenol (Compound 264)

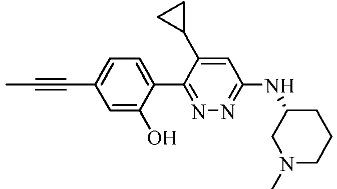

### Steps:

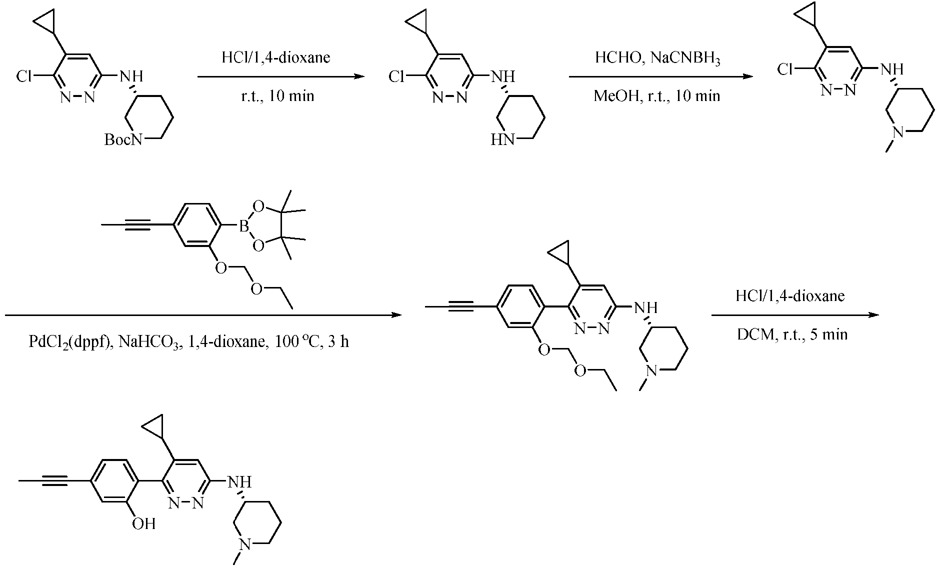

### Step 1: Synthesis of Intermediate (R)-6-chloro-5-cyclopropyl-N-(piperidin-3-yl)pyridazin-3-amine

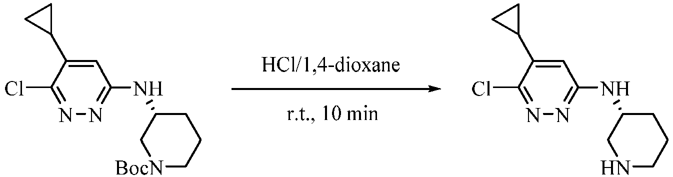

(R)-3-((6-chloro-5-cyclopropylpyridazin-3-yl)amino)piperidin-1-tert-butyl carboxylate(4.0 g, 11.34 mmol) was dissolved in dichloromethane (20 mL); the resulting mixture was dripped into the 1,4-dioxane solution of hydrogen chloride (40 mL, 4 mol/L), to allow for reaction for 5 min at 25°C; and TLC detection showed that the reaction was complete. The reaction mixture was poured into saturated sodium hydrogen carbonate in water (50 mL), and extracted with dichloromethane /methanol (10:1, 50 mL); and the organic phases were combined, dried, and concentrated to obtain a product 1.93 g, yield: 67.2%).

### Step 2: Synthesis of Intermediate (R)-6-chloro-5-cyclopropyl-N-(1-methylpiperidin-3-yl)pyridazin-3-amine

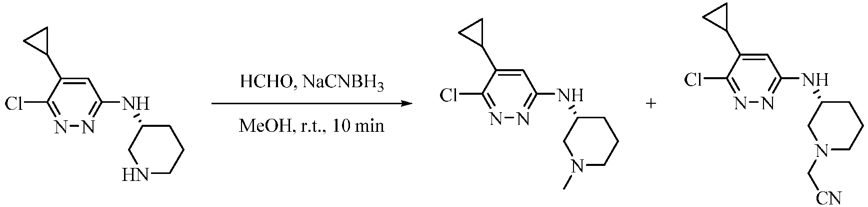

(*R*)-6-chloro-5-cyclopropyl-A-(piperidin-3-yl)pyridazin-3-amine (1.93 g, 7.64 mmol, 1.0 eq.) was dissolved in methanol (20 mL); 37% formaldehyde in water (620 mg, 7.64 mmol, 1.0 eq.) and sodium cyanoborohydride (528 mg, 8.40 mmol, 1.1 eq.) were added to the resulting mixture, to allow for reaction for 5 min at 25°C; and TLC detection showed that the reaction was complete. The reaction mixture was concentrated, added with saturated saline (15 mL), and extracted with dichloromethane/methanol (10:1, 20 mL × 3); the organic phases were combined, dried, and concentrated; and the resulting crude product was treated by silica gel column chromatography (dichloromethane:methanol = 100:1-30:1) to obtain a product (645 mg, yield: 31.6%) and a by-product (R)-2-(3-(6-chloro-5-cyclopropyl-pyridazin-3-yl)amino)piperidin-1-yl)acetonitrile (110 mg, yield: 4.9%).

### Step 3: Synthesis of Intermediate (R)-5-cyclopropyl-6-(2-ethoxymethoxy)-4-(propyl-1-alkyn-1-yl)phenyl)-N-(1-methylpiperidin-3 -yl)pyridazin-3 -amine

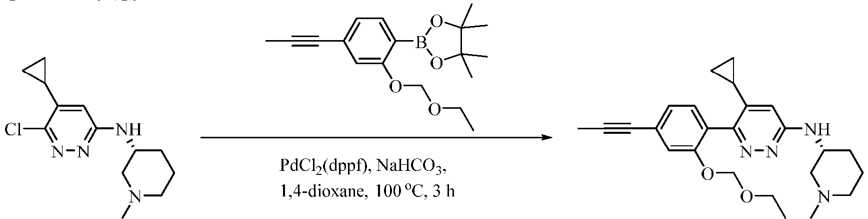

(*R*)-6-chloro-5-cyclopropyl-*N*-(1-methylpiperidin-3-yl)pyridazin-3-amine (125 mg, 0.469 mmol, 1.0 eq.) was dissolved in 1,4-dioxane (2 mL); 2-(2-(ethoxymethoxy)-4-(propyl-1-alkyn-1-ylphenyl)-4,4,5,5-trimethyl-1,3,2-dioxoboracyclopentane (223 mg, 0.704 mmol, 1.5 eq.), sodium hydrogen carbonate (79 mg, 0.938 mmol, 2.0 eq.), PdCl₂(dppf) (34 mg, 0.469 mmol, 0.1 eq.) and water (0.5 mL) were added to the resulting mixture; under the protection of nitrogen, the temperature was increased to 100°C to allow for reaction for 3 h; and LC-MS detection showed that the reaction was complete. The reaction mixture was filtered by means of diatomite, dried, and concentrated; and the resulting crude product was purified by silica gel column chromatography (dichloromethane:methanol = 50:1-20:1) to obtain a product (110 mg, yield:55.8%).

### Step 4: Synthesis of Compound (R)-2-(4-cyclopropyl-6-((1-methylpiperidin-3-yl)amino)pyridazin-3-yl)-5-(propyl-1-alkyn-1-yl)phenol

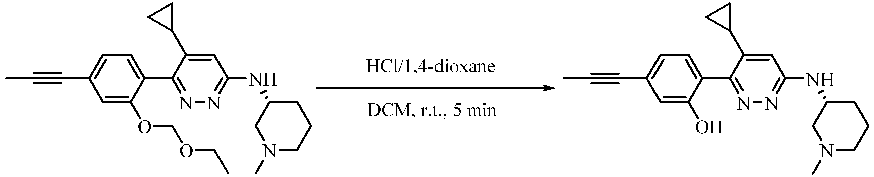

(*R*)-5-cyclopropyl-6-(2-ethoxymethoxy)-4-(prop-1-yn-1-yl)phenyl)-*N*-(1-methylpiperidin-3 -yl)pyridazin-3 -amine (110 mg, 0.262 mmol) was dissolved in dichloromethane (1 mL); the resulting mixture was dripped into the 1,4-dioxane solution of hydrogen chloride (1 mL, 4 mol/L) to allow for reaction for 5 min 25°C; and LC-MS detection showed that the reaction was complete. The reaction mixture was poured into saturated sodium hydrogen carbonate in water (5 mL), and extracted with dichloromethane /methanol (10:1,10 mL×5); the organic phases were combined, dried with anhydrous magnesium sulfate, and concentrated; and the resulting crude product was purified by silica gel column chromatography (dichloromethane:methanol = 100:1-20:1) to obtain a product (123 mg, yield: 59.4%). ¹H-NMR (400 MHz, DMSO-*d*₆) δ(ppm): 10.00 (s, 1H), 7.17-7.15 (d, 1H), 6.94-6.90 (m, 3H), 6.33 (s, 1H), 4.28 (s, 1H), 3.18-3.16 (m, 1H), 2.94-2.78 (m, 1H), 2.72 (s, 3H), 2.05 (s, 3H), 2.01-1.89 (m, 2H), 1.80-1.77 (m, 1H), 1.61-1.54 (m, 1H), 1.46-1.42 (m, 1H), 1.30-1.24 (m, 2H), 0.93-0.84 (m, 2H), 0.66-0.60 (m, 2H).

Molecular formula: C₂₂H₂₆N₄O Accurate molecular weight: 362.21 LC-MS(*m*/*z*): 363.20 [M+H]⁺

### Example 33: Synthesis of Compound (R)-2-(3-((5-cyclopropyl-6-(4-ethynyl-2-hydroxyphenyl)pyridazin-3-yl)amino)piperidin-1-yl)acetonitrile (Compound 267)

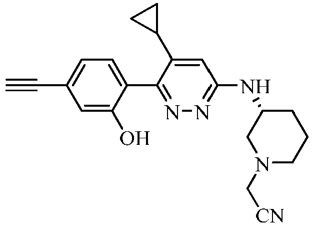

### Step 1: Synthesis of Intermediate (R)-2-(5-cyclopropyl-6-(2-ethoxymethoxy)-4-formylphenyl)pyridazin-3-yl)amino)piperidin-1-yl)acetonitrile

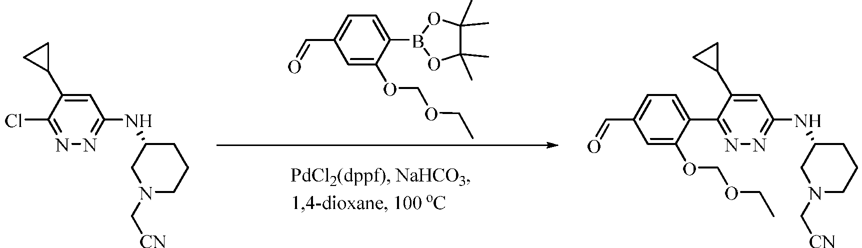

(*R*)-2-(3-(6-chloro-5-cyclopropyl-pyridazin-3-yl)amino)piperidin-1-yl)acetonitrile (110 mg, 0.377 mmol, 1.0 eq.) was dissolved in 1,4-dioxane (2 mL); 3-(ethoxymethoxy)-4-(4,4,5,5-trimethyl-1,3,2-dioxoboracyclopentan-2-yl)benzaldehyde (173 mg, 0.566 mmol, 1.5 eq.), sodium hydrogen carbonate (64 mg, 0.754 mmol, 2.0 eq.), PdCl₂(dppf) (28 mg, 0.0377 mmol, 0.1 eq.) and water (0.5 mL) were added to the resulting mixture; under the protection of nitrogen, the temperature was increased to 100°C to allow for reaction for 1 h; and TLC detection showed that the reaction was complete. The reaction mixture was filtered by means of diatomite, and concentrated; and the resulting crude product was purified by silica gel column chromatography (dichloromethane:methanol = 100:1-70:1) to obtain a product (90 mg, yield: 54.9%).

### Step 2: Synthesis of Intermediate (R)-2-(5-cyclopropyl-6-(2-ethoxymethoxy)-4-ethynylphenyl)pyridazin-3-yl)amino)piperidin-1-yl)acetonitrile

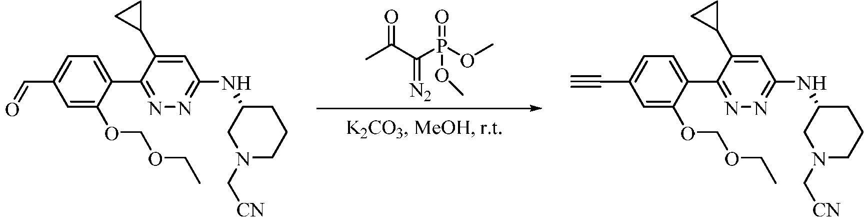

(*R*)-2-(5 -cyclopropyl-6-(2-ethoxymethoxy)-4-formylphenyl)pyridazin-3-yl)amino)piperidin-1-yl)acetonitrile (90 mg, 0.207 mmol, 1.0 eq.) was dissolved in methanol (1 mL); potassium carbonate (57 mg, 0.414 mmol, 2.0 eq.) and (1-diazo-2-oxopropyl)dimethyl phosphonate (60 mg, 0.311 mmol, 1.5 eq.) were successively added to the resulting mixture, to allow for reaction for 20 min at 25°C; and TLC detection showed that the reaction was complete. The reaction mixture was concentrated, added with water (10 mL), and extracted with dichloromethane (10 mL × 3); the organic phases were combined, dried with anhydrous magnesium sulfate, and concentrated; and the resulting crude product was purified by silica gel column chromatography (dichloromethane:methanol = 100:1) to obtain a crude product, which was directly used in a next step.

### Step 3: Synthesis of Compound (R)-2-(3-((5-cyclopropyl-6-(4-ethynyl-2-hydroxyphenyl)pyridazin-3-yl)amino)piperidin-1 -yl)acetonitrile

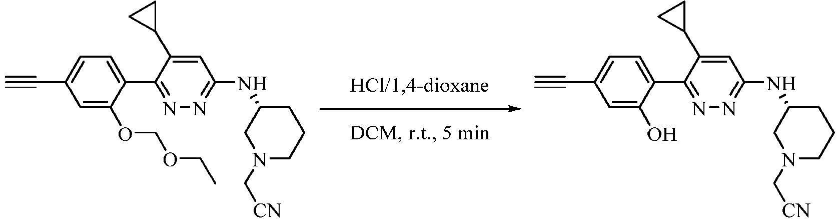

The crude product (*R*)-2-(5-cyclopropyl-6-(2-ethoxymethoxy)-4-ethynylphenyl)pyridazin-3-yl)amino)piperidin-1-yl)acetonitrile from the previous step was dissolved in dichloromethane (1 mL); the resulting mixture was dripped into the 1,4-dioxane solution of hydrogen chloride (1 mL, 4 mol/L) to allow for reaction for 5 min at 25°C; and LC-MS detection showed that the reaction was complete. The reaction mixture was poured into saturated sodium hydrogen carbonate in water (5 mL), and extracted with dichloromethane/methanol (10:1, 10 mL×5); the organic phases were combined, dried with anhydrous magnesium sulfate, and concentrated; and the resulting crude product was purified by preparative thin-layer chromatography (dichloromethane:methanol = 10:1) to obtain a product (5 mg, two-step yield: 6.5%).

¹H-NMR (400 MHz, DMSO-*d*₆) δ(ppm): 10.07 (s, 1H), 7.24-7.22 (d, 1H), 7.02-6.99 (m, 2H), 6.58-6.56 (d, 1H), 6.31 (s, 1H), 4.19 (s, 1H), 4.09-4.07 (m, 1H), 3.77 (s, 2H), 2.97-2.95 (m, 1H), 2.68-2.63 (m, 1H), 2.28-2.23 (m, 1H), 2.13-2.08 (s, 1H), 1.86 -1.83 (m, 1H), 1.78-1.75 (m, 1H), 1.61-1.52 (m, 2H), 1.34-1.28 (m, 1H), 0.88-0.84 (m, 2H), 0.63-0.60 (m, 2H).

Molecular formula: C₂₂H₂₃N₅O Accurate molecular weight: 373.19 LC-MS(*m*/*z*): 374.11 [M+H]⁺

### Example 34: Synthesis of (R)-5-ethynyl-2-(6-((1-(2-hydroxyethyl)piperidin-3-yl)amino)-4-methylpyridazin-3-yl)phenol (Compound 253)

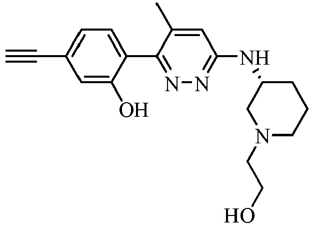

### Step 1: Synthesis of (R)-2-(3-((6-chloro-5-methylpyridazin-3-yl)amino)piperidin-1-yl)ethan-1-ol

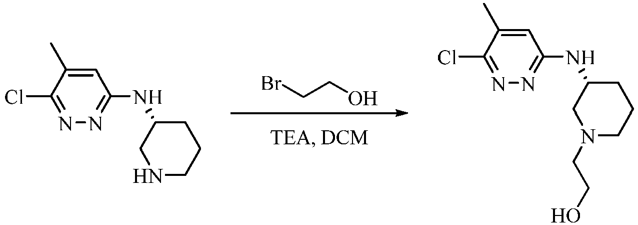

(*R*)-6-chloro-5-methyl-A-(piperidin-3-yl)pyridazin-3-amine (250 mg, 1.10 mmol, 1.0 eq.) was dissolved in DCM (5 mL); triethylamine (223 mg, 2.20 mmol, 2.0 eq.) and 2-bromoethanol (275 mg, 2.20 mmol, 2.0 eq.) were added; and the resulting mixture was stirred for 22 h at room temperature. The resulting mixture was concentrated, and the resulting crude product was purified by silica gel column chromatography (DCM:MeOH = 50:1-15:1) to obtain a product (282 mg, yield: 94.4%).

### Step 2: Synthesis of (R)-3-(ethoxymethoxy)-4-(6-((1-(2-hydroxyethyl)piperidin-3-yl)atnino)-4-methylpyridazin-3-yl)benzaldehyde

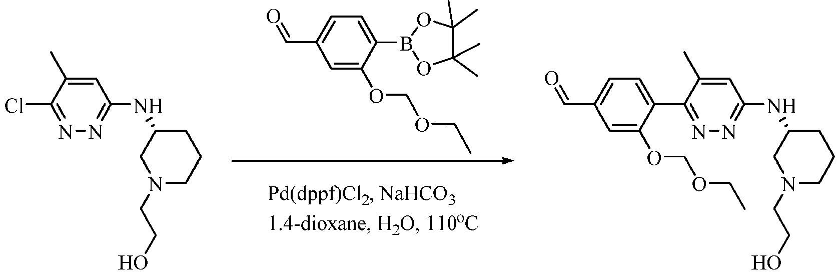

3-(ethoxymethoxy)-4-(4,4,5,5-tetramethyl-1,3,2-dioxoboracyclopentan-2-yl)benzaldehyde (382 mg, 1.25 mmol, 1.2 eq.), (*R*)-2-(3-((6-chloro-5-methylpyridazin-3-yl)amino)piperidin-1-yl)ethan-1-ol (282 mg, 1.04 mmol, 1.0 eq.), Pd(dppf)Cl₂ (76.1 mg, 0.104 mmol, 0.1 eq.) and NaHCO₃ (175 mg, 2.08 mmol, 2.0 eq.) were successively added to 1,4-dioxane (10 mL); H₂O (5 mL) was added; and the resulting mixture was heated to 110°C under the protection of nitrogen to allow for reaction for 2 h. The reaction mixture was cooled to room temperature, quenched by adding water (30 mL), and extracted with EA (30 mL × 2); the organic phases were dried with anhydrous sodium sulfate and filtered; the filtrate was concentrated under reduced pressure; and the resulting crude product was purified by silica gel column chromatography (DCM:MeOH = 50:1-15:1) to obtain a product (314 mg, yield: 72.7%).

### Step 3: Synthesis of (R)-2-(3-((6-(2-(ethoxymethoxy)-4-ethynylphenyl)-5-methylpyridazin-3-yl)amino)piperidin-1-yl)ethan-1-ol

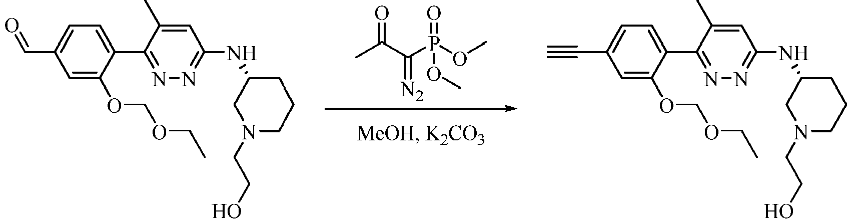

(*R*)-3-(ethoxymethoxy)-4-(6-((1-(2-hydroxyethyl)piperidin-3-yl)amino)-4-methylpyridazin-3-yl)benzaldehyde (314 mg, 0.758 mmol, 1.0 eq.) was dissolved in MeOH(10 mL); K₂CO₃ (210 mg, 1.52 mmol, 2.0 eq.) and (1-diazo-2-oxopropyl)dimethyl phosphonate (218 mg, 1.14 mmol, 1.5 eq.) were added ; and the resulting mixture was stirred for 2 h at room temperature. The resulting mixture was concentrated, and the resulting crude product was purified by silica gel column chromatography (DCM:MeOH = 50:1-15:1) to obtain a product (253 mg, yield: 81.4%).

### Step 4: Synthesis of (R)-5-ethynyl-2-(6-((1-(2-hydroxyethyl)piperidin-3-yl)amino)-4-methylpyridazin-3-yl)phenol

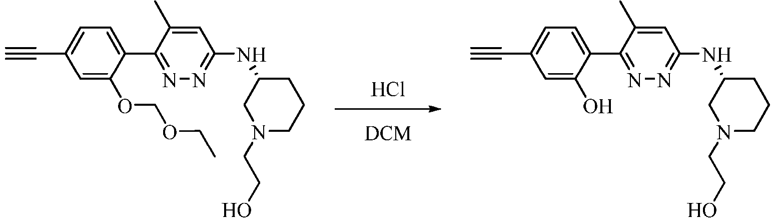

(*R*)-2-(3 -((6-(2-(ethoxymethoxy)-4-ethynylphenyl)-5 -methylpyridazin-3-yl)amino)piperidin-1-yl)ethan-1-ol (253 mg, 0.616 mmol, 1.0 eq.) was dissolved in DCM (5 mL); then the 1,4-dioxane solution of hydrogen chloride (4 mol/L, 0.46 mL, 1.85 mmol, 3.0 eq.) was dropwise added; and the resulting mixture was stirred for 2 h at room temperature. The resulting mixture was adjusted to a pH value of 8 by using saturated NaHCOs in water, and extracted with DCM (20 mL × 2); the organic phases were dried with anhydrous sodium sulfate and filtered; the filtrate was concentrated under reduced pressure; and the resulting crude product was purified by preparative thin-layer chromatography (DCM:MeOH = 8:1) to obtain a product (158 mg, yield: 72.7%).

¹HNMR(400MHz, DMSO-*d*₆) δ(ppm): 10.15 (s, 1H), 7.17 (d, *J* = 7.7 Hz, 1H), 7.02-6.98 (m, 2H), 6.74 (s, 1H), 6.69 (s, 1H), 4.61 (s, 1H), 4.20 (s, 1H), 4.13 (s, 1H), 3.57 (s, 2H), 3.11 (s, 1H), 2.83 (s, 1H), 2.60 (s, 2H), 2.35-2.19 (m, 2H), 2.02 (s, 3H), 1.86 (s, 1H), 1.74 (s, 1H), 1.62 (s, 1H), 1.38 (s, 1H).

Molecular formula: C₂₀H₂₄N₄O₂ Accurate molecular weight: 352.19 LC-MS(Pos, *m*/*z*)=353.17[M+H]⁺.

### Example 35: Synthesis of (R)-2-(3-((6-(4-ethynyl-2-hydroxyphenyl)-5-methylpyridazin-3-yl)amino)piperidin-1-yl)acetic acid (Compound 256)

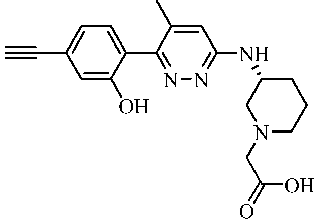

### Step 1: Synthesis of (R)-2-(3-((6-chloro-5-methylpyridazin-3-yl)amino)piperidin-1-yl)ethyl acetate

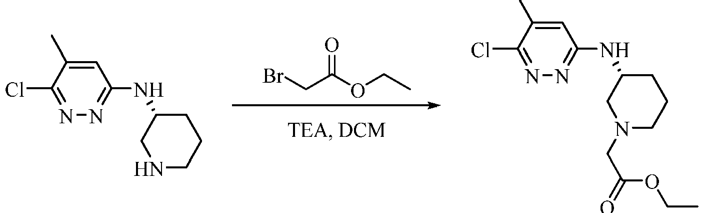

(*R*)-6-chloro-5-methyl-*N*-(piperidin-3-yl)pyridazin-3-amine (250 mg, 1.10 mmol, 1.0 eq.) was dissolved in DCM (5 mL); triethylamine (223 mg, 2.20 mmol, 2.0 eq.) and bromoethyl acetate (367 mg, 2.20 mmol, 2.0 eq.) were added; and the resulting mixture was stirred for 20 h at room temperature. The resulting mixture was concentrated, and the resulting crude product was purified by silica gel column chromatography (DCM:MeOH = 50:1-15:1) to obtain a product (325 mg, yield: 94.2%).

### Step 2: Synthesis of (R)-2-(3-((6-(2-(ethoxymethoxy)-4-formylphenyl)-5-methylpyridazin-3-yl)atnino)piperidin-1-yl)ethyl acetate

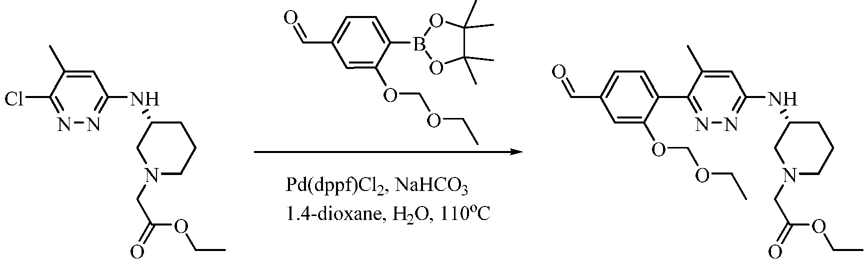

3-(ethoxymethoxy)-4-(4,4,5,5-tetramethyl-1,3,2-dioxoboracyclopentan-2-yl)benzaldehyde (382 mg, 1.25 mmol, 1.2 eq.), (*R*) -2-(3-((6-chloro-5-methylpyridazin-3-yl)amino)piperidin-1-yl)ethan-1-ol (282 mg, 1.04 mmol, 1.0 eq.), Pd(dppf)Cl₂ (76.1 mg, 0.104 mmol, 0.1 eq.) and NaHCOs (175 mg, 2.08 mmol, 2.0 eq.) were successively added to 1,4-dioxane (10 mL); H₂O (5 mL) was added; and the resulting mixture was heated to 110°C under the protection of nitrogen to allow for reaction for 1 h. The reaction mixture was cooled to room temperature, quenched by adding water (30 mL), and extracted with EA (30 mL × 2); the organic phases were dried with anhydrous sodium sulfate and filtered; the filtrate was concentrated under reduced pressure; and the resulting crude product was purified by silica gel column chromatography (DCM:MeOH = 100:1-50:1) to obtain a product (402 mg, yield: 84.7%).

### Step 3: Synthesis of (R)-2-(3-((6-(2-(ethoxymethoxy)-4-ethynylphenyl)-5-methylpyridazin-3-yl)amino)piperidin-1-yl)methyl acetate

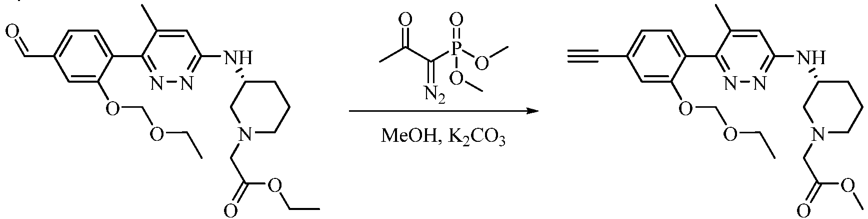

(*R*)-2-(3 -((6-(2-(ethoxymethoxy)-4-formylphenyl)-5 -methylpyridazin-3-yl)amino)piperidin-1-yl)ethyl acetate(402 mg, 0.881 mmol, 1.0 eq.) was dissolved in MeOH(10 mL); K₂CO₃ (243 mg, 1.76 mmol, 2.0 eq.) and (1-diazo-2-oxopropyl)dimethyl phosphonate (254 mg, 1.32 mmol, 1.5 eq.) were added; and the resulting mixture was stirred for 2 h at room temperature. The resulting mixture was concentrated, and the resulting crude product was purified by silica gel column chromatography (DCM:MeOH = 100:1-50:1) to obtain a product (352 mg, yield: 91.2%).

### Step 4: Synthesis of (R)-2-(3-((6-(4-ethynyl-2-hydroxyphenyl)-5-methylpyridazin-3-yl)amino)piperidin-1-yl)acetic acid

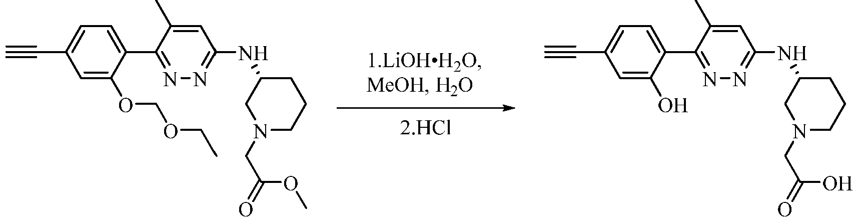

(*R*)-2-(3-((6-(2-(ethoxymethoxy)-4-ethynylphenyl)-5-methylpyridazin-3-yl)amino)piperidin-1-yl)methyl acetate (330 mg, 0.753 mmol, 1.0 eq.) was dissolved in MeOH (2 mL); H₂O (2 mL) and lithium hydroxide monohydrate (63.2 mg, 1.51 mmol, 2.0 eq.) were added; and the resulting mixture was stirred at room temperature for 2 h. The mixture was concentrated, adjusted to a pH value of 2 by using dilute hydrochloric acid , stirred for 1 h at room temperature, and purified by reversed-phase column chromatography (ACN:H₂O = 20:80) to obtain a product (182 mg, yield: 66.0%).

¹HNMR(400MHz, DMSO-*d*₆) δ(ppm): 10.37 (s, 1H), 7.25 (s, 1H), 7.17 (d, *J =* 7.8 Hz, 1H), 7.07 (d, *J =* 1.4 Hz, 1H), 7.01-6.99 (m, 1H), 6.80 (s, 1H), 4.34-4.33 (m, 1H), 4.21 (s, 1H), 3.85 (s, 2H), 3.54 (d, *J =* 10.0 Hz, 1H), 3.28 (d, *J =* 11.3Hz, 1H), 2.92-2.87 (m, 1H), 2.82-2.76 (m, 1H), 2.04 (s, 3H), 1.98-1.95 (m, 1H), 1.90-1.86 (m, 1H), 1.83-1.79 (m, 1H), 1.52-1.44 (m, 1H).

Molecular formula: C₂₀H₂₂N₄O₃ Accurate molecular weight: 366.17 LC-MS(Pos, *m*/*z*)=367.14[M+H]⁺.

### Example 36: Synthesis of 5-ethyl-2-(6-((3-hydroxybenzyl)amino)-4-methylpyridazin-3-yl)phenol (Compound 261)

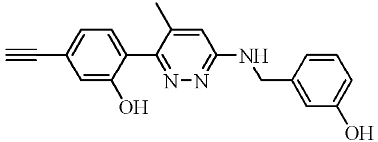

### Step 1: Synthesis of 3-(((6-chloro-5-methylpyridazin-3-yl)amino)methyl)phenol

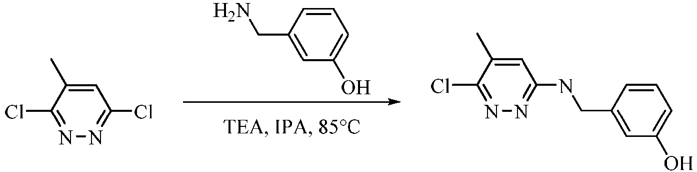

3,6-dicholoro-4-methylpyridazine (2.0 g, 12.3 mmol, 1.0 eq.), 3-(aminomethyl)phenol (1.51 g, 12.3 mmol, 1.0 eq.) and triethylamine (6.22 g, 61.5 mmol, 5.0 eq.) were added to isopropanol (30 mL), and heated to 85°C to allow for reaction for 24 h. The reaction mixture was concentrated, dissolved by addition of EA(30 mL), and washed with water (20 mL × 2); the organic phases were dried with anhydrous sodium sulfate and filtered; the filtrate was concentrated under reduced pressure; and the resulting crude product was purified by silica gel column chromatography (DCM:MeOH = 100:1-60:1) to obtain a product (300 mg, yield: 9.8%).

### Step 2: Synthesis of 3-(ethoxymethoxy)-4-(6-((3-hydroxybenzyl)amino)-4-methylpyridazin-3-yl)benzaldehyde

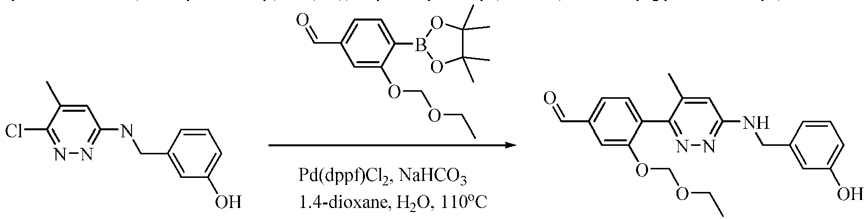

3-(ethoxymethoxy)-4-(4,4,5,5-tetramethyl-1,3,2-dioxoboracyclopentan-2-yl)benzaldehyde (294 mg, 0.961 mmol, 1.2 eq.), 3-(((6-chloro-5-methylpyridazin-3-yl)amino)methyl)phenol (200 mg, 0.801 mmol, 1.0 eq.), Pd(dppf)Cl₂ (58.6 mg, 0.0801 mmol, 0.1 eq.) and NaHCO₃ (135 mg, 1.60 mmol, 2.0 eq.) were successively added to 1,4-dioxane (10 mL); H₂O(5 mL) was added; and the resulting mixture was heated to 110°C under the protection of nitrogen to allow for reaction for 1 h. The reaction mixture was cooled to room temperature, quenched by adding water (30 mL), and extracted with EA (20 mL × 2); the organic phases were dried with anhydrous sodium sulfate and filtered; the filtrate was concentrated under reduced pressure; and the resulting crude product was purified by silica gel column chromatography (DCM:MeOH = 100:1-50:1) to obtain a product (248 mg, yield: 78.7%).

### Step 3: Synthesis of 3-((6-(2-(ethoxymethoxy)-4-ethynylphenyl)-5-methylpyridazin-3-yl)amino)methyl)phenol

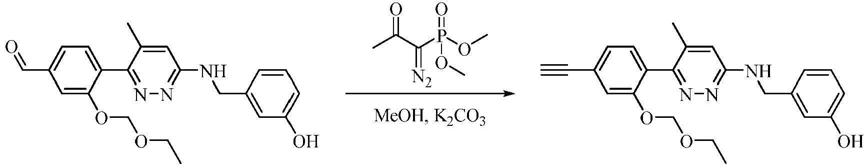

3-(ethoxymethoxy)-4-(6-((3-hydroxybenzyl)amino)-4-methylpyridazin-3-yl)benzaldehyde (248 mg, 0.630 mmol, 1.0 eq.) was dissolved in MeOH(10 mL); K₂CO₃ (174 mg, 1.26 mmol, 2.0 eq.) and (1-diazo-2-oxopropyl)dimethyl phosphonate (182 mg, 0.945 mmol, 1.5 eq.) were added; and the resulting mixture was stirred for 2 h at room temperature. The resulting mixture was concentrated, and the resulting crude product was purified by silica gel column chromatography (DCM:MeOH = 100:1-50:1) to obtain a product (215 mg, yield: 87.6%).

### Step 4: Synthesis of 5-ethyl-2-(6-((3-hydroxybenzyl)amino)-4-methylpyridazin-3-yl)phenol

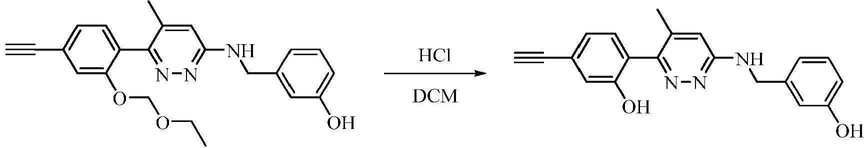

3-((6-(2-(ethoxymethoxy)-4-ethynylphenyl)-5-methylpyridazin-3-yl)amino)methyl)phenol (215 mg, 0.552 mmol, 1.0 eq.) was dissolved in DCM (5 mL); then, the 1,4-dioxane solution of hydrogen chloride (4 mol/L, 0.42 mL, 1.66 mmol, 3.0 eq.) was dropwise added; and the resulting mixture was stirred for 1 h at room temperature. The resulting mixture was adjusted to a pH value of 8 by using saturated NaHCOs in water, and extracted with DCM (20 mL × 3); the organic phases were dried with anhydrous sodium sulfate and filtered; the filtrate was concentrated under reduced pressure; and the resulting crude product was purified by silica gel column chromatography (DCM:MeOH = 100:1-20:1) to obtain a product (116 mg, yield: 63.4%).

¹HNMR (400MHz, DMSO-*d*₆) δ(ppm): 10.10 (s, 1H), 9.34 (s, 1H), 7.31-7.28 (m, 1H), 7.18 (d, *J =* 8.1 Hz, 1H), 7.14-7.10 (m, 1H), 6.99 (d, *J* = 5.6 Hz, 2H), 6.79 (d, *J* = 6.6 Hz, 2H), 6.71 (s, 1H), 6.63 (d, *J* = 8.3 Hz, 1H), 4.51 (d, *J=* 5.7 Hz, 2H), 4.19 (s, 1H), 2.03 (s, 3H).

Molecular formula: C₂₀H₁₇N₃O₂ Accurate molecular weight: 331.13 LC-MS(Pos, *m*/*z*)=332.10[M+H]⁺.

### Example 37: Synthesis of (R)-2-(5-(dimethylamino)-3-((1-methylpiperidin-3-yl)amino)-1,2,4-triazin-6-yl)-5-ethynylphenol (Compound 181)

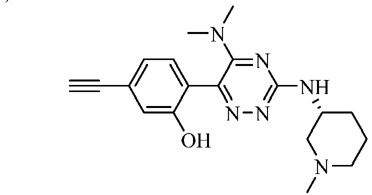

### Step 1: Synthesis of 6-chloro-N,N-dimethyl-3-(methylthio)-1,2,4-triazin-5-amine

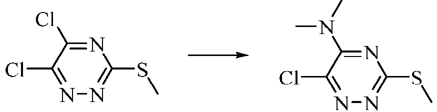

5,6-dicholoro-3-(methylthio)-1,2,4-triazine (6.85 g, 34.92 mmol, 1.0 eq.) was dispersed in THF (100 mL) and dimethylamine in a tetrahydrofuran solution (2 mol/L, 30 mL) to allow for reaction for 20 min at room temperature. The reaction mixture was concentrated under reduced pressure, and the resulting crude product was purified by silica gel column chromatography (EA:PE = 1:10) to obtain a product (1.2 g, yield: 16.8%).

### Step 2: Synthesis of 4-(5-(dimethylamino)-3-(methylthio)-1,2,4-triazin-6-yl)-3-(ethoxymethoxy)benzaldehyde

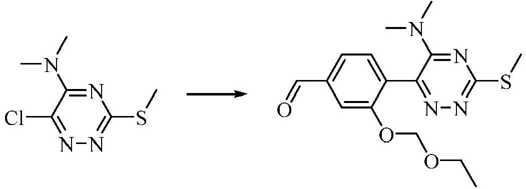

6-chloro-*N*,*N*-dimethyl-3-(methylthio)-1,2,4-triazin-5-amine (1.2 g, 5.86 mmol, 1.0 eq.), 3-(ethoxymethoxy)-4-(4,4,5,5-tetramethyl-1,3,2-dioxoboracyclopentan-2-yl)benzaldehyde (2.15 g, 7.03 mmol, 1.2 eq.), Pd(dppf)Cl₂ (432 mg, 0.59 mmol, 0.1 eq.) and NaHCOs(984 mg, 11.72 mmol, 2.0 eq.) were dissolved in 1,4-dioxane (20 mL) and H₂O (4 mL) to allow for reaction for 3 h at 110°C under the protection of nitrogen. TLC detection showed that the reaction was complete; the reaction mixture was poured into water (30 mL), and extracted with EA(30 mL × 3); and the organic phases were dried, concentrated, and purified by silica gel column chromatography (MeOH:DCM = 1:100) to obtain a product (1.7 g, yield: 83.3%).

### Step 3: Synthesis of 6-(2-(ethoxymethoxy)-4-ethynylphenyl)-N,N-dimethyl-3-(methylthio)-1,2,4-triazin-5-amine

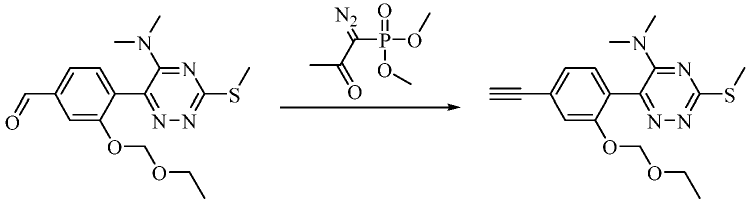

4-(5-(dimethylamino)-3-(methylthio)-1,2,4-triazin-6-yl)-3-(ethoxymethoxy)benzaldehyde (1.7 g, 4.88 mmol, 1.0 eq.) and potassium carbonate (1.01 g, 7.32 mmol, 1.5 eq.) were dispersed in MeOH (30 mL); and after 5 min, (1-diazo-2-oxopropyl)dimethyl phosphonate (1.41 g, 7.32 mmol, 1.5 eq.) was added to allow for reaction for 2 h. TLC detection showed that the reaction was complete; the reaction mixture was concentrated under reduced pressure; the resulting crude product was dispersed with water (30 mL), and extracted with EA(20 mL × 2); and the organic phases were dried and concentrated to obtain a product (1.5 g, yield: 89.3%).

### Step 4: Synthesis of 6-(2-(ethoxymethoxy)-4-ethynylphenyl)-N,N-dimethyl-3-(methylsulfinyl)-1,2,4-triazin-5-amine

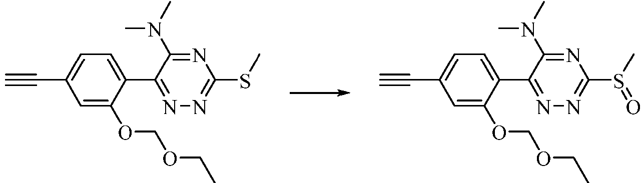

6-(2-(ethoxymethoxy)-4-ethynylphenyl)-*N,N*-dimethyl-3-(methylthio)-1,2,4-triazin-5-amine (1.5 g, 4.35 mmol, 1.0 eq.) was dissolved in DCM (30 mL); and metachloroperbenzoic acid (883 mg, 4.35 mmol, 1.0 eq.) with a mass fraction of 85% was added to allow for reaction for 30 min at room temperature. TLC monitoring showed that the reaction was complete; and the reaction mixture was concentrated under reduced pressure to obtain a crude product (1.57 g, yield: 100%).

### Step 5: Synthesis of (R)-3-((5-(dimethylatnino)-6-(2-(ethoxymethoxy)-4-ethynylphenyl)-1,2,4-triazin-3-yl)amino)piperidin-1-tert-butyl carboxylate

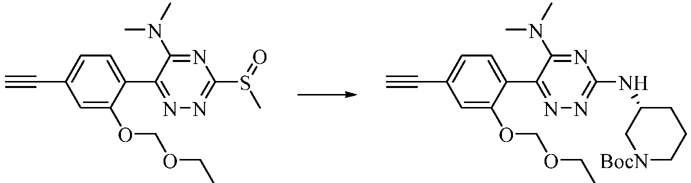

6-(2-(ethoxymethoxy)-4-ethynylphenyl)-*N,N*-dimethyl-3-(methylsulfinyl)-1,2,4-triazin-5-amine (1.57 mg, 4.35 mmol, 1.0 eq.), (R)-3-aminopiperidin-1-tert-butyl carboxylate (1.30 g, 6.52 mmol, 1.5 eq.) and TEA (880 mg, 8.70 mmol, 2.0 eq.) were dissolved in dioxane (30 mL), and allowed to react for 23 h at 100°C. TLC monitoring showed that the reaction was complete; the reaction mixture was concentrated; the resulting crude product was dispersed with water (20 mL), and extracted with EA(20 mL × 3); and, the organic phases were dried, and concentrated to obtain a product (1.5 g, yield: 69.4%).

### Step 6: Synthesis of (R)-2-(5-(dimethylamino)-3-(piperidin-3-ylamino)-1,2,4-triazin-6-yl)-5-ethynylphenol

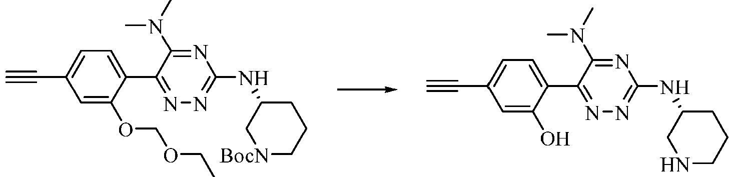

*(R)-3 -((5 -(dimethylamino)-6-(2-(ethoxymethoxy)-4-ethynylphenyl)-1,2,4-triazin-3 -yl)amino)piperidin-1 -tert-*butyl carboxylate (1.5 g, 3.02 mmol, 1.0 eq.) was dissolved in DCM (30 mL); then, the 1,4-dioxane solution of hydrogen chloride (4 mol/L, 6 mL) was dropwise added to allow for reaction for 2 h at room temperature. LC-MS monitoring showed that the reaction was complete; the reaction mixture was poured into water (30 mL), and separated; the aqueous phase was reserved, adjusted to a pH value of about 10 by using NaHCOs in water, and then extracted with DCM (20 mL × 2); the organic phases were dried and concentrated; and the resulting crude product was purified by silica gel column chromatography (MeOH:DCM = 1:15) to obtain a product (600 mg, yield: 58.8%).

### Step 7: Synthesis of (R)-2-(5-(dimethylamino)-3-((1-methylpiperidin-3-yl)amino)-1,2,4-triazin-6-yl)-5-ethynylphenol

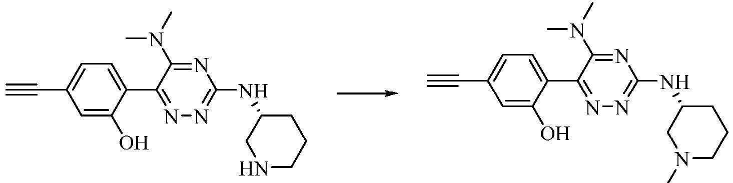

(*R*)-2-(5-(dimethylamino)-3-(piperidin-3-ylamino)-1,2,4-triazin-6-yl)-5-ethynylphenol (600 mg, 1.77 mmol, 1.0 eq.) and formaldehyde in water (37%) (144 mg, 1.77 mmol, 1.0 eq.) were dissolved in methanol (6 mL), stirred for 5 min at room temperature; and sodium cyanoborohydride (111 mg, 1.77 mmol, 1.0 eq.) was added to allow for reaction for 5min at room temperature. TLC monitoring showed that the reaction was complete; the reaction mixture was concentrated under reduced pressure; the resulting crude product was dispersed in water (10 mL), and extracted with DCM (20 mL × 3); the organic phases were dried and concentrated; and the resulting crude product was purified by silica gel column chromatography (MeOH:DCM = 1: 15) to obtain a product (200 mg, yield: 32.1%).

¹HNMR (400MHz, DMSO-*d₆*) δ(ppm): 9.91 (s, 1H), 7.29-7.27 (d, 1H), 7.01-6.99 (d, 1H), 6.95 (s, 1H), 6.75 (s, 1H), 4.18 (s, 1H), 3.91 (s, 1H), 2.88-2.86 (d, 1H), 2.82 (s, 6H), 2.61-2.58 (d, 1H), 2.18 (s, 3H), 1.95-1.79 (m, 3H), 1.71-1.66 (m, 1H), 1.57-1.47 (m, 1H), 1.35-1.27 (m, 1H).

Molecular formula: C₁₉H₂₄N₆O Accurate molecular weight: 352.20 LC-MS(Pos, m/z)=353.15 [M+H]⁺.

### Example 38: Synthesis of (R)-5-ethynyl-2-(5-methoxy-3-((1-methylpiperidin-3-yl)amino)-1,2,4-triazin-6-yl)phenol (Compound 182)

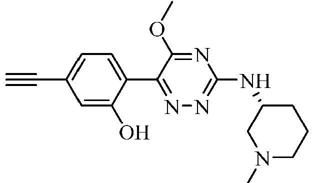

### Step 1: Synthesis of 6-chloro-5-methoxy-3-(methylthio)-1,2,4-triazine

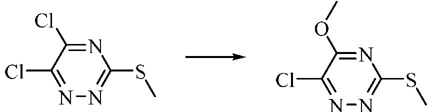

5,6-dicholoro-3-(methylthio)-1,2,4-triazine (6.85 g, 34.92 mmol, 1.0 eq.) wase dispersed in MeOH (50 mL) to allow for reaction for 10 min at room temperature. The reaction mixture was concentrated under reduced pressure, and the resulting crude product was purified by silica gel column chromatography (EA:PE = 1:20 AM) to obtain a product (1.5 g, yield: 22.4%).

### Step 2: Synthesis of 3-(ethoxymethoxy)-4-(5-methoxy-3-(methylthio)-1,2,4-triazin-6-yl)benzaldehyde

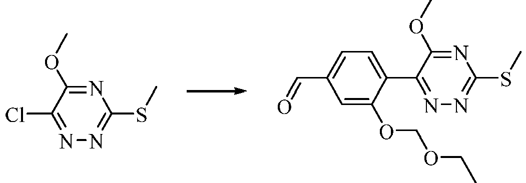

6-chloro-5-methoxy-3-(methylthio)-1,2,4-triazine (1.5 g, 7.82 mmol, 1.0 eq.), 3-(ethoxymethoxy)-4-(4,4,5,5-tetramethyl-1,3,2-dioxoboracyclopentan-2-yl)benzaldehyde (2.63 g, 8.60 mmol, 1.1 eq.), Pd(dppf)Cl₂ (571 mg, 0.78 mmol, 0.1 eq.) and NaHCO₃ (1.31 g, 15.64 mmol, 2.0 eq.) were dissolved in 1,4-dioxane (20 mL) and H₂O (4 mL) to allow for reaction for 2 h at 110°C under the protection of nitrogen. TLC detection showed that the reaction was complete; the reaction mixture was poured into water (20 mL), and extracted with EA (20 mL × 3); the organic phases were dried and concentrated; and the resulting crude product was purified by silica gel column chromatography (EA:PE = 1:20) to obtain a product (1.0 g, yield: 38.2%).

### Step 3: Synthesis of 6-(2-(ethoxymethoxy)-4-ethynylphenyl)-5-methoxy-3-(methylthio)-1,2,4-triazine

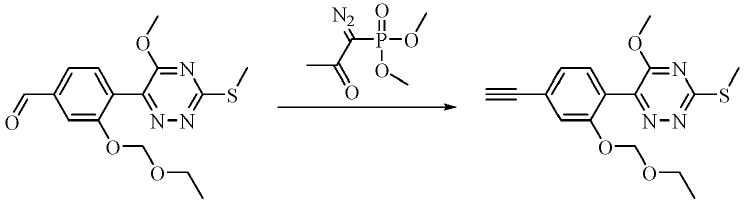

3-(ethoxymethoxy)-4-(5-methoxy-3-(methylthio)-1,2,4-triazin-6-yl)benzaldehyde (1.0 g, 2.98 mmol, 1.0 eq.), (1-diazo-2-oxopropyl)dimethyl phosphonate (859 mg, 4.47 mmol, 1.5 eq.) and potassium carbonate (617 mg, 4.47 mmol, 1.5 eq.) were dispersed in MeOH(20 mL) to allow for reaction for 1 h. TLC detection showed that the reaction was complete; the reaction mixture was concentrated under reduced pressure; the resulting crude product was dispersed with water (30 mL), and extracted with EA (20 mL × 2); and the organic phases were dried, and concentrated to obtain a product (988 mg, yield: 100%).

### Step 4: Synthesis of 6-(2-(ethoxymethoxy)-4-ethynylphenyl)-N,N-dimethyl-3-(methylsulfinyl)-1,2,4-triazin-5-amine

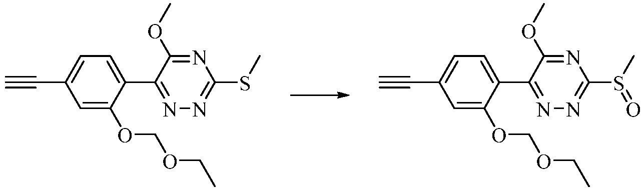

6-(2-(ethoxymethoxy)-4-ethynylphenyl)-5-methoxy-3-(methylthio)-1,2,4-triazine (988 mg, 2.98 mmol, 1.0 eq.) was dissolved in DCM (20 mL); metachloroperbenzoic acid (605 mg, 2.98 mmol, 1.0 eq.) with a mass fraction of 85% was added to allow for reaction for 20 min at room temperature. TLC monitoring showed that the reaction was complete; the reaction mixture was poured into saturated sodium carbonate in water (20 mL), and extracted with DCM (20 mL × 2); and the organic phases were dried, and concentrated to obtain a product (1.03 g, yield: 100%).

### Step 5: Synthesis of (R)-3-((6-(2-(ethoxymethoxy)-4-ethynylphenyl)-5-methoxy-1,2,4-triazin-3-yl)amino)piperidin-1-tert-butyl carboxylate

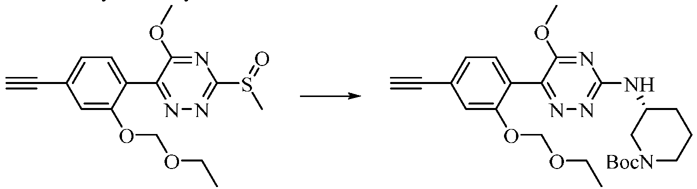

6-(2-(ethoxymethoxy)-4-ethynylphenyl)-*N,N*-dimethyl-3-(methylsulfinyl)-1,2,4-triazin-5-amine (1.03 mg, 2.98 mmol, 1.0 eq.) and (R)-3-aminopiperidin-1-tert-butyl carboxylate (1.20 g, 5.96 mmol, 2.0 eq.) were dissolved in 1,4-dioxane (20 mL) to allow for reaction for 1 h at 100°C. LC-MS monitoring showed that the reaction was complete; the reaction mixture was concentrated; and the resulting crude product was purified by silica gel column chromatography (EA:PE = 1: 1) to obtain a product (600 mg, yield: 41.7%).

### Step 6: Synthesis of (R)-5-ethynyl-2-(5-methoxy-3-(piperidin-3-ylamino)-1,2,4-triazin-6-yl)phenol

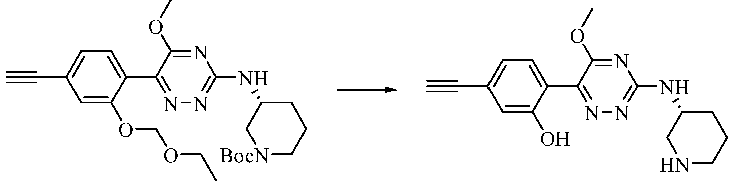

(*R*)-3 -((6-(2-(ethoxymethoxy)-4-ethynylphenyl)-5 -methoxy-1,2,4-triazin-3-yl)amino)piperidin-1-tert-butyl carboxylate (0.6 g, 1.03 mmol, 1.0 eq.) was dissolved in DCM (10 mL); and then, the 1,4-dioxane solution of hydrogen chloride (4 mol/L, 1 mL) was dropwise added to allow for reaction for 2.5 h at room temperature. LC-MS monitoring showed that the reaction was complete; the reaction mixture was washed with water (20 mL); the aqueous phase was reserved, adjusted to a pH value of about 9 by using NaHCOs in water, and extracted with DCM (20 mL × 2); and the organic phases were dried, and concentrated to obtain a product (250 mg, yield: 74.6%).

### Step 7: Synthesis of (R)-5-ethynyl-2-(5-methoxy-3-((1-methylpiperidin-3-yl)atnino)-1,2,4-triazin-6-yl)phenol

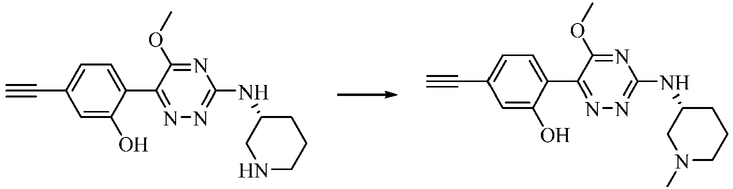

(*R*)-5-ethynyl-2-(5-methoxy-3-(piperidin-3-ylamino)-1,2,4-triazin-6-yl)phenol (250 mg, 0.77 mmol, 1.0 eq.) and 37% formaldehyde in water (62 mg, 0.77 mmol, 1.0 eq.) were dissolved in methanol (5 mL), and stirred at room temperature for 5min; and sodium cyanoborohydride (48 mg, 0.77 mmol, 1.0 eq.) was added to allow for reaction for 5min at room temperature. TLC monitoring showed that the reaction was complete; the reaction mixture was concentrated under reduced pressure; the resulting crude product was dispersed with water (15 mL), and extracted with DCM (20 mL × 2); and the organic phases were dried, concentrated, and purified by preparative thin-layer chromatography (MeOH:DCM = 1:8) to obtain a product (120 mg, yield: 46.0%).

¹HNMR (400MHz, DMSO-*d*₆) δ(ppm): 10.61 (s, 1H), 7.94 (s, 1H), 7.42-7.40 (d, 1H), 7.00-6.98 (d, 1H), 4.22 (s, 2H), 3.92 (s, 3H), 3.00 (s, 1H), 2.51 (s, 6H), 1.91-1.83 (d, 2H), 1.73 (s, 1H), 1.49 (s, 1H).

Molecular formula: C₁₈H₂₁N₅O₂ Accurate molecular weight: 339.17 LC-MS(Pos, *m*/*z*)=340.19 [M+H]⁺.

### Example 39: Synthesis of Compound 5-ethynyl-2-(((tetrahydrofuran-2-yl)methyl)amino)phthalazin-1-yl)phenol (Compound 63)

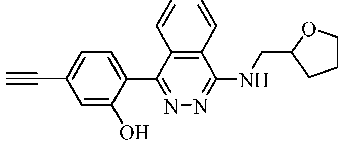

### Steps:

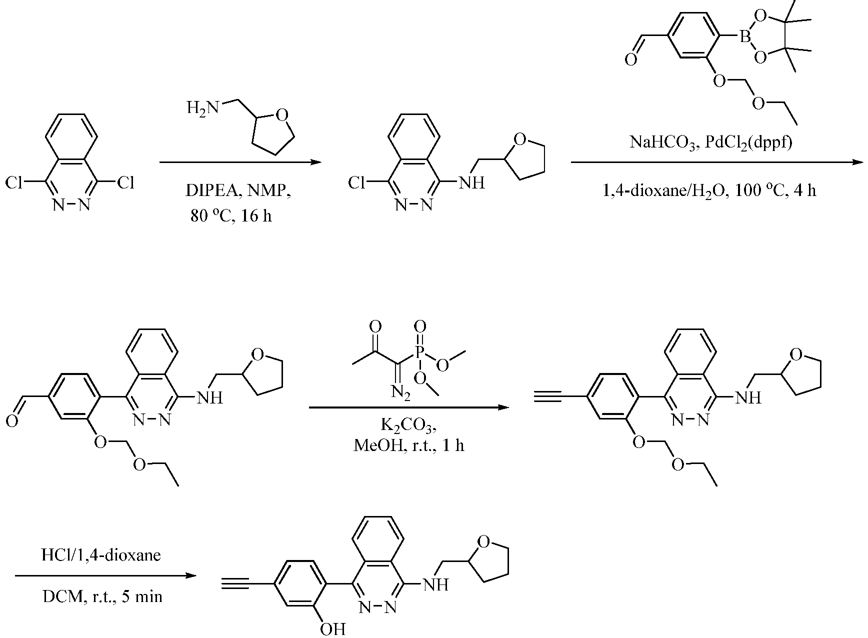

### Step 1: Synthesis of Intermediate 4-chloro-N-(tetrahydrofuran-2-yl)methyl)phthalazin-1-amine

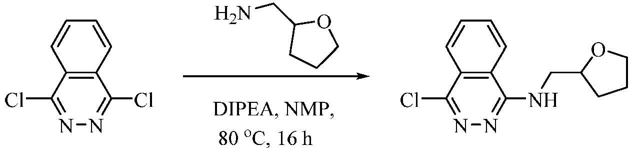

1,4-dicholorophthalazine (1.0 g, 5.0 mmol, 1.0 eq.) was dissolved in NMP (10 mL); 2-tetrahydrofurfurylamine (506 mg, 5.0 mmol, 1.2 eq.) and DIPEA (2.26 g, 17.5 mmol, 3.5 eq.) were added to the resulting mixture, to allow for reaction for 16 h at 80°C; and TLC detection showed that the reaction was complete. The reaction mixture was poured into water (20 mL), and extracted with dichloromethane (20 mL × 2); the organic phases were combined, dried, and concentrated; and the resulting crude product was purified by silica gel column chromatography (petroleum ether:ethyl acetate = 5:1-1:1) to obtain a crude product, which was directly used in a next step.

### Step 2: Compound 3-(ethoxymethoxy)-4-(4-(((tetrahydrofuran-2-yl)methyl)amino)phthalazin-1-yl)benzaldehyde

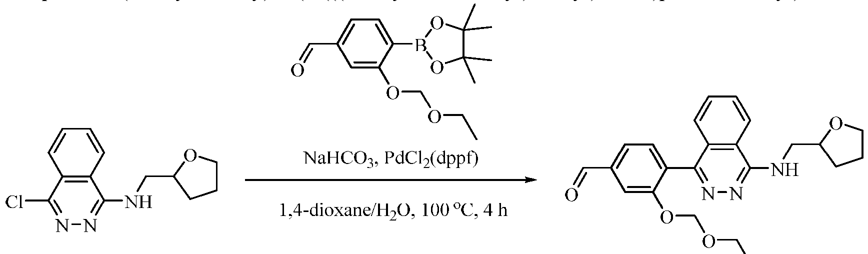

4-chloro-*N*-(tetrahydrofuran-2-yl)methyl)phthalazin-1-amine (500 mg, 1.89 mmol, 1.0 eq.) was dissolved in 1,4-dioxane (5 mL); 3-(ethoxymethoxy)-4-(4,4,5,5-trimethyl-1,3,2-dioxoboracyclopentan-2-yl)benzaldehyde (753 mg, 2.46 mmol, 1.3 eq.), sodium hydrogen carbonate (318 mg, 3.78 mmol, 2.0 eq.), PdCl₂(dppf) (69 mg, 0.095 mmol, 0.05 eq.) and water (1 mL) were added to the resulting mixture; nitrogen replacement was conducted; the temperature was increased to 100°C to allow for reaction for 4 h; and TLC detection showed that the reaction was complete. The reaction mixture was filtered by means of diatomite; the filter cake was washed with dichloromethane (20 mL); the filtrate was dried with anhydrous magnesium sulfate, and concentrated; and the resulting crude product was purified by silica gel column chromatography (petroleum ether:ethyl acetate = 2:1-ethyl acetate) to obtain a product (377 mg, two-step yield: 52.4%).

### Step 3: Synthesis of Intermediate 4-(2-ethoxymethoxy)-4-ethynylphenyl-N-(tetrahydrofuran-2-yl)methyl)phthalazin-1-amine

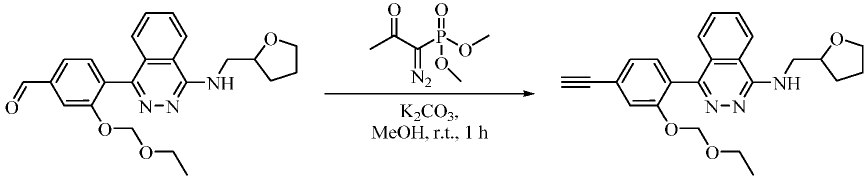

3-(ethoxymethoxy)-4-(4-(((tetrahydrofuran-2-yl)methyl)amino)phthalazin-1-yl)benzaldehyde (377 mg, 0.925 mmol, 1.0 eq.) was dissolved in methanol (4 mL); potassium carbonate (256 mg, 1.85 mmol, 2.0 eq.) and (1-diazo-2-oxopropyl)dimethyl phosphonate (267 mg, 1.388 mmol, 1.5 eq.) were successively added to the resulting mixture, to allow for reaction for 1 h at 25°C; and TLC detection showed that the reaction was complete. The reaction mixture was concentrated, added with water (10 mL), and extracted with DCM (10 mL×3); the organic phases were combined, dried with anhydrous magnesium sulfate, and concentrated; and the resulting crude product was directly used in the next step.

### Step 4: Synthesis of Compound 5-ethynyl-2-(4-(((tetrahydrofuran-2-yl)methyl)amino)phthalazin-1-yl)phenol

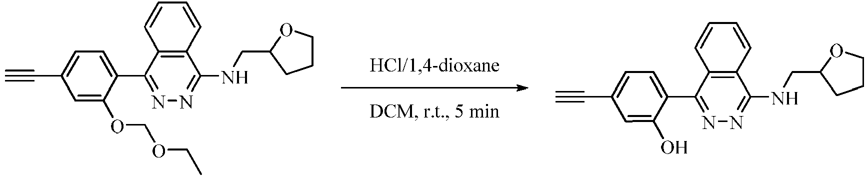

4-(2-ethoxymethoxy)-4-ethynylphenyl-*N*-(tetrahydrofuran-2-yl)methyl)phthalazin-1-amine (crude product, 0.925 mmol, 1.0 eq.) was dissolved in dichloromethane (2 mL); the resulting mixture was dripped to the 1,4-dioxane solution of hydrogen chloride (2 mL, 4 mol/L), to allow for reaction for 5 min at 25°C; and TLC detection showed that the reaction was complete. The reaction mixture was slowly poured into saturated sodium hydrogen carbonate in water (10 mL), and extracted with dichloromethane (10 mL × 3); the organic phases were combined, dried, and concentrated; and the resulting crude product was first purified by silica gel column chromatography (dichloromethane:methanol = 20:1), and then pulped with ethyl acetate (2 mL) and dried to obtain a product (101 mg, yield: 31.7%).

¹H-NMR (400 MHz, DMSO-*d*₆) δ(ppm): 9.97 (s, 1H), 8.37-8.35 (d, 1H), 7.87-7.83 (m, 1H), 7.80-7.76 (m, 1H), 7.63-7.60 (t, 1H), 7.49-7.47 (d, 1H), 7.31-7.29 (m, 1H), 7.08-7.06 (m, 2H), 4.31-4.24 (m, 1H), 4.22 (s, 1H), 3.89-3.82 (m, 1H), 3.70-3.64 (m, 3H), 2.03-1.95 (m, 1H), 1.94-1.79 (m, 2H), 1.73-1.64 (m, 1H).

Molecular formula: C₂₁H₁₉N₃O₂ Accurate molecular weight: 345.15 LC-MS(*m*/*z*): 346.06 [M+H]⁺

### Example 40: Synthesis of Compound (R)-5-ethynyl-2-(4-((1-(2-(methylsulfonyl)ethyl)piperidin-3-yl)amino)phthalazin-1-yl)phenol (Compound 62)

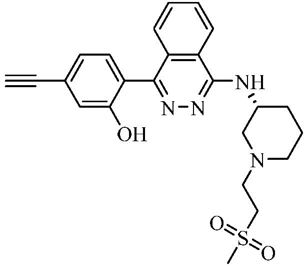

### Steps:

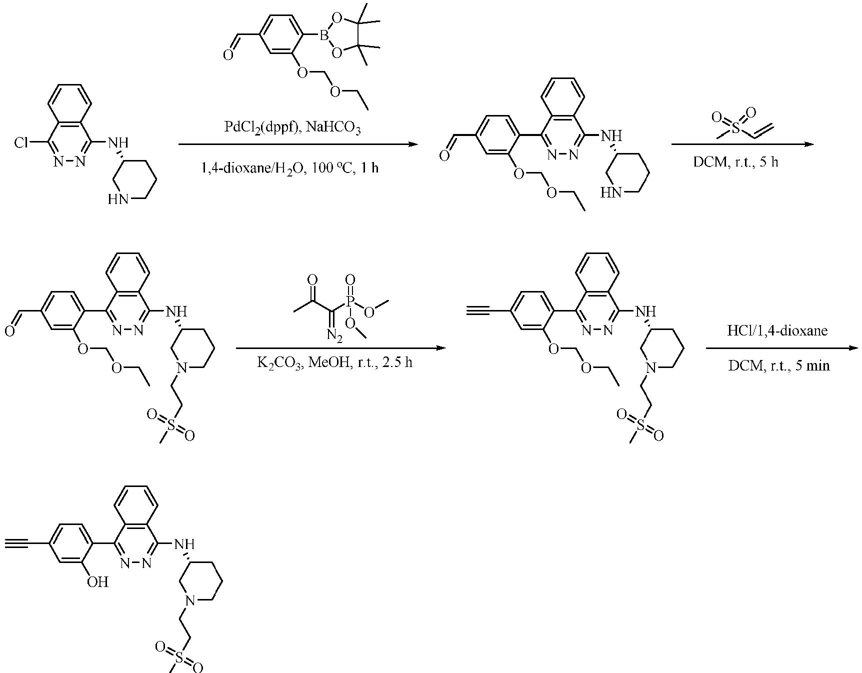

### Step 1: Synthesis of Intermediate (R)-3-(ethoxymethoxy)-4-(4-(piperidin-3-ylamino)phthalazin-1-yl)benzaldehyde

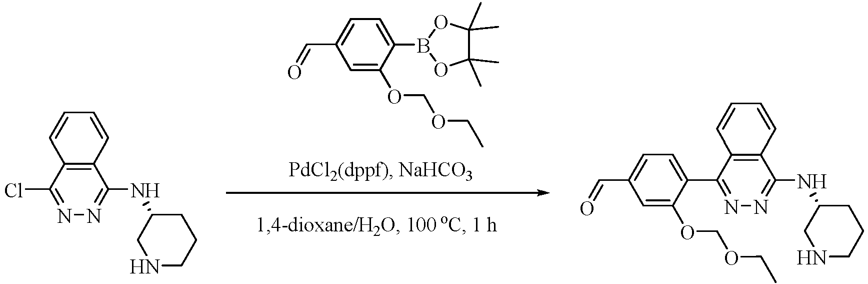

(*R*)-6-chloro-5-methyl-*N*-(piperidin-3-yl)pyridazin-3-atnine (500 mg, 1.9 mmol, 1.0 eq.) was dissolved in 1,4-dioxane (4 mL); 3-(ethoxymethoxy)-4-(4,4,5,5-trimethyl-1,3,2-dioxoboracyclopentan-2-yl)benzaldehyde (873 mg, 2.85 mmol, 1.5 eq.), sodium hydrogen carbonate (319 mg, 3.8 mmol, 2.0 eq.), PdCl₂(dppf) (139 mg, 0.19 mmol, 0.1 eq.) and water (1 mL) were added to the resulting mixture; nitrogen replacement was conducted; the temperature was increased to 100°C to allow for reaction for 1 h; and TLC detection showed that the reaction was complete. The reaction mixture was filtered by means of diatomite; the filter cake was successively washed with dichloromethane (10 mL) and water (5 mL); the filtrate was separated; the organic phases were dried and concentrated; and the resulting crude product was purified by silica gel column chromatography (dichloromethane:methanol = 50:1-7:1) to obtain a product (560 mg, yield: 72.5%).

### Step 2: Synthesis of Intermediate (R)-3-(ethoxymethoxy)-4-(4-((1-(2-(methylsulfonyl)ethyl)piperidin-3-amino)phthalazin-1 -yl)benzaldehyde

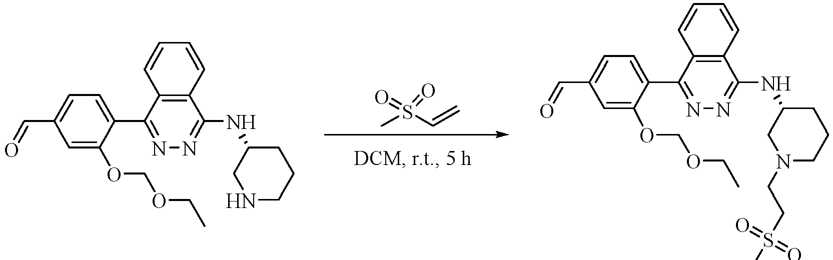

(R)-3-(ethoxymethoxy)-4-(4-(piperidin-3-ylamino)phthalazin-1-yl)benzaldehyde (200 mg, 0.492 mmol, 1.0 eq.) was dissolved in dichloromethane (2 mL); methyl vinyl sulfone (52 mg, 0.492 mmol, 1.0 eq.) was added to the resulting mixture, to allow for reaction for 5 h at 25°C; and TLC detection showed that the reaction was complete. The reaction mixture was concentrated, and directly used in a next step.

### Step 3: Synthesis of Intermediate (R)-4-(2-ethoxymethoxy)-4-ethynylphenyl)-N-(1-(2-methylsulfonyl)ethyl)piperidin-3-yl)phthalazin-1-amine

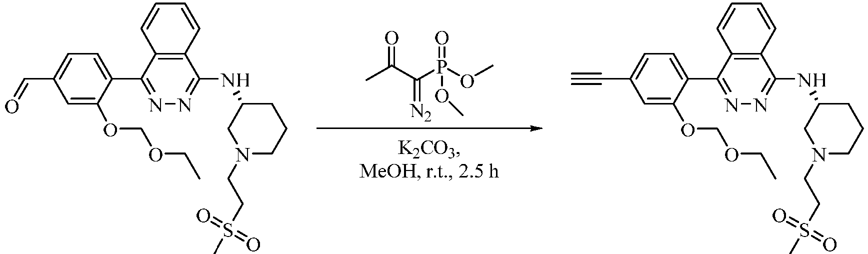

(*R*)-3-(ethoxymethoxy)-4-(4-((1-(2-(methylsulfonyl)ethyl)piperidin-3-amino)phthalazin-1-yl)benzaldehyde (crude product, 0.492 mmol, 1.0 eq.) was dissolved in methanol (2 mL); potassium carbonate (136 mg, 0.984 mmol, 2.0 eq.) and (1-diazo-2-oxopropyl)dimethyl phosphonate (142 mg, 0.738 mmol, 1.5 eq.) were successively added to the resulting mixture, to allow for reaction for 2.5 h at 25°C; and TLC detection showed that the reaction was complete. The reaction mixture was concentrated, added with water (5 mL), and extracted with DCM (10 mL×3); the organic phases were combined, dried with anhydrous magnesium sulfate, and concentrated; and the resulting crude product was directly used in the next step.

### Step 4: Synthesis of Compound (R)-5-ethynyl-2-(4-((1-(2-(methylsulfonyl)ethyl)piperidin-3-amino)phthalazin-1-yl)phenol

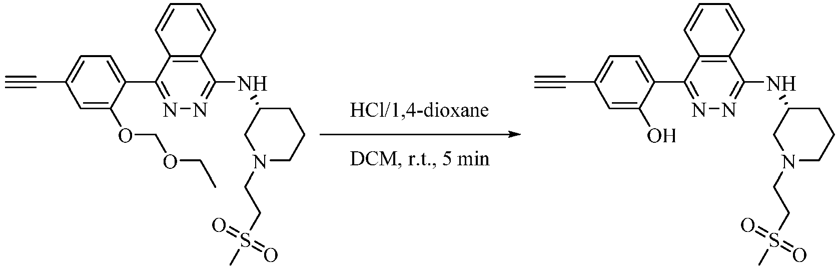

(*R*)-4-(2-ethoxymethoxy)-4-ethynylphenyl)-*N*-(1-(2-methylsulfonyl)ethyl)piperidin-3-yl)phthalazin-1-amine (crude product, 0.492 mmol) was dissolved in dichloromethane (2 mL); the resulting mixture was dripped into the 1,4-dioxane solution of hydrogen chloride (2 mL, 4 mol/L) to allow for reaction for 5 min at 25°C; and LC-MS detection showed that the reaction was complete. The reaction mixture was poured into saturated sodium hydrogen carbonate in water (15 mL), and extracted with dichloromethane /methanol=20:1 (20 mL×3); the organic phases were combined, dried with anhydrous magnesium sulfate, and concentrated; and the resulting crude product was purified by silica gel column chromatography (dichloromethane:methanol = 100:1-50:1) to obtain a product (112 mg, three-step yield: 50.7%).

¹H-NMR (400 MHz, DMSO-*d*₆) δ(ppm): 10.00 (s, 1H), 8.39-8.37 (d, 1H), 7.89-7.83 (m, 1H), 7.80-7.76 (m, 1H), 7.49-7.47 (d, 1H), 7.31-7.29 (d, 1H), 7.11-7.06 (m, 3H), 4.43-4.42 (m, 1H), 4.22 (s, 1H), 3.35-3.28 (m, 2H), 3.18-3.16 (m, 1H), 3.07 (s, 3H), 2.85-2.77 (m, 3H), 2.14-2.09 (m, 2H), 2.00-1.98 (m, 1H), 1.79-1.76 (m, 1H), 1.61-1.53 (m, 2H).

Molecular formula: C₂₄H₂₆N₄O₃S Accurate molecular weight: 450.17 LC-MS(*m*/*z*): 451.12 [M+H]⁺

### Example 41: Synthesis of Compound (R)-3-ethynyl-2-fluoro-6-(4-((tetrahydrofuran-3-yl)amino)phthalazin-1-yl)phenol (Compound 46)

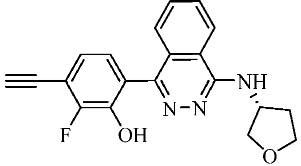

### Steps:

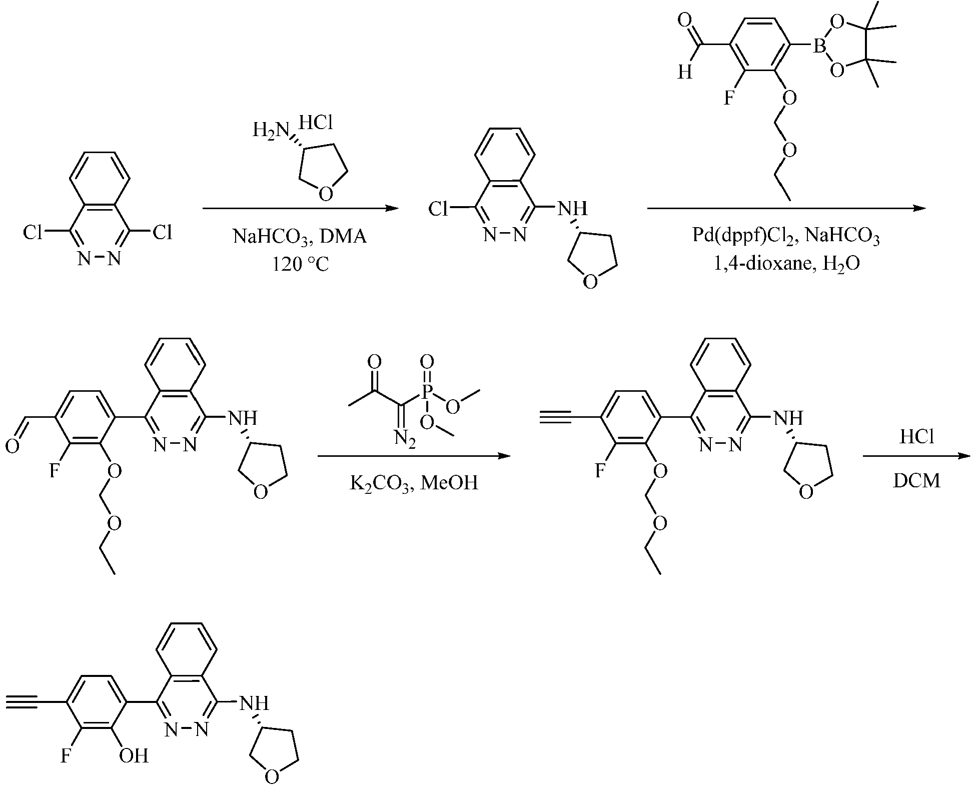

### Step 1: Synthesis of Intermediate (R)-4-chloro-N-(tetrahydrofuran-3-yl)phthalazin-1-amine

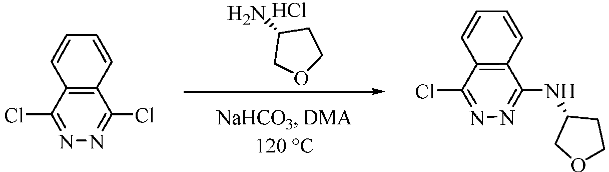

1,4-dicholorophthalazine (1 g, 5.02 mmol, 1.0 eq.) was dissolved in dimethylacetamide (10 mL); (*R*)-tetrahydrofuran-3-amine hydrochloride (683 mg, 5.53 mmol, 1.1 eq.) and sodium hydrogen carbonate (924 mg, 11.04 mmol, 2.2 eq.) were added; the resulting mixture was stirred for 3 h at 120°C; and TLC detection showed that the reaction was complete. The reaction mixture was added with water (50 mL) and dichloromethane (25 mL), and separated; the aqueous phase was extracted with dichloromethane (25 mL); the organic phases were combined, washed successively with water (15 mL × 2) and saturated saline (15 mL × 2), dried with anhydrous sodium sulfate, filtered, and concentrated; and the resulting crude product was purified by silica gel column chromatography (dichloromethane:methanol = 40:1-20:1) to obtain a product 1.25 g, yield: 99.7%).

### Step 2: Synthesis of Intermediate (R)-3-(ethoxymethoxy)-2-fluoro-4-(4-((tetrahydrofuran-3-yl)amino)phthalazin-1-yl)benzaldehyde

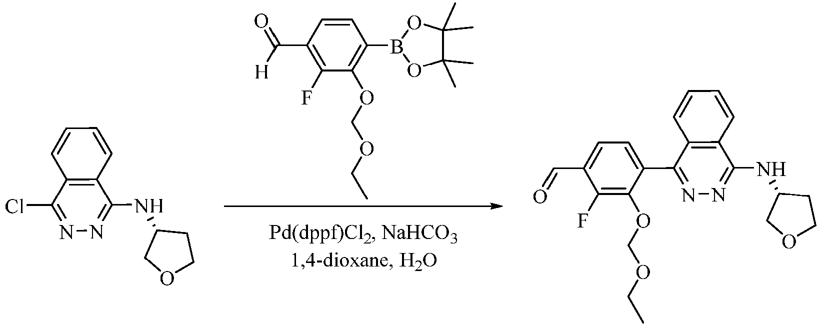

(*R*)-4-chloro-*N*-(tetrahydrofuran-3-yl)phthalazin-1-amine (400 mg, 1.60 mmol, 1.0 eq.) obtained from the previous step was dissolved in 1,4-dioxane (4 mL) and water (2 mL); 3-(ethoxymethoxy)-2-fluoro-4-(4,4,5,5-tetramethyl-1,3,2-dioxaboracyclopentan-2-yl)benzaldehyde (675 mg, 2.08 mmol, 1.3 eq.), sodium hydrogen carbonate (269 mg, 3.20 mmol, 2.0 eq.) and Pd(dppf)Cl₂ (117 mg, 0.16 mmol, 0.1 eq.) were successively added; and the resulting mixture was stirred for 2 h at 90°C under the protection of nitrogen. LCMS detection showed that the reaction was complete. The reaction mixture was concentrated, added with water (5 mL), and extracted with DCM (5 mL × 3); the organic phases were dried and filtered by suction; the filtrate was concentrated; and the resulting crude product was purified by silica gel column chromatography (DCM:MeOH = 50:1-20:1) to obtain a product (420 mg, yield: 63.8%).

### Step 3: Synthesis of Intermediate (R)-4-(2-(ethoxymethoxy)-4-ethynyl-3-fluorophenyl)-N-(tetrahydrofuran-3-yl)phthalazin-1-amine

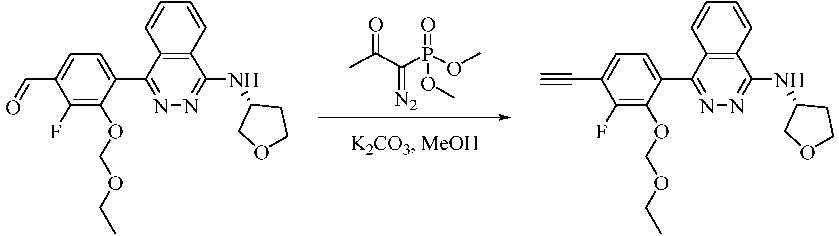

(*R*)-3-(ethoxymethoxy)-2-fluoro-4-(4-((tetrahydrofuran-3-yl)amino)phthalazin-1-yl)benzaldehyde (400 mg, 0.97 mmol, 1.0 eq.) was dissolved in methanol (5 mL); (1-diazo-2-oxopropyl)dimethyl phosphonate (224 mg, 1.17 mmol, 1.2 eq.) and potassium carbonate (251 mg, 1.82 mmol, 2.0 eq.) were sucessively added; and the resulting mixture was stirred for 1 h at room temperature. LCMS detection showed that the reaction was complete. The reaction mixture was concentrated, added with water (5 mL), and extracted with dichloromethane (5 mL × 3); and the organic phases were dried, and concentrated to obtain a crude product (550 mg).

### Step 4: Synthesis of Compound (R)-3-ethynyl-2-fluoro-6-(4-((tetrahydrofuran-3-yl)amino)phthalazin-1-yl)phenol

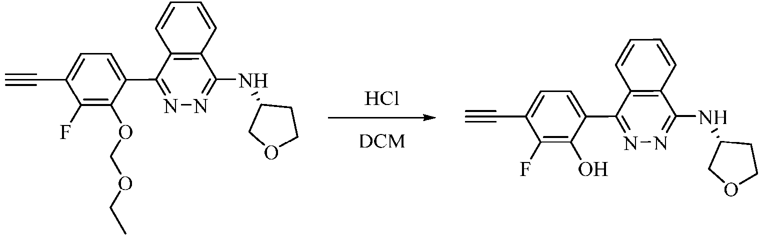

The crude product of (*R*)-4-(2-(ethoxymethoxy)-4-ethynyl-3-fluorophenyl)-*N*-(tetrahydrofuran-3-yl)phthalazin-1-amine (550 mg) obtained from the previous step was dissolved in dichloromethane (10 mL); 4 mol/L hydrogen chloride/1,4-dioxane solution (1.25 mL) was added; the resulting mixture was stirred for 30 min at room temperature; and LC-MS detection showed that the reaction was complete. The reaction mixture was poured into water, adjusted to a pH value of about 8 by using sodium bicarbonate, and separated; then, the aqueous phase was extracted with DCM (5 mL×2), and the organic phases were dried, filtered by suction, and concentrated; and the resulting crude product was purified by preparative thin-layer chromatography (130 mg, two-step yield: 38.3%).

¹H-NMR (400 MHz, DMSO-*d*₆) δ(ppm): 10.23 (s, 1H), 8.45 (d, J=8.2 Hz, 1H), 7.90-7.86 (m, 1H), 7.81-7.78 (m, 1H), 7.52-7.50 (m, 2H), 7.15-7.06 (m, 2H), 4.82-4.76 (m, 1H), 4.54 (s, 1H), 4.07-4.03 (m, 1H), 3.98-3.93 (m, 1H), 3.82-3.74 (m, 2H), 2.36-2.27 (m, 1H), 2.16-2.08 (m, 1H).

Molecular formula: C₂₀H₁₆FN₃O₂ Accurate molecular weight: 349.12 LC-MS(*m*/*z*): 350.03 [M+H]⁺

### Example 42: Synthesis of Compound 2-fluoro-6-(4-(((cis)-3-hydroxyl-3-methylcyclobutyl)amino)phthalazin-1-yl)-3-ethenylphenol (Compound 171)

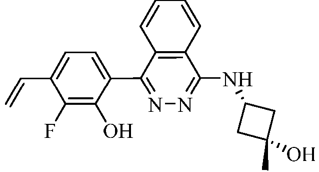

### Steps:

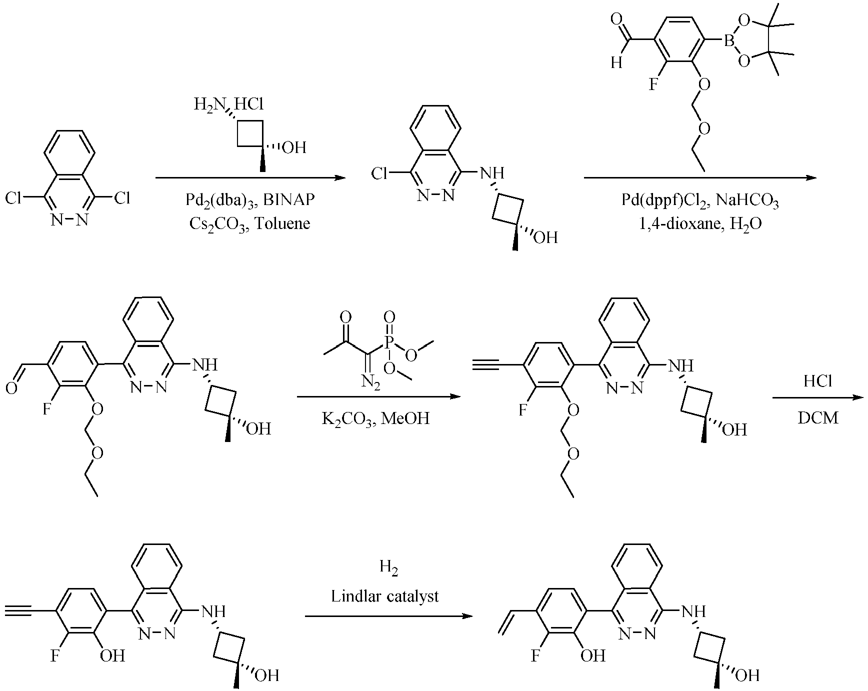

### Step 1: Synthesis of Intermediate (cis)-3-((4-chlorophthalazin-1-yl)amino)-1-methylcyclobutan-1-ol

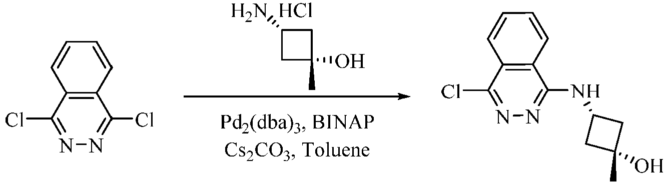

1,4-dicholorophthalazine (2 g, 10.04 mmol, 1.0 eq.) was dissolved in 1,4-dioxane (40 mL); (cis)-3-amino-1-methylcyclobutan-1-ol hydrochloride (683 mg, 5.53 mmol, 1.1 eq.), BINAP(1.25 g, 2.01 mmol, 0.2 eq.), Pd₂(dba)₃ (915 mg, 1.00 mmol, 0.1 eq.) and cesium carbonate (9.78 g, 30.12 mmol, 3.0 eq.) were successively added to the resulting mixture; under the protection of nitrogen, the mixture was stirred overnight at 90°C; and TLC detection showed that the reaction was complete. Suction filtration was conducted by means of diatomite; the filtrate was concentrated; and the resulting crude product was purified by silica gel column chromatography (dichloromethane:methanol = 50:1-10:1) to obtain a product (1.50 g, yield: 56.9%).

### Step 2: Synthesis of Intermediate 3-(ethoxymethoxy)-2-fluoro-4-(4-(((cis)-3-hydroxyl-3-methylcyclobutyl)amino)phthalazin-1-yl)benzaldehyde

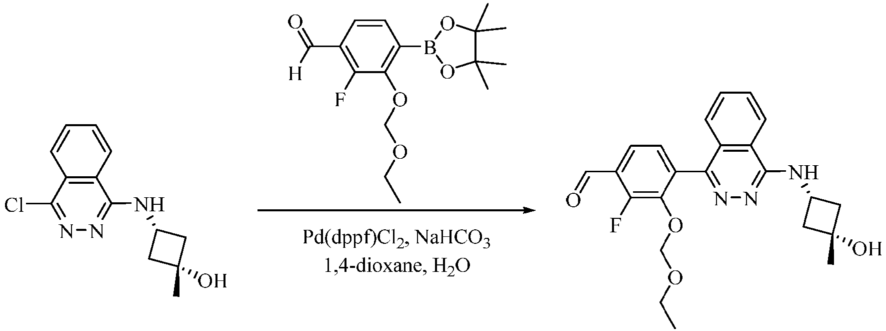

(Cis)-3-((4-chlorophthalazin-1-yl)amino)-1-methylcyclobutan-1-ol (600 mg, 2.28 mmol, 1.0 eq.) obtained from the previous step was dissolved in 1,4-dioxane (6 mL) and water (2 mL); 3-(ethoxymethoxy)-2-fluoro-4-(4,4,5,5-tetramethyl-1,3,2-dioxaboracyclopentan-2-yl)benzaldehyde (958 mg, 2.96 mmol, 1.3 eq.), sodium hydrogen carbonate (383 mg, 4.56 mmol, 2.0 eq.) and Pd(dppf)Cl₂ (167 mg, 0.23 mmol, 0.1 eq.) were successively added; and the resulting mixture was stirred for 2 h at 90°C under the protection of nitrogen. LCMS detection showed that the reaction was complete. The reaction mixture was concentrated, added with water (5 mL), and extracted with DCM (5 mL × 3); the organic phases were dried and filtered by suction; the filtrate was concentrated; and the resulting crude product was purified by silica gel column chromatography (DCM:MeOH = 50:1-20:1) to obtain a product (700 mg, yield: 72.2%).

### Step 3: Synthesis of Intermediate (cis)-3-((4-(2-(ethoxymethoxy)-4-ethynyl-3-fluorophenyl)phthalazin-1-yl)amino)-1 -methylcyclobutan-1 -ol

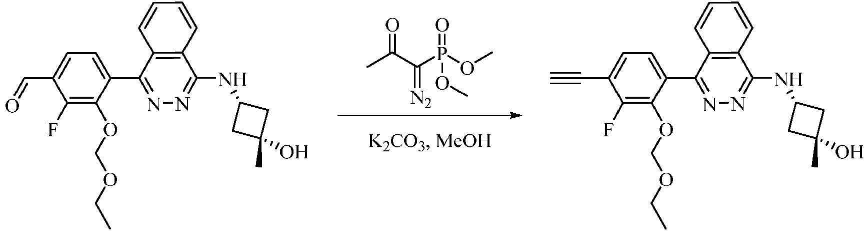

3 -(ethoxymethoxy)-2-fluoro-4-(4-(((cis)-3 -hydroxyl-3-methylcyclobutyl)amino)phthalazin-1-yl)benzaldehyde (500 mg, 1.18 mmol, 1.0 eq.) was dissolved in methanol (5 mL); (1-diazo-2-oxopropyl)dimethyl phosphonate (271 mg, 1.41 mmol, 1.2 eq.) and potassium carbonate (326 mg, 2.36 mmol, 2.0 eq.) were sucessively added; and the resulting mixture was stirred for 2 h at room temperature. LCMS detection showed that the reaction was complete. The reaction mixture was concentrated, added with water (5 mL), and extracted with DCM (5 mL × 3); the organic phases were dried and concentrated; and the resulting crude product was purified by silica gel column chromatography (DCM:MeOH = 20:1) to obtain a product (300 mg, yield: 60.3%).

### Step 4: Synthesis of Intermediate 3-ethynyl-2-fluoro-6-(4-(((cis)-3-hydroxyl-3-methylcyclobutyl)amino)phthalazin-1-yl)phenol

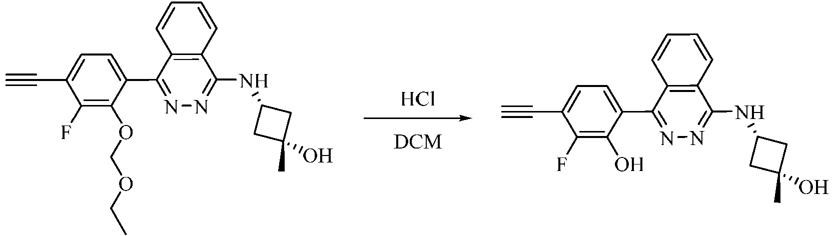

(Cis)-3-((4-(2-(ethoxymethoxy)-4-ethynyl-3-fluorophenyl)phthalazin-1-yl)atnino)-1-methylcyclobutan-1-ol (300 mg, 0.71 mmol, 1.0 eq.) obtained from the previous step was dissolved in dichloromethane (3 mL); 4 mol/L hydrogen chloride /1,4-dioxane solution (0.9 mL) was added; the resulting mixture was stirred for 30 min at room temperature; and LC-MS detection showed that the reaction was complete. The reaction mixture was poured into water, adjusted to a pH value of about 8 by using sodium bicarbonate, and separated; then, the aqueous phase was extracted with DCM (5 mL×2), and the organic phases were dried, filtered by suction, and concentrated; and the resulting crude product was purified by preparative thin-layer chromatography (220 mg, yield: 85.3%).

### Step 5: Synthesis of Compound 2-fluoro-6-(4-(((cis)-3-hydroxyl-3-methylcyclobutyl)atnino)phthalazin-1-yl)-3-ethenylphenol

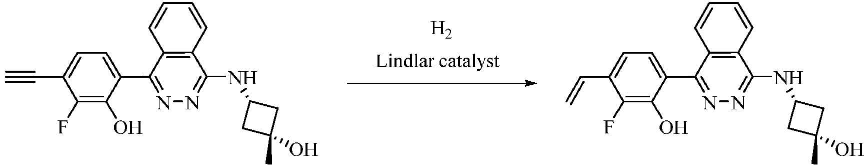

3-ethynyl-2-fluoro-6-(4-(((cis)-3-hydroxyl-3-methylcyclobutyl)amino)phthalazin-1-yl)phenol (120 mg, 0.33 mmol, 1.0 eq.) obtained from the previous step was dissolved in methanol (4 mL); the Lindlar catalyst (12 mg, 12wt%) was added; the resulting mixture was stirred for 1 h at room temperature; LC-MS detection showed that a large amount of raw materials were left; the Lindlar catalyst (12 mg, 12wt%) was supplemented to allow for reaction overnight; and LC-MS detection showed that the reaction was complete. Suction filtration was conducted by means of diatomite; the filtrate was concentrated; and the resulting crude product was purified by preparative thin-layer chromatography to obtain a product (25 mg, yield: 20.7%).

¹H-NMR (400 MHz, DMSO-*d*₆) δ(ppm): 10.03 (s, 1H), 8.45 (d, J=8.1 Hz, 1H), 7.89-7.85 (m, 1H), 7.82-7.78 (m, 1H), 7.68-7.67 (m, 1H), 7.56-7.54 (m, 1H), 7.24-7.20 (m, 1H), 7.15-7.13 (m, 1H), 6.96-6.89 (m, 1H), 6.01-5.96 (m, 1H), 5.51-5.48 (m, 1H), 5.01 (s, 1H), 4.30-4.21 (m, 1H), 2.49-2.46 (m, 2H), 2.22-2.17 (m, 2H), 1.35 (s, 3H)_{∘} Molecular formula: C₂₁H₂₀FN₃O₂ Accurate molecular weight: 365.15 LC-MS(m/z): 366.10 [M+H]⁺

### Example 43: Synthesis of Compound 5-(bromoethynyl)-2-(4-(((cis)-3-hydroxyl-3-methylcyclobutyl)amino)phthalazin-1-yl)phenol (Compound 111)

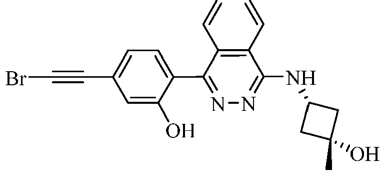

### Steps:

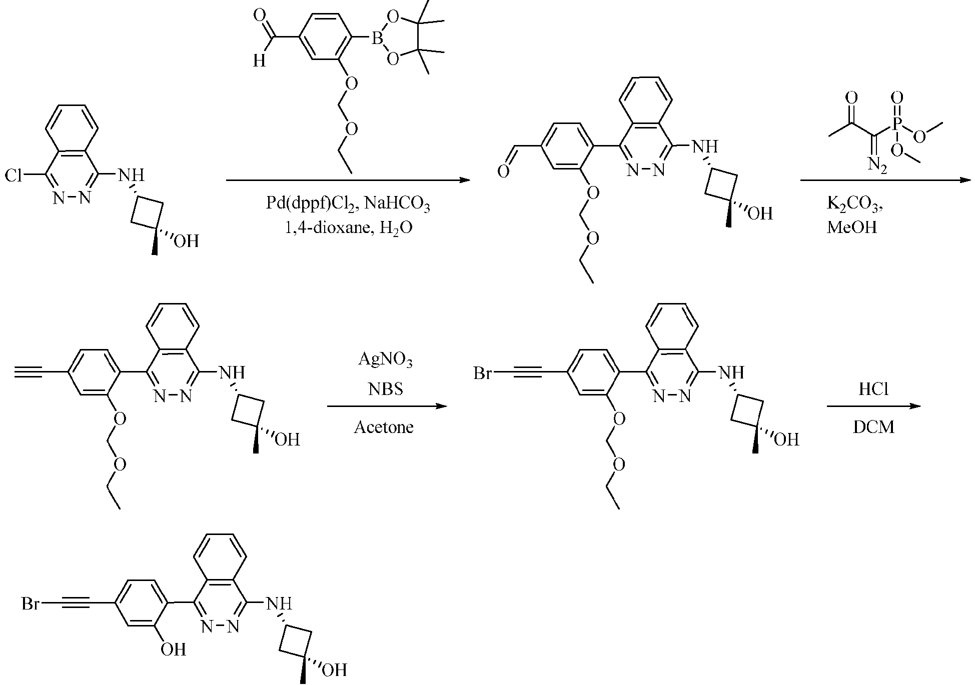

### Step 1: Synthesis of Intermediate 3-(ethoxymethoxy)-4-(4-(((cis)-3-hydroxyl-3-methylcyclobutyl)amino)phthalazin-1 -yl)benzaldehyde

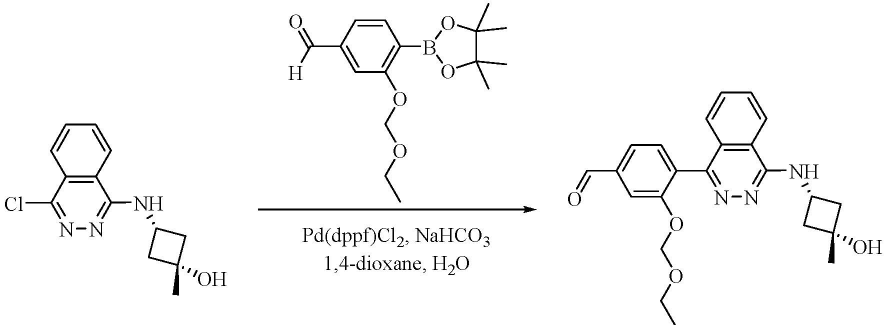

(Cis)-3-((4-chlorophthalazin-1-yl)amino)-1-methylcyclobutan-1-ol (486 mg, 1.85 mmol, 1.0 eq.) was dissolved in 1,4-dioxane (5 mL) and water (2 mL); 3-(ethoxymethoxy)-4-(4,4,5,5-tetramethyl-1,3,2-dioxaboracyclopentan-2-yl)benzaldehyde (849 mg, 2.78 mmol, 1.5 eq.), sodium hydrogen carbonate (311 mg, 3.70 mmol, 2.0 eq.) and Pd(dppf)Cl₂ (135 mg, 0.19 mmol, 0.1 eq.) were successively added; and the resulting mixture was stirred for 2 h at 90°C under the protection of nitrogen. LCMS detection showed that the reaction was complete. The reaction mixture was concentrated, added with water (5 mL), and extracted with DCM (5 mL × 3); the organic phases were dried and filtered by suction; the filtrate was concentrated; and the resulting crude product was purified by silica gel column chromatography (DCM:MeOH = 50:1-20:1) to obtain a product (450 mg, yield: 60.0%).

### Step 2: Synthesis of Intermediate (cis)-3-((4-(2-(ethoxymethoxy)-4-ethynylphenyl)phthalazin-1-yl)amino)-1-methylcyclobutan-1-ol

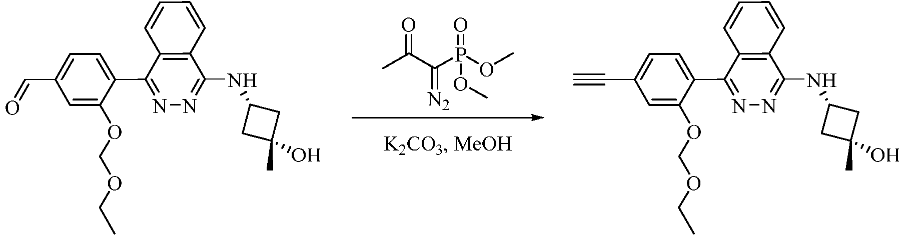

3-(ethoxymethoxy)-4-(4-(((cis)-3-hydroxyl-3-methylcyclobutyl)amino)phthalazin-1-yl)benzaldehyde (450 mg, 1.10 mmol, 1.0 eq.) was dissolved in methanol (5 mL); (1-diazo-2-oxopropyl)dimethyl phosphonate (254 mg, 1.33 mmol, 1.2 eq.) and potassium carbonate (304 mg, 2.20 mmol, 2.0 eq.) were sucessively added; and the resulting mixture was stirred for 1 h at room temperature. LCMS detection showed that the reaction was complete. The reaction mixture was concentrated, added with water (5 mL), and extracted with DCM (5 mL × 3); the organic phases were dried and concentrated; and the resulting crude product was purified by silica gel column chromatography (DCM:MeOH = 20:1) to obtain a product (430 mg, yield: 96.8%).

### Step 3: Synthesis of Intermediate (cis)-3-((4-(4-(bromoethynyl)-2-(ethoxymethoxy)phenyl)phthalazin-1-yl)amino)-1-methylcyclobutan-1-ol

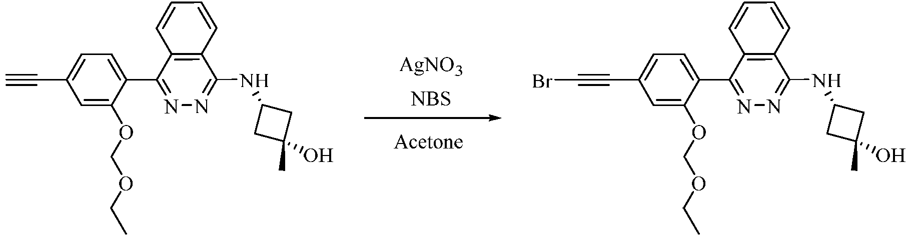

(Cis)-3-((4-(2-(ethoxymethoxy)-4-ethynylphenyl)phthalazin-1-yl)amino)-1-methylcyclobutan-1-ol (300 mg, 0.74 mmol, 1.0 eq.) obtained from the previous step was dissolved in acetone (6 mL); NBS (158 mg, 0.89 mmol, 1.2 eq.) and silver nitrate (13 mg, 0.074 mmol, 0.1 eq.) were added; the resulting mixture was stirred overnight at room temperature; and LC-MS detection showed that the reaction was complete. The reaction mixture was concentrated, added with water (5 mL), and extracted with dichloromethane (5 mL × 3); and the organic phases were dried, and concentrated to obtain a product (350 mg of crude product).

### Step 4: Synthesis of Compound 5-(bromoethynyl)-2-(4-(((cis)-3-hydroxyl-3-methylcyclobutyl)amino)phthalazin-1-yl)phenol

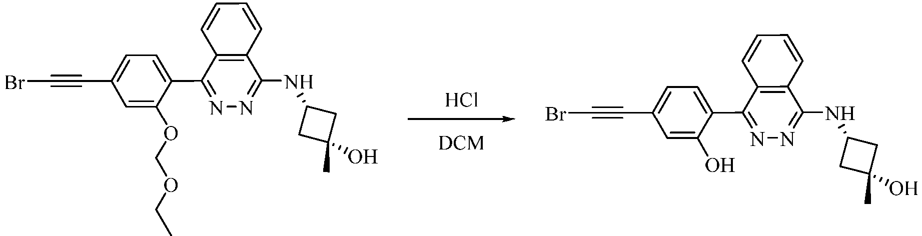

(Cis)-3-((4-(4-(bromoethynyl)-2-(ethoxymethoxy)phenyl)phthalazin-1-yl)amino)-1-methylcyclobutan-1-ol (350 mg of crude product) obtained from the previous step was dissolved in dichloromethane (6 mL); 4 mol/L hydrogen chloride/1,4-dioxane solution (0.9 mL) was added; the resulting mixture was stirred for 30 min at room temperature; and LC-MS detection showed that the reaction was complete. The reaction mixture was poured into water, adjusted to a pH value of about 8 by using sodium bicarbonate, and separated; then, the aqueous phase was extracted with DCM (5 mL×2), and the organic phases were dried, filtered by suction, and concentrated; and the resulting crude product was purified by preparative thin-layer chromatography (30 mg, two-step yield: 28.3%).

¹H-NMR (400 MHz, DMSO-*d*₆) δ(ppm): 9.90 (s, 1H), 8.42 (d, J=8.2 Hz, 1H), 7.87-7.76 (m, 2H), 7.60 (s, 1H), 7.47 (m, d, J=8.2 Hz, 1H), 7.30 (d, J=8.0 Hz, 1H), 7.08-7.06 (m, 2H), 5.00 (s, 1H), 4.23 (s, 1H), 2.48-2.47 (m, 2H), 2.21-2.16 (m, 2H), 1.35 (s, 3H)_{∘}

Molecular formula: C₂₁H₁₈BrN₃O₂ Accurate molecular weight: 423.06 LC-MS(*m*/*z*): 423.98,425.98 [M+H]⁺

### Example 44: Synthesis of Compound (R)-3-ethynyl-2-fluoro-6-(4-((tetrahydro-2H-pyran-3-yl)amino)phthalazin-1-yl)phenol (Compound 45)

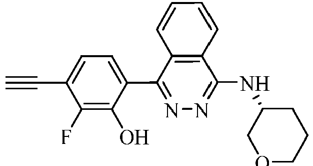

### Steps:

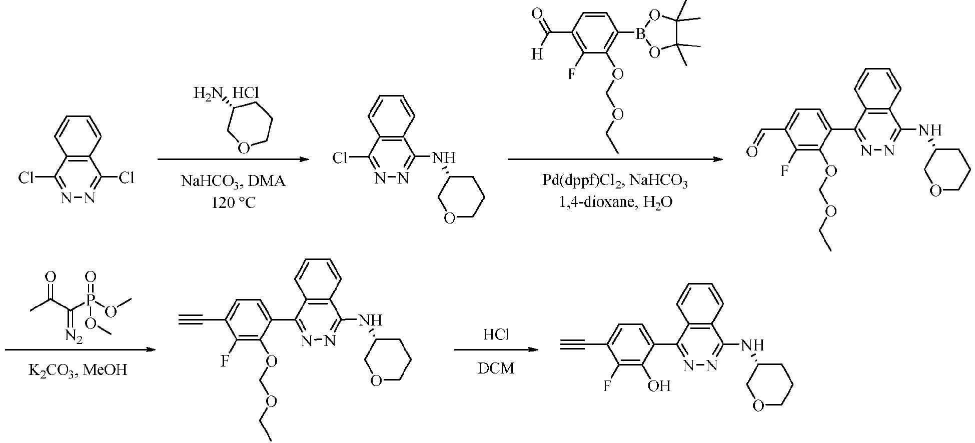

This compound was synthesized by referring to the steps in the foregoing embodiments.

¹H-NMR (400 MHz, DMSO-*d*₆) δ(ppm): 10.16 (s, 1H), 8.43 (d, J=8.2 Hz, 1H), 7.90-7.79 (m, 2H), 7.50 (d, J=8.2 Hz, 1H), 7.23-7.10 (m, 3H), 4.57 (s, 1H), 4.45-4.41 (m, 1H), 4.10-4.06 (m, 1H), 3.86-3.83 (m, 1H), 3.39-3.24 (m, 2H), 2.17-2.08 (m, 1H), 1.80-1.66 (m, 3H)_{∘}

Molecular formula: C₂₁H₁₈FN₃O₂ Accurate molecular weight: 363.14 LC-MS(m/z): 364.09 [M+H]⁺

### Example 45: Synthesis of 5-ethynyl-2-(6-(((cis)-3-hydroxyl-3-methylcyclobutyl)amino)-4-methylpyridazin-3-yl)phenol (Compound 55)

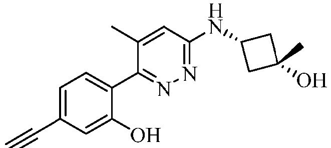

### Step 1: Synthesis of 3-(ethoxymethoxy)-4-(6-(((cis)-3-hydroxyl-3-methylcyclobutyl)amino)-4-methylpyridazin-3-yl)benzaldehyde

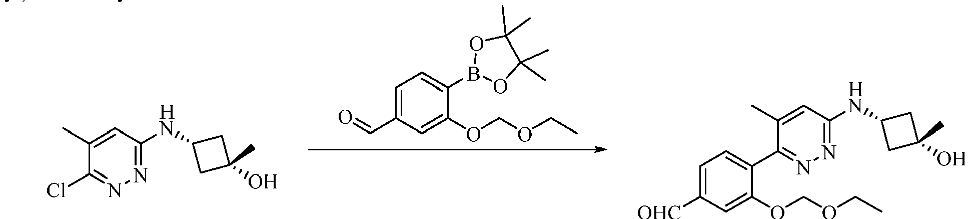

(*cis*)-3-((6-chloro-5-methylpyridazin-3-yl)amino)-1-methylcyclobutan-1-ol (266.0 mg, 1.16 mmol, 1.0 eq.), 3-(ethoxymethoxy)-4-(4,4,5,5-tetratnethyl-1,3,2-dioxaboracyclopentan-2-yl)benzaldehyde (536.4 mg, 1.75 mmol, 1.5 eq.), sodium hydrogen carbonate (196.2 mg, 2.33 mmol, 2.0 eq.) and Pd(dppf)Cl₂ (42.7 mg, 0.05 mmol, 0.05 eq.) were added to the mixed solution of 1,4-dioxane (12.0 mL) and water (4.0 mL), to allow for reaction for 3 h at 110°C under the protection of nitrogen; and TLC monitoring showed that the reaction was complete. The reaction mixture was added with water (50.0 mL), and extracted with ethyl acetate (100.0 mL); the organic phase was dried with anhydrous sodium sulfate and filtered; the filtrate was concentrated under reduced pressure; and the resulting crude product was purified by silica gel column chromatography (DCM:MeOH = 80:1-20:1) to obtain a product (300.0 mg, yield: 69.2%).

### Step 2: Synthesis of (cis)-3-((6-(2-(ethoxymethoxy)-4-ethynylphenyl)-5-methylpyridazin-3-yl)amino)-1-methylcyclobutan-1 -ol

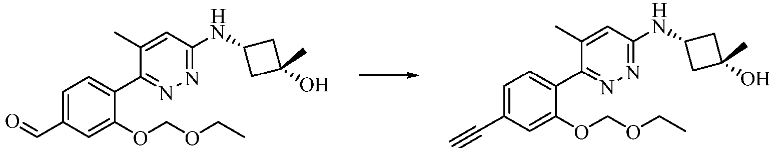

3 -(ethoxymethoxy)-4-(6-(((cis)-3 -hydroxyl-3 -methylcyclobutyl)amino)-4-methylpyridazin-3 -yl)benzaldehyde (300.0 mg, 0.80 mmol, 1.0 eq.), (1-diazo-2-oxopropyl)dimethyl phosphonate (232.7 mg, 1.21 mmol, 1.5 eq.) and anhydrous potassium carbonate (223.2 mg, 1.60 mmol, 2.0 eq.) were added to methanol (15.0 mL) to allow for reaction for 2 h at room temperature; and TLC monitoring showed that the reaction was complete. The reaction mixture was concentrated under reduced pressure; and the resulting crude product was purified by silica gel column chromatography (dichloromethane:methanol = 60:1-20:1) to obtain a product (270.0 mg, yield: 91.0%).

### Step 3: Synthesis of 5-ethynyl-2-(6-(((cis)-3-hydroxyl-3-methylcyclobutyl)amino)-4-methylpyridazin-3-yl)phenol

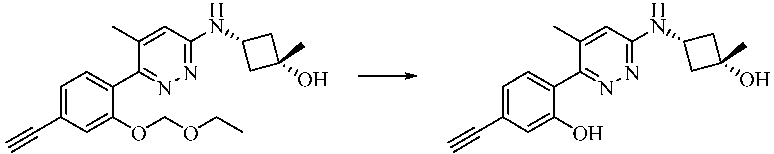

(*cis*)-3-((6-(2-(ethoxymethoxy)-4-ethynylphenyl)-5-methylpyridazin-3-yl)amino)-1-methylcyclobutan-1-ol (270.0 mg, 0.73 mmol, 1.0 eq.) was added to dichloromethane (2.0 mL); a hydrogen chloride-1,4-dioxane solution (4.0 mol/L, 4.0 mL) was dropwise added to allow for reaction for 1 h at room temperature; and TLC monitoring showed that the reaction was complete. The mixture was adjusted to pH = 8-9 by using saturated sodium carbonate in water, and extracted with dichloromethane (100.0 mL); the organic phase was dried with anhydrous sodium sulfate and filtered; the filtrate was concentrated under reduced pressure; and the resulting crude product was purified by silica gel column chromatography (dichloromethane:methanol = 50:1-20:1) to obtain a product (150.0 mg, yield: 66.0%) ¹HNMR(400MHz, DMSO-*d₆*) δ(ppm): 10.09 (s, 1H), 7.18-7.16 (d, *J*=8 Hz, 1H), 6.99 (s, 3H), 6.61 (s, 1H), 4.98 (s, 1H), 4.17 (s, 1H), 3.93-3.88 (m, 1H), 2.43-2.39 (m, 2H), 2.03 (s, 3H), 1.97-1.92 (m, 2H), 1.29 (s, 3H).

Molecular formula: C₁₈H₁₉N₃O₂ Molecular weight: 309.15 LC-MS(Pos, *m*/*z*)=310.11[M+H]⁺*.*

### Example 46: Synthesis of (R)-5-cyclopropyl-2-(5-methyl-3-((1-methylpiperidin-3-yl)amino)-1,2,4-triazin-6-yl)phenol (Compound 192)

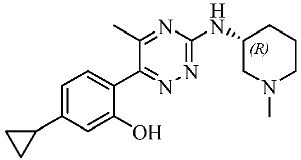

### Step 1: Synthesis of 4-bromo-2-methoxymethyl benzoate

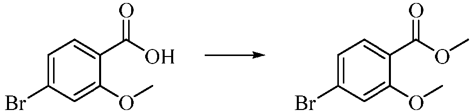

4-bromo-2-methoxybenzoic acid (15.0 g, 64.92 mmol, 1.0 eq.), iodomethane (11.97 g, 84.40 mmol, 1.3 eq.) and anhydrous potassium carbonate (11.60 g, 84.40 mmol. 1.3 eq.) were added to the mixed solution of acetonitrile (50.0 mL) and *N,N*-dimethylformamide (50.0 mL), to allow for reaction at room temperature for 12 h; and TLC monitoring showed that the reaction was complete. The reaction mixture was added with water (150.0 mL), and extracted with ethyl acetate (200.0 mL); the organic phase was dried with anhydrous sodium sulfate and filtered; the filtrate was concentrated under reduced pressure; and the resulting crude product was purified by silica gel column chromatography (silica gel: 100-200 meshes, heptane:ethyl acetate = 20:1) to obtain a product (15.0 g, yield: 94.3%).

### Step 2: Synthesis of 4-cyclopropyl-2-methoxymethyl benzoate

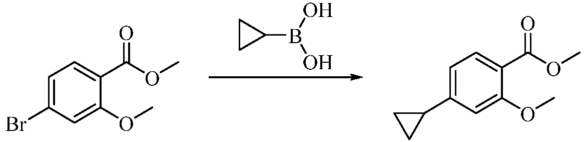

4-bromo-2-methyl methoxybenzoate (6.0 g, 24.48 mmol, 1.0 eq.), cyclopropylboric acid (4.2 g, 48.96 mmol, 2.0 eq.), potassium phosphate (15.6 g, 73.44 mmol, 3.0 eq.), palladium acetate (1.0 g, 4.89 mmol, 0.2 eq.) and tricyclohexylphosphine (2.7 g, 9.79 mmol, 0.4 eq.) were added to the mixed solution of 1,4-dioxane (30.0 mL) and water (6.0 mL), and allowed to react for 12 h at 100°C under the protection of nitrogen; TLC monitoring showed that the reaction was complete; the reaction mixture was added with water (50.0 mL), and extracted with ethyl acetate (100.0 mL); the organic phase was dried with anhydrous sodium sulfate and filtered; the filtrate was concentrated under reduced pressure; and the resulting crude product was purified by silica gel column chromatography (heptane:ethyl acetate = 100:1-40:1) to obtain a product (4.4 g, yield: 80.0%).

### Step 3: Synthesis of 4-cyclopropyl-2-methoxybenzoic acid

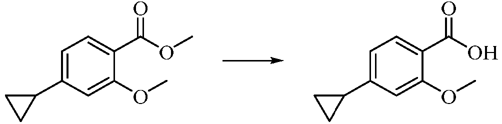

4-cyclopropyl-2-methoxymethyl benzoate (4.4 g, 21.33 mmol, 1.0 eq.) was added to methanol (20.0 mL); lithium hydroxide monohydrate (4.4 g, 106.67 mmol, 5.0 eq.) in water (10.0 mL) was dropwise added to allow for reaction at room temperature for 12 h; TLC monitoring showed that the reaction was complete; hydrochloric acid (2.0 mol/L) was dropwise added to adjust pH to 2-3, after which white solids were precipitated and then filtered; and the filter cake was dried to obtain a product (3.8 g, yield: 92.6%).

### Step 4: Synthesis of 1-bromo-4-cyclopropyl-2-methoxybenzene

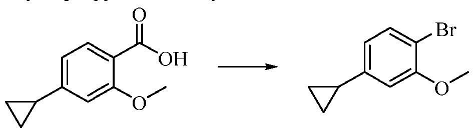

4-cyclopropyl-2-methoxybenzoic acid (3.8 g, 19.77 mmol, 1.0 eq.), potassium phosphate (4.2 g, 19.77 mmol, 1.0 eq.), and tetrabutylammonium tribromide (9.53 g, 19.77 mmol, 1.0 eq.) were added to acetonitrile (40.0 mL), to allow for reaction for 2 h at 100°C; and TLC monitoring showed that the reaction was complete. The reaction mixture was added with water (50.0 mL), and extracted with ethyl acetate (100.0 mL); the organic phase was dried with anhydrous sodium sulfate and filtered; the filtrate was concentrated under reduced pressure; and the resulting crude product was purified by silica gel column chromatography (heptane:ethyl acetate = 200:1) to obtain a product (3.5 g, yield: 78.1%).

### Step 5: Synthesis of 2-bromo-5-cyclopropylphenol

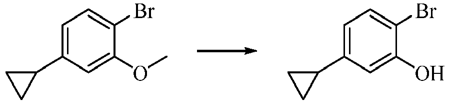

1-bromo-4-cyclopropyl-2-methoxybenzene (3.5 g, 15.41 mmol, 1.0 eq.) was added to dichloromethane (20.0 mL); in the ice water bath, boron tribromide (11.5g, 46.23 mmol, 3.0 eq.) was dropwise added; the temperature was increased to room temperature to allow for reaction for 1 h; TLC monitoring showed that the reaction was complete; in the ice water bath, quenching was conducted with methanol (10.0 mL); the reaction mixture was concentrated under reduced pressure, added with dichloromethane (100.0 mL), and reversely extracted with water (50.0 mL); the aqueous phase was adjusted to pH = 7-8 by using saturated sodium hydrogen carbonate in water, and extracted with dichloromethane (100.0 mL); the organic phase was dried with anhydrous sodium sulfate and filtered; the filtrate was concentrated under reduced pressure; and the resulting crude product was purified by silica gel column chromatography (heptane:ethyl acetate = 10: 1) to obtain a product (2.6 g, yield: 79.2%).

### Step 6: Synthesis of 1-bromo-4-cyclopropyl-2-(ethoxymethoxy)benzene

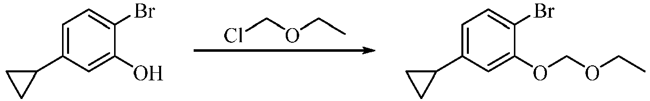

2-bromo-5-cyclopropylphenol (2.1 g, 9.85 mmol, 1.0 eq.) were added to dried tetrahydrofuran (20.0 mL); the resulting mixture was cooled to 0°C under the protection of nitrogen; sodium hydride (mass fraction: 60%, 591.3 mg, 14.78 mmol, 1.5 eq.) was slowly portionwise added, to allow for reaction for 0.5 h; chloromethyloxyethane (1.39 g, 14.78 mmol, 1.5 eq.) was added; the temperature was increased to room temperature to allow for reaction for2 h; and TLC monitoring showed that the reaction was complete. The reaction mixture was added with water (20.0 mL), and extracted with ethyl acetate (100.0 mL); the organic phase was dried with anhydrous sodium sulfate and filtered; the filtrate was concentrated under reduced pressure; and the resulting crude product was purified by silica gel column chromatography (silica gel: 100-200 meshes, heptane:ethyl acetate = 100:1) to obtain a product (2.5 g, yield: 96.1%).

### Step 7: Synthesis of (4-cyclopropyl-2-(ethoxymethoxy)phenyl)boric acid

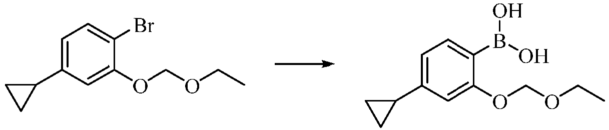

1-bromo-4-cyclopropyl-2-(ethoxymethoxy)benzene (2.5 g, 9.21 mmol, 1.0 eq.) was added to dry tetrahydrofuran (20.0 mL); the resulting mixture was cooled to -70°C under the protection of nitrogen; the n-hexane solution of n-butyl lithium (2.5 mol/L, 5.5 mL, 13.82 mmol, 1.5 eq.) was slowly dropwise added to allow for reaction for 1 h; triisopropyl borate (2.25 g, 11.98 mmol, 1.3 eq.) was added; the temperature was increased to room temperature to allow for reaction for 3 h; and TLC monitoring showed that the reaction was complete. Saturated ammonium chloride in water (50.0 mL) was added; the resulting mixture was extracted with ethyl acetate (100.0 mL); the organic phase was dried with anhydrous sodium sulfate and filtered; and the filtrate was concentrated under reduced pressure to obtain a product (1.5 g, yield: 69.1%)

### Step 8: Synthesis of (R)-6-(4-cyclopropyl-2-(ethoxymethoxy)phenyl)-5-methyl-N-(1-methylpiperidin-3-yl)-1,2,4-triazin-3-amine

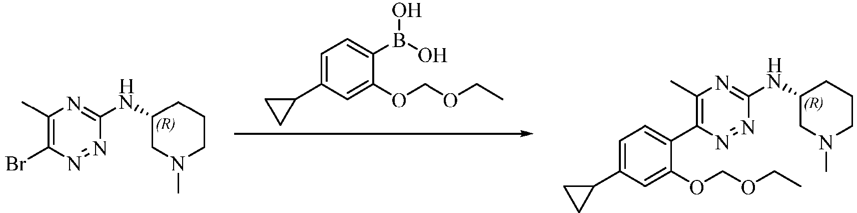

(*R*)-6-bromo-5-methyl-*N*-(1-methylpiperidin-3-yl)-1,2,4-triazin-3-amine (311.0 mg, 1.08 mmol, 1.0 eq.), (4-cyclopropyl-2-(ethoxymethoxy)phenyl)boric acid (384.8 mg, 1.63 mmol, 1.5 eq.), sodium hydrogen carbonate(182.4 mg, 2.17 mmol, 2.0 eq.) and Pd(dppf)Cl₂ (79.4 mg, 0.10 mmol, 0.1 eq.) were added to the mixed solution of 1,4-dioxane (10.0 mL) and water (5.0 mL), and allowed to react for 2 h at 110°C under the protection of nitrogen; TLC monitoring showed that the reaction was complete; the reaction mixture was added with water (50.0 mL), and extracted with ethyl acetate (100.0 mL); the organic phase was dried with anhydrous sodium sulfate and filtered; the filtrate was concentrated under reduced pressure; and the resulting crude product was purified by silica gel column chromatography (dichloromethane:methanol = 80:1-40:1) to obtain a product (333.0 mg, yield: 77.2%).

### Step 9: Synthesis of (R)-5-cyclopropyl-2-(5-methyl-3-((1-methylpiperidin-3-yl)amino)-1,2,4-triazin-6-yl)phenol

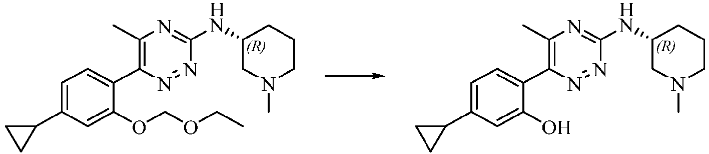

(R)-6-(4-cyclopropyl-2-(ethoxymethoxy)phenyl)-5-methyl-*N*-(1-methylpiperidin-3 -yl)-1,2,4-triazin-3 -amine (300.0 mg, 0.75 mmol, 1.0 eq.) was added to dichloromethane (4.0 mL); a hydrogen chloride-1,4-dioxane solution (4.0 mol/L, 4.0 mL) was dropwise added to allow for reaction for 1 h at room temperature; and TLC monitoring showed that the reaction was complete. The mixture was adjusted to pH = 8-9 by using saturated sodium carbonate in water, and extracted with dichloromethane (100.0 mL); the organic phase was dried with anhydrous sodium sulfate and filtered; the filtrate was concentrated under reduced pressure; and the resulting crude product was purified by preparative thin-layer chromatography (dichloromethane:methanol = 10:1) to obtain a product (200.0 mg, yield: 78.1%)

¹HNMR (400 MHz, DMSO-*d₆*) δ(ppm): 9.61 (s, 1H), 7.36 (s, 1H), 7.11-7.09 (d, *J*=8 Hz, 1H), 6.64-6.62 (m, 2H), 3.97 (s, 1H), 2.88 (s, 1H), 2.65-2.62 (m, 1H), 2.18-2.17 (m, 6H), 1.92-1.85 (m, 4H), 1.72-1.68 (m, 1H), 1.57-1.47 (m, 1H), 1.35-1.23 (m, 1H), 0.99-0.92 (m, 2H), 0.68-0.64 (m, 2H).

Molecular formula: C₁₉H₂₅N₅O Molecular weight: 339.21 LC-MS(Pos, *m*/*z*)=340.15[M+H]⁺.

### Example 47: Synthesis of (R)-5-ethynyl-2-(5-methyl-3-(((tetrahydrofuran-2-yl)methyl)amino)-1,2,4-triazin-6-yl)phenol (Compound 250)

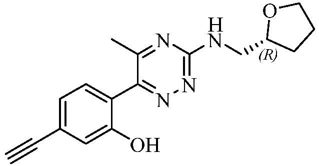

### Step 1: Synthesis of (R)-5-methyl-N-((tetrahydrofuran-2-yl)methyl)-1,2,4-triazin-3-amine

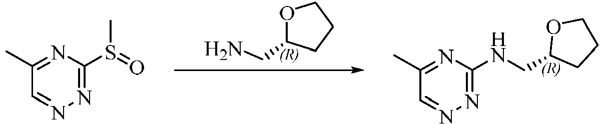

5-methyl-3-(methylsulfinyl)-1,2,4-triazine (1.39 g, 8.85 mmol, 1.0 eq.), (R)-(tetrahydrofuran-2-yl)methylamine (1.34 g, 13.27 mmol, 1.5 eq.) and triethylamine (2.68 g, 26.55 mmol, 3.0 eq.) were added to 1,4-dioxane (20.0 mL), and stirred for 1 h at 100°C; and TLC monitoring showed that the reaction was complete. The reaction mixture was concentrated under reduced pressure, added with saturated ammonium chloride in water (100.0 mL), and extracted with dichloromethane (100.0 mL); the organic phase was dried with anhydrous sodium sulfate and filtered; the filtrate was concentrated under reduced pressure; and the resulting crude product was purified by silica gel column chromatography (dichloromethane:methanol = 100:1-80:1) to obtain a product (1.27 g, yield: 73.8%).

### Step 2: Synthesis of (R)-6-bromo-5-methyl-N-((tetrahydrofuran-2-yl)methyl)-1,2,4-triazin-3-amine

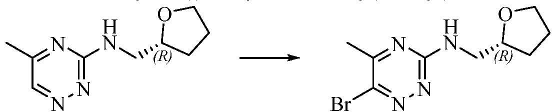

(*R*)-5-methyl-*N*-((tetrahydrofuran-2-yl)methyl)-1,2,4-triazin-3-amine (1.27 g, 6.53 mmol, 1.0 eq.) and *N-*bromosuccinimide (1.22 g, 6.86 mmol, 1.05 eq.) were added to *N,N*-dimethyl formamide (10.0 mL) to allow for reaction for 2 h at room temperature; and TLC monitoring showed that the reaction was complete. The reaction mixture was added with water (50.0 mL), and extracted with ethyl acetate (100.0 mL); the organic phase was dried with anhydrous sodium sulfate and filtered; the filtrate was concentrated under reduced pressure; and the resulting crude product was purified by silica gel column chromatography (heptane:ethyl acetate = 10:1-6:1) to obtain a product (520.0 mg, yield: 29.1%).

### Step 3: Synthesis of (R)-3-(ethoxymethoxy)-4-(5-methyl-3-(((tetrahydrofuran-2-yl)methyl)amino)-1,2,4-triazin-6-yl)benzaldehyde

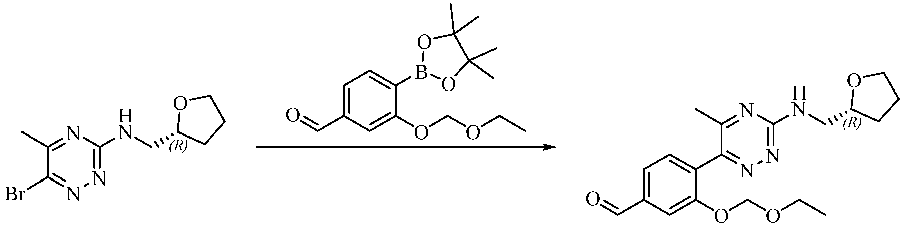

(*R*)-6-bromo-5-methyl-*N*-((tetrahydrofuran-2-yl)methyl)-1,2,4-triazin-3-amine (520.0 mg, 1.90 mmol, 1.0 eq.), 3-(ethoxymethoxy)-4-(4,4,5,5-tetratnethyl-1,3,2-dioxaboracyclopentan-2-yl)benzaldehyde (874.3 mg, 2.85 mmol, 1.5 eq.), sodium hydrogen carbonate (319.7 mg, 3.80 mmol, 2.0 eq.) and Pd(dppf)Cl₂ (69.6 mg, 0.09 mmol, 0.05 eq.) were added to the mixed solution of 1,4-dioxane (10.0 mL) and water (5.0 mL), to allow for reaction for 3 h at 110°C under the protection of nitrogen; and TLC monitoring showed that the reaction was complete. The reaction mixture was added with water (50.0 mL), and extracted with ethyl acetate (100.0 mL); the organic phase was dried with anhydrous sodium sulfate and filtered; the filtrate was concentrated under reduced pressure; and the resulting crude product was purified by silica gel column chromatography (DCM:MeOH = 100:1-80:1) to obtain a product (630.0 mg, yield: 88.9%).

### Step 4: Synthesis of (R)-6-(2-(ethoxymethoxy)-4-ethynylphenyl)-5-methyl-N-((tetrahydrofuran-2-yl)methyl)-1,2,4-triazin-3-amine

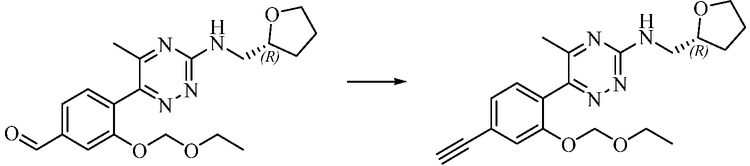

(*R*)-3-(ethoxymethoxy)-4-(5-methyl-3-(((tetrahydrofuran-2-yl)methyl)amino)-1,2,4-triazin-6-yl)benzaldehyde (315.0 mg, 0.84 mmol, 1.0 eq.), (1-diazo-2-oxopropyl)dimethyl phosphonate (243.7 mg, 1.26 mmol, 1.5 eq.) and anhydrous potassium carbonate (350.3 mg, 2.53 mmol, 3.0 eq.) were added to methanol (10.0 mL), to allow for reaction for 2 h at room temperature; and TLC monitoring showed that the reaction was complete. The reaction mixture was concentrated under reduced pressure; and the resulting crude product was purified by silica gel column chromatography (dichloromethane:methanol = 100:1-80:1) to obtain a product (290.0 mg, yield: 93.2%)

### Step 5: Synthesis of (R)-5-ethynyl-2-(5-methyl-3-(((tetrahydrofuran-2-yl)methyl)amino)-1,2,4-triazin-6-yl)phenol

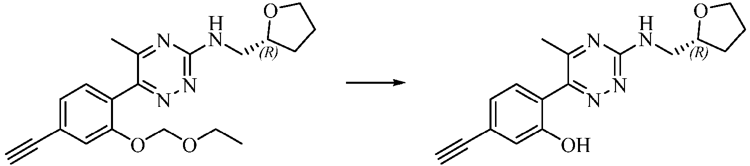

(*R*)-6-(2-(ethoxymethoxy)-4-ethynylphenyl)-5-methyl-*N*-((tetrahydrofuran-2-yl)methyl)-1,2,4-triazin-3-amine (280.0 mg, 0.75 mmol, 1.0 eq.) was added to dichloromethane (3.0 mL); a hydrogen chloride-1,4-dioxane solution (4.0 mol/L, 3.0 mL) was dropwise added to allow for reaction for 1 h at room temperature; and TLC monitoring showed that the reaction was complete. The mixture was adjusted to pH = 8-9 by using saturated sodium carbonate in water, and extracted with dichloromethane (100.0 mL); the organic phase was dried with anhydrous sodium sulfate and filtered; the filtrate was concentrated under reduced pressure; and the resulting crude product was purified by preparative thin-layer chromatography (dichloromethane:methanol = 20:1) to obtain a product (150.0 mg, yield: 63.6%).

¹HNMR (400 MHz, DMSO-*d₆*) δ(ppm): 10.11 (s, 1H), 7.63 (s, 1H), 7.27-7.25 (d, *J*=8 Hz, 1H), 7.04 (s, 1H), 7.01 (s, 1H), 4.23 (s, 1H), 4.06-4.03 (m, 1H), 3.81-3.76 (m, 1H), 3.66-3.60 (m, 1H), 3.50-3.46 (m, 1H), 3.38 (s, 1H), 2.18 (s, 3H), 1.98-1.75 (m, 3H), 1.66-1.58 (m, 1H).

Molecular formula: C₁₇H₁₈N₄O₂ Molecular weight: 310.14 LC-MS(Pos, *m*/*z*)=311.10[M+H]⁺*.*

### Example 48: Synthesis of (R)-5-ethynyl-2-(5-methyl-3-((1-((tetrahydro-2H-pyran-4-yl)methyl)piperidin-3-yl)amino)-1,2,4-triazin-6-yl)phenol (Compound 255)

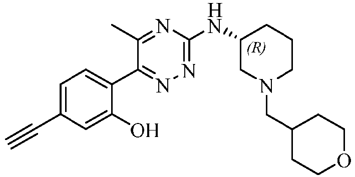

### Step 1: Synthesis of (R)-5-ethynyl-2-(5-methyl-3-((1-((tetrahydro-2H-pyran-4-yl)methyl)piperidin-3-yl)amino)-1,2,4-triazin-6-yl)phenol

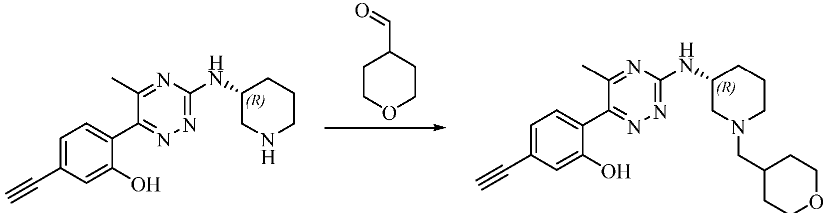

(*R*)-5-ethynyl-2-(5-methyl-3-(piperidin-3-ylatnino)-1,2,4-triazin-6-yl)phenol (211.7 mg, 0.68 mmol, 1.0 eq.) and formaldehyde in water (mass fraction: 37%, 117.1 mg, 1.02 mmol, 1.5 eq.) were added to methanol (10.0 mL), to allow for reaction at room temperature for 0.5 h; sodium cyanoborohydride (64.5 mg, 1.02 mmol, 1.5 eq.) was added, to allow to continue the reaction for 1 h at room temperature; and TLC monitoring showed that the reaction was complete. Saturated sodium hydrogen carbonate in water (50.0 mL) was added; the resulting mixture was stirred for 30 min, and extracted with dichloromethane (100.0 mL); the organic phase was dried with anhydrous sodium sulfate and filtered; the filtrate was concentrated under reduced pressure; and the resulting crude product was purified by preparative thin-layer chromatography (dichloromethane:methanol =10:1) to obtain a product (80.0 mg, yield: 28.7%).

¹HNMR (400MHz, DMSO-*d₆*) δ(ppm): 10.11 (s, 1H), 7.63 (s, 1H), 7.27-7.25 (d, *J*=8 Hz, 1H), 7.04-7.01 (m, 2H), 4.23 (s, 1H), 3.98 (s, 1H), 3.83-3.80 (m, 2H), 3.29-3.23 (m, 3H), 2.94-2.92 (m, 1H), 2.70-2.68 (m, 1H), 2.17 (s, 3H), 2.15-2.13 (m, 2H), 1.93-1.85 (m, 5H), 1.61-1.47 (m, 4H), 1.40-1.33 (m, 1H).

Molecular formula: C₂₃H₂₉N₅O₂ Molecular weight: 407.23 LC-MS(Pos, *m*/*z*)=408.18[M+H]⁺.

### Example 49: Synthesis of 5-ethynyl-2-(7-((cis-3-hydroxyl-3-methylcyclobutyl)amino)thieno[2,3-d]pyridazin-4-yl)phenol (Compound 11)

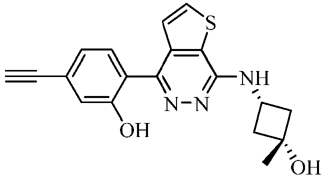

### Step 1: Synthesis of cis-3-((4-chlorothiophen[2,3-d]pyridazin-7-yl)amino)-1-methylcyclobutan-1-ol

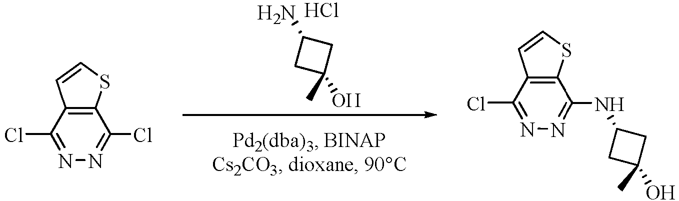

4,7-dicholorothieno[2,3-*d*]pyridazine (1.00 g, 4.88 mmol, 1.0 eq.), cis-3-amino-1-methylcyclobutanol hydrochloride(671 mg, 4.88 mmol, 1.1 eq.), Pd₂(dba)₃(447 mg, 4.88 mmol, 0.1 eq.), BINAP (607 mg, 0.976 mmol, 0.2 eq.) and Cs₂CO₃ (3.18 g, 9.76 mmol, 2.0 eq.) were successively added to 1,4-dioxane (50 mL), and heated to 90°C under the protection of nitrogen to allow for reaction for 2 h. The reaction mixture was cooled to room temperature, quenched by adding water (50 mL), and extracted with EA (30 mL × 2); the organic phases were dried with anhydrous sodium sulfate and filtered; the filtrate was concentrated under reduced pressure; and the resulting crude product was purified by preparative thin-layer chromatography (DCM:MeOH = 20:1) to obtain a product (240 mg, yield: 18.2%).

### Step 2: Synthesis of 3-(ethoxymethoxy)-4-(7-((cis-3-hydroxyl-3-methylcyclobutyl)amino)thiophen[2,3-d]pyridazin-4-yl)benzaldehyde

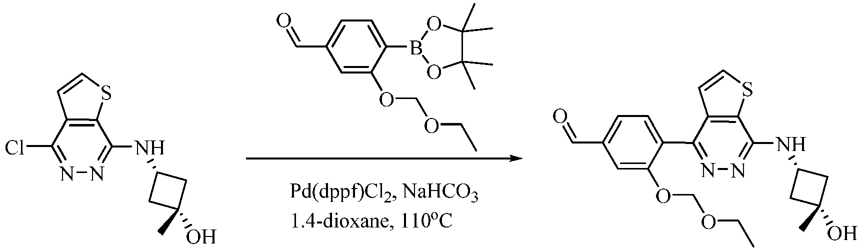

3-(ethoxymethoxy)-4-(4,4,5,5-tetramethyl-1,3,2-dioxaboracyclopentan-2-yl)benzaldehyde (409 mg, 1.34 mmol, 1.5 eq.), cis-3-((4-chlorothiophen[2,3-*d*]pyridazin-7-yl)amino)-1-methylcyclobutan-1-ol (240 mg, 0.890 mmol, 1.0 eq.), Pd(dppf)Cl₂ (65.1 mg, 0.0890 mmol, 0.1 eq.) and NaHCOs(150 mg, 1.78 mmol, 2.0 eq.) were successively added to 1,4-dioxane (6 mL); H₂O (3 mL) was added; the resulting mixture was heated to 100°C under the protection of nitrogen to allow for reaction for 2 h. The reaction mixture was cooled to room temperature, quenched by adding water (20 mL), and extracted with EA (30 mL × 2); the organic phases were dried with anhydrous sodium sulfate and filtered; the filtrate was concentrated under reduced pressure; and the resulting crude product was purified by silica gel column chromatography (DCM:MeOH = 100:1-20:1) to obtain a product (214 mg, yield: 58.2%).

### Step 3: Synthesis of cis-3-((4-(2-(ethoxymethoxy)-4-ethynylphenyl)thieno[2,3-d]pyridazin-7-yl)amino)-1-methylcyclobutan-1 -ol

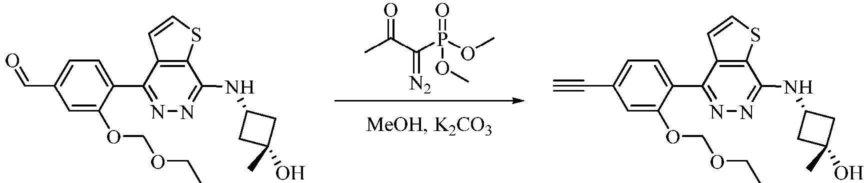

3-(ethoxymethoxy)-4-(7-((cis-3-hydroxyl-3-methylcyclobutyl)amino)thiophen[2,3-*d*]pyridazin-4-yl)benzaldehyde (214 mg, 0.518 mmol, 1.0 eq.) was dissolved in MeOH (4 mL); K₂CO₃ (144 mg, 1.04 mmol, 2.0 eq.) and (1-diazo-2-oxopropyl)dimethyl phosphonate(149 mg, 0.777 mmol, 1.5 eq.) were added; and the resulting mixture was stirred for 2 h at room temperature. The reaction mixture was quenched by adding water (10 mL), and extracted with EA (10 mL × 2); the organic phases were dried with anhydrous sodium sulfate and filtered; the filtrate was concentrated under reduced pressure; and the resulting crude product was purified by silica gel column chromatography (DCM:MeOH = 100:1-30:1) to obtain a product (192 mg, yield: 90.6%).

### Step 4: Synthesis of 5-ethynyl-2-(7-((cis-3-hydroxyl-3-methylcyclobutyl)amino)thieno[2,3-d]pyridazin-4-yl)phenol

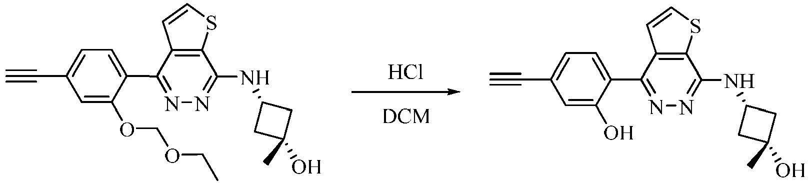

Cis-3-((4-(2-(ethoxymethoxy)-4-ethynylphenyl)thieno[2,3-*d*]pyridazin-7-yl)atnino)-1-methylcyclobutan-1-ol (192 mg, 0.469 mmol, 1.0 eq.) was dissolved in DCM (3 mL); then, the 1,4-dioxane solution of hydrogen chloride (4 mol/L, 0.59 mL, 2.35 mmol, 5.0 eq.) was dropwise added; and the resulting mixture was stirred for 1 h at room temperature. The resulting mixture was adjusted to a pH value of 8 by using saturated NaHCO₃ in water, and extracted with the mixed solvent of DCM:MeOH=10:1 (20 mL × 3); the organic phases were dried with anhydrous sodium sulfate and filtered; the filtrate was concentrated under reduced pressure; and the resulting crude product was purified by silica gel column chromatography (DCM:MeOH = 50:1-20:1) to obtain a product (142 mg, yield: 86.2%).

¹HNMR (400MHz, DMSO-*d*₆) δ(ppm): 11.52 (s, 1H), 8.12 (d, *J* = 5.3 Hz, 1H), 7.63-7.59 (m, 2H), 7.50 (d, *J* = 5.3 Hz, 1H), 7.07 (d, *J* = 6.9 Hz, 2H), 4.99 (s, 1H), 4.29-4.20 (m, 2H), 2.47-2.45 (m, 2H), 2.19-2.14 (m, 2H), 1.34 (s, 3H).

Molecular formula: C₁₉H₁₇N₃O₂S Accurate molecular weight: 351.10 LC-MS (Pos, m/z)=352.05[M+H]⁺.

### Example 50: Synthesis of 5-(1-hydroxyl-2-alkyn-1-yl)-2-(4-methyl-6-(((R)-1-methylpiperidin-3-yl)amino)pyridazin-3-yl)phenol (Compound 175)

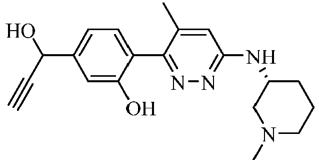

### Step 1: Synthesis of 1-(3-(ethoxymethoxy)-4-(4-methyl-6-(((R)-1-methylpiperidin-3-yl)amino)pyridazin-3-yl)phenyl)prop-2-yn-1 -ol

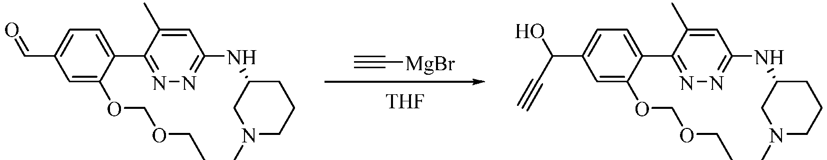

(*R*)-3-(ethoxymethoxy)-4-(4-methyl-6-((1-methylpiperidin-3-yl)amino)pyridazin-3-yl)benzaldehyde (160 mg, 0.416 mmol, 1.0 eq.) was dissolved in THF (3 mL); and under the protection of nitrogen, 0.5 mol/L ethynylmagnesium bromide in tetrahydrofuran solution (2.50 mL, 1.25 mmol, 3.0 eq.) was dropwise added at room temperature to allow for reaction for 3 h at room temperature. The reaction mixture was quenched by addition of saturated ammonium chloride in water, and extracted with DCM (15 mL × 2); the organic phases were dried with anhydrous sodium sulfate and filtered; the filtrate was concentrated under reduced pressure; and the resulting crude product was purified by silica gel column chromatography (DCM:MeOH = 50:1-10:1) to obtain a product (112 mg, yield: 65.6%).

### Step 2: Synthesis of 5-(1-hydroxyl-2-alkyn-1-yl)-2-(4-methyl-6-(((R)-1-methylpiperidin-3-yl)amino)pyridazin-3-yl)phenol

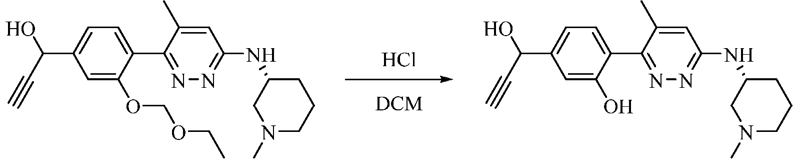

1-(3-(ethoxymethoxy)-4-(4-methyl-6-(((*R*)-1-methylpiperidin-3-yl)amino)pyridazin-3-yl)phenyl)prop-2-yn-1-ol (90 mg, 0.219 mmol, 1.0 eq.) was dissolved in DCM (2 mL); then, the 1,4-dioxane solution of hydrogen chloride (4 mol/L, 0.16 mL, 0.675 mmol, 3.0 eq.) was dropwise added; the resulting mixture was stirred at room temperature for 1 h, adjusted to a pH value of 8 by using saturated NaHCOs in water, and then extracted with a mixed solvent (DCM:MeOH = 10:1, 15 mL × 3); the organic phases were dried with anhydrous sodium sulfate and filtered; the filtrate was concentrated under reduced pressure; and the resulting crude product was purified by preparative thin-layer chromatography (DCM:MeOH = 8:1) to obtain a product (46.3 mg, yield: 59.9%).

¹HNMR (400 MHz, DMSO-*d*₆) δ(ppm): 9.89 (s, 1H), 7.14 (d, *J =* 7.7 Hz, 1H), 7.09 (s, 1H), 6.97 (d, *J =* 7.8 Hz, 1H), 6.90 (s, 1H), 6.72 (s, 1H), 6.07 (d, *J* = 5.9 Hz, 1H), 5.33 (d, *J* = 3.8 Hz, 1H), 4.25 (s, 1H), 3.51 (d, *J* = 1.6 Hz, 1H), 3.00 (s, 1H), 2.55 (s, 3H), 2.05 (s, 3H), 2.01-1.98 (m, 1H), 1.90 (s, 2H), 1.75-1.73 (m, 2H), 1.48-1.40 (m, 2H). Molecular formula: C₂₀H₂₄N₄O₂ Accurate molecular weight: 352.19 LC-MS(Pos, *m*/*z*)=353.12[M+H]⁺.

### Example 51: Synthesis of (R)-2-(3-((6-(4-ethynyl-2-hydroxyphenyl)-5-methylpyridazin-3-yl)amino)piperidin-1-yl)acetonitrile (Compound 222)

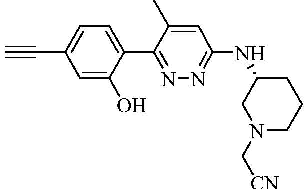

### Step 1: Synthesis of (R)-2-(3-((6-(2-(ethoxymethoxy)-4-formylphenyl)-5-methylpyridazin-3-yl)atnino)piperidin-1-yl)acetonitrile

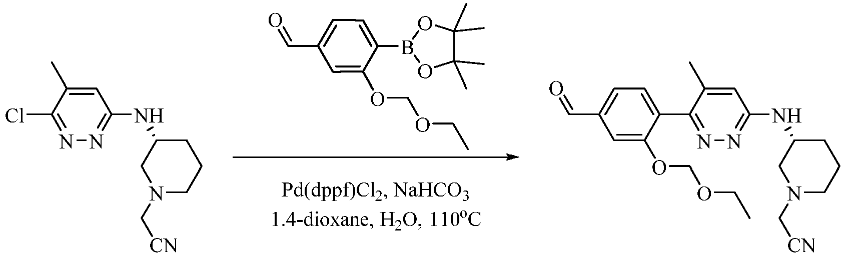

3-(ethoxymethoxy)-4-(4,4,5,5-tetramethyl-1,3,2-dioxaboracyclopentan-2-yl)benzaldehyde (346 mg, 1.13 mmol, 1.5 eq.), (*R*)-2-(3-((6-chloro-5-methylpyridazin-3-yl)amino)piperidin-1-yl)acetonitrile (200 mg, 0.753 mmol, 1.0 eq.), Pd(dppf)Cl₂ (55.1 mg, 0.0753 mmol, 0.1 eq.) and NaHCO₃ (127 mg, 1.51 mmol, 2.0 eq.) were successively added to 1,4-dioxane (6 mL); H₂O (3 mL) was added; and the resulting mixture was heated to 110°C under the protection of nitrogen to allow for reaction for 2 h. The reaction mixture was cooled to room temperature, quenched by adding water (20 mL), and extracted with EA (20 mL × 2); the organic phases were dried with anhydrous sodium sulfate and filtered; the filtrate was concentrated under reduced pressure; and the resulting crude product was purified by silica gel column chromatography (DCM:MeOH = 100:1-20:1) to obtain a product (126 mg, yield: 40.9%).

### Step 2: Synthesis of (R)-2-(3-((6-(2-(ethoxymethoxy)-4-ethynylphenyl)-5-methylpyridazin-3-yl)amino)piperidin-1-yl)acetonitrile

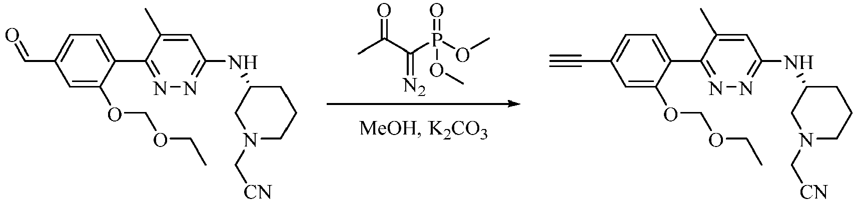

(*R*)-2-(3-((6-(2-(ethoxymethoxy)-4-formylphenyl)-5-methylpyridazin-3-yl)amino)piperidin-1-yl)acetonitrile (126 mg, 0.308 mmol, 1.0 eq.) was dissolved in MeOH (4 mL); K₂CO₃(85.1 mg, 0.616 mmol, 2.0 eq.) and (1-diazo-2-oxopropyl)dimethyl phosphonate (88.7 mg, 0.462 mmol, 1.5 eq.) were added; and the resulting mixture was stirred for 2 h at room temperature. The reaction mixture was quenched by adding water (20 mL), and extracted with EA (20 mL × 2); the organic phases were dried with anhydrous sodium sulfate and filtered; the filtrate was concentrated under reduced pressure; and the resulting crude product was purified by silica gel column chromatography (DCM:MeOH = 100:1-50:1) to obtain a product (72.0 mg, yield: 57.7%).

### Step 3: Synthesis of (R)-2-(3-((6-(4-ethynyl-2-hydroxyphenyl)-5-methylpyridazin-3-yl)amino)piperidin-1-yl)acetonitrile

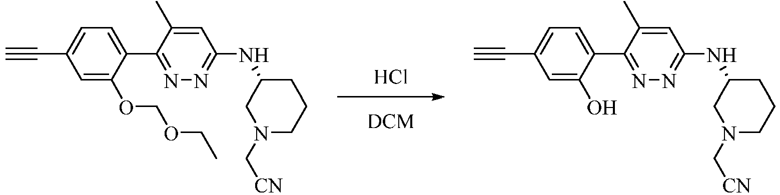

(*R*)-2-(3-((6-(2-(ethoxymethoxy)-4-ethynylphenyl)-5-methylpyridazin-3-yl)amino)piperidin-1-yl)acetonitrile (72.0 mg, 0.178 mmol, 1.0 eq.) was dissolved in DCM (2 mL); then, the 1,4-dioxane solution of hydrogen chloride (4 mol/L, 0.13 mL, 0.534 mmol, 3.0 eq.) was dropwise added; and the resulting mixture was stirred for 1 h at room temperature. The resulting mixture was adjusted to a pH value of 8 by using saturated NaHCO₃ in water, and extracted with DCM (10 mL × 3); the organic phases were dried with anhydrous sodium sulfate and filtered; the filtrate was concentrated under reduced pressure; and the resulting crude product was purified by silica gel column chromatography (DCM:MeOH = 100:1-50:1) to obtain a product (26.0 mg, yield: 42.1%).

¹HNMR (400 MHz, DMSO-*d*₆) δ(ppm): 10.12 (s, 1H), 7.17 (d, *J* = 7.6 Hz, 1H), 7.01-6.98 (m, 2H), 6.72-6.69 (m, 2H), 4.18 (s, 1H), 4.09-4.07 (m, 1H), 3.77 (s, 2H), 2.99 (d, *J* = 87.3 Hz, 1H), 2.68-2.66 (m, 1H), 2.26-2.21 (m, 1H), 2.14-2.09 (m, 1H), 2.03 (s, 3H), 1.91-1.87 (m, 1H), 1.80-1.76 (m, 1H), 1.62-1.54 (m, 1H), 1.34-1.30 (m, 1H). Molecular formula: C₂₀H₂₁N₅O₂ Accurate molecular weight: 347.17 LC-MS(Pos, *m*/*z*)=348.14[M+H]⁺.

### Example 52: Synthesis of (R)-2-(5-methyl-3-((1-methylpiperidin-3-yl)amino)-1,2,4-triazin-6-yl)-5-ethenylphenol (Compound 189)

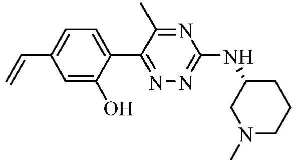

### Step 1: Synthesis of (R)-2-(5-methyl-3-((1-methylpiperidin-3-yl)amino)-1,2,4-triazin-6-yl)-5-ethenylphenol

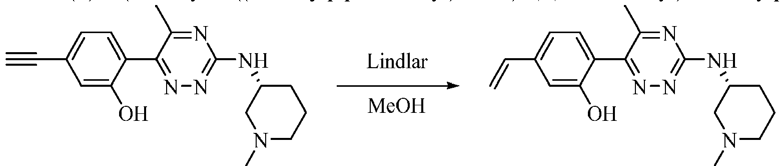

(R)-5-ethynyl-2-(5-methyl-3-((1-methylpiperidin-3-yl)amino)-1,2,4-triazin-6-yl)phenol (80.0 mg, 0.247 mmol, 1.0 eq.) was dissolved in MeOH(5 mL); the Lindlar catalyst (8 mg, 10%) was added; and nitrogen replacement was conducted to allow for reaction for 1 h at room temperature. The mixture was subjected to suction filtration by means of diatomite; and the filtrate was concentrated under reduced pressure, and pulped with the mixed solvent of PE:EA = 5:1 to obtain a product (65.0 mg, yield: 80.7%).

¹HNMR (400 MHz, DMSO-*d*₆) δ(ppm): 9.96 (s, 1H), 7.61 (s, 1H), 7.23 (d, *J =* 7.8 Hz, 1H), 7.05 (d, *J =* 7.5 Hz, 2H), 5.75-6.68 (m, 1H), 5.79 (d, *J* = 17.6 Hz, 1H), 5.29 (d, *J =* 11.0 Hz, 1H), 4.15 (s, 1H), 3.17 (s, 1H), 2.91 (s, 1H), 2.44 (s, 3H), 2.33 (s, 2H), 2.21 (s, 3H), 1.92-1.90 (m, 1H), 1.82-1.79 (m, 1H), 1.72-1.66 (m, 1H), 1.44-1.40 (m, 1H). Molecular formula: C₁₈H₂₃N₅O Accurate molecular weight: 325.19 LC-MS(Pos, *m*/*z*)=326.12[M+H]⁺.

### Example 53: Synthesis of 5-ethynyl-2-(3-((cis-3-hydroxyl-3-methylcyclobutyl)amino)-5-methyl-1,2,4-triazin-6-yl)phenol (Compound 59)

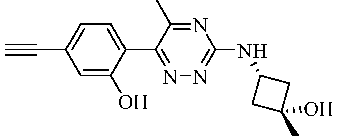

### Step 1: Synthesis of 1-amino-S-methylisothiourea hydriodate

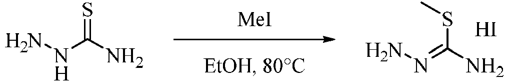

Aminothiourea (100 g, 1.10 mol, 1.0 eq.) and iodomethane (187 g, 1.32 mol, 1.2 eq.) were added to ethanol (400 mL), and heated to 80°C to allow for reaction for 16 h. The reaction mixture was cooled to room temperature to allow for precipitation of solids, which were subjected to suction filtration; and the filter cake was dried to obtain a product (233 g, yield: 91.1%).

### Step 2: Synthesis of 5-methyl-3-(methylthio)-1,2,4-triazine

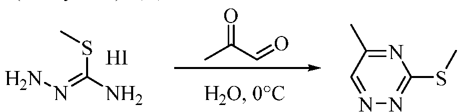

40% pyruvic aldehyde in water (180 g, 1.00 mol, 1.0 eq.) was dissolved in H₂O (500 mL), and cooled to 0°C in the ice water bath; then, NaHCO₃ (84.0 g, 1.00 mol, 1.0 eq.) was added; 1-amino-*S*-methylisothiourea hydriodate (233 g, 1.00 mol, 1.0 eq.) was then dissolved in water, and dropwise added to pyruvic aldehyde in water at 0°C 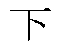 to allow for reaction for 6 h. The resulting mixture was extracted with DCM (300 mL × 24); the organic phases were dried with anhydrous sodium sulfate and filtered; the filtrate was concentrated under reduced pressure; and the resulting crude product was purified by silica gel column chromatography (PE:EA = 50:1-10:1) to obtain a crude product, which was recrystallized with the mixed solvent of PE:EA = 20:1 to obtain a product (85.0 g, yield: 60.2%). Step 3: Synthesis of 5-methyl-3-(methylsulfinyl)-1,2,4-triazine

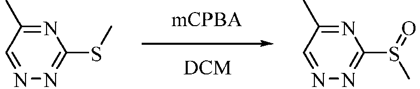

5-methyl-3-(methylthio)-1,2,4-triazine (2.00 g, 0.0142 mol, 1.0 eq.) was dissolved in DCM (20 mL), and stirred at room temperature; metachloroperbenzoic acid (2.88 g, 0.0142 mol, 1.0 eq.) was portionwise added; and the resulting mixture was stirred for 1 h at room temperature. The resulting mixture was subjected to suction filtration; and the filtrate was concentrated under reduced pressure, which was directly used in a next step.

### Step 4: Synthesis of cis-1-methyl-3-((5-methyl-1,2,4-triazin-3-yl)atnino)cyclobutan-1-ol

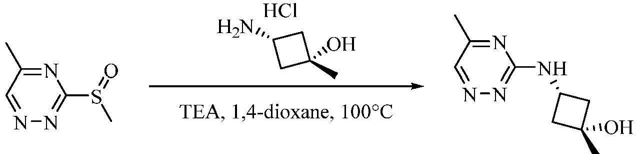

5-methyl-3-(methylsulfinyl)-1,2,4-triazine was dissolved in 1,4-dioxane (30 mL); triethylamine (2.87 g, 0.0284 mol, 2.0 eq.) and cis-3-amino-1-methylcyclobutanol hydrochloride(1.95 g, 0.0142 mol, 1.0 eq.) were added; and the resulting mixture was heated to 100°C to allow for reaction for 1 h. The reaction mixture was cooled to room temperature, quenched by adding water (100 mL), and extracted with EA (30 mL × 3); the organic phases were dried with anhydrous sodium sulfate and filtered; the filtrate was concentrated under reduced pressure; and the resulting crude product was purified by silica gel column chromatography (DCM:MeOH = 100:1-20:1) to obtain a product (2.10 g, two-step yield: 76.3%).

### Step 5: Synthesis of cis-3-((6-bromo-5-methyl-1,2,4-triazin-3-yl)atnino)-1-methylcyclobutan-1-ol

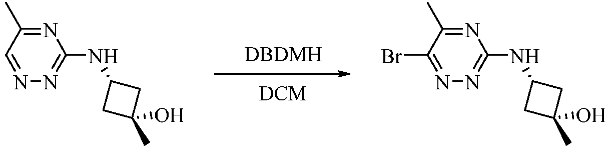

Cis-1-methyl-3-((5-methyl-1,2,4-triazin-3-yl)atnino)cyclobutan-1-ol (1.20 g, 6.18 mmol, 1.0 eq.) was dissolved in DCM (20 mL); and dibromohydantoin (3.53 g, 12.4 mmol, 2.0 eq.) was added to allow for reaction for 1 h at room temperature. Saturated sodium thiosulfate in water was used for quenching; the resulting mixture was extracted with DCM (50 mL × 2); the organic phases were dried with anhydrous sodium sulfate and filtered; the filtrate was concentrated under reduced pressure; and the resulting crude product was purified by silica gel column chromatography (DCM:MeOH = 100:1 -20:1) to obtain a product (550 mg, yield: 32.6%).

### Step 6: Synthesis of 3-(ethoxymethoxy)-4-(3-((cis-3-hydroxyl-3-methylcyclobutyl)amino)-5-methyl-1,2,4-triazin-6-yl)benzaldehyde

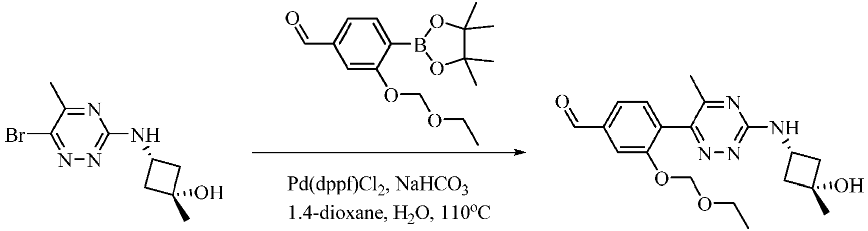

3-(ethoxymethoxy)-4-(4,4,5,5-tetramethyl-1,3,2-dioxaboracyclopentan-2-yl)benzaldehyde (840 mg, 2.75 mmol, 1.5 eq.), cis-3-((6-bromo-5-methyl-1,2,4-triazin-3-yl)amino)-1-methylcyclobutan-1-ol (500 mg, 1.83 mmol, 1.0 eq.), Pd(dppf)Cl₂ (134 mg, 0.183 mmol, 0.1 eq.) and NaHCO₃ (307 mg, 3.66 mmol, 2.0 eq.) were successively added to 1,4-dioxane (10 mL); H₂O (5 mL) was added; and the resulting mixture was heated to 110°C under the protection of nitrogen to allow for reaction for 2 h. The reaction mixture was cooled to room temperature, quenched by adding water (30 mL), and extracted with EA (30 mL × 2); the organic phases were dried with anhydrous sodium sulfate and filtered; the filtrate was concentrated under reduced pressure; and the resulting crude product was purified by silica gel column chromatography (DCM:MeOH = 100:1-20:1) to obtain a product (510 mg, yield: 74.8%).

### Step 7: Synthesis of cis-3-((6-(2-(ethoxymethoxy)-4-ethynylphenyl)-5-methyl-1,2,4-triazin-3-yl)atnino)-1-methylcyclobutan-1 -ol

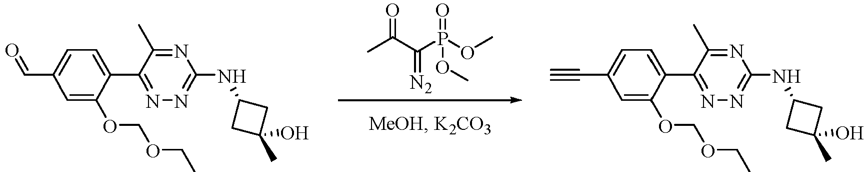

3 -(ethoxymethoxy)-4-(3 -((cis-3 -hydroxyl-3 -methylcyclobutyl)amino)-5 -methyl-1,2,4-triazin-6-yl)benzaldehyde (510 mg, 1.37 mmol, 1.0 eq.) was dissolved in MeOH (10 mL); K₂CO₃ (379 mg, 2.74 mmol, 2.0 eq.) and (1-diazo-2-oxopropyl)dimethyl phosphonate (395 mg, 2.06 mmol, 1.5 eq.) were added; and the resulting mixture was stirred for 2 h at room temperature. The resulting mixture was concentrated, and the resulting crude product was purified by silica gel column chromatography (DCM:MeOH = 100:1-20:1) to obtain a product (495 mg, yield: 98.1%).

### Step 8: Synthesis of 5-ethynyl-2-(3-((cis-3-hydroxyl-3-methylcyclobutyl)amino)-5-methyl-1,2,4-triazin-6-yl)phenol

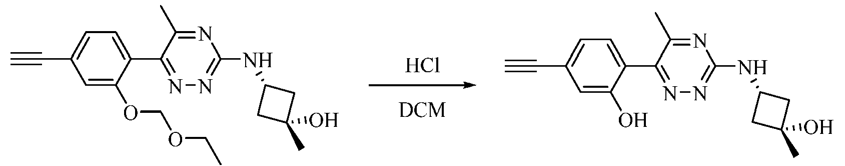

Cis-3-((6-(2-(ethoxymethoxy)-4-ethynylphenyl)-5-methyl-1,2,4-triazin-3-yl)atnino)-1-methylcyclobutan-1-ol (490 mg, 1.33 mmol, 1.0 eq.) was dissolved in DCM (10 mL); the 1,4-dioxane solution of hydrogen chloride (4 mol/L, 1.0 mL, 3.99 mmol, 3.0 eq.) was dropwise added; and the resulting mixture was stirred for 1 h at room temperature. The resulting mixture was adjusted to a pH value of 8 by using saturated NaHCOs in water, and extracted with DCM (20 mL × 3); the organic phases were dried with anhydrous sodium sulfate and filtered; the filtrate was concentrated under reduced pressure; and the resulting crude product was purified by silica gel column chromatography (DCM:MeOH = 100:1-20:1) to obtain a product (310 mg, yield: 75.1%).

¹HNMR (400 MHz, DMSO-*d*₆) δ(ppm): 10.09 (s, 1H), 7.85 (s, 1H), 7.26 (d, *J =* 7.6 Hz, 1H), 7.04-7.02 (m, 2H), 4.99 (s, 1H), 4.21 (s, 1H), 3.95 (s, 1H), 2.39-2.35 (m, 2H), 2.18 (s, 3H), 2.08-2.03 (m, 2H), 1.28 (s, 3H). Molecular formula: C₁₇H₁₈N₄O₂ Accurate molecular weight: 310.14 LC-MS(Pos, *m*/*z*)=311.11[M+H]⁺.

### Example 54: Synthesis of (R)-6-(4-ethynyl-2-hydroxyphenyl)-5-methyl-3-((1-methylpiperidin-3-yl)amino)-1,2,4-triazin-2-oxide (Compound 249)

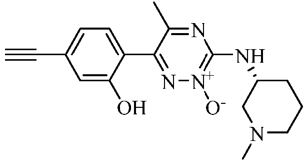

### Step 1: Synthesis of (R)-6-bromo-3-((1-(tert-butoxycarbonyl)piperidin-3-yl)amino)-5-methyl-1,2,4-triazin-2-oxide

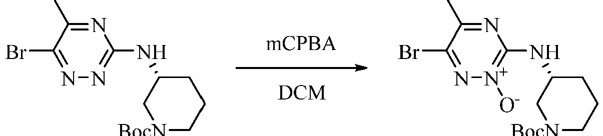

(R)-3-((6-bromo-5-methyl-1,2,4-triazin-3-yl)amino)piperidin-1-tert-butyl formate (1.00 g, 2.69 mmol, 1.0 eq.) was dissolved in DCM (10 mL), and stirred at room temperature; metachloroperbenzoic acid (464 mg, 2.69 mmol, 1.0 eq.) was added; and the resulting mixture was stirred for 4 h at room temperature. The resulting mixture was quenched by using saturated NaHCOs in water, and extracted with DCM (20 mL × 2); the organic phases were dried with anhydrous sodium sulfate and filtered; the filtrate was concentrated under reduced pressure; and the resulting crude product purified by silica gel column chromatography (PE:EA = 10:1-2:1) to obtain a product (945 mg, yield: 90.6%).

### Step 2: Synthesis of (R)-3-((1-(tert-butoxycarbonyl)piperidin-3-yl)atnino)-6-(2-(ethoxymethoxy)-4-formylphenyl)-5-methyl-1,2,4-triazin-2-oxide

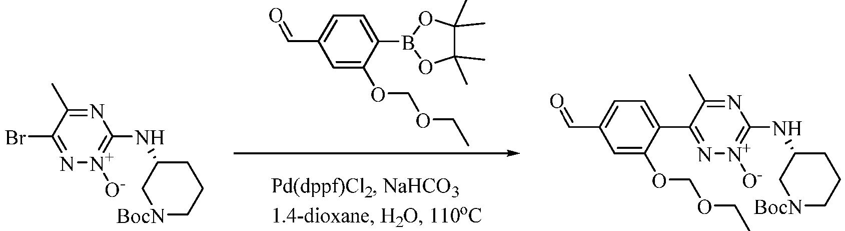

3-(ethoxymethoxy)-4-(4,4,5,5-tetramethyl-1,3,2-dioxaboracyclopentan-2-yl)benzaldehyde (712 mg, 2.33 mmol, 1.5 eq.), (*R*)-6-bromo-3-((1-(tert-butoxycarbonyl)piperidin-3-yl)amino)-5-methyl-1,2,4-triazin-2-oxide (600 mg, 1.55 mmol, 1.0 eq.), Pd(dppf)Cl₂ (113 mg, 0.155 mmol, 0.1 eq.) and NaHCOs (260 mg, 3.10 mmol, 2.0 eq.) were successively added to 1,4-dioxane (20 mL); H₂O(5 mL) was added; and the resulting mixture was heated to 110°C under the protection of nitrogen to allow for reaction for 1 h. The reaction mixture was cooled to room temperature, quenched by adding water (50 mL), and extracted with EA (30 mL × 2); the organic phases were dried with anhydrous sodium sulfate and filtered; the filtrate was concentrated under reduced pressure; and the resulting crude product was purified by silica gel column chromatography (DCM:MeOH = 100:1-20:1) to obtain a product (650 mg, yield: 86.3%).

### Step 4: Synthesis of (R)-3-((1-(tert-butoxycarbonyl)piperidin-3-yl)amino)-6-(2-(ethoxymethoxy)-4-ethynylphenyl)-5-methyl-1,2,4-triazin-2-oxide

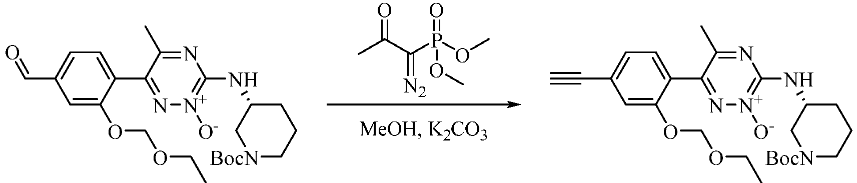

(*R*)-3-((1-(tert-butoxycarbonyl)piperidin-3-yl)amino)-6-(2-(ethoxymethoxy)-4-formylphenyl)-5-methyl-1,2,4-triazin-2-oxide (650 mg, 1.33 mmol, 1.0 eq.) was dissolved in MeOH(15 mL); K₂CO₃ (368 mg, 2.66 mmol, 2.0 eq.) and (1-diazo-2-oxopropyl)dimethyl phosphonate (384 mg, 2.00 mmol, 1.5 eq.) were added; and the resulting mixture was stirred for 1 h at room temperature. The reaction mixture was concentrated; and the resulting crude product was purified by silica gel column chromatography (DCM: MeOH = 100:1-20:1) to obtain a product (582 mg, yield: 90.3%).

### Step 4: Synthesis of (R)-6-(4-ethynyl-2-hydroxyphenyl)-5-methyl-3-(piperidin-3-ylamino)-1,2,4-triazin-2-oxide

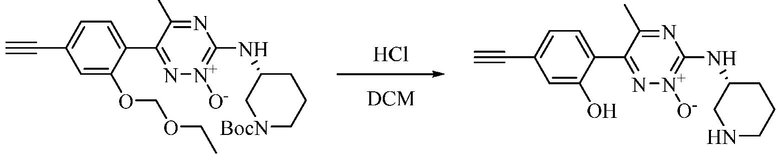

(*R*)-3-((1-(tert-butoxycarbonyl)piperidin-3-yl)amino)-6-(2-(ethoxymethoxy)-4-ethynylphenyl)-5-methyl-1,2,4-triazin-2-oxide (580 mg, 1.20 mmol, 1.0 eq.) was dissolved in DCM (10 mL); the 1,4-dioxane solution of hydrogen chloride (4 mol/L, 1.2 mL, 4.80 mmol, 4.0 eq.) was dropwise added; and the resulting mixture was stirred for 2 h at room temperature. The resulting mixture was adjusted to a pH value of 8 by using saturated NaHCOs in water, and extracted with DCM (30 mL × 2); the organic phases were dried with anhydrous sodium sulfate and filtered; the filtrate was concentrated under reduced pressure; and the resulting crude product was purified by silica gel column chromatography (DCM:MeOH = 50:1-10:1) to obtain a product (348 mg, yield: 89.2%).

### Step 5: Synthesis of (R)-6-(4-ethynyl-2-hydroxyphenyl)-5-methyl-3-((1-methylpiperidin-3-yl)amino)-1,2,4-triazin-2-oxide

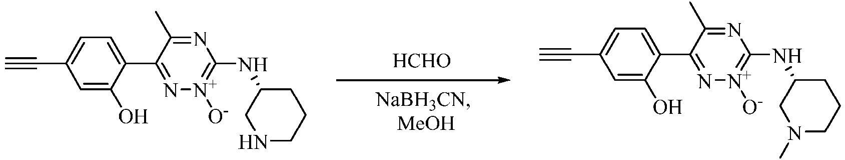

(R)-6-(4-ethynyl-2-hydroxyphenyl)-5-methyl-3-(piperidin-3-ylamino)-1,2,4-triazin-2-oxide (345 mg, 1.06 mmol, 1.0 eq.) was dissolved in MeOH (30 mL); formaldehyde in water (mass fraction: 37%, 103 mg, 1.27 mmol, 1.2 eq.) was added; and the resulting mixture was stirred at room temperature for 20 min. Then, NaBH₃CN (80.0 mg, 1.27 mmol, 1.2 eq.) was added to allow for reaction for 1 hour. The resulting mixture was concentrated, dissolved by addition of DCM (30 mL), and washed with saturated NaHCOs in water; the organic phases were dried with anhydrous sodium sulfate and filtered; the filtrate was concentrated under reduced pressure; and the resulting crude product was purified by silica gel column chromatography (DCM:MeOH = 100:1-50:1) to obtain a product (312 mg, yield: 86.7%).

¹HNMR (400 MHz, DMSO-*d*₆) δ(ppm): 10.12 (s, 1H), 7.90 (d, *J* = 8.3 Hz, 1H), 7.24-7.22 (m, 1H), 7.03-7.01 (m, 2H), 4.25 (s, 1H), 4.04-4.02 (m, 1H), 2.66 (d, *J =* 9.1 Hz, 1H), 2.45 (s, 1H), 2.20 (s, 3H), 2.19 (s, 3H), 2.12 (s, 1H), 1.69-1.65 (m, 2H), 1.61-1.52 (m, 2H), 1.28-1.26 (m, 1H).

Molecular formula: C₁₈H₂₁N₅O₂ Accurate molecular weight: 339.17 LC-MS(Pos, *m*/*z*)=340.15[M+H]⁺.

### Example 55: Synthesis of Compound 5-ethynyl-2-(4-(((cis)-3-hydroxyl-3-methylcyclobutyl)amino)-6,7-dihydro-5H-cyclopentan[d]pyridazin-1-yl)phenol (Compound 8)

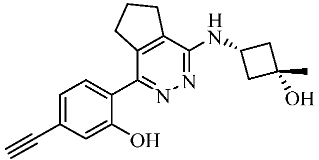

### Synthetic Route

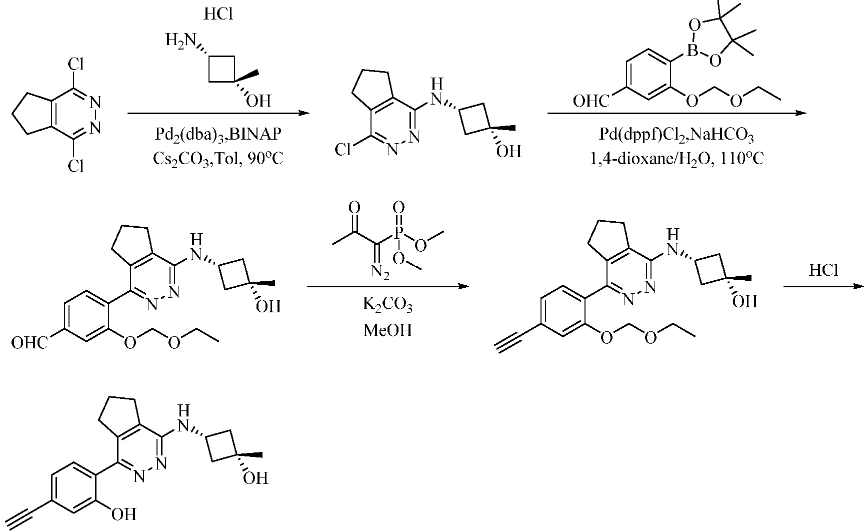

This compound was synthesized by referring to the steps in the foregoing embodiments.

¹HNMR (400 MHz, DMSO-*d₆*) δ(ppm): 13.65 (s, 1H), 7.58-7.56 (d, *J*=8 Hz, 1H), 7.00-6.97 (m, 2H), 6.84-6.82 (d, *J*=8 Hz, 1H), 4.95 (s, 1H), 4.21 (s, 1H), 4.14-4.09 (m, 1H), 3.15-3.11 (m, 2H), 2.78 (t, *J*=8 Hz, 2H), 2.44-2.40 (m, 2H), 2.11-2.03 (m, 4H), 1.31 (s, 3H).

Molecular formula: C₂₀H₂₁N₃O₂ Accurate molecular weight: 335.16 LC-MS (Pos, m/z)=336.09[M+H]⁺

### Example 56: Synthesis of Compound (cis)-3-((4-(4-ethynyl-2-methylphenyl)-5,6,7,8-tetrahydrophthalazin-1-yl)amino)-1-methylcyclobutan-1-ol (Compound 49)

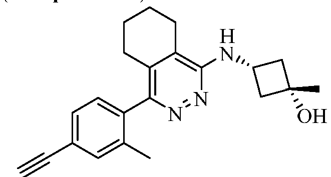

### Synthetic Route

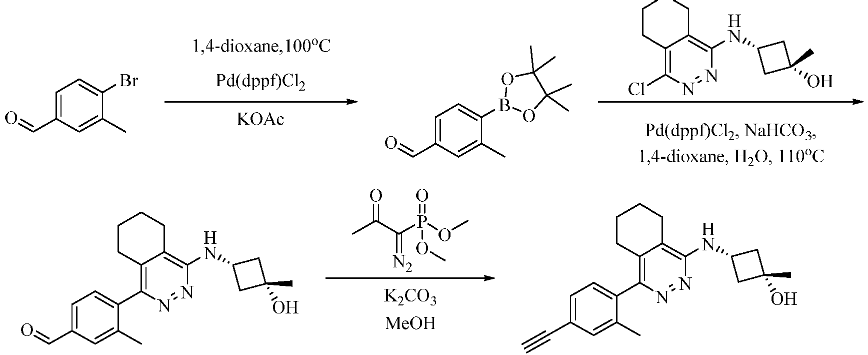

This compound was synthesized by referring to the steps in the foregoing embodiments.

¹HNMR (400 MHz, DMSO-*d₆*) δ(ppm): 7.43 (s, 1H), 7.36-7.34 (m, 1H), 7.14-7.12 (d, *J*=8 Hz, 1H), 6.11-6.10 (d, *J=*4 Hz, 1H), 4.90 (s, 1H), 4.19 (s, 1H), 4.14-4.09 (m, 1H), 2.42-2.37 (m, 4H), 2.10-2.08 (m, 4H), 1.99 (s, 3 H), 1.74-1.73 (m, 2 H), 1.61-1.59 (m, 2H), 1.30 (s, 3H).

Molecular formula: C₂₂H₂₅N₃O Accurate molecular weight: 347.20 LC-MS (Pos, m/z)=348.13[M+H]⁺

### Example 57: Synthesis of (cis)-3-((4-(4-ethynylphenyl)phthalazin-1-yl)amino)-1-methylcyclobutan-1-ol (Compound 148)

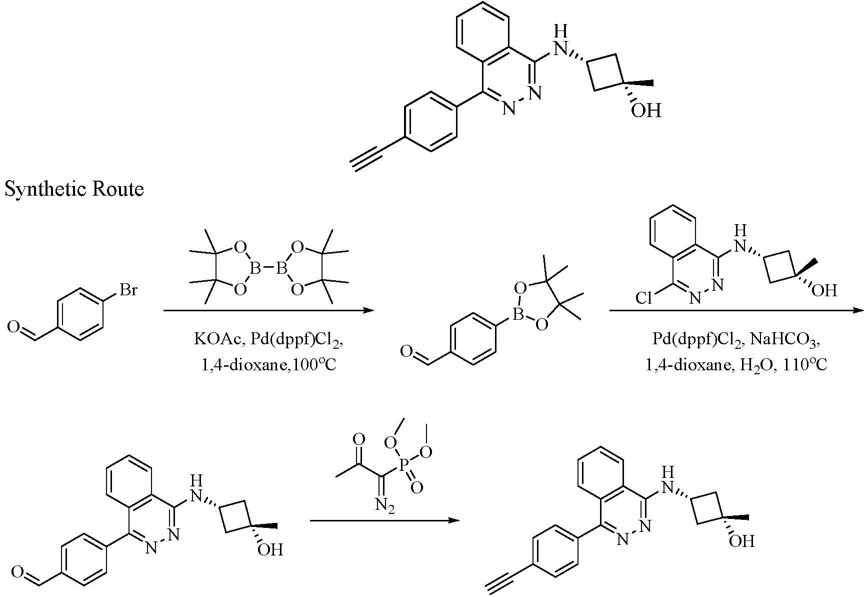

This compound was synthesized by referring to the steps in the foregoing embodiments.

¹HNMR (400MHz, DMSO-*d₆*) δ(ppm): 8.48-8.46 (d, *J*=8 Hz, 1H), 7.92-7.77 (m, 3H), 7.66-7.62 (m, 4H), 4.99 (s, 1H), 4.30-4.23 (m, 2H), 2.51-2.47 (m, 2H), 2.21-2.16 (m, 2H), 1.34 (s, 3 H).

Molecular formula: C₂₁H₁₉N₃O Accurate molecular weight: 329.15 LC-MS(Pos, *m*/*z*)=330.11[M+H]⁺

### Example 58: Synthesis of (cis)-3-((4-(4-ethynyl-2-fluorophenyl)phthalazin-1-yl)amino)-1-methylcyclobutan-1-ol (Compound 149)

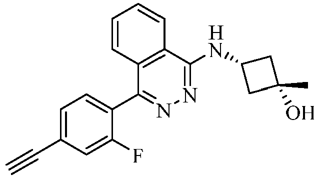

### Synthetic Route

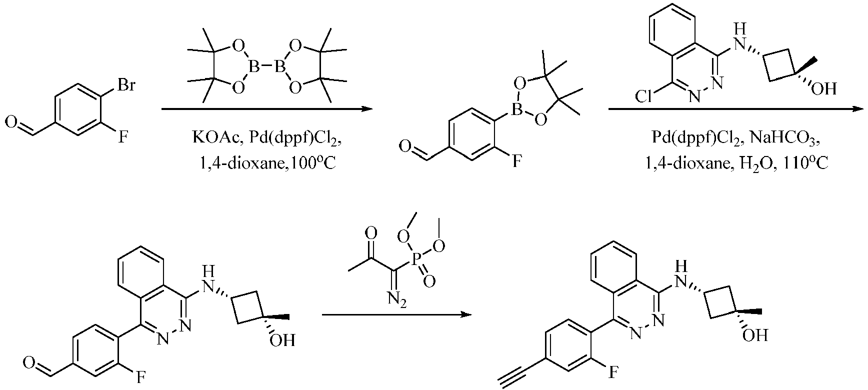

This compound was synthesized by referring to the steps in the foregoing embodiments.

¹HNMR (400 MHz, DMSO-*d₆*) δ(ppm): 8.48-8.46 (d, *J*=8 Hz, 1H), 7.92-7.72 (m, 3H), 7.59-7.46 (m, 4H), 5.00 (s, 1H), 4.29-4.24 (m, 1H), 2.51-2.47 (m, 2H), 2.21-2.16 (m, 2H), 1.34 (s, 3H)

Molecular formula: C₂₁H₁₈FN₃O Accurate molecular weight: 347.14 LC-MS(Pos, *m*/*z*)=348.08[M+H]⁺

### Example 59: Synthesis of (R)-5-ethynyl-2-(5-methyl-3-((1-(tetrahydro-2H-pyran-4-yl)piperidin-3-yl)amino)-1,2,4-triazin-6-yl)phenol (Compound 248)

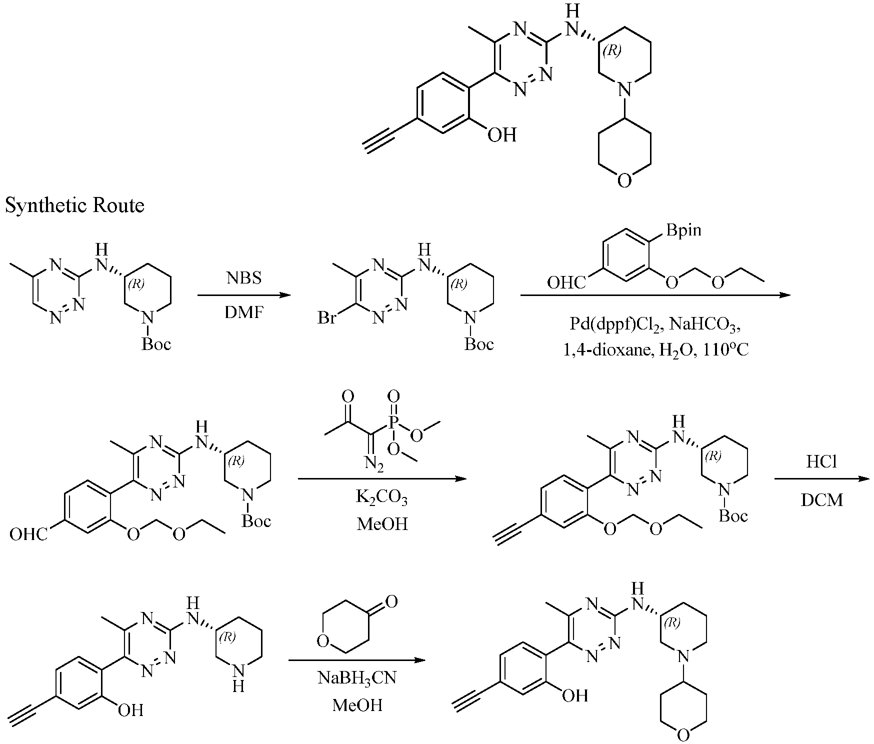

This compound was synthesized by referring to the steps in the foregoing embodiments.

¹HNMR (400MHz, DMSO-*d₆*) δ(ppm): 10.11 (s, 1H), 7.49 (s, 1H), 7.27-7.25 (d, *J*=8 Hz, 1H), 7.04-7.02 (m, 2H), 4.23 (s, 1H), 3.97-3.82 (m, 3H), 3.26 (t, *J*=12Hz, 1H), 3.04 (s, 1H), 2.77 (s, 1H), 2.05-2.18 (m, 5 H), 1.91-1.86 (m, 1 H), 1.65-1.33 (m, 7H).

Molecular formula: C₂₂H₂₇N₅O₂ Accurate molecular weight: 393.22 LC-MS(Pos, m/z)=394.15[M+H]⁺

### Example 60: Synthesis of (S)-5-ethynyl-2-(5-methyl-3-(((tetrahydrofuran-2-yl)methyl)amino)-1,2,4-triazin-6-yl)phenol (Compound 251)

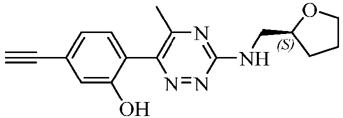

### Synthetic Route

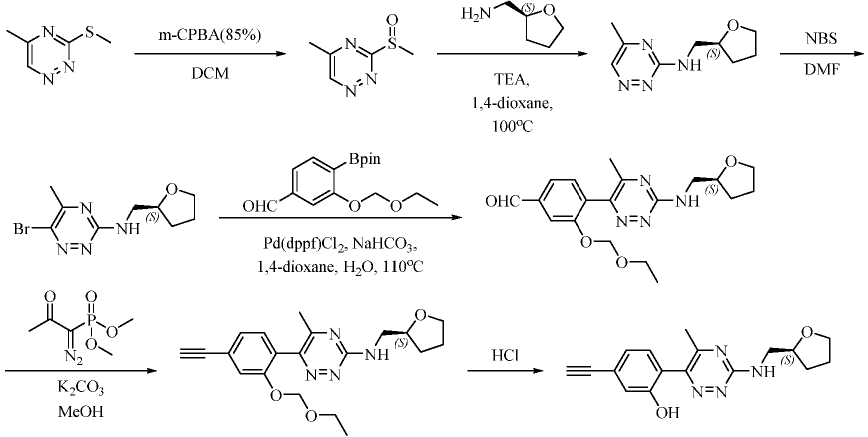

This compound was synthesized by referring to the steps in the foregoing embodiments.

¹HNMR (400 MHz, DMSO-*d₆*) δ(ppm): 10.11 (s, 1H), 7.79 (s, 1H), 7.27-7.25 (d, *J*=8 Hz, 1H), 7.04-7.01 (m, 2H), 4.23 (s, 1H), 4.06-4.03 (m, 1H), 3.81-3.76 (m, 1H), 3.66-3.60 (m, 1H), 3.50-3.46 (m, 1H), 3.38 (s, 1H), 2.18 (s, 3H), 1.98-1.75 (m, 3H), 1.66-1.58 (m, 1H).

Molecular formula: C₁₇H₁₈N₄O₂ Accurate molecular weight: 310.14 LC-MS(Pos, *m*/*z*)=311.08[M+H]⁺

### Example 61: Synthesis of (R)-2-(3-((6-(4-ethynyl-2-hydroxyphenyl)-5-methyl-1,2,4-triazin-3-yl)amino)piperidin-1-yl)acetic acid (Compound 258)

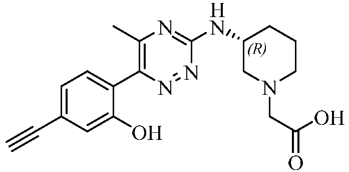

### Synthetic Route

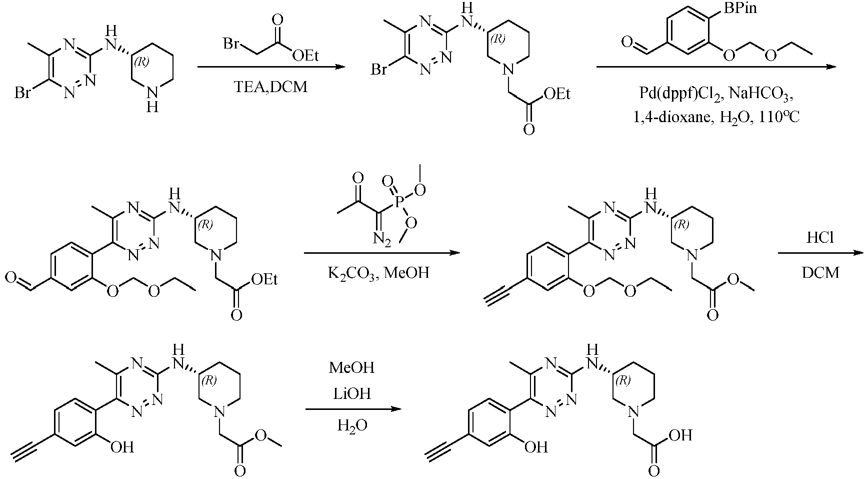

This compound was synthesized by referring to the steps in the foregoing embodiments.

¹HNMR (400 MHz, DMSO-*d₆*) δ(ppm): 10.30 (s, 1H), 7.66 (s, 1H), 7.26-7.24 (d, *J*=8 Hz, 1H), 7.06-7.01 (m, 2H), 4.23-4.16 (m, 2H), 3.51 (s, 2H), 3.31-3.29 (m, 1H), 3.06-3.03 (m, 1H), 2.64-2.50 (m, 2H), 2.19 (s, 3H), 1.91-1.69 (m, 3H), 1.51-1.43 (m, 1H).

Molecular formula: C₁₉H₂₁N₅O₃ Accurate molecular weight: 367.16 LC-MS(Pos, m/z)=368.13[M+H]⁺

### Example 62: Synthesis of 2-(4-(((1S,3S)-3-aminocyclopentyl)amino)-5,6,7,8-tetrahydrophthalazin-1-yl)-5-ethynylphenol (Compound 53)

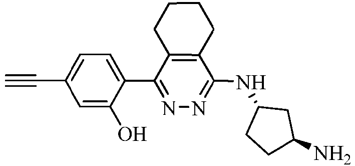

### Synthetic Route:

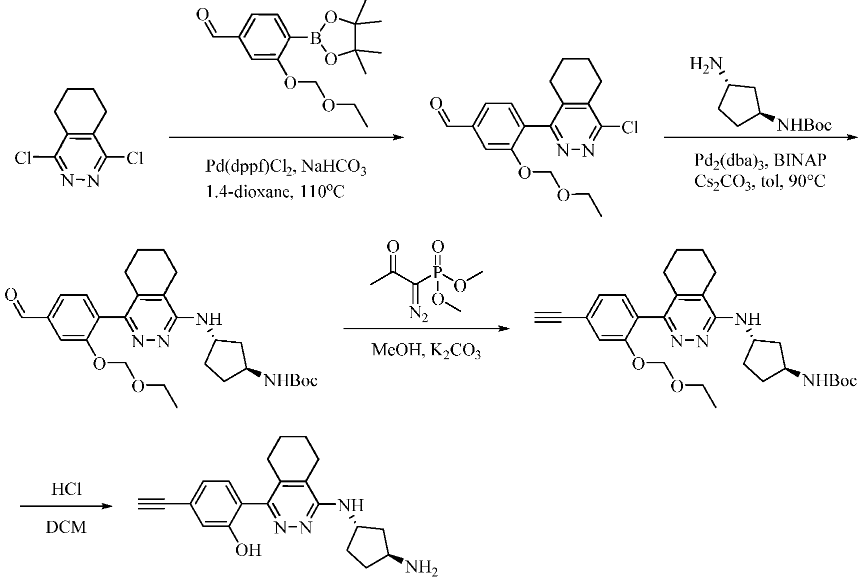

This compound was synthesized by referring to the steps in the foregoing embodiments.

¹HNMR (400MHz, DMSO-*d*₆) δ(ppm): 7.13 (d, *J* = 8.2 Hz, 1H), 6.98-6.96 (m, 2H), 5.71 (d, *J* = 6.9 Hz, 1H), 4.65-4.60 (m, 1H), 4.16 (s, 1H), 3.45-3.42 (m, 4H), 2.38-2.31 (m, 4H), 2.21-2.14 (m, 1H), 1.99-1.91 (m, 1H), 1.78-1.74 (m, 4H), 1.61-1.51 (m, 3H).

Molecular formula: C₂₁H₂₄N₄O Accurate molecular weight: 348.20 LC-MS (Pos, m/z)=349.12[M+H]⁺.

### Example 63: Synthesis of 2-(4-((cis-3-aminocyclobutyl)amino)-5,6,7,8-tetrahydrophthalazin-1-yl)-5-ethynylphenol (Compound 51)

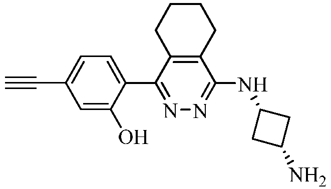

### Synthetic Route:

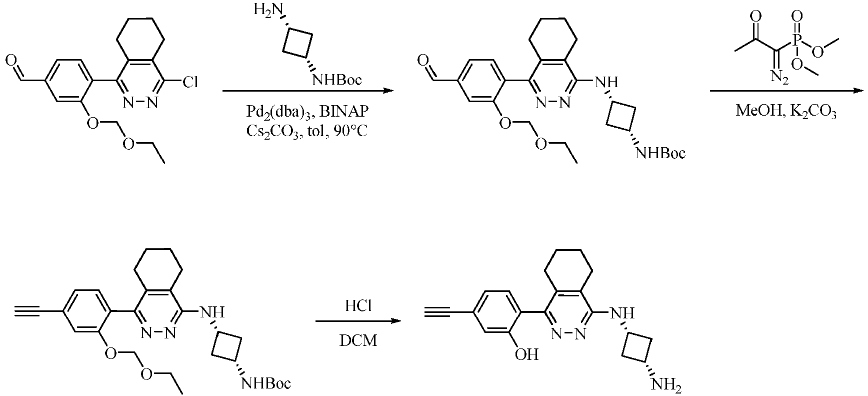

This compound was synthesized by referring to the steps in the foregoing embodiments.

¹HNMR(400MHz, DMSO-*d*₆)δ(ppm): 7.11 (d, *J* = 7.7 Hz, 1H), 7.05 (d, *J* = 1.3 Hz, 1H), 6.97-6.95 (m, 1H), 6.09 (d, *J* = 6.7 Hz, 1H), 4.17 (s, 1H), 4.15-4.11 (m, 1H), 3.39 (s, 2H), 3.16-3.09 (m, 1H), 2.69-2.62 (m, 2H), 2.41-2.38 (m, 2H), 2.33-2.30 (m, 2H), 1.84-1.74 (m, 4H), 1.61-1.58 (m, 2H).

Molecular formula: C₂₀H₂₂N₄O Accurate molecular weight: 334.18 LC-MS (Pos, m/z)=335.11[M+H]⁺.

### Example 64: Synthesis of 2-(4-((trans-3-aminocyclobutyl)amino)-5,6,7,8-tetrahydrophthalazine-1-yl)-5-ethynylphenol (Compound 52)

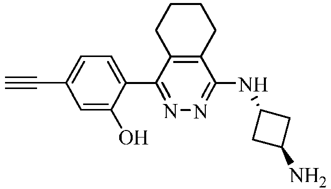

### Synthetic Route:

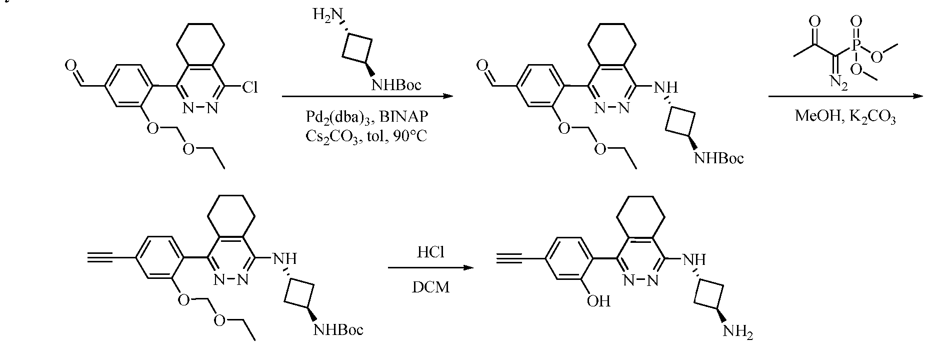

This compound was synthesized by referring to the steps in the foregoing embodiments.

¹HNMR (400MHz, DMSO-*d*₆) δ(ppm): 8.38 (s, 1H), 7.11 (d, *J* = 7.7 Hz, 1H), 7.07 (d, *J* = 1.4 Hz, 1H), 6.98-6.96 (m, 1H), 6.34 (d, *J =* 6.3 Hz, 1H), 4.81-4.76 (m, 1H), 4.17 (s, 1H), 3.77-3.75 (m, 2H), 2.47-2.41 (m, 6H), 2.34-2.31 (m, 2H), 2.03-1.98 (m, 1H), 1.76-1.75 (m, 2H), 1.62-1.61 (m, 2H).

Molecular formula: C₂₀H₂₂N₄O Accurate molecular weight: 334.18 LC-MS(Pos, *m*/*z*)=335.13 [M+H]⁺.

### Example 65: Synthesis of 5-ethynyl-2-(4-((cis-3-hydroxyl-3-methylcyclobutyl)amino)-5,6,7,8-tetrahydro-5,8-ethylphthalazine-1-yl)phenol (Compound 12)

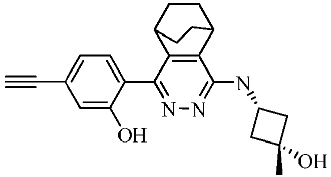

### Synthetic Route:

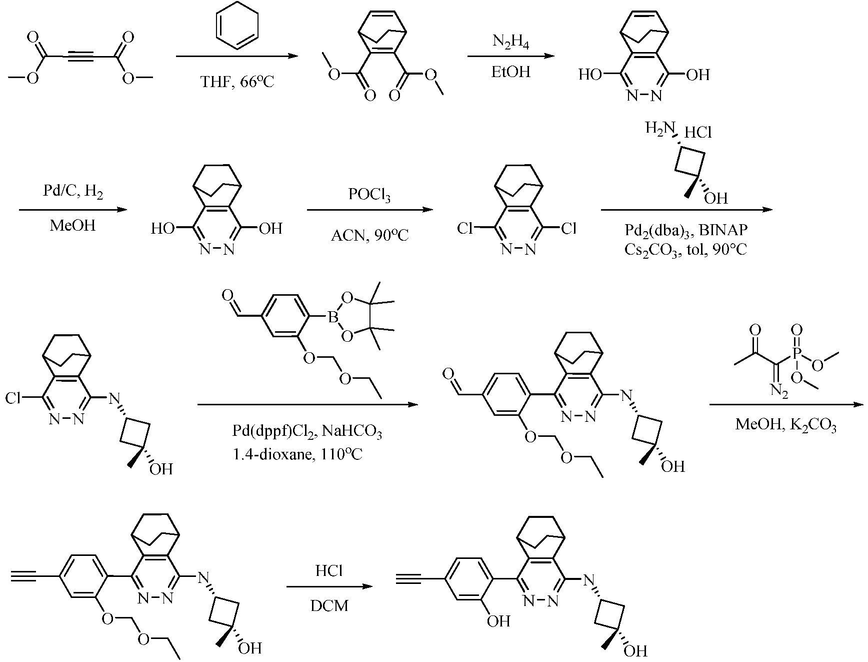

This compound was synthesized by referring to the steps in the foregoing embodiments.

¹HNMR (400MHz, DMSO-*d*₆) δ(ppm): 10.40 (s, 1H), 7.25-7.23 (m, 1H), 7.02-7.00 (m, 2H), 6.74 (s, 1H), 4.94 (s, 1H), 4.18 (s, 1H), 4.10-4.04 (m, 1H), 3.44 (s, 1H), 2.79 (s, 1H), 2.45-2.40 (m, 2H), 2.11-2.06 (m, 2H), 1.75-1.67 (m, 4H), 1.31 (s, 3H), 1.27-1.20 (m, 4H).

Molecular formula: C₂₃H₂₅N₃O₂ Accurate molecular weight: 375.19 LC-MS (Pos, m/z)=376.15[M+H]⁺.

### Example 66: Synthesis of 2-(4-((cis-3-hydroxyl-3-methylcyclobutyl)amino)-5,6,7,8-tetrahydro-5,8-ethylphthalate-1-yl)-5-ethenylphenol (Compound 21)

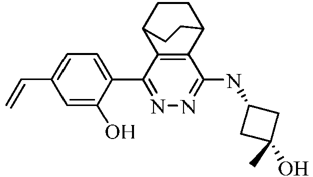

### Synthetic Route:

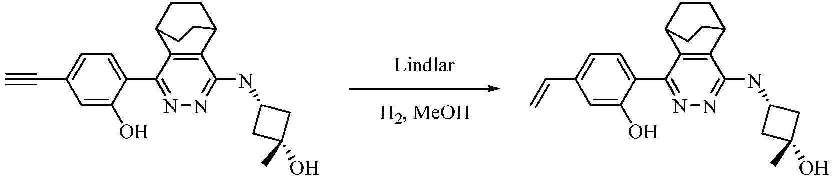

This compound was synthesized by referring to the steps in the foregoing embodiments.

¹HNMR (400 MHz, DMSO-*d*₆) δ(ppm): 7.26-7.24 (m, 1H), 7.06-7.05 (m, 2H), 6.76-6.69 (m, 1H), 5.81 (d, *J =* 17.6 Hz, 1H), 5.81 (d, *J =* 11.2 Hz, 1H), 5.02 (s, 1H), 4.06-4.00 (m, 1H), 3.57 (s, 1H), 2.89 (s, 1H), 2.48-2.43 (m, 2H), 2.17-2.12 (m, 2H), 1.78-1.71 (m, 4H), 1.31 (s, 3H), 1.27-1.24 (m, 4H).

Molecular formula: C₂₃H₂₇N₃O₂ Accurate molecular weight: 377.21 LC-MS(Pos, *m*/*z*)=378.15[M+H]⁺.

### Example 67: Synthesis of (R)-6-(5-ethylpyridin-2-yl)-5-methyl-N-(1-methylpiperidin-3-yl)pyridazin-3-amine (Compound 176)

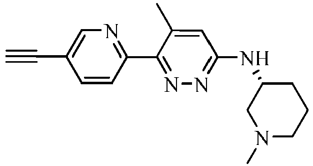

### Synthetic Route:

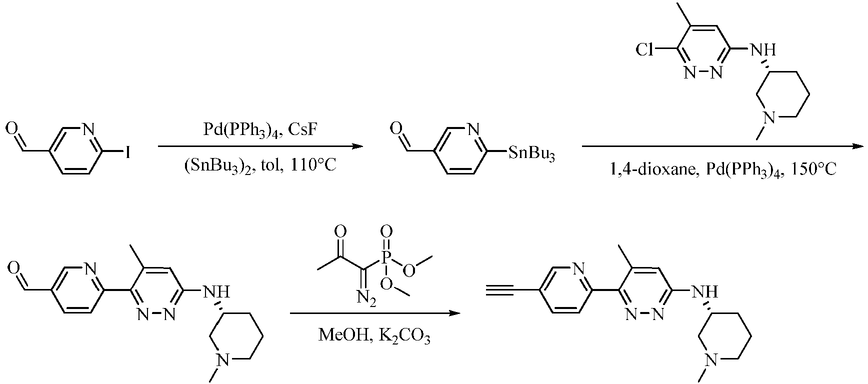

This compound was synthesized by referring to the steps in the foregoing embodiments.

¹HNMR(400MHz, DMSO-*d*₆)δ(ppm): 8.72-8.71 (m, 1H), 8.02-7.99 (m, 1H), 7.96-7.94 (m, 1H), 6.89 (d, *J =* 7.9 Hz, 1H), 6.73 (s, 1H), 4.50 (s, 1H), 4.10-4.08 (m, 1H), 2.87 (d, *J* = 8.7 Hz, 1H), 2.57-2.54 (m, 1H), 2.38 (s, 3H), 2.19 (s, 3H), 2.06-2.01 (m, 1H), 1.94-1.91 (m, 1H), 1.86-1.82 (m, 1H), 1.74-1.69 (m, 1H), 1.59-1.51 (m, 1H), 1.36-1.26 (m, 1H).

Molecular formula: C₁₈H₂₁N₅ Accurate molecular weight: 307.18 LC-MS(Pos, *m*/*z*)=308.09[M+H]⁺.

### Example 68: Synthesis of (3R)-3-((6-(4-ethynyl-2-hydroxyphenyl)-5-methyl-1,2,4-triazin-3-yl)amino)-1-methylpiperidin-1-oxide(Compound 247)

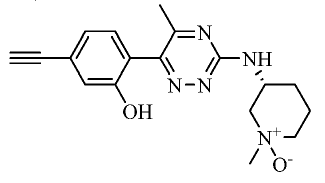

### Synthetic Route:

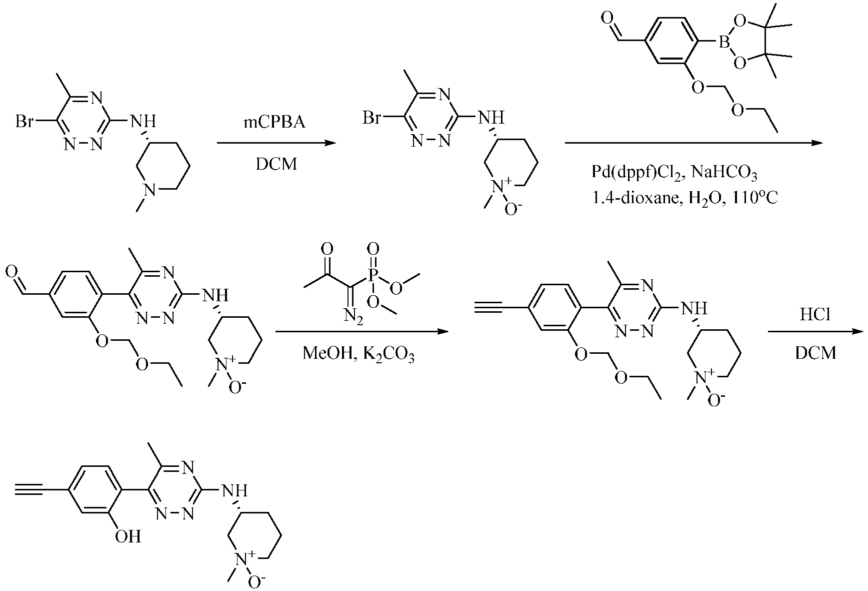

This compound was synthesized by referring to the steps in the foregoing embodiments.

¹HNMR (400MHz, DMSO-*d*₆) δ(ppm): 11.89 (s, 1H), 9.00 (s, 1H), 7.24 (d, *J* = 7.8 Hz, 1H), 7.07 (d, *J* = 1.3Hz, 1H), 7.00-6.98 (m, 1H), 4.48 (s, 1H), 4.20 (s, 1H), 3.67-3.63 (m, 1H), 3.29-3.21 (m, 3H), 3.16 (s, 3H), 2.33-2.27 (s, 1H), 2.20 (s, 3H), 1.84 (s, 1H), 1.69-1.63 (m, 2H).

Molecular formula: C₁₈H₂₁N₅O₂ Accurate molecular weight: 339.17 LC-MS(Pos, *m*/*z*)=340.10[M+H]⁺.

### Example 69: Synthesis of 5-ethynyl-2-(4-(((tetrahydrofuran-2-yl)methyl)amino)-5,6,7,8-tetrahydrophthalazin-1-yl)phenol (Compound 44)

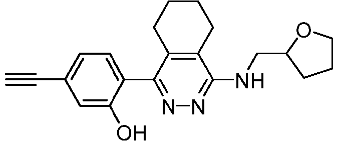

### Synthetic Route:

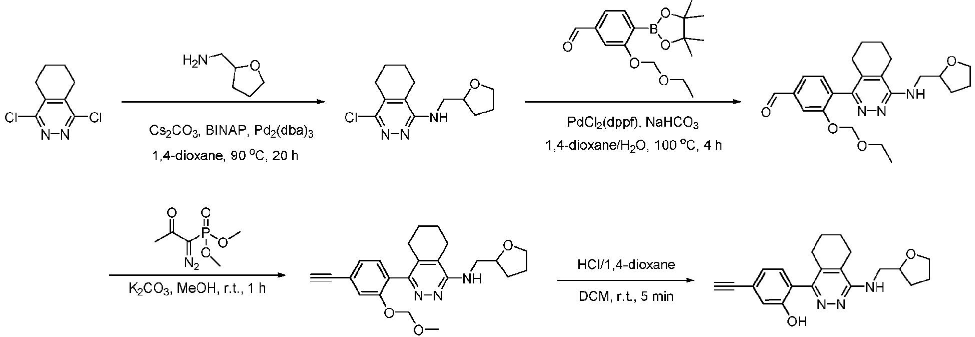

This compound was synthesized by referring to the steps in the foregoing embodiments.

¹H-NMR (400 MHz, DMSO-*d*₆) δ(ppm): 10.03 (s, 1H), 7.14-7.12 (m, 1H), 6.99-6.97 (m, 2H), 6.09-6.06 (m, 1H), 4.17-4.13 (m, 2H), 3.83-3.78 (m, 1H), 3.67-3.62 (m, 1H), 3.53-3.50 (m, 2H), 2.38-2.32 (m, 4H), 2.00-1.81 (m, 3H), 1.79-1.75 (m, 2H), 1.68-1.60 (m, 3H).

Molecular formula: C₂₁H₂₃N₃O₂ Accurate molecular weight: 349.18 LC-MS(m/z): 350.09 [M+H]⁺

### Example 70: Synthesis of 3-ethynyl-2-fluoro-6-(4-((cis-3-hydroxyl-3-methylcyclobutyl)amino)-5,6,7,8-tetrahydrophthalazin-1-yl)phenol (Compound 26)

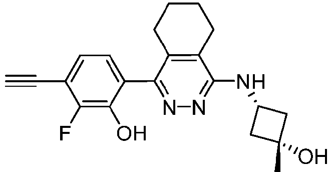

### Synthetic Route:

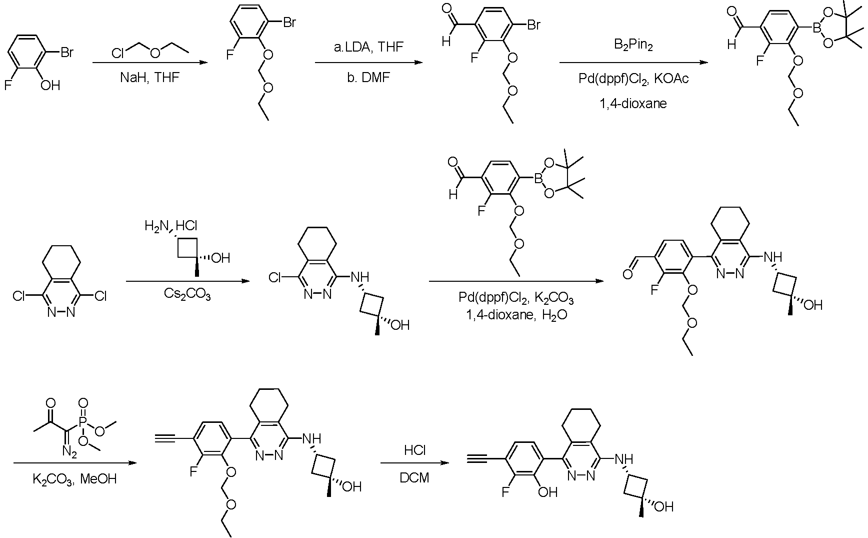

This compound was synthesized by referring to the steps in the foregoing embodiments.

¹H-NMR (400 MHz, DMSO-*d*₆) δ(ppm): 10.33 (s, 1H), 7.04-6.97 (m, 2H), 6.19-6.17 (m, 1H), 4.93 (s, 1H), 4.52 (s, 1H), 4.12-4.07 (m, 1H), 3.17 (s, 1H), 2.40-2.33 (m, 5H), 2.11-2.06 (m, 2H), 1.76-1.60 (m, 4H), 1.30 (s, 3H). Molecular formula: C₂₁H₂₂FN₃O₂ Accurate molecular weight: 367.17 LC-MS(*m*/*z*): 368.08 [M+H]⁺

### Example 71: Synthesis of 3-ethynyl-2-fluoro-6-(4-((cis-3-hydroxyl-3-methylcyclobutyl)amino)phthalazin-1-yl)phenol (Compound 24)

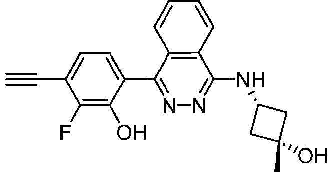

### Synthetic Route:

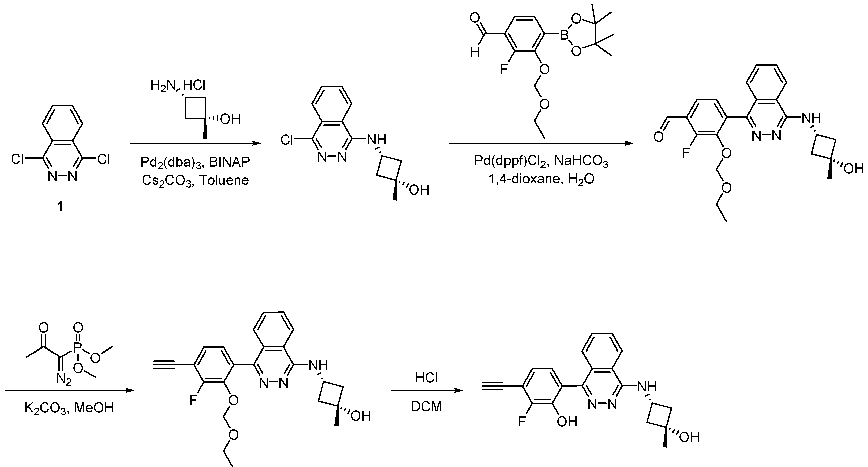

This compound was synthesized by referring to the steps in the foregoing embodiments.

¹H-NMR (400 MHz, DMSO-*d*₆) δ(ppm): 9.86(s, 1H), 8.95-8.93 (d, *J*=8.1 Hz, 1H), 8.12-8.03 (m, 2H), 7.66-7.64 (d, *J*=7.7 Hz, 1H), 7.21-7.16 (m, 2H), 5.23 (s, 1H), 4.65 (s, 1H), 4.24-4.11 (m, 2H), 2.64-2.59 (m, 2H), 2.42-2.37 (m, 2H), 1.35 (s, 3H).

Molecular formula: C₂₁H₁₈FN₃O₂ Accurate molecular weight: 363.14 LC-MS(*m*/*z*): 364.06 [M+H]⁺

### Example 72: Synthesis of 2-(4-((cis-3-hydroxyl-3-methylcyclobutyl)amino)phthalazin-1-yl)-5-(prop-1-yn-1-yl)phenol (Compound 103)

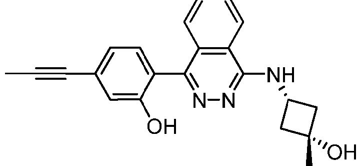

### Synthetic Route:

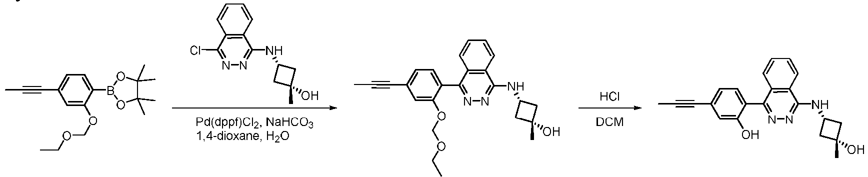

This compound was synthesized by referring to the steps in the foregoing embodiments.

¹H-NMR (400 MHz, DMSO-*d*₆) δ(ppm): 9.89 (s, 1H),8.41 (d, *J*=8.2 Hz, 1H), 7.86-7.76 (m, 2H), 7.56-7.47 (m, 2H), 7.25 (d, *J*=7.8 Hz, 1H), 6.98 (s, 2H), 5.00 (s, 1H), 4.27-4.22 (m, 1H), 3.81 (d, *J*=5.0 Hz, 1H), 2.20-2.15 (m, 2H), 2.08 (s, 3H), 1.35 (s, 3H).

Molecular formula: C₂₂H₂₁N₃O₂ Accurate molecular weight: 359.16 LC-MS(*m*/*z*): 360.19[M+H]⁺

### Example 73: Synthesis of 5-((E)-(2-hydroxycyclobutenyl)methyl)-2-(5-methyl-3-(((R)-1-methylpiperidin-3-yl)amino)-1,2,4-triazin-6-yl)phenol (Compound 252)

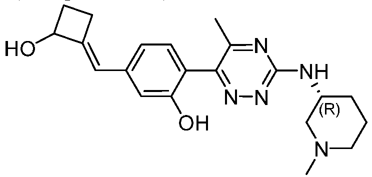

### Synthetic Route:

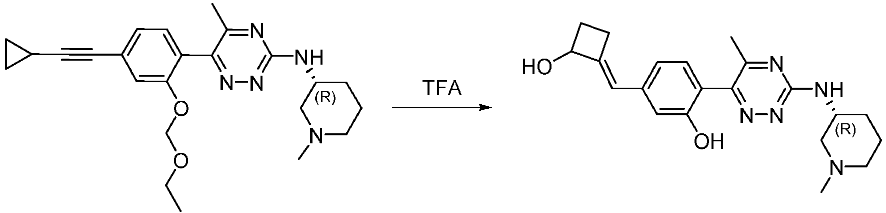

This compound was synthesized by referring to the steps in the foregoing embodiments.

¹H-NMR (400 MHz, DMSO-*d*₆) δ(ppm): 9.75 (s, 1H), 7.40 (s, 1H), 7.20 (d, *J*=7.8 Hz, 1H), 6.86-6.81 (m, 2H), 6.26 (d, *J*=1.7 Hz, 1H), 5.52 (d, *J*=7.6 Hz, 1H), 4.69-4.67 (m, 1H), 3.99 (s, 1H), 2.88 (s, 1H), 2.65-2.63 (m, 3H), 2.35-2.23 (m, 1H), 2.19 (s, 3H), 2.18 (s, 3H), 1.91-1.85 (m, 4H), 1.71-1.68 (m, 1H), 1.55-1.52 (m, 1H), 1.31-1.29 (m, 1H).

Molecular formula: C₂₁H₂₇N₅O₂ Accurate molecular weight: 381.22 LC-MS(m/z): 382.35[M+H]⁺

### Example 74: Synthesis of (R)-5-ethynyl-2-(4-((tetrahydrofuran-3-yl)amino)phthalazin-1-yl)phenol (Compound 64)

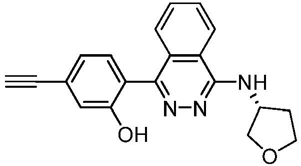

### Step 1: Synthesis of (R)-3-(ethoxymethoxy)-4-(4-((tetrahydrofuran-3-yl)amino)phthalazin-1-yl)benzaldehyde

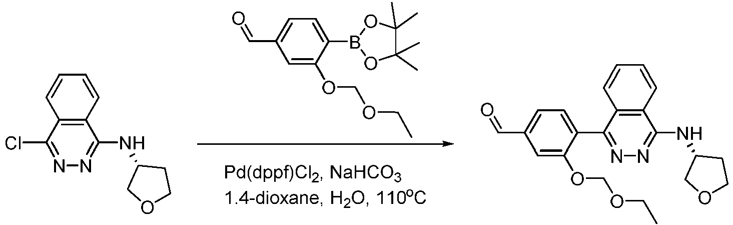

3-(ethoxymethoxy)-4-(4,4,5,5-tetramethyl-1,3,2-dioxoboracyclopentan-2-yl)benzaldehyde (642.3 mg, 2.098 mmol, 1.3 eq.), (R)-4-chloro-N-(tetrahydrofuran-3-yl)phthalazin-1-amine (403.0 mg, 1.613 mmol, 1.0 eq.), Pd(dppf)Cl₂ (59.0 mg, 0.08 mmol, 0.05 eq.), and NaHCOs (271.0 mg, 3.226 mmol, 2.0 eq.) were successively added to 1,4-dioxane (10 mL); H₂O(5 mL) was added; nitrogen replacement was conducted; and in an oil bath, the resulting mixture was heated to 110°C to allow for reaction for 4 h. The reaction mixture was cooled to room temperature, quenched by adding water, and extracted with EA; the organic phase was dried with anhydrous sodium sulfate and then filtered; the filtrate was concentrated under reduced pressure; and the resultant was separated by silica gel column chromatography (DCM:MeOH = 100:1-50:1) to obtain a product (387.8 mg, yield: 61.1%).

### Step 2: Synthesis of (R)-4-(2-(ethoxymethoxy)-4-ethynylphenyl)-N-(tetrahydrofuran-3-yl)phthalazin-1-amine

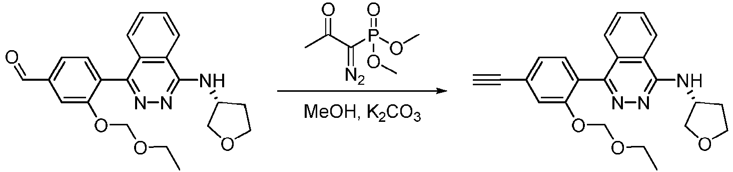

(R)-3-(ethoxymethoxy)-4-(4-((tetrahydrofuran-3-yl)amino)phthalazin-1-yl)benzaldehyde (387.8 mg, 0.985 mmol, 1.0 eq.) was dissolved in MeOH(15 mL); K₂CO₃ (272.4 mg, 1.971 mmol, 2.0 eq.) and (1-diazo-2-oxopropyl)dimethyl phosphonate (284.0 mg, 1.478 mmol, 1.5 eq.) were added; and the resulting mixture was stirred for 12 h at room temperature. The system was concentrated, and separated by silica gel column chromatography (DCM:MeOH = 80:1) to obtain a product (320.0 mg, yield:83.5%).

### Step 3: Synthesis of (R)-5-ethynyl-2-(4-((tetrahydrofuran-3-yl)amino)phthalazin-1-yl)phenol

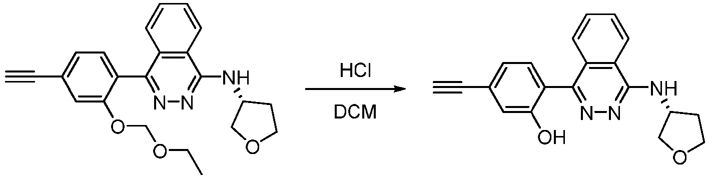

(*R*)-4-(2-(ethoxymethoxy)-4-ethynylphenyl)-*N*-(tetrahydrofuran-3-yl)phthalazin-1-amine (320.0 mg, 0.821 mmol, 1.0 eq.) was dissolved in DCM (4 mL); hydrochloric acid in a 1,4-dioxane solution (4 mol/L, 4 mL) was dropwise added; and the resulting mixture was stirred for 1 h at room temperature. The resulting mixture was adjusted to a pH value of 8-9 by using saturated NaHCOs in water, and extracted with DCM; the organic phases were dried with anhydrous sodium sulfate and filtered; the filtrate was concentrated under reduced pressure; and the resultant was separated by silica gel column chromatography (DCM:MeOH = 50:1-20:1) to obtain a product (160.0 mg, yield: 58.8%).

¹HNMR(400MHz, DMSO-*d*₆)δ(ppm): 9.97 (s, 1H), 8.44 (d, *J* = 8.2 Hz, 1H), 7.89-7.85 (m, 1H), 7.81-7.77 (m, 1H), 7.49-7.47 (m, 2H), 7.32-7.30 (m, 1H), 7.08-7.06 (m, 2H), 4.81-4.77 (m, 1H), 4.24 (s, 1H), 4.06-4.02 (m, 1H), 3.98-3.92 (m, 1H), 3.81-3.74 (m, 2H), 2.36-2.27 (m, 1H), 2.15-2.07(m, 1H).

Molecular formula: C₂₀H₁₇N₃O₂ Accurate molecular weight: 331.13 LC-MS (Pos, m/z)=332.07[M+H]⁺.

### Example 75: Synthesis of (R)-2-(6-((1-(2-hydroxyethyl)piperidin-3-yl)amino)-4-methylpyridazin-3-yl)-5-(prop-1-yn-1-yl)phenol (Compound 269)

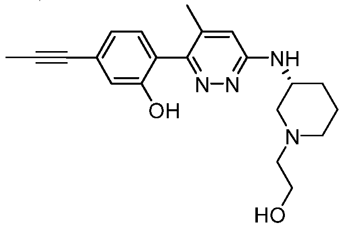

### Step 1: Synthesis of (2-(ethoxymethoxy)-4-(prop-1-yn-1-yl)phenyl)boric acid

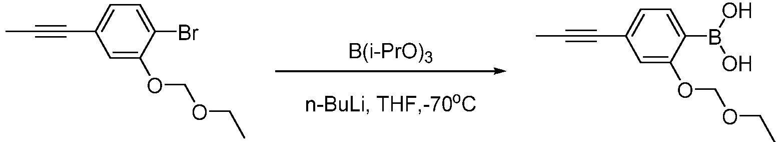

1-bromo-2-(ethoxymethoxy)-4-(prop-1-yn-1-yl)benzene (1.00 g, 3.715 mmol, 1.0 eq.) was dissolved in dry THF (20 mL); nitrogen replacement was conducted for protection; the resulting mixture was cooled to -70°C; then, n-butyl lithium (2.22 mL, 5.573 mmol, 1.5 eq.) was dropwise added to allow for reaction for 1 h; then, triisopropyl borate (1.0 g, 5.573 mmol, 1.5 eq.) was dropwise added; and the temperature was increased to room temperature to allow for reaction for 3 h. Saturated ammonium chloride in water was used for quenching; the reaction mixture was extracted with EA (50 mL); the organic phases were dried with anhydrous sodium sulfate, and then filtered; and the filtrate was concentrated under reduced pressure to obtain a product (800.0 mg, yield: 92.0%).

### Step 2: Synthesis of (R)-2-(3-((6-(2-(ethoxymethoxy)-4-(prop-1-yn-1-yl)phenyl)-5-methylpyridazin-3-yl)amino)piperidin-1 -yl)ethan-1 -ol

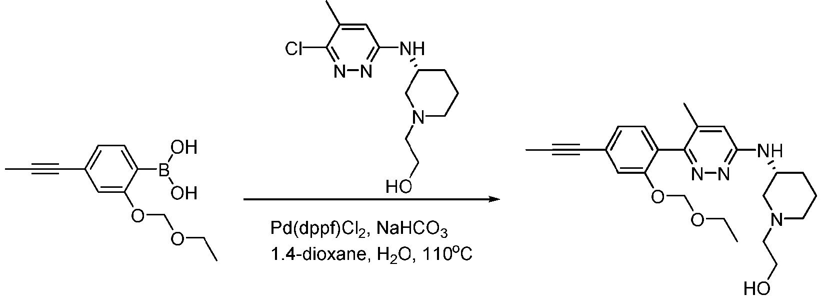

(2-(ethoxymethoxy)-4-(prop-1-yn-1-yl)phenyl)boric acid (348.3 mg, 1.488 mmol, 1.3 eq.), (R)-2-(3-((6-chloro-5-methylpyridazin-3-yl)atnino)piperidin-1-yl)ethan-1-ol (310.0 mg, 1.145 mmol, 1.0 eq.), Pd(dppf)Cl₂ (83.4 mg, 0.114 mmol, 0.1 eq.), and NaHCO₃ (192.3 mg, 2.29 mmol, 2.0 eq.) were successively added to 1,4-dioxane (10 mL); H₂O(5 mL) was added; nitrogen replacement was conducted for protection; and in an oil bath, the resulting mixture was heated to 110°C to allow for reaction for 4 h. The reaction mixture was cooled to room temperature, quenched by addition of water, and extracted with DCM; the organic phases were dried with anhydrous sodium sulfate and then filtered; and the filtrate was concentrated under reduced pressure to obtain a produce that was directly used in a next step.

### Step 3: Synthesis of (R)-2-(6-((1-(2-hydroxyethyl)piperidin-3-yl)atnino)-4-methylpyridazin-3-yl)-5-(prop-1-yn-1-yl)phenol

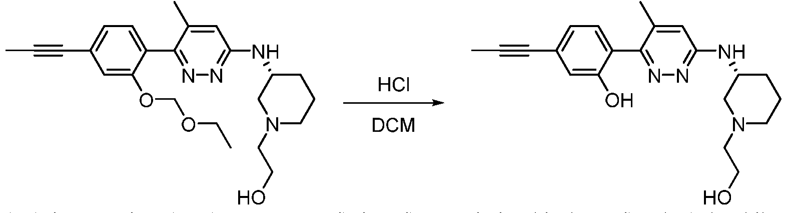

(*R*)-2-(3-((6-(2-(ethoxymethoxy)-4-(prop-1-yn-1-yl)phenyl)-5-methylpyridazin-3-yl)amino)pipendin-1-yl)ethan-1-ol (510 mg, 1.145 mmol, 1.0 eq.) was dissolved in DCM (4 mL); then, hydrochloric acid in a 1,4-dioxane solution (4 mol/L, 3 mL) was dropwise added; and the resulting mixture was stirred for 1 h at room temperature. The resulting mixture was adjusted to a pH value of 8-9 by using saturated NaHCO₃ in water, and extracted with DCM; the organic phases were dried with anhydrous sodium sulfate and filtered; the filtrate was concentrated under reduced pressure; and the resultant was separated by silica gel column chromatography (DCM:MeOH = 50:1-10:1) to obtain a product (90.0 mg, yield: 21.4%).

¹HNMR(400MHz, DMSO-*d*₆)δ(ppm): 10.01 (s, 1H), 7.13 (d, *J* = 8.1 Hz, 1H), 6.91-6.88 (m, 2H), 6.67-6.60 (m, 2H), 4.41 (s, 1H), 4.05 (s, 1H), 3.51 (d, *J* = 4.0 Hz, 2H), 2.96 (s, 1H), 2.68 (s, 1H), 2.44 (s, 2H), 2.18 (s, 1H), 2.05 (s, 3H), 2.02 (s, 3H), 1.83 (d, *J =* 8.0 Hz, 1H), 1.70 (s, 1H), 1.55-1.53 (m, 1H), 1.40-1.33 (m, 2H).

Molecular formula: C₂₁H₂₆N₄O₂ Accurate molecular weight: 366.21 LC-MS (Pos, m/z)=367.10[M+H]⁺.

Other compounds of the present invention were synthesized by referring to the steps in the foregoing embodiments.

### Experimental Example 1: Cell NLRP3 Inflammasome Inhibitory Activity Test of Compounds of the Present Invention

Compounds under test: the compounds of the present invention, which are prepared according to the methods in the embodiments.

THP-1 was an immortalized human macrophage cell line.

### Test Instrument: Microplate Reader (PE)

### Test Method:

1. THP-1 cells were cultured with a 1640 complete medium (500 ml of 1640 + 56 ml of FBS + 560 ul of 1000 × P/S + 2 ul of mercaptoethanol), and they were available within 3-20 passages.
2. Coated culture plate: 100 ul of polylysine solution was added into a 96-well cell culture plate, held for 30 min at 37 degrees Celsius, and discarded, and the plate was washed twice with PBS, and set aside.
3. Induced differentiation of THP-1: THP-1 cells were resuspended with an appropriate amount of complete medium containing 10 ng/ml PMA to a cell suspension density of 5×10⁵ cells/ml, added to the 96-well plate with 100 ul of cell suspension per well, and incubated for 16 h overnight in a carbon dioxide cell culture incubator at 37 degrees Celsius.
4. Stimulation of THP-1:
   a. A serum-free THP-1 medium containing LPS was added at a final concentration of 500 ng/ml and incubated for 3 h in a carbon dioxide cell culture incubator at 37 degrees Celsius.
   b. The compounds to be tested were prepared with DMSO into mother solutions with gradient concentrations; the mother solutions were added to cells, evenly mixed, and finally diluted at 1: 1000; and the cells were incubated for 1 h in the carbon dioxide cell culture incubator at 37 degrees Celsius.
   c. Nigericin was added to each well at a final concentration of 10 g/ml and incubated for 30 min in the carbon dioxide cell culture incubator at 37 degrees Celsius.
   d. The culture medium in each well was transferred to a new culture plate, and then centrifuged for 5 min at 3,000 rpm, and the supernatant was transferred to a new 96-well plate.
   e. The collected supernatant samples of the cell culture medium were tested for human IL-1β content by using a commercially available Elisa assay kit according to the specifications in the instructions.

The test results were shown in Table 2 below.

**Table 2 Inhibitory activity of compounds of the present invention against NLRP3 in THP-1 cells**

| Compounds under test | IC50 (nM) |
|---|---|
| Compound 1 | 0.6 |
| Compound 2 | 7 |
| Compound 3 | 5 |
| Compound 5 | 2 |
| Compound 11 | 12 |
| Compound 54 | 0.2 |
| Compound 59 | 14 |
| Compound 62 | 3 |
| Compound 63 | 10 |
| Compound 64 | 7 |
| Compound 76 | 3 |
| Compound 99 | 0.4 |
| Compound 100 | 0.5 |
| Compound 111 | 7 |
| Compound 172 | 1 |
| Compound 173 | 0.7 |
| Compound 174 | 1 |
| Compound 177 | 0.6 |
| Compound 179 | 2 |
| Compound 181 | 10 |
| Compound 182 | 3 |
| Compound 189 | 0.2 |
| Compound 195 | 1 |
| Compound 197 | 4 |
| Compound 203 | 2 |
| Compound 221 | 11 |
| Compound 222 | 7 |
| Compound 253 | 0.8 |
| Compound 254 | 1 |
| Compound 255 | 16 |
| Compound 259 | 1 |
| Compound 260 | 2 |
| Compound 261 | 0.2 |
| Compound 263 | 7 |
| Compound 264 | 1 |
| Compound 267 | 9 |
| Compound 268 | 0.2 |
| Compound 270 | 2 |

As can be seen from the experimental results in Table 2, the compounds of the present invention show good inhibitory activity against the NLRP3 inflammasomes. Therefore, the compounds of the present invention can be used to prevent and/or treat NLRP3 inflammasome-related diseases.

### Experimental Example 2: Pharmacokinetic Study of Compounds in C57BL/6J Mice

Compounds under test: the compounds of the present invention, which are prepared according to the methods in the embodiments.

### Animal Administration and Sample Collection:

Compound 100, Compound 54, Compound 174, Compound 179, and Compound 253 were prepared into solutions with a mixed solvent formulated from 5% dimethyl sulfoxide, 20% (30% solutol) and 75% normal saline, respectively; the solution of each compound was administered to C57BL/6J female mice by gavage at a dose of 10 mg/kg; and the time points for blood collection were: 15 min, 30 min, 1 h, 2 h, 4 h, 6 h, 8 h and 24 h. The solution of the compound was administered to C57BL/6J female mice by intravenous bolus at a dose of 5 mg/kg, and the time points for blood collection were as follows: 5 min, 15 min, 30 min, 1 h, 2 h, 4 h, 6 h, 8 h, and 24 h.

Compound 1 was prepared into a solution with a mixed solvent formulated from 5% DMF, 20% (30% solutol) and 75% normal saline; the solution of this compound was administered to C57BL/6J female mice by gavage at a dose of 10 mg/kg; and the time points for blood collection were: 15 min, 30 min, 1 h, 2 h, 4 h, 6 h, 8 h, and 24 h. The solution of the compound was administered to C57BL/6J female mice by intravenous bolus at a dose of 5 mg/kg, and the time points for blood collection were as follows: 5 min, 15 min, 30 min, 1 h, 2 h, 4 h, 6 h, 8 h, and 24 h.

Compound 195 was prepared into a solution with a mixed solvent formulated from 5% dimethyl sulfoxide, 20% PEG400 and 75% normal saline; the solution of this compound was administered to C57BL/6J female mice by gavage at a dose of 10 mg/kg; and the time points for blood collection were 15 min, 30 min, 1 h, 2 h, 4 h, 6 h, 8 h, and 24 h. The solution of the compound was administered to C57BL/6J female mice by intravenous bolus at a dose of 5 mg/kg, and the time points for blood collection were as follows: 5 min, 15 min, 30 min, 1 h, 2 h, 4 h, 6 h, 8 h, and 24 h.

Approximately 100 µL of blood was collected through the retroorbital venous plexus, and was then placed in an anticoagulant tube containing EDTA-K2. Blood samples were centrifuged at 12,000 rpm for 10 min at 4°C to obtain plasma samples, and plasma was prepared within 30 min after blood collection. The plasma was stored in a -80°C freezer before testing.

### Sample Analysis Method:

1. The samples were taken from the -80°C freezer, thawed naturally at room temperature, and then vortexed for 5 min.
2. 10 µL of a plasma sample was precisely pipetted from different individuals into a 1.5 mL centrifuge tube.
3. 300 µL of an internal standard working solution (the acetonitrile solution of tolbutamide) at a concentration of 100 ng/mL was added and mixed well.
4. After vortexing for 5 min, the mixture was centrifuged at 12,000 rpm for 5 min.
5. 50 µL of the supernatant was accurately pipetted into a 96-well plate preloaded with water at 150 µL/well.
6. Vortexing was performed for 5 min for sufficient mixing, and LC-MS/MS analysis was then conducted.

### Data Processing Method:

The results of the concentrations of the compounds under test were output using Analyst 1.6.3 of AB. Parameters such as mean, standard deviation, coefficient of variation was calculated by Microsoft Excel (parameters directly output by Analyst 1.6.3 needed no calculation), and PK parameters were calculated by WinNonlin Phenoix 8.2 software NCA (Tₘₐₓ as the median).

### Results

| Compound | Dose iv/po (mg/kg) | t_{z1/2} iv/po (h) | Vₛₛ iv (L/kg) | Cl_{_obs} iv (L/h/kg) | Tₘₐₓ po (h) | Cₘₐₓ po (ng/mL) | AUCₗₐₛₜ iv/po (h*ng/mL) | F% |
|---|---|---|---|---|---|---|---|---|
| Compound 100 | 5/10 | 3.04/2.76 | 3.16 | 0.783 | 2.00 | 1530 | 6365/10112 | 79.4 |
| Compound 1 | 5/10 | 3.85/3.00 | 5.22 | 2.64 | 0.250 | 428 | 1819/1887 | 49.9 |
| Compound 54 | 5/10 | 2.97/3.04 | 6.59 | 1.83 | 1.00 | 820 | 2395/4126 | 75.6 |
| Compound 174 | 5/10 | 3.95/3.82 | 2.64 | 0.574 | 0.500 | 3160 | 8613/20057 | 116 |
| Compound 179 | 5/10 | 2.01/2.19 | 5.14 | 1.97 | 0.500 | 1120 | 2474/3858 | 79.2 |
| Compound 195 | 5/10 | 3.89/3.12 | 5.14 | 1.39 | 1.00 | 1230 | 3577/7858 | 110 |
| Compound 253 | 5/10 | 1.50/3.49 | 2.76 | 1.53 | 0.500 | 2320 | 3202/6902 | 106 |

| | | | | | | | | |
|---|---|---|---|---|---|---|---|---|
| **Note:** t_{z1/2}: terminal elimination half-life; Cl_{_obs}: clearance rate; Vₛₛ: volume of distribution; Tₘₐₓ: peak time of plasma concentration; Cₘₐₓ: peak concentration; AUCₗₐₛₜ: area under the drug-time curve for 0-24 h; and F%: absolute bioavailability. Conclusion: The compounds of the present invention have good pharmacokinetic properties in the C57BL/6J mice. | | | | | | | | |

### Experimental Example 3: Pharmacokinetic Study of Compounds in SD Male Rats

Compounds under test: the compounds of the present invention, which are prepared according to the methods in the embodiments.

### Animal Administration and Sample Collection:

Compound 54, Compound 55, Compound 174, Compound 173, and Compound 264 were prepared into solutions with a mixed solvent formulated from 5% dimethyl sulfoxide, 20% (30% solutol) and 75% normal saline, respectively; the solution of each compound was administered to SD male rats by gavage at a dose of 5.0 mg/kg; and the time points for blood collection were: 15 min, 30 min, 1 h, 2 h, 4 h, 6 h, 8 h, 10 h and 24 h. The solution of the compound was administered to male SD rats by intravenous bolus at a dose of 1.0 mg/kg, and the time points for blood collection were as follows: 5 min, 15 min, 30 min, 1 h, 2 h, 4 h, 6 h, 8 h, 10 h, and 24 h.

On the day before administration, the jugular vein of each animal was cannulated; and after administration, about 300 µL of blood was collected through the jugular vein, and then placed in an anticoagulant tube containing EDTA-K₂. Blood samples were centrifuged at 12,000 rpm for 10 min at 4°C to obtain plasma samples, and plasma was prepared within 30 min after blood collection. The plasma was stored in a -80°C freezer before testing.

### Sample Analysis Method:

1. The samples were taken from the -80°C freezer, thawed naturally at room temperature, and then vortexed for 5 min.
2. 30 µL of a plasma sample was precisely pipetted into a 1.5 mL centrifuge tube.
3. 750 µL of an internal standard working solution (the acetonitrile solution of tolbutamide) at a concentration of 100 ng/mL was added and mixed well.
4. After vortexing for 5 min, the mixture was centrifuged at 12,000 rpm for 5 min.
5. 50 µL of the supernatant was accurately pipetted into a 96-well plate preloaded with water at 150 µL/well.
6. Vortexing was performed for 5 min for sufficient mixing, and LC-MS/MS analysis was then conducted.

### Data Processing Method:

The results of the concentrations of the compounds under test were output using Analyst 1.6.3 of AB. Parameters such as mean, standard deviation, coefficient of variation was calculated by Microsoft Excel (parameters directly output by Analyst 1.6.3 needed no calculation), and PK parameters were calculated by WinNonlin Phenoix 8.2 software NCA (Tₘₐₓ as the median).

### Results

| Compound | Dose iv/po (mg/kg) | t_{z1/2} iv/po (h) | Vₛₛ (L/kg) iv | Cl_{_obs} iv (L/h/kg) | Tₘₐₓ po (h) | Cₘₐₓ po (ng/mL) | AUCₗₐₛₜ iv/po (h*ng/mL) | F% |
|---|---|---|---|---|---|---|---|---|
| Compound 54 | 1/5 | 2.97/3.94 | 5.01 | 1.21 | 1.00 | 442 | 749/3791 | 93.1 |
| Compound 55 | 1/5 | 5.24/7.18 | 1.12 | 0.190 | 2.00 | 3047 | 5133/38545 | 163 |
| Compound 174 | 1/5 | 3.60/3.51 | 4.82 | 1.09 | 2.00 | 465 | 813/4433 | 97.0 |
| Compound 173 | 1/5 | 6.13/5.72 | 6.92 | 0.856 | 4.00 | 395 | 1097/4692 | 85.6 |
| Compound 264 | 1/5 | 8.89/7.26 | 12.5 | 1.05 | 4.00 | 230 | 811/3118 | 72.8 |

| | | | | | | | | |
|---|---|---|---|---|---|---|---|---|
| **Note:** t_{z1/2}: terminal elimination half-life; Cl_{_obs}: clearance rate; Vₛₛ: volume of distribution; Tₘₐₓ: peak time of plasma concentration; Cₘₐₓ: peak concentration; AUCₗₐₛₜ: area under the drug-time curve for 0-24 h; and F%: absolute bioavailability. Conclusion: The compounds of the present invention have good pharmacokinetic properties in the SD male rats. | | | | | | | | |

### Experimental Example 4: Pharmacokinetic Study of Compounds in Beagles

Compounds under test: the compounds of the present invention, which are prepared according to the methods in the embodiments.

### Animal Administration and Sample Collection:

Compound 174 was prepared into a solution with a mixed solvent formulated from 5% dimethyl sulfoxide, 20% (30% solutol) and 75% normal saline; the solution of this compound was administered to male beagles by gavage at a dose of 1.0 mg/kg; and the time points for blood collection were: 15 min, 30 min, 1 h, 2 h, 4 h, 6 h, 8 h, 10 h and 24 h. The solution of the compound was administered to male beagles by intravenous bolus at a dose of 1.0 mg/kg, and the time points for blood collection were as follows: 5 min, 15 min, 30 min, 1 h, 2 h, 4 h, 6 h, 8 h, 10 h, and 24 h.

Compound 177 and Compound 195 were prepared into solutions with a mixed solvent formulated from 5% dimethyl sulfoxide, 20% (30% solutol) and 75% normal saline, respectively; the solutions of these compounds were administered to male beagles by gavage at a dose of 1.0 mg/kg; and the time points for blood collection were: 15 min, 30 min, 1 h, 2 h, 4 h, 6 h, 8 h, 10 h and 24 h. The solution of the compound was administered to male beagles by intravenous bolus at a dose of 1.0 mg/kg, and the time points for blood collection were as follows: 5 min, 15 min, 30 min, 1 h, 2 h, 4 h, 6 h, 8 h, 10 h, and 24 h.

Approximately 400 µL of blood was collected through the medial cephalic vein of the forelimb and placed in an anticoagulant tube containing EDTA-K₂. Blood samples were centrifuged at 12,000 rpm for 10 min at 4°C to obtain plasma samples, and plasma was prepared within 30 min after blood collection. The plasma was stored in a -80°C freezer before testing.

### Results

| Compound | Dose iv/po (mg/kg) | t_{z1/2} iv/po (h) | Vₛₛ iv (L/kg) | Cl_{_obs} iv (L/h/kg) | Tₘₐₓ po (h) | Cₘₐₓ po (ng/mL) | AUCₗₐₛₜ iv/po (h*ng/mL) | F% |
|---|---|---|---|---|---|---|---|---|
| Compound 174 | 1/1 | 15.8/14.7 | 7.39 | 0.332 | 4.00 | 105 | 1981/1660 | 83.8 |
| Compound 177 | 1/1 | 5.58/5.90 | 5.53 | 0.780 | 1.00 | 293 | 1245/1918 | 154 |
| Compound 195 | 1/1 | 8.16/11.0 | 3.74 | 0.395 | 4.00 | 206 | 2321/1901 | 90.7 |

| | | | | | | | | |
|---|---|---|---|---|---|---|---|---|
| **Note:** t_{z1/2}: terminal elimination half-life; Cl_{_obs}: clearance rate; Vₛₛ: volume of distribution; Tₘₐₓ: peak time of plasma concentration; Cₘₐₓ: peak concentration; AUCₗₐₛₜ: area under the drug-time curve for 0-24 h; and F%: absolute bioavailability. Conclusion: The compounds of the present invention have good pharmacokinetic properties in the male beagles. | | | | | | | | |

### Experimental Example 5: Test for Inhibition of hERG Potassium Ion Channels by Compound

Experimental Objective: To study the inhibitory effect of the compound on hERG potassium ion channels by a manual patch-clamp technique.

### Experimental Method

### 1. Cell Culture

1.1 The HEK-293 cell line stably expressing the hERG potassium channels were cultured in a DMEM medium containing 10% fetal bovine serum and 0.8 mg/mL G418 at 37°C and 5% carbon dioxide.
1.2 In order to maintain the electrophysiological activity of the cells, the cell density should not exceed 80%.
1.3 Before the patch-clamp assay, the cells were isolated by TrypLE^{™} Express, and 4×10³ cells were plated onto a coverslip, cultured in a 24-well plate (final volume: 500 µL) and were assayed after 18 hours.

### 2. Preparation method for dosing preparation stock

2.1 Blank control: DMSO stock.
2.2 Positive control: 10 mM stock formulated from a appropriate amount of Cisapride and dimethyl sulfoxide (DMSO). The stock was aliquoted and stored at -20°C.
2.3 Compound: An appropriate amount of compound was weighed; the volume of DMSO required was calculated according to the formula: DMSO volume = actual amount × purity (content) / (molecular weight × theoretical concentration), and the corresponding volume of DMSO was pipetted; then, the weighed compound was dissolved with the pipetted DMSO, and the mass of DMSO was measured at the same time; and finally, the actual stock concentration was calculated according to the final amount of DMSO used. In general, the actual stock concentration was slightly different from the theoretical concentration.

### 3. Preparation method for working solution of dosing preparation

Before the hERG channel current test, the blank control stock was diluted into an appropriate amount of extracellular fluid as a working solution. The stocks of the positive control and the compound as stored at -20°C were taken out, and each diluted into an appropriate amount of extracellular fluid as a working solution.

The highest detection concentration of the compound was achieved by diluting the stock directly using the extracellular fluid, or the stock needed to be further diluted with DMSO. For other concentrations, the diluted solutions were achieved by successive dilution from high concentration to low concentration with DMSO, and then diluted to the working solution concentration with the extracellular fluid. The concentration of DMSO in each working solution was 0.3%. Before the patch-clamp test, the working solution of the compound was ultrasonically treated for 20 min.

The blank control stock (DMSO) was stored at room temperature, and the blank control working solution was prepared on the day of testing and stored at room temperature. The stocks for the compound and the positive control were stored at -20°C, and the working solutions for the compound and the positive control were prepared on the day of testing and stored at room temperature.

### 4. Basis for Concentration Selection

The default detection concentrations for the compound were 30, 10, 3, 1, and 0.3 µM. The blank control is 0.3% DMSO. The detection concentrations for the positive control (Cisapride) were: 1000, 100, 10, and 1 nM.

### 5. Electrophysiological Recording

### 5.1 Recording of liquids used

### 5.1.1 Extracellular fluid: K-007-1

140 mM NaCl, 3.5 mM KCl, 1 mM MgCl2•6H₂O, 2 mM CaCl₂•2H₂O, 10 mM D-Glucose, 10 mM HEPES, 1.25 mM NaH₂PO₄•2H₂O, NaOH for adjustment to pH = 7.4.

### 5.1.2 Intracellular fluid: K-002-2

20 mM KCl, 115 mM K-Aspartic, 1 mM MgCl₂•6H₂O, 5 mM EGTA, 10 mM HEPES, 2 mM Na₂-ATP, KOH for adjustment to pH = 7.2.

The shelf life of the extracellular fluid was 2 weeks; and the intracellular fluid prepared was aliquoted as 1 mL per tube, frozen in a -20°C freezer, and the freshly thawed intracellular fluid was used for daily experiment. All intracellular fluids were used up within three months. The intracellular fluid beyond three months were discarded and prepared again.

### 5.2 Patch-clamp Testing

The voltage stimulation protocol for whole-cell patch-clamp recording of hERG current was as follows: the cell membrane voltage was clamped to -80 mV when whole-cell closure was formed. The clamping voltage was depolarized from -80 mV to -50 mV for 0.5 s (for leakage current detection), then stepped to 30 mV for 2.5 s, and then quickly returned to -50 mV for 4 s, such that the tail current of the hERG channel could be excited. The data were collected repeatedly every 10 s to observe the effect of the compound on the hERG tail current. The stimulation at -50 mV for 0.5 s was used for leakage current detection. The test data were collected by an EPC 10 amplifier (HEKA) and stored in the PatchMaster (HEKA) software.

The capillary glass tube was pulled into a recording electrode with a microelectrode puller. The electrode filled with the intracellular fluid was installed into the electrode head, and the microelectrode manipulator is manipulated under an inverted microscope to immerse the electrode in the extracellular fluid and record the electrode resistance (Rpip). The electrode was allowed to contact with the cell surface, and negative pressure suction was applied to form high-resistance seal (GQ). At this point, fast capacitance compensation was performed, and then negative pressure was continued to be applied to suck and break through the cell membrane to form a whole-cell recording pattern. Then, slow capacitance compensation was performed, and the experimental parameters such as membrane capacitance (Cm) and series resistance (Rs) were recorded. No leakage compensation was given.

Dosing is initiated when the hERG current in the whole-cell recording was stable; each compound concentration was detected for 5 min (or till the current became stable), and then the next concentration was detected; and multiple concentrations were detected for each compound under test. The cell-lined coverslip was placed in a recording bath under the inverted microscope; the extracellular fluid for the blank control and the working solution for the compound to be tested were allowed to successively flow through the recording bath from low to high concentration by gravity perfusion to act on the cells; and a peristaltic pump was used for liquid exchange during recording. For each cell, the current detected in the compound-free extracellular fluid was taken as its own control group. For each concentration, independent testing was conducted three times by using at least three cells. All the electrophysiology tests were performed at room temperature.

### 6. Data analysis

IC₅₀ calculation and curve fitting were performed using GraphPad Prism software.

### 7. Test Results

The IC50 value of the compound for the hERG potassium ion channel was shown in the table below.

| Compound | hERG IC50 (µM) |
|---|---|
| Compound 54 | >10 µM |

| | |
|---|---|
| Conclusion: The compound of the present invention has no significant inhibitory effect on the hERG potassium ion channel. | |

The above description only provides the preferred embodiments of the present invention, and is not intended to limit the present invention. Any modification, equivalent substitution, improvement or others made within the spirit and principle of the present invention shall be included within the protection scope of the present invention.

## Claims

1. A compound of general formula (A) or a pharmacologically acceptable salt, stereoisomer or tautomer thereof:
Y-W-R₃ (A)
wherein, W is selected from 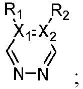
X₁ and X₂ are each independently selected from C or N;
R₁ is selected from hydrogen, hydroxyl, amino, carboxyl, cyano, nitro, halogen, carbonyl, C₁₋₆ alkyl, halo C₁₋₆alkyl, C₁₋₆ alkoxy, halo C₁₋₆ alkoxy, C₂₋₆ alkenyl, C₂₋₆ alkynyl, 3-7 membered heterocyclyl, 3-7 membered cycloalkyl, aryl, 5-7 membered heteroaryl, -N(C₁₋₆ alkyl)₂, -S-C₁₋₆ alkyl, or absent;
R₂ is selected from hydrogen, hydroxyl, amino, carboxyl, cyano, nitro, halogen, carbonyl, C₁₋₆ alkyl, halo C₁₋₆alkyl, C₁₋₆ alkoxy, C₁₋₆ haloalkoxy, C₂₋₆ alkenyl, C₂₋₆ alkynyl, 3-7 membered heterocyclyl, 3-7 membered cycloalkyl, aryl, 5-7 membered heteroaryl, -N(C₁₋₆ alkyl)₂, -S-C₁₋₆ alkyl, or absent;
R₁ and R₂ are each optionally substituted by 1-3 substituents selected from hydroxyl, amino, carboxyl, cyano, nitro, halogen, carbonyl, C₁₋₆ alkyl, C₁₋₆ haloalkyl, C₁₋₆ alkoxy, C₁₋₆ haloalkoxy, 3-7 membered heterocyclyl, 3-7 membered cycloalkyl, aryl, and 5-7 membered heteroaryl; or
R₁ and R₂, together with the carbon or nitrogen atom to which they are attached, form a 5-12 membered ring A, the 5-12 membered ring A is optionally substituted by 1-4 substituents selected from the group consisting of hydroxyl, amino, carboxyl, cyano, nitro, halogen, carbonyl, C₁₋₆ alkyl, -NH-C₁₋₆ alkyl, C₁₋₆ haloalkyl, C₁₋₆ alkoxy, C₁₋₆ haloalkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂;
R₃ is selected from the group consisting of -(C₁₋₆ alkylene)₀₋₂-NR₄R₅, -(C₁₋₆ alkylene)₀₋₂-NR₄-COR₅, -(C₁₋₆ alkylene)₀₋₂-CO-NR₄-R₅, and -(C₁₋₆ alkylene)₀₋₂-NR₄-C₁₋₆ alkylene-R₅;
R₄ is selected from hydrogen or C₁₋₆ alkyl;
R₅ is selected from the group consisting of 3-7 membered heterocyclyl, 3-7 membered cycloalkyl, aryl, and 5-7 membered heteroaryl; R₅ is optionally substituted by 1-4 substituents selected from the group consisting of halogen, cyano, amino, hydroxyl, C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, C₂₋₆ alkenylcarbonyl, sulfonyl, and C₁₋₆ alkylcarbonyl;
when R₅ is substituted,
the substituent on R₅, which is C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from the group consisting of halogen, cyano, amino, hydroxy, carbonyl, C₁₋₆ alkyl, 3-6 membered cycloalkyl, 3-6 membered heterocyclyl, carboxyl, and C₁₋₆ alkylsulfonyl;
Y is selected from the group consisting of aryl, 5-14 membered heteroaryl, 3-14 membered heterocyclyl, and 3-12 membered cycloalkyl;
(1) when X₁ and X₂ are each selected from C,
Y is substituted by C₂₋₆ alkenyl or C₂₋₆ alkynyl, and is optionally substituted by 1-2 substituents selected from the group consisting of halogen, cyano, amino, hydroxy, carbonyl, C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, and sulfonyl;
(2) when either one or two of X₁ and X₂ is/are selected from N,
Y is optionally substituted by 1-3 substituents selected from the group consisting of C₂₋₆ alkenyl, C₂₋₆ alkynyl, halogen, cyano, amino, hydroxy, carbonyl, C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, sulfonyl, -N(C₁₋₆ alkyl)₂, and -S-C₁₋₆ alkyl;
in (1) and (2) above, when Y is substituted,
the substituent on Y, which is C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from the group consisting of halogen, cyano, amino, hydroxy, carbonyl, C₁₋₆ alkyl, and 3-6 membered cycloalkyl; or
the substituent on Y, which is C₂₋₆ alkenyl or C₂₋₆ alkynyl, is optionally substituted by 1-3 substituents selected from the group consisting of halogen, cyano, amino, hydroxy, carbonyl, unsubstituted or hydroxy-substituted C₁₋₆ alkyl, unsubstituted or hydroxy-substituted 3-6 membered cycloalkyl, and C₁₋₆ haloalkyl.

2. The compound or the pharmaceutically acceptable salt, stereoisomer or tautomer thereof according to claim 1, which has a structure of general formula (I): 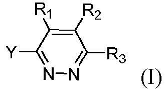 wherein,
R₁ is selected from the group consisting of hydrogen, hydroxy, amino, carboxyl, cyano, nitro, halogen, C₁₋₆ alkyl, C₁₋₆ haloalkyl, C₁₋₆ alkoxy, C₁₋₆ haloalkoxy, C₂₋₆ alkenyl, C₂₋₆ alkynyl, 3-7 membered heterocyclyl, 3-7 membered cycloalkyl, aryl, and 5-7 membered heteroaryl;
R₂ is selected from the group consisting of hydrogen, hydroxy, amino, carboxyl, cyano, nitro, halogen, C₁₋₆ alkyl, C₁₋₆ haloalkyl, C₁₋₆ alkoxy, C₁₋₆ haloalkoxy, C₂₋₆ alkenyl, C₂₋₆ alkynyl, 3-7 membered heterocyclyl, 3-7 membered cycloalkyl, aryl, and 5-7 membered heteroaryl;
R₁ and R₂ are each optionally substituted by 1-3 substituents selected from the group consisting of hydroxy, amino, carboxyl, cyano, nitro, halogen, carbonyl, C₁₋₆ alkyl, haloC₁₋₆ alkyl, C₁₋₆ alkoxy, C₁₋₆ haloalkoxy, 3-7 membered heterocyclyl, 3-7 membered cycloalkyl, aryl, and 5-7 membered heteroaryl;
or
R₁ and R₂, together with a carbon atom attached thereto, form a 5-12 membered ring A; the 5-12 membered ring A is optionally substituted by 1-4 substituents selected from the group consisting of hydroxy, amino, carboxyl, cyano, nitro, halogen, carbonyl, C₁₋₆ alkyl, -NH-C₁₋₆ alkyl, C₁₋₆ haloalkyl, C₁₋₆ alkoxy, C₁₋₆ haloalkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂;
R₃ is selected from the group consisting of -(C₁₋₆ alkylene)₀₋₂-NR₄R₅, -(C₁₋₆ alkylene)₀₋₂-NR₄-COR₅, -(C₁₋₆ alkylene)₀₋₂-CO-NR₄-R₅, and -(C₁₋₆ alkylene)₀₋₂-NR₄-C₁₋₆ alkylene-R₅;
R₄ is selected from hydrogen or C₁₋₆ alkyl;
R₅ is selected from the group consisting of 3-7 membered heterocyclyl, 3-7 membered cycloalkyl, aryl, and 5-7 membered heteroaryl; R₅is optionally substituted by 1-4 substituents selected from the group consisting of halogen, cyano, amino, hydroxy, C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, C₂₋₆ alkenylcarbonyl, sulfonyl, and C₁₋₆ alkylcarbonyl;
Y is selected from the group consisting of aryl, 5-14 membered heteroaryl, 3-14 membered heterocyclyl, and 3-12 membered cycloalkyl, and Y is substituted by C₂₋₆ alkenyl or C₂₋₆ alkynyl, and is optionally substituted by 1-2 substituents selected from the group consisting of halogen, cyano, amino, hydroxy, carbonyl, C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, and sulfonyl; when R₅ is substituted,
the substituent on R₅, which is C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from the group consisting of halogen, cyano, amino, hydroxy, carbonyl, C₁₋₆ alkyl, 3-6 membered cycloalkyl, and C₁₋₆ alkylsulfonyl;
when Y is substituted,
the substituent on Y, which is C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from the group consisting of halogen, cyano, amino, hydroxy, carbonyl, C₁₋₆ alkyl, and 3-6 membered cycloalkyl; or
the substituent on Y, which is C₂₋₆ alkenyl or C₂₋₆ alkynyl, is optionally substituted by 1-3 substituents selected from the group consisting of halogen, cyano, amino, hydroxy, carbonyl, C₁₋₆ alkyl, 3-6 membered cycloalkyl, and C₁₋₆ haloalkyl.

3. The compound of general formula (2) or the pharmaceutically acceptable salt, stereoisomer or tautomer thereof according to claim 2, 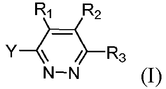 wherein,
R₁ is selected from the group consisting of hydrogen, hydroxy, amino, carboxyl, cyano, nitro, halogen, C₁₋₆ alkyl, C₁₋₆ haloalkyl, C₁₋₆ alkoxy, C₁₋₆ haloalkoxy, C₂₋₆ alkenyl, C₂₋₆ alkynyl, 3-7 membered heterocyclyl, 3-7 membered cycloalkyl, aryl, and 5-7 membered heteroaryl;
R₂ is selected from the group consisting of hydrogen, hydroxy, amino, carboxyl, cyano, nitro, halogen, C₁₋₆ alkyl, C₁₋₆ haloalkyl, C₁₋₆ alkoxy, C₁₋₆ haloalkoxy, C₂₋₆ alkenyl, C₂₋₆ alkynyl, 3-7 membered heterocyclyl, 3-7 membered cycloalkyl, aryl, and 5-7 membered heteroaryl;
R₁ and R₂ are each optionally substituted by 1-3 substituents selected from the group consisting of hydroxy, amino, carboxyl, cyano, nitro, halogen, carbonyl, C₁₋₆ alkyl, C₁₋₆ haloalkyl, C₁₋₆ alkoxy, C₁₋₆ haloalkoxy, 3-7 membered heterocyclyl, 3-7 membered cycloalkyl, aryl, and 5-7 membered heteroaryl;
or
R₁ and R₂, together with a carbon atom attached thereto, form a 5-12 membered ring A; the 5-12 membered ring A is optionally substituted by 1-4 substituents selected from the group consisting of hydroxy, amino, carboxyl, cyano, nitro, halogen, carbonyl, C₁₋₆ alkyl, -NH-C₁₋₆ alkyl, haloC₁₋₆ alkyl, C₁₋₆ alkoxy, C₁₋₆ haloalkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂;
R₃ is selected from the group consisting of -(C₁₋₆ alkylene)₀₋₂-NR₄R₅, -(C₁₋₆ alkylene)₀₋₂-NR₄-COR₅, and -(C₁₋₆ alkylene)₀₋₂-CO-NR₄-R₅;
R₄ is selected from hydrogen or C₁₋₆ alkyl;
R₅ is selected from the group consisting of 3-7 membered heterocyclyl, 3-7 membered cycloalkyl, aryl, and 5-7 membered heteroaryl; R₅ is optionally substituted by 1-4 substituents selected from the group consisting of halogen, cyano, amino, hydroxy, C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, C₂₋₆ alkenylcarbonyl, sulfonyl, and C₁₋₆ alkylcarbonyl;
Y is selected from the group consisting of aryl, 5-14 membered heteroaryl, 3-14 membered heterocyclyl, and 3-12 membered cycloalkyl, and Y is substituted by C₂₋₆ alkenyl or C₂₋₆ alkynyl, and is optionally substituted by 1-2 substituents selected from the group consisting of halogen, cyano, amino, hydroxy, carbonyl, C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, C₁₋₆ alkylamino, C₁₋₆ alkylcarbonylamino, C₁₋₆ alkylsulfonyl, aminocarbonyl, C₁₋₆ alkylaminocarbonyl, and sulfonyl; when R₅ is substituted,
the substituent on R₅, which is C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from the group consisting of halogen, cyano, amino, hydroxy, carbonyl, C₁₋₆ alkyl, and 3-6 membered cycloalkyl; when Y is substituted,
the substituent on Y, which is C₁₋₆ alkyl, C₁₋₆ haloalkyl, C₁₋₆ alkoxy, 3-7 membered heterocyclyl, 3-7 membered cycloalkyl, aryl, 5-7 membered heteroaryl, or sulfonyl, is optionally substituted by 1-3 substituents selected from the group consisting of halogen, cyano, amino, hydroxy, carbonyl, C₁₋₆ alkyl, and 3-6 membered cycloalkyl; or the substituent on Y, which is C₂₋₆ alkenyl or C₂₋₆ alkynyl, is not substituted.

4. The compound or the pharmaceutically acceptable salt, stereoisomer or tautomer thereof according to claim 1, which has a structure of general formula (B): 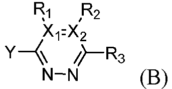
R₁ is selected from hydrogen, hydroxy, amino, carboxyl, cyano, nitro, halogen, carbonyl, C₁₋₆ alkyl, C₁₋₆ haloalkyl, C₁₋₆ alkoxy, C₁₋₆ haloalkoxy, C₂₋₆ alkenyl, C₂₋₆ alkynyl, 3-7 membered heterocyclyl, 3-7 membered cycloalkyl, aryl, 5-7 membered heteroaryl, -N(C₁₋₆ alkyl)₂, -S-C₁₋₆ alkyl, or absent;
R₂ is selected from hydrogen, hydroxy, amino, carboxyl, cyano, nitro, halogen, carbonyl, C₁₋₆ alkyl, C₁₋₆ haloalkyl, C₁₋₆ alkoxy, C₁₋₆ haloalkoxy, C₂₋₆ alkenyl, C₂₋₆ alkynyl, 3-7 membered heterocyclyl, 3-7 membered cycloalkyl, aryl, 5-7 membered heteroaryl, -N(C₁₋₆ alkyl)₂, -S-C₁₋₆ alkyl, or absent;
R₁ and R₂ are each optionally substituted by 1-3 substituents selected from the group consisting of hydroxy, amino, carboxyl, cyano, nitro, halogen, carbonyl, C₁₋₆ alkyl, C₁₋₆ haloalkyl, C₁₋₆ alkoxy, C₁₋₆ haloalkoxy, 3-7 membered heterocyclyl, 3-7 membered cycloalkyl, aryl, and 5-7 membered heteroaryl;
either one or two of X₁ and X₂ is/are selected from N; and
Y is substituted by hydroxy and is optionally substituted by 1-2 substituents selected from the group consisting of C₂₋₆ alkenyl, C₂₋₆ alkynyl, halogen, cyano, amino, hydroxy, carbonyl, C₁₋₆ alkyl, C₁₋₆ haloalkyl, C₁₋₆ alkoxy, 3-7 membered heterocyclyl, 3-7 membered cycloalkyl, C₁₋₆ alkylamino, C₁₋₆ alkylcarbonylamino, C₁₋₆ alkylsulfonyl, aminocarbonyl, C₁₋₆ alkylaminocarbonyl, sulfonyl, -N(C₁₋₆ alkyl)₂, and -S-C₁₋₆ alkyl.

5. The compound or the pharmaceutically acceptable salt, stereoisomer or tautomer thereof according to any one of claims 1-3, which has a structure of general formula (II): 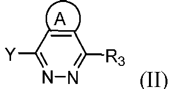 wherein,
the ring A is selected from the group consisting of 5-7 membered cycloalkenyl, 5-7 membered cycloalkyl, 5-7 membered heterocyclyl, phenyl, and 5-7 membered heteroaryl; and the ring A is optionally substituted by 1-4 substituents selected from the group consisting of hydroxy, amino, carboxyl, cyano, nitro, halogen, C₁₋₆ alkyl, -NH-C₁₋₆ alkyl, C₁₋₆ haloalkyl, C₁₋₆ alkoxy, C₁₋₆ haloalkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂.

6. The compound or the pharmaceutically acceptable salt, stereoisomer or tautomer thereof according to claim 5,
wherein,
the ring A is selected from the group consisting of phenyl and 5-7 membered heteroaryl; and the ring A is optionally substituted by 1-4 substituents selected from the group consisting of hydroxy, amino, carboxyl, cyano, nitro, halogen, C₁₋₆ alkyl, -NH-C₁₋₆ alkyl, C₁₋₆ haloalkyl, C₁₋₆ alkoxy, C₁₋₆ haloalkoxy, 3-7 membered heterocyclyl, 3-7 membered cycloalkyl, C₁₋₆ alkylsulfonyl, and -N(C₁₋₆ alkyl)₂.

7. The compound or the pharmaceutically acceptable salt, stereoisomer or tautomer thereof according to any one of claims 1-3,
wherein,
Y is selected from the group consisting of phenyl, 5-7 membered heteroaryl, 3-8 membered heterocyclyl, and 3-7 membered cycloalkyl; Y is substituted by C₂₋₆ alkenyl or C₂₋₆ alkynyl, and is optionally substituted by 1-2 substituents selected from the group consisting of halogen, cyano, amino, hydroxy, carbonyl, C₁₋₆ alkyl, C₁₋₆ haloalkyl, C₁₋₆ alkoxy, 3-7 membered heterocyclyl, 3-7 membered cycloalkyl, C₁₋₆ alkylamino, C₁₋₆ alkylcarbonylamino, C₁₋₆ alkylsulfonyl, aminocarbonyl, C₁₋₆ alkylaminocarbonyl, and sulfonyl; and
when Y is substituted, the substitute on Y, which is C₁₋₆ alkyl, C₁₋₆ haloalkyl, C₁₋₆ alkoxy, 3-7 membered heterocyclyl, 3-7 membered cycloalkyl, or sulfonyl, is optionally substituted by 1-3 substituents selected from the group consisting of halogen, cyano, amino, hydroxy, carbonyl, and C₁₋₆ alkyl.

8. The compound or the pharmaceutically acceptable salt, stereoisomer or tautomer thereof according to claim 6, wherein,
Y is selected from the group consisting of phenyl, 5-7 membered heteroaryl, 3-8 membered heterocyclyl, and 3-7 membered cycloalkyl; Y is substituted by C₂₋₆ alkenyl, or C₂₋₆ alkynyl, and is optionally substituted by 1-2 substituents selected from the group consisting of halogen, cyano, amino, hydroxy, carbonyl, C₁₋₆ alkyl, C₁₋₆ haloalkyl, C₁₋₆ alkoxy, 3-7 membered heterocyclyl, 3-7 membered cycloalkyl, C₁₋₆ alkylamino, C₁₋₆ alkylcarbonylamino, C₁₋₆ alkylsulfonyl, aminocarbonyl, C₁₋₆ alkylaminocarbonyl, and sulfonyl;
when Y is substituted, the substituent on Y, which is C₁₋₆ alkyl, C₁₋₆ haloalkyl, C₁₋₆ alkoxy, 3-7 membered heterocyclyl, 3-7 membered cycloalkyl, or sulfonyl, is optionally substituted by 1-3 substituents selected from the group consisting of halogen, cyano, amino, hydroxy, carbonyl, and C₁₋₆ alkyl; and
R₃ is selected from -NH-R₅, and R₅ is 3-7 membered heterocyclyl substituted by 1-2 substituents selected from C₁₋₆ alkyl.

9. The compound or the pharmaceutically acceptable salt, stereoisomer or tautomer thereof according to any one of claims 1-3,
wherein,
the ring A is selected from the group consisting of 5-12 membered cycloalkyl, 5-12 membered cycloalkenyl, 5-12 membered heterocyclyl, aryl, and 5-12 membered heteroaryl; and preferably, the ring A is selected from the group consisting of 5-8 membered cycloalkyl, 5-8 membered cycloalkenyl, 5-8 membered heterocyclyl, phenyl, and 5-8 membered heteroaryl.

10. The compound or the pharmaceutically acceptable salt, stereoisomer or tautomer thereof according to any one of claims 1-3,
wherein, R₃ is selected from the group consisting of -NR₄R₅, and -NR₄-C₁₋₆ alkylene-R₅, R₄ is selected from hydrogen, and R₅ is selected from the group consisting of 3-7 membered cycloalkyl, and 3-7 membered heterocyclyl; and preferably, R₃ is selected from -NHR₅, and R₅ is selected from the group consisting of 4-6 membered cycloalkyl and 4-6 membered heterocyclyl.

11. The compound or the pharmaceutically acceptable salt, stereoisomer or tautomer thereof according to claim 10,
wherein Y is selected from the group consisting of phenyl and 5-6 membered heteroaryl, and Y is substituted by C₂₋₆ alkenyl or C₂₋₆ alkynyl, and is optionally substituted by 1-2 substituents selected from the group consisting of halogen, cyano, amino, hydroxy, carbonyl, C₁₋₆ alkyl, C₁₋₆ haloalkyl, C₁₋₆ alkoxy, 3-7 membered heterocyclyl, and 3-7 membered cycloalkyl; and C₂₋₆ alkenyl and C₂₋₆ alkynyl are substituted by 1-3 substituents selected from the group consisting of halogen, cyano, amino, hydroxy, carbonyl, C₁₋₆ alkyl, 3-6 membered cycloalkyl, and C₁₋₆ haloalkyl.

12. The compound or the pharmaceutically acceptable salt, stereoisomer or tautomer thereof according to claim 5, wherein, the ring A is selected from the group consisting of 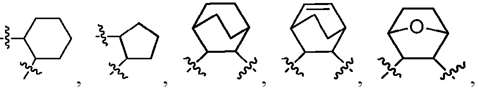 and 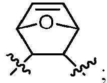 and the ring A is optionally substituted by 1-4 substituents selected from the group consisting of hydroxy, amino, carboxyl, cyano, nitro, halogen, C₁₋₆ alkyl, -NH-C₁₋₆ alkyl, C₁₋₆ haloalkyl, C₁₋₆ alkoxy, C₁₋₆ haloalkoxy, 3-7 membered heterocyclyl, 3-7 membered cycloalkyl, C₁₋₆ alkylsulfonyl, and -N(C₁₋₆ alkyl)₂.

13. The compound or the pharmaceutically acceptable salt, stereoisomer or tautomer thereof according to claim 10,
wherein, the ring A is selected from the group consisting of 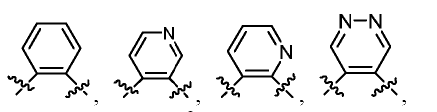 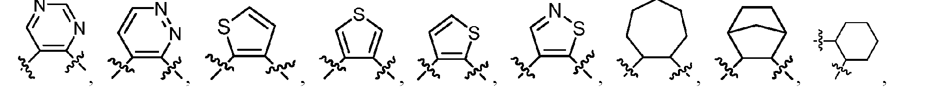 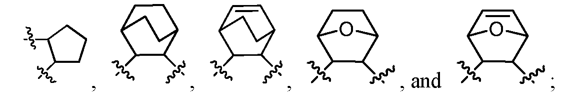 and the ring A is optionally substituted by 1-2 substituents selected from the group consisting of cyano, nitro, halogen, C₁₋₆ alkyl, C₁₋₆ haloalkyl, C₁₋₆ alkoxy, 3-7 membered cycloalkyl, 5-7 membered heteroaryl, C₁₋₆ alkylsulfonyl, and -N(C₁₋₆ alkyl)₂.

14. The compound or the pharmaceutically acceptable salt, stereoisomer or tautomer thereof according to claim 4 or 10,
wherein, Y is selected from the group consisting of

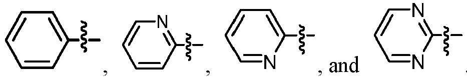

15. A compound or a pharmaceutically acceptable salt, stereoisomer or tautomer thereof, which is shown as below:

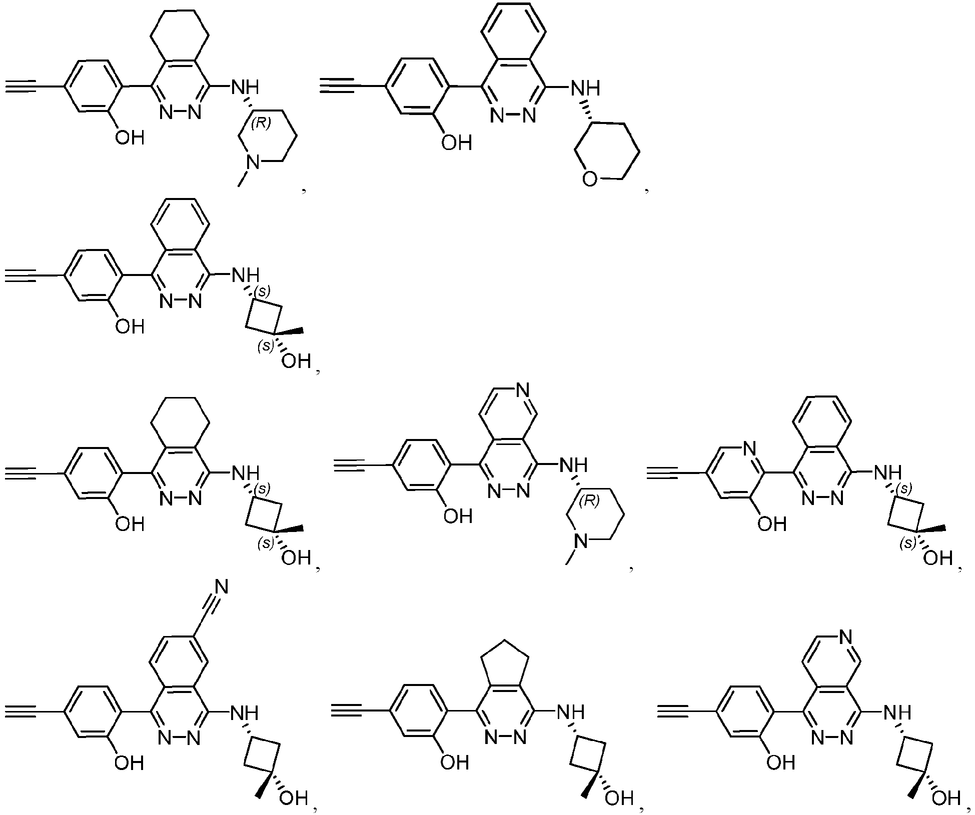

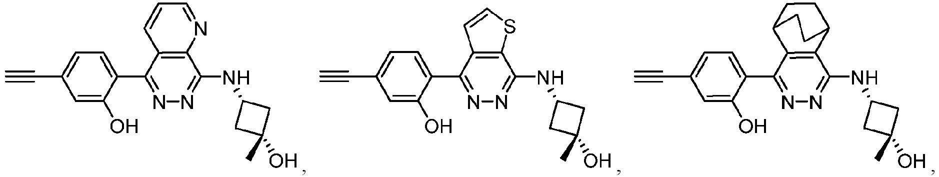

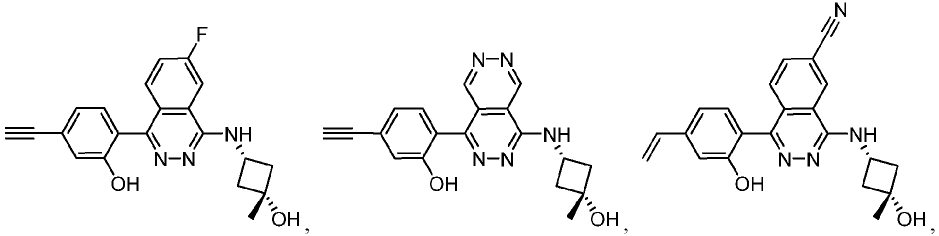

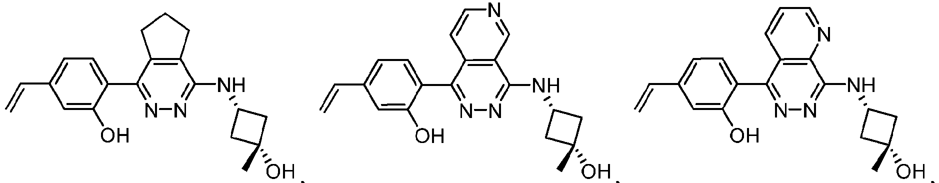

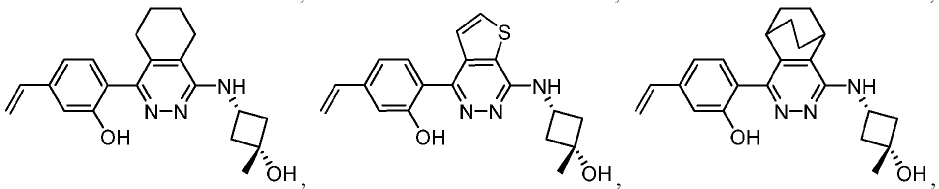

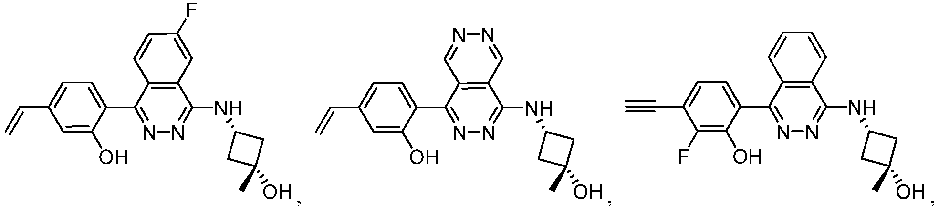

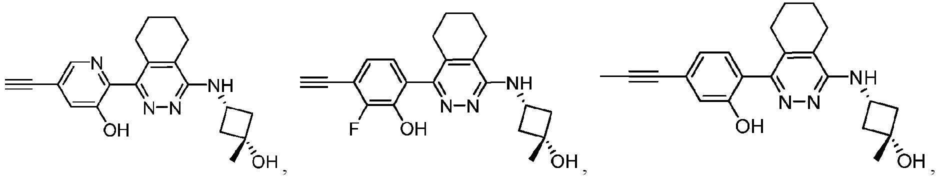

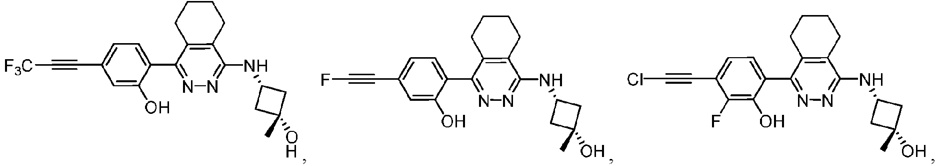

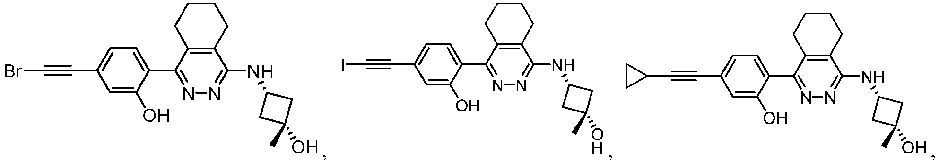

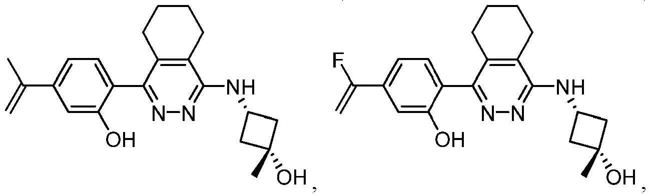

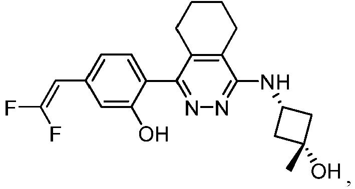

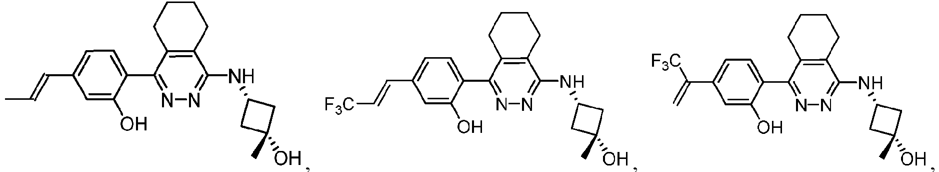

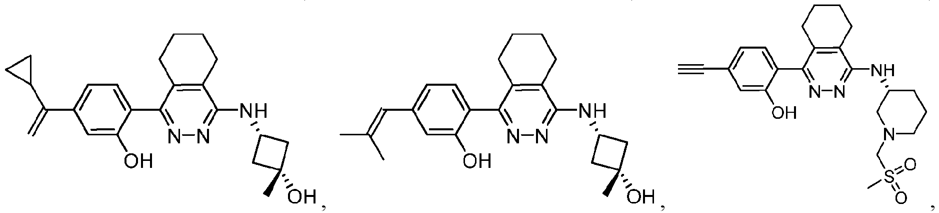

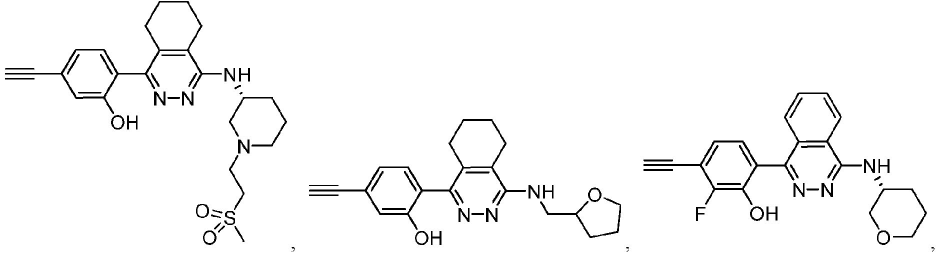

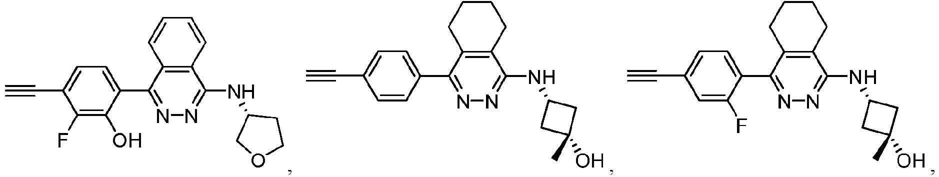

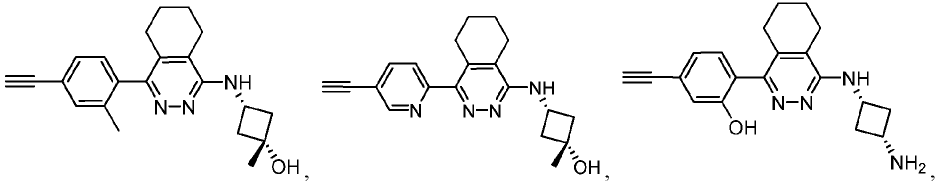

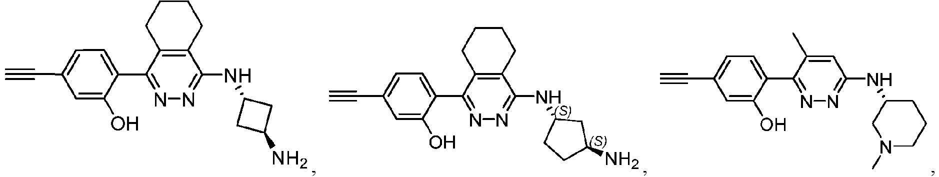

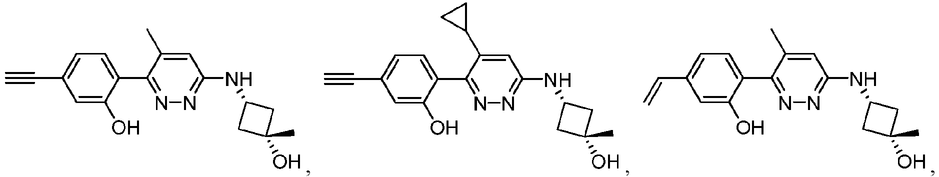

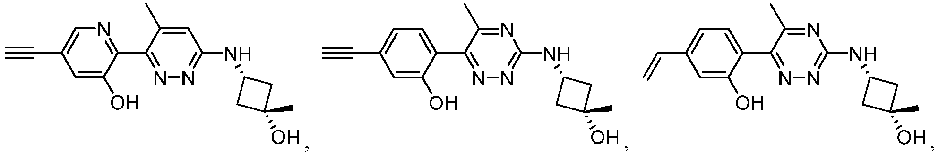

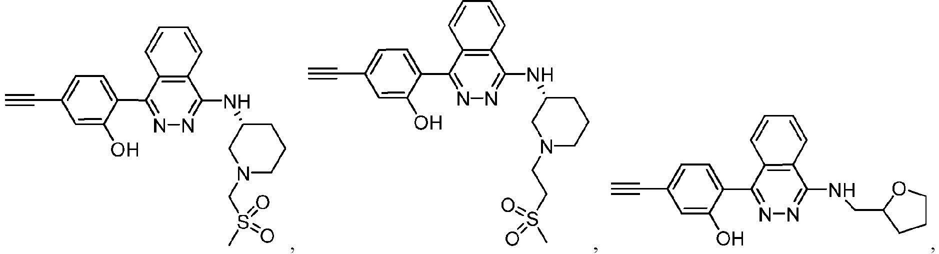

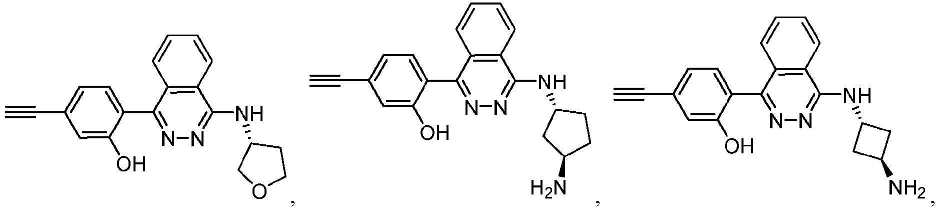

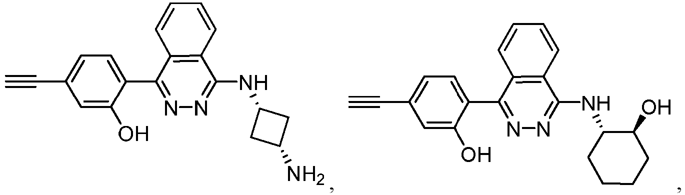

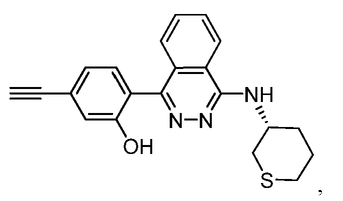

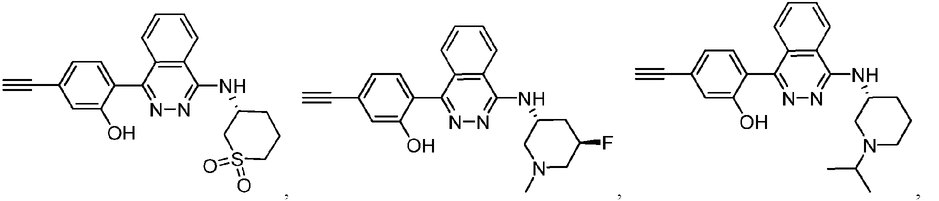

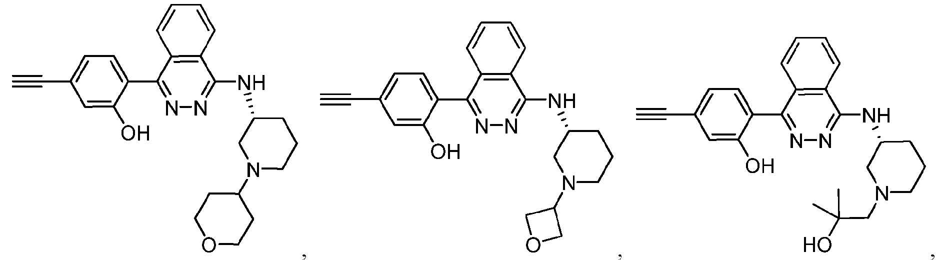

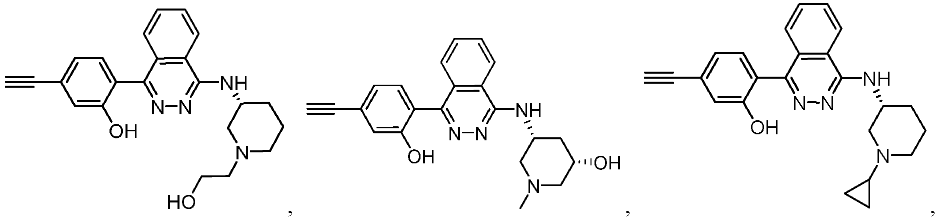

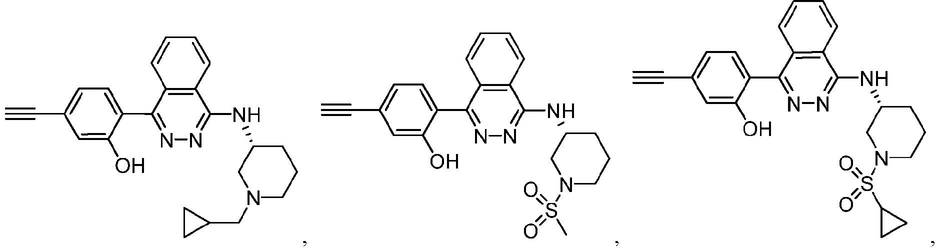

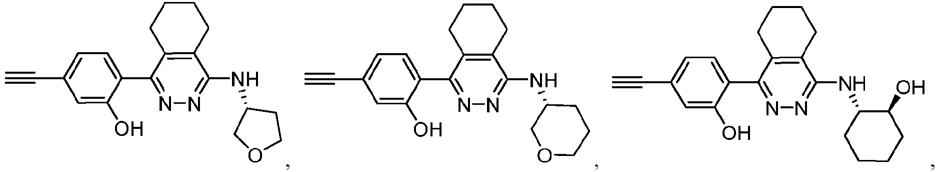

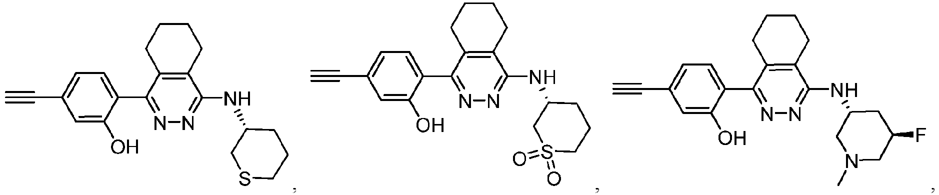

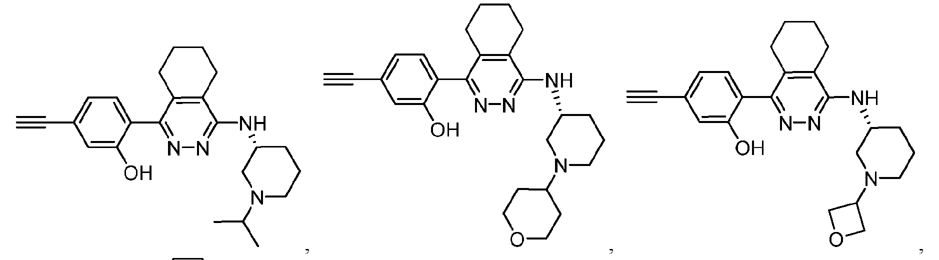

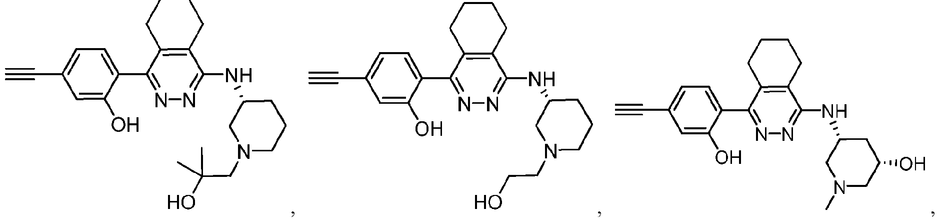

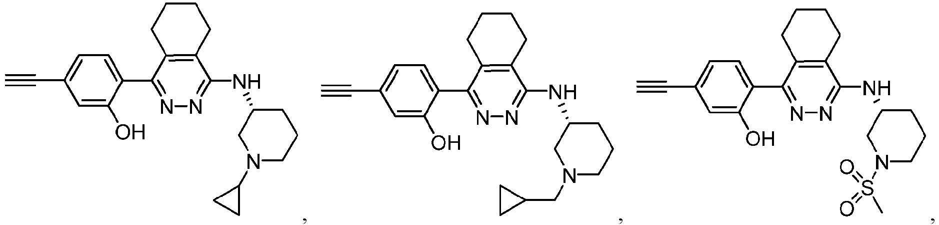

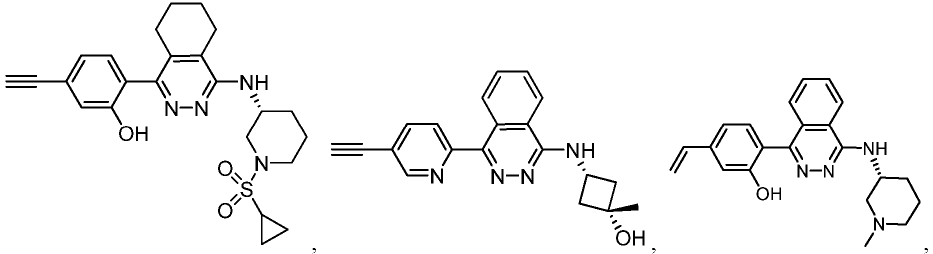

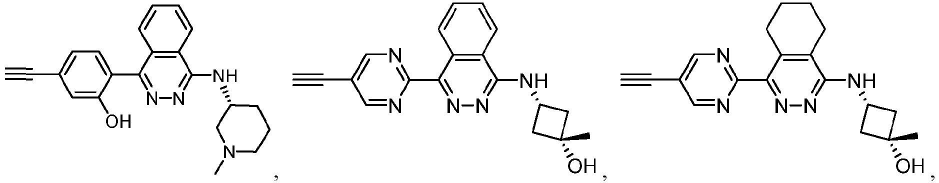

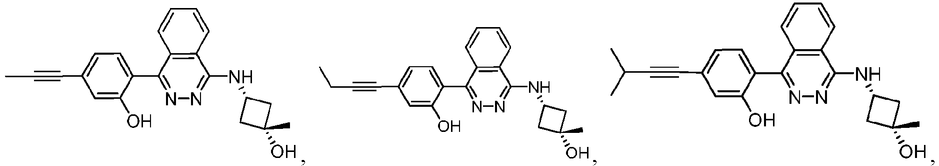

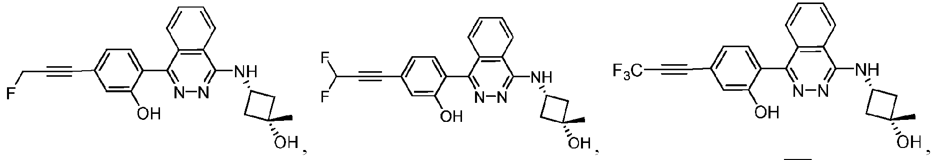

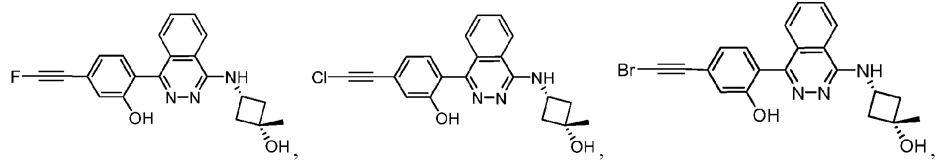

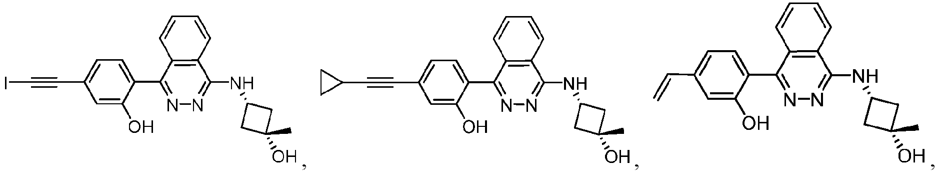

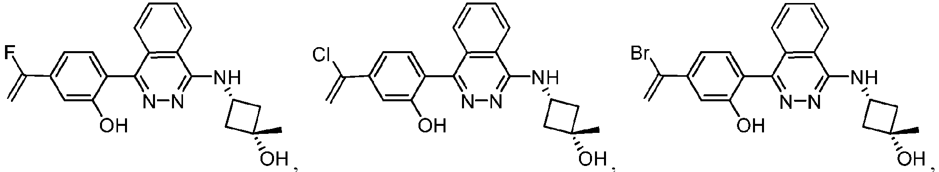

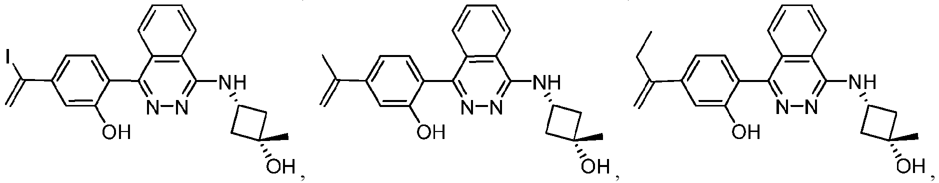

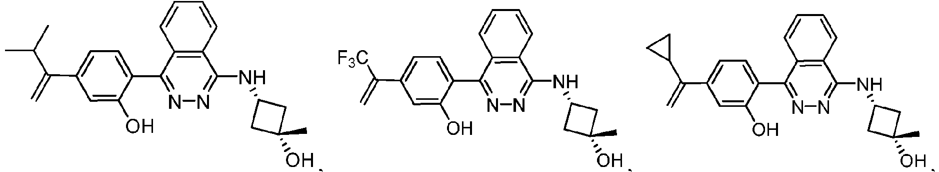

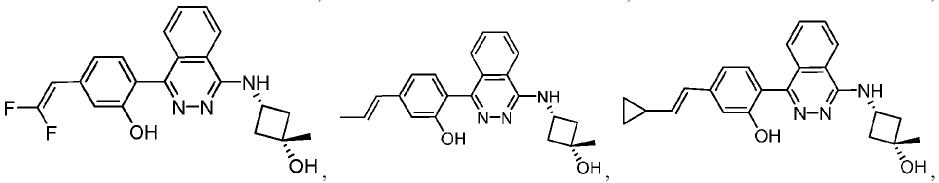

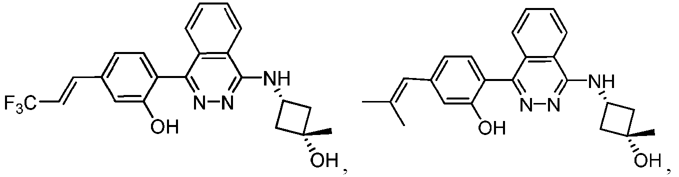

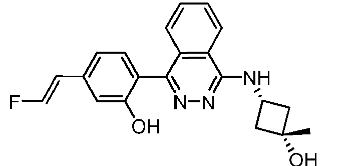

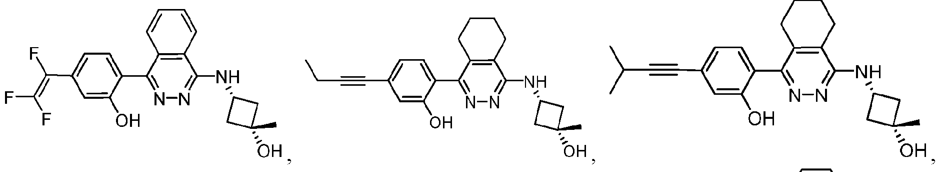

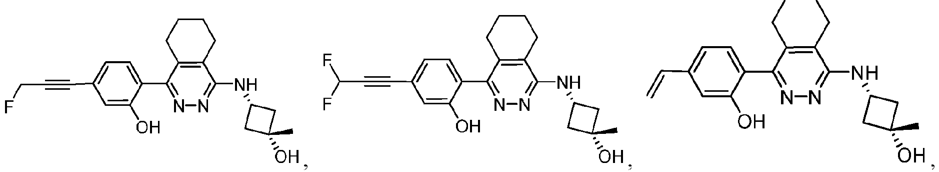

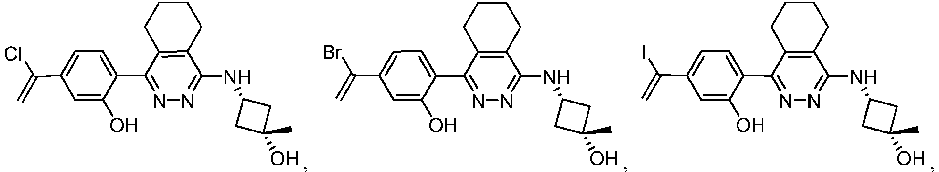

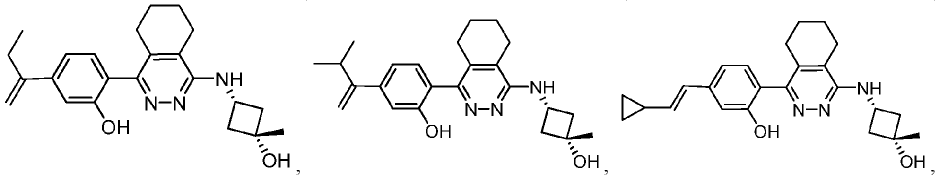

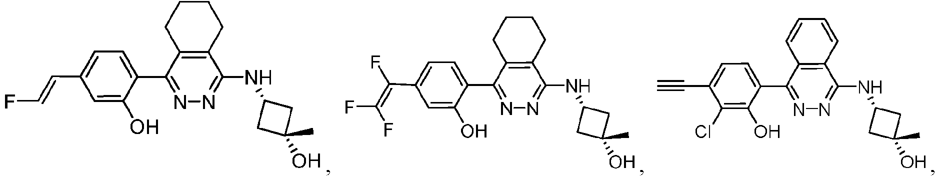

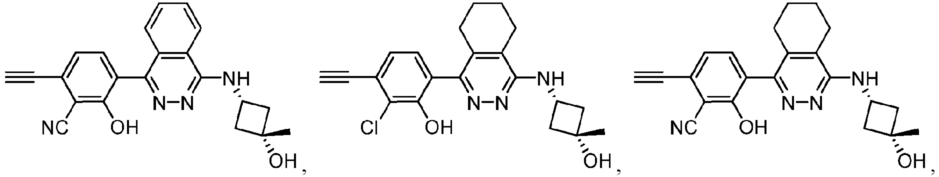

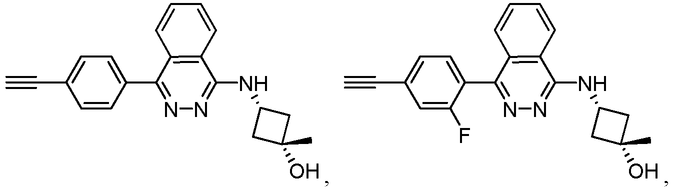

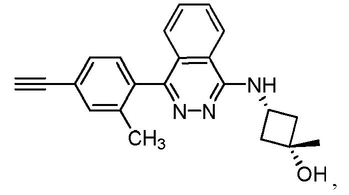

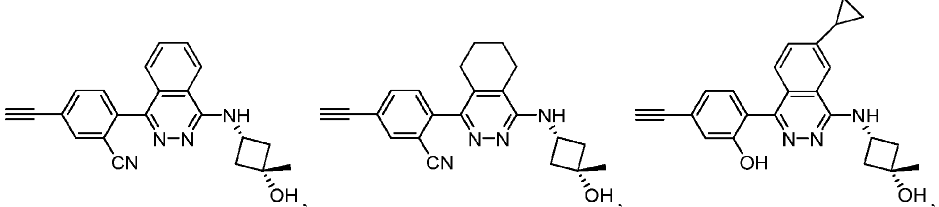

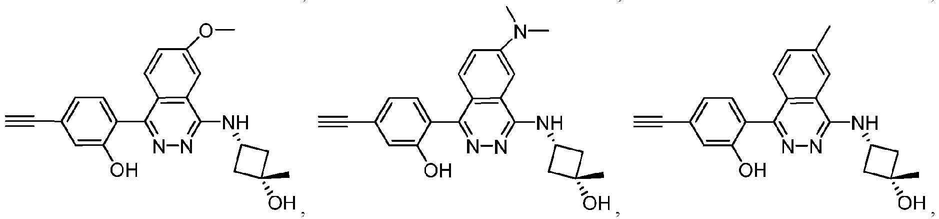

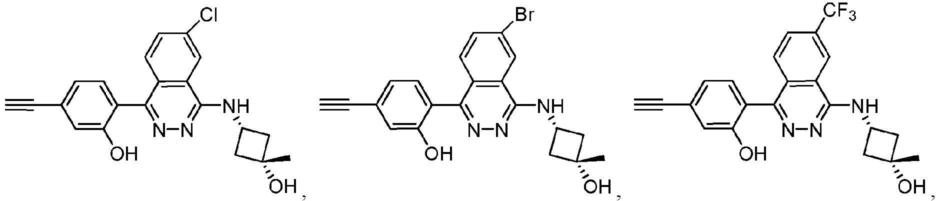

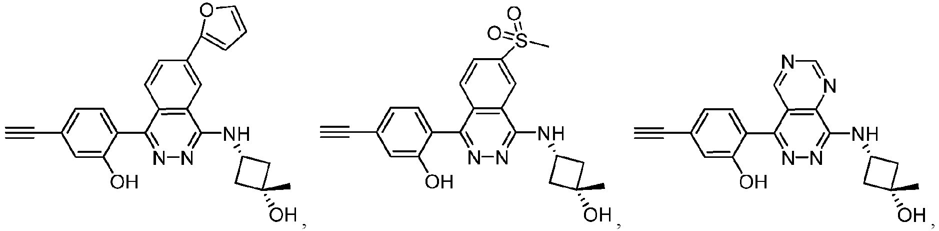

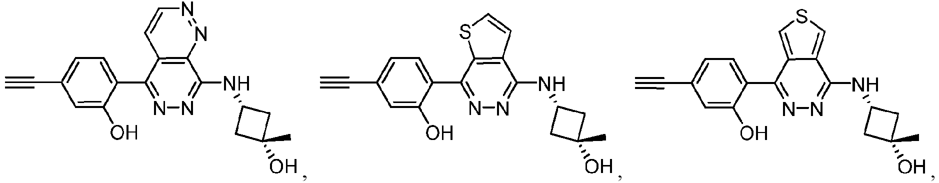

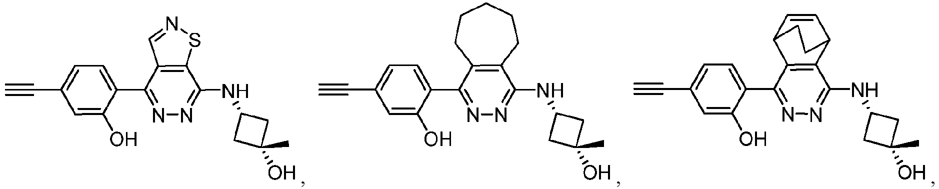

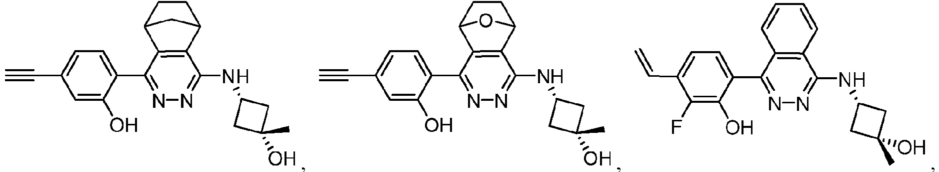

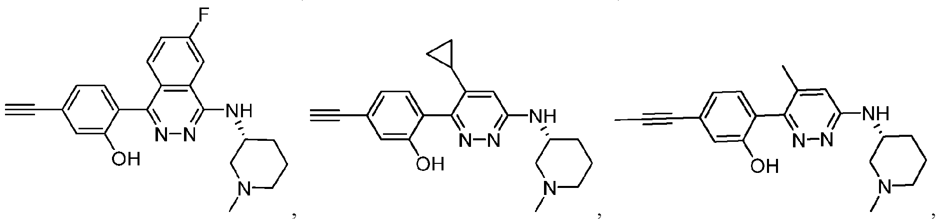

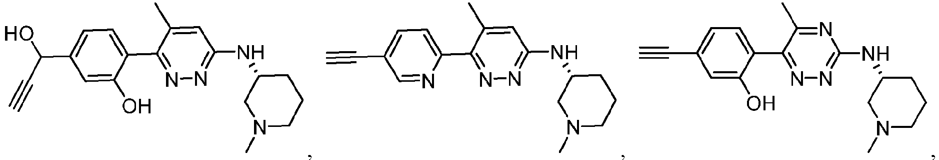

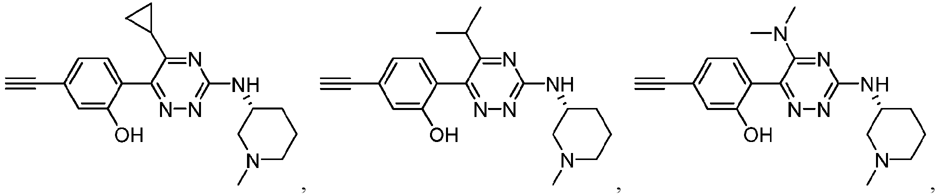

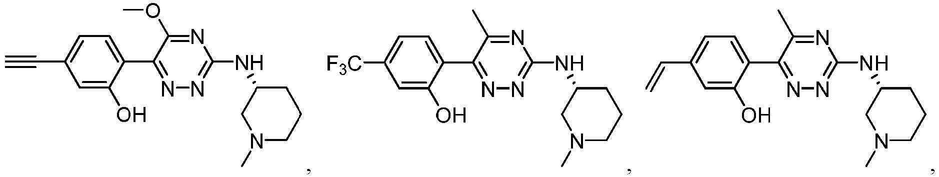

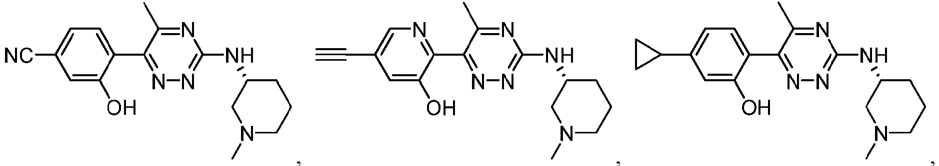

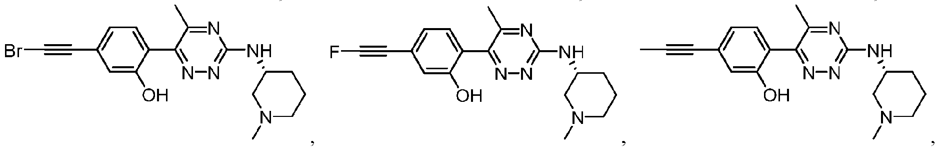

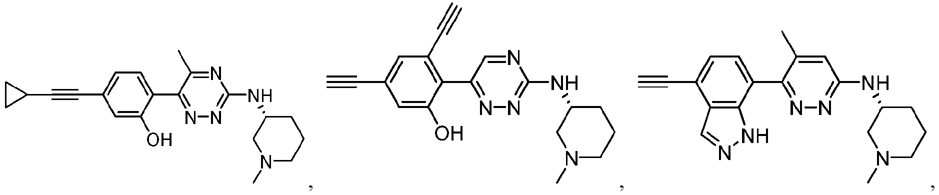

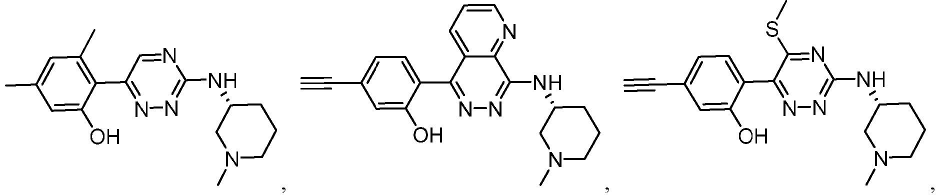

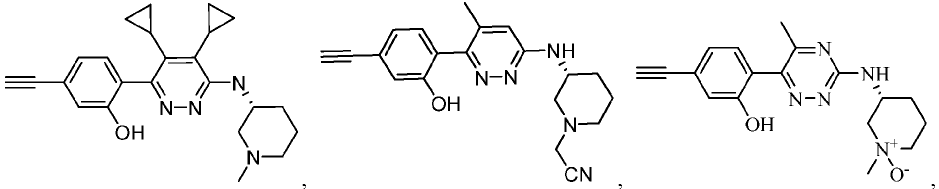

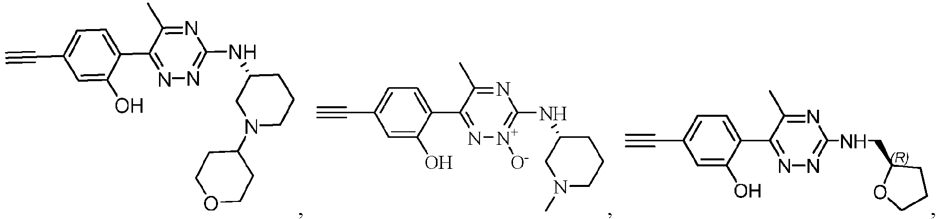

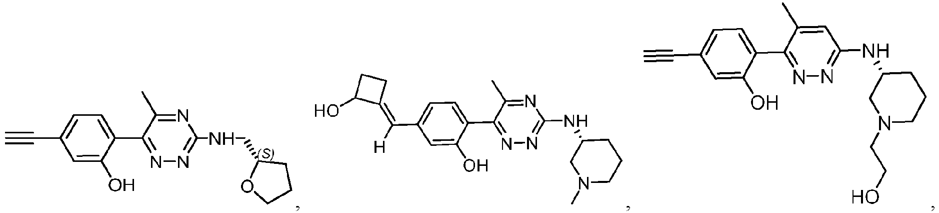

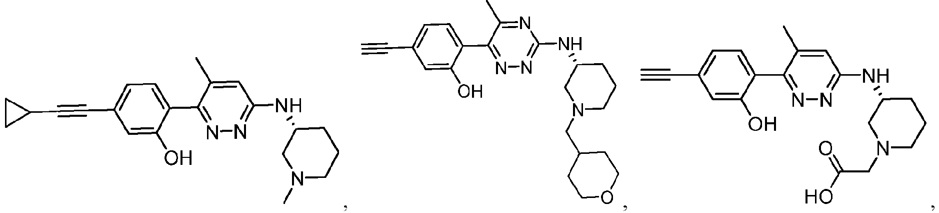

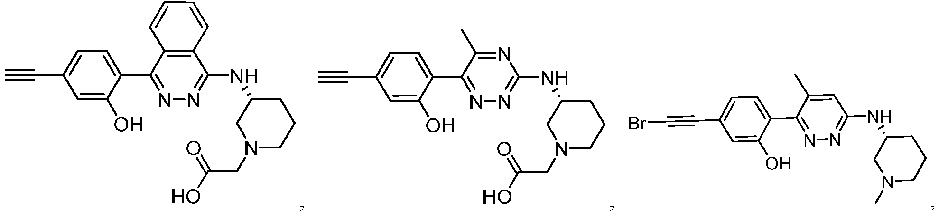

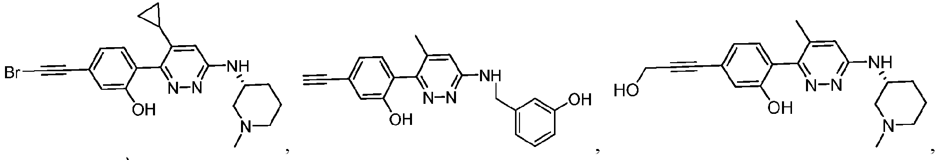

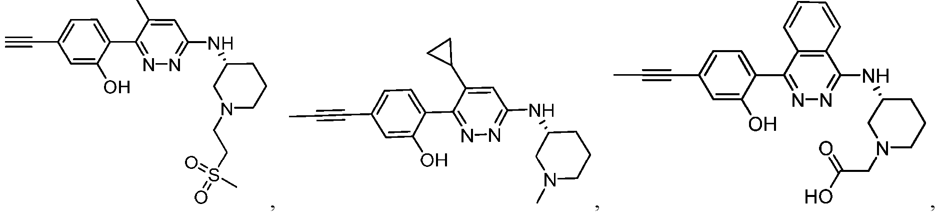

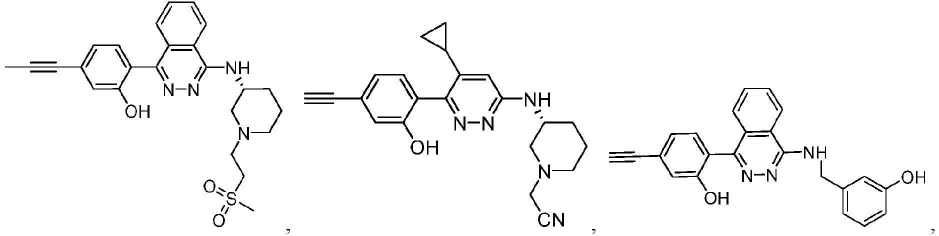

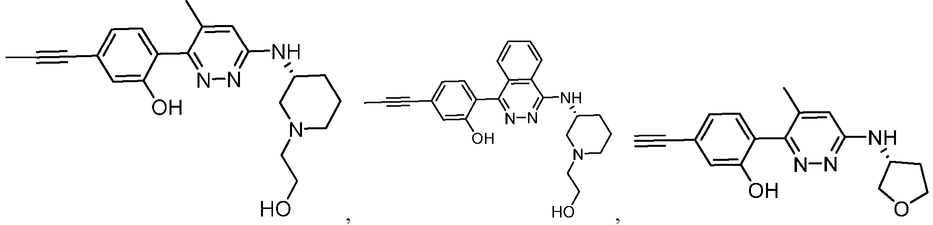

16. A pharmaceutical composition, comprising the compound or the pharmaceutically acceptable salt, stereoisomer or tautomer thereof according to any one of claims 1-15, and a pharmaceutically acceptable carrier.

17. Use of the compound or the pharmaceutically acceptable salt, stereoisomer or tautomer thereof according to any one of claims 1-15, or the pharmaceutical composition according to claim 16, in preparation of a medicament for prevention and/or treatment of NLRP3 inflammasome-related diseases.

18. Use of the compound or the pharmaceutically acceptable salt, stereoisomer or tautomer thereof according to any one of claims 1-15, or the pharmaceutical composition according to claim 16, in preparation of a medicament for prevention and/or treatment of NLRP3 inflammasome-related diseases, immune diseases, inflammatory diseases, autoimmune diseases, or autoinflammatory diseases.
